(19) Europäisches Patentamt
European Patent Office
Office européen des brevets

(11) EP 4 744 680 A1

(12) **EUROPEAN PATENT APPLICATION**
published in accordance with Art. 153(4) EPC

(43) Date of publication:
**20.05.2026 Bulletin 2026/21**

(21) Application number: **24842465.7**

(22) Date of filing: **16.08.2024**

(51) International Patent Classification (IPC):
***A61K 47/54*** *(2017.01)* ***A61K 45/00*** *(2006.01)*
***A61P 35/00*** *(2006.01)*

(86) International application number:
**PCT/CN2024/112770**

(87) International publication number:
**WO 2025/016480 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.07.2023 CN 202310871629**

(71) Applicant: **Bai Yao Zhi Da (Beijing) Nanobio Technology Co., Ltd.**
**Beijing 102206 (CN)**

(72) Inventors:
- **WANG, Liyuan**
  **Beijing 102206 (CN)**
- **WEI, Hong**
  **Beijing 102206 (CN)**
- **WANG, Meng**
  **Beijing 102206 (CN)**

(74) Representative: **Winter, Brandl - Partnerschaft mbB**
**Alois-Steinecker-Straße 22**
**85354 Freising (DE)**

Remarks:
- A request for restoration of the right of priority by the EPO as designated Office has been granted (R. 49ter.2 PCT, Art.122 EPC)
- The complete document including Reference Table(s) and the Sequence Listing(s) can be downloaded from the EPO website

# (54) TARGETED CHEMICAL DRUG AND PREPARATION METHOD THEREFOR, PHARMACEUTICAL COMPOSITION, AND USE OF TARGETED CHEMICAL DRUG

(57) Provided is a targeted chemical drug, a method for its preparation, a pharmaceutical composition including the same, and the use thereof. The targeted chemical drug includes a targeted nucleic acid carrier and a small molecule chemical drug conjugated to the carrier. The targeted nucleic acid carrier includes a nucleic acid carrier and a targeting molecule connected thereto. The nucleic acid carrier is a DNA carrier or RNA carrier, and includes sequence a, sequence b, and sequence c, which together form an assembly structure. This targeted chemical drug enables stable and reliable delivery of the small molecule chemical drug and produces a synergistic therapeutic effect in combination with other drugs carried by the carrier.

Fig. 13

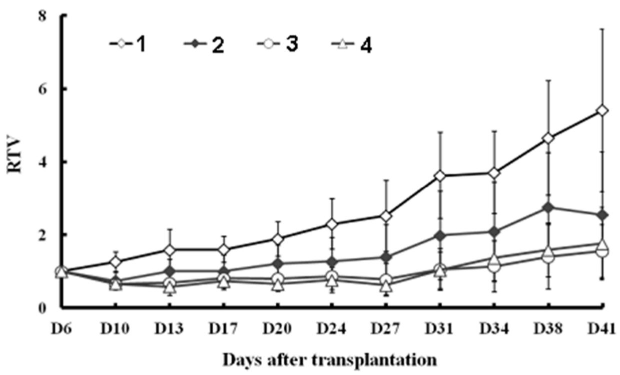


EP 4 744 680 A1

## Description

This application is based upon and claims priority to Chinese Patent Application No. 202310871629.2 filed on July 14, 2023, the application of which is hereby incorporated by reference in its entirety.

## Technical Field

The present invention relates to the field of pharmaceuticals and, more particularly, to a targeted chemical drug, a method for the preparation thereof, a pharmaceutical composition including the same, and uses of the targeted chemical drug.

## Background

Conventional chemotherapy for the treatment of tumors is based on the premise that tumor cells, due to their higher proliferative rate, are more susceptible to anticancer chemotherapeutic agents. However, representative cytotoxic chemotherapeutics such as doxorubicin, paclitaxel, and cisplatin lack selectivity between tumor cells and normal cells. This non-specificity results in substantial collateral toxicity and may even lead to more deleterious adverse effects. In particular, the high doses of cytotoxic agents required for the intended eradication of tumors place patients at risk of systemic cytotoxicity. Accordingly, there is an urgent need for efficient cancer therapy employing targeted delivery systems capable of exploiting inherent differences between tumor cells and normal cells. The development and accumulated know-how of multi-ligand, targeting-mediated nucleic acid nanocarriers provide a prospect for specific (targeted) delivery. Nevertheless, the reliability of delivering small-molecule chemical drugs by existing nucleic acid nanocarriers remains to be further improved.

## Summary

The principal objective of the present invention is to provide a targeted chemical drug, a method for its preparation, a pharmaceutical composition including the same, and uses of the targeted chemical drug, so as to address the following issues in the prior art: targeted delivery of small-molecule chemical drugs, attenuation of toxicity with enhanced efficacy, and expansion of the therapeutic window.

According to one aspect of the invention, there is provided a **targeted chemical drug** including a targeted nucleic acid carrier and a small-molecule chemical drug conjugated to the targeted nucleic acid carrier, wherein the targeted nucleic acid carrier includes a nucleic acid carrier and a targeting molecule linked to the nucleic acid carrier; the nucleic acid carrier is a DNA carrier or an RNA carrier and includes a sequence **a,** a sequence **b,** and a sequence c that form a self-assembled structure, wherein the sequence a is SEQ ID NO: 1: 5'-ACGAGCGTTCCG-3'; the sequence b is SEQ ID NO: 2: 5'-CGGTTCGCCG-3'; and the sequence c is SEQ ID NO: 3: 5'-CGGCCATAGCCGT-3'; or, alternatively, the sequence **a** is SEQ ID NO: 4: 5'-ACGAGCGUUCCG-3'; the sequence **b** is SEQ ID NO: 5: 5'-CGGUUCGCCG-3'; and the sequence c is SEQ ID NO: 6: 5'-CGGCCAUAGCCGU-3'. **Optionally,** at least one base substitution, insertion, or deletion occurs in any of the sequences **a, b,** and **c.**

According to a second aspect of the invention, there is provided a pharmaceutical composition including the targeted chemical drug described herein and a pharmaceutically acceptable carrier and/or excipient.

According to a third aspect of the invention, there is provided the use of the targeted chemical drug described herein in the manufacture of a medicament for the treatment of tumors.

Further, the tumor is gastric cancer and/or lung cancer; preferably, the route of administration during use is intratumoral administration, intravenous administration, or intraperitoneal administration.

According to a fourth aspect of the invention, there is further provided a method for preventing and/or treating cancer, including: (a) providing the targeted chemical drug or the pharmaceutical composition including the same; and (b) administering an effective amount of the targeted chemical drug or the pharmaceutical composition to a subject in need thereof.

Further, the tumor is selected from andrological tumors, gynecologic tumors, respiratory system tumors, digestive system tumors, hematologic tumors, urinary system tumors, bone tumors, neurologic tumors, dermatologic tumors, general surgical tumors, and otorhinolaryngologic/ophthalmologic tumors; preferably, the andrological tumor is prostate cancer, penile cancer, testicular tumor, or male urethral carcinoma; the gynecologic tumor is ovarian cancer, cervical cancer, endometrial cancer, uterine fibroid, vulvar cancer, or malignant hydatidiform mole; the respiratory system tumor is lung cancer, non-small cell lung cancer, small cell lung cancer, nasopharyngeal carcinoma, tracheal tumor, metastatic lung cancer, pulmonary inflammatory pseudotumor, or radiation-induced lung cancer; the digestive system tumor is liver cancer, gastric cancer, colorectal cancer, gallbladder cancer, esophageal cancer, rectal cancer, pancreatic cancer, or colon cancer; the hematologic tumor is leukemia, lymphoma, lymphosarcoma, or multiple myeloma; the urinary

system tumor is kidney cancer, bladder cancer, or urinary tract cancer; the bone tumor is giant cell tumor of bone, osteochondroma, or osteosarcoma; the neurologic tumor is brain tumor, meningioma, cerebral tuberculoma, pituitary tumor, neuroblastoma, glioblastoma, or glioma; the dermatologic tumor is skin cancer or melanoma; the general surgical tumor is breast cancer, lipoma, thyroid cancer, or thyroid tumor; and the otorhinolaryngologic/ophthalmologic tumor is oral cancer, tongue cancer, laryngeal cancer, middle-ear cancer, gingival cancer, or orbital tumor. Preferably, the route of administration during use is intratumoral, intravenous, or intraperitoneal administration; preferably, the daily dose is 0.1 μg/kg to 100 mg/kg.

According to a fifth aspect of the present invention, there is provided a method for preparing the targeted chemical drug, the method including: forming, by sequence self-assembly, a targeted nucleic acid carrier bearing optional oligonucleotide effector molecule(s) and optional immunostimulant(s); and linking a small-molecule chemical drug to the targeted nucleic acid carrier to obtain the targeted chemical drug.

The targeted chemical drug provided herein includes a targeted nucleic acid carrier and a small-molecule chemical drug conjugated to the targeted nucleic acid carrier. The targeted nucleic acid carrier includes a nucleic acid carrier and a targeting molecule linked to the nucleic acid carrier; the nucleic acid carrier is a DNA carrier or an RNA carrier and includes sequences a, b, and c that form a self-assembled structure.On the one hand, coating by the DNA carrier encloses the small-molecule chemical drug within the carrier so that it does not detach during delivery; on the other hand, after modification with targeting aptamers the carrier exhibits improved targetability relative to other nucleic acid carriers; moreover, the small-molecule chemical drug and other agents borne by the carrier produce a combined (synergistic) therapeutic effect.In sum, the targeted chemical drug enables more stable and reliable delivery of the small-molecule chemical drug and allows the small-molecule chemical drug to exert a combined therapeutic effect together with other agents.

## Brief Description of the Drawings

The accompanying drawings, which form a part of the present specification, serve to further illustrate the invention and, together with the exemplary embodiments described herein, explain the invention without unduly limiting it.In the drawings:

**FIG.1** shows the HPLC chromatogram (SEC) of the crude targeted nucleic acid carrier after sequence assembly in Example 6;

**FIG.2** shows the HPLC chromatogram (SEC) of the targeted nucleic acid carrier after HPLC purification in Example 6;

**FIG.3** shows the reverse-phase HPLC chromatogram of the crude targeted chemical drug after loading epirubicin in Example 6;

**FIG.4** shows the reverse-phase HPLC chromatogram of the targeted chemical drug after HPLC purification in Example 6;

**FIG.5** shows the reverse-phase HPLC chromatogram of the targeted chemical drug obtained at an epirubicin-to-carrier molar ratio of 10: 1 in Example 6;

**FIG.6** shows the reverse-phase HPLC chromatogram of the targeted chemical drug obtained at an epirubicin-to-carrier molar ratio of 20: 1 in Example 6;

**FIG.7** shows the reverse-phase HPLC chromatogram of the targeted chemical drug obtained at an epirubicin-to-carrier molar ratio of 30: 1 in Example 6;

**FIG.8** shows the relative body-weight growth curves of each mouse group in the in vivo efficacy study of the targeted epirubicin drug "Apt AS1411-Apt EGFR-Apt A15-2*Bio-DNA-epirubicin" in the N87 mouse gastric cancer model in Example 5;

**FIG.9** shows the tumor growth curves of each mouse group in the in vivo efficacy study of the targeted epirubicin drug "Apt AS1411-Apt EGFR-Apt A15-2*Bio-DNA-epirubicin" in the N87 mouse gastric cancer model in Example 5;

**FIG.10** shows the changes in tumor volume for each mouse group in the in vivo efficacy study of the targeted epirubicin drug "Apt AS1411-Apt EGFR-Apt A15-2*Bio-DNA-epirubicin" in the N87 mouse gastric cancer model in Example 5;

**FIG.11** shows the body-weight change curves, in a comparative in vivo efficacy study in the N87 mouse gastric cancer model, for the targeted epirubicin drugs prepared in Example 5 (Apt AS1411-Apt EGFR-Apt *A15-2Bio-DNA-epirubicin) and Example 7* (*Apt AS1411-Apt EGFR-Apt A15-2*Bio-AmiR-21-DNA-epirubicin);

**FIG.12** shows the mouse tumor volume change curves, in a comparative in vivo efficacy test in the N87 mouse gastric cancer model, for the targeted epirubicin drugs prepared in Example 5 (Apt AS1411-Apt EGFR-Apt *A15-2Bio-DNA-epirubicin) and Example 7* (*Apt AS1411-Apt EGFR-Apt A15-2*Bio-AmiR-21-DNA-epirubicin);

**FIG.13** shows the mouse relative tumor volume change curves, in a comparative in vivo efficacy test in the N87 mouse gastric cancer model, for the targeted epirubicin drugs prepared in Example 5 (Apt AS1411-Apt *EGFR-Apt A15-2Bio-DNA-epirubicin) and Example 7* (*Apt AS1411-Apt EGFR-Apt A15-2*Bio-AmiR-21-DNA-epirubicin);

**FIG.14** shows the mouse tumor volume change curves, in an in vivo efficacy test in the A549 mouse lung cancer model, for the targeted epirubicin drug prepared in Example 3 (Apt AS1411-4*Bio-DNA-epirubicin);

**FIG.15** shows the curves of mouse relative tumor volume changes, in an in vivo efficacy test in the A549 mouse lung cancer model, for the targeted epirubicin drug prepared in Example 3 (Apt AS1411-4*Bio-DNA-epirubicin);

**FIG.16** shows the mouse tumor volume change curves, in an in vivo efficacy test in the A549 mouse lung cancer model, for the targeted epirubicin drugs prepared in Example 6 (Apt AS 1411-Apt EGFR-Apt A15-DNA-epirubicin) and Example 20 (Apt AS1411-Apt EGFR-Apt A15-AmiR-21-DNA-epirubicin);

**FIG.17** shows the mouse relative tumor volume change curves, in an in vivo efficacy test in the A549 mouse lung cancer model, for the targeted epirubicin drugs prepared in Example 6 (Apt AS1411-Apt EGFR-AptA15-DNA-epirubicin) and Example 20 (*Apt AS1411-Apt* EGFR-Apt A15-AmiR-21-DNA-epirubicin);

**FIG.18** shows the mouse tumor volume change curves, in an in vivo efficacy test in the HepG2 mouse hepatocellular carcinoma model, for the targeted epirubicin drug prepared in Example 6 (Apt AS1411-Apt EGFR-AptA15-DNA-epirubicin);

**FIG.19** shows the mouse relative tumor volume change curves, in an in vivo efficacy test in the HepG2 mouse hepatocellular carcinoma model, for the targeted epirubicin drug prepared in Example 6 (*Apt AS1411-Apt* EGFR-AptA15-DNA-epirubicin);

**FIG.20** shows the tumor images from Groups 1, 2, and 4 in the in vivo efficacy test of the epirubicin-targeted drug Apt TfRA3-Apt AS1411-AptA15-DNA-epirubicin, prepared in Example 9, in the U251 glioblastoma mouse model;

**FIG.21** shows the body weight change curves for the mice in each group in the in vivo efficacy test of the epirubicin-targeted drug Apt TfRA4-Apt AS1411-AptA15-DNA-epirubicin (1), prepared in Example 8, in the GL261-Luc glioblastoma mouse model;

**FIG.22** shows the percentage change in body weight for the mice in each group in the in vivo efficacy test of the epirubicin-targeted drug Apt TfRA4-Apt AS1411-AptA15-DNA-epirubicin (1), prepared in Example 8, in the GL261-Luc glioblastoma mouse model;

**FIG.23** shows the dorsal in vivo imaging of the mice in the in vivo efficacy test of the epirubicin-targeted drug Apt TfRA4-Apt AS1411-AptA15-DNA-epirubicin (1), prepared in Example 8, in the GL261-Luc glioblastoma mouse model;

**FIG.24** shows the left-side in vivo imaging of the mice in the in vivo efficacy test of the epirubicin-targeted drug Apt TfRA4-Apt AS1411-AptA15-DNA-epirubicin (1), prepared in Example 8, in the GL261-Luc glioblastoma mouse model;

**FIG.25** shows the right-side in vivo imaging of the mice in the in vivo efficacy test of the epirubicin-targeted drug Apt TfRA4-Apt AS1411-AptA15-DNA-epirubicin (1), prepared in Example 8, in the GL261-Luc glioblastoma mouse model;

**FIG.26** shows the ventral in vivo imaging of the mice in the in vivo efficacy test of the epirubicin-targeted drug Apt

TfRA4-Apt AS1411-AptA15-DNA-epirubicin (1), prepared in Example 8, in the GL261-Luc glioblastoma mouse model;

**FIG.27** shows the changes in BLI values of the in vivo imaging of the mice in the in vivo efficacy test of the epirubicin-targeted drug Apt TfRA4-Apt AS1411-AptA15-DNA-epirubicin (1), prepared in Example 8, in the GL261-Luc glioblastoma mouse model; where (a) represents the changes in BLI values of the dorsal in vivo imaging, (b) represents the changes in BLI values of the left-side in vivo imaging, (c) represents the changes in BLI values of the right-side in vivo imaging, and (d) represents the changes in BLI values of the ventral in vivo imaging;

**FIG.28** shows the survival curves of the mice in each group in the in vivo efficacy test of the epirubicin-targeted drug Apt TfRA4-Apt AS1411-AptA15-DNA-epirubicin (1), prepared in Example 8, in the GL261-Luc glioblastoma mouse model.

**Detailed Description of the Embodiments**

It should be noted that, in the absence of conflicting elements, the embodiments and features within the embodiments in this application can be combined with each other. The following descriptions will provide a more detailed explanation of the present invention with reference to the accompanying drawings and embodiments.

Glossary of Terms:

As used herein, the term "oligonucleotide" refers to molecules that include small interfering RNA (siRNA), antisense oligonucleotides (ASO), microRNA (miRNA), and nucleic acid aptamers (Aptamers).Oligonucleotide drugs, composed of nucleotides, represent a completely new class of drugs distinct from small molecule drugs and antibody drugs. Compared to traditional chemical drug molecules, oligonucleotide drugs have advantages such as strong targeting specificity, high efficiency, long-lasting effects, ease of drug design, and a wide range of potential targets. The main types of oligonucleotide drugs are siRNA drugs and antisense nucleic acid drugs. These primarily act on cytoplasmic mRNA by base-pairing recognition to inhibit the target mRNA and regulate protein expression to treat diseases. The nucleic acids or small nucleic acids referred to in this application are specifically small nucleic acids.

ASO refers to antisense oligonucleotides, which are short synthetic oligonucleotides (typically 16-53 nucleotides) used to block the function of RNA (including mRNA or miRNA). These oligonucleotides typically contain chemically modified nucleotides or specific sequences added to their ends to improve their affinity for target sequences and resist intracellular nucleic acid degradation.

When targeting mRNA, ASOs are typically designed to form complementary pairs with specific regions of target mRNA to block the reading of the translation machinery, induce mRNA degradation, or alter mRNA splicing.

When targeting miRNA, ASOs (also known as anti-miRNA molecules based on their functions) are typically designed to complement specific miRNAs, preventing miRNA from binding to its target mRNA and thus inhibiting miRNA function.miRNAs are short, non-coding RNAs that regulate the stability and translation of target mRNA by base-pairing with its 3' untranslated region (UTR), which in turn affects protein expression.In this application, the ASOs acting as anti-miRNA molecules include, but are not limited to, A-miR21, A-miR-10a, A-miR-30c, and AmiR1306.

MicroRNA (miRNA): miRNAs are short, non-coding RNAs approximately 22 nucleotides in length. Their mechanism of action is that miRNAs regulate target mRNA stability or translation by base-pairing with the 3' untranslated region (3' UTR) of the mRNA, thereby influencing protein expression.miRNAs have been shown to play key roles in many biological processes, including development, differentiation, proliferation, and apoptosis.

miRNAs exist in various forms, with the most original form being pri-miRNA.After one processing step, pri-miRNA becomes pre-miRNA, the precursor to microRNA. Pre-miRNA is further processed by the Dicer enzyme to produce mature miRNA.In actual research, pre-miRNA is the most widely used form, and many commercially available miRNA libraries are in the pre-miRNA form. Recently, studies have shown that the dual arms of miRNA play a crucial role in the formation of mature miRNA, and therefore, natural pri-miRNA forms are increasingly being used in research.

Additionally, as part of the therapeutic strategy, in order to increase the levels of specific miRNAs in the body (such as miR-34a or miR-122), existing research has proposed the concept of miRNA supplements. These supplements are typically analogs or mimics of miRNA that may be in RNA formor DNA form.When in the DNA form, as long as the DNA molecules are capable of being transcribed into RNA with the same sequence, they can execute the same biological function as normal miRNA within cells.

The use of miRNA supplements in the form of DNA offers several advantages.First, DNA exhibits greater stability and resistance to enzymatic degradation in vivo.Second, DNA can be read by the cell's transcription machinery and transcribed into the corresponding RNA, thereby increasing the intracellular levels of miRNA.Finally, DNA is relatively easier to synthesize and are more cost-effective to producethan RNA molecules, facilitating their design, large-scale

manufacturing and practical application.

For example, miR-34a has been demonstrated to exerta significant anti-tumor effect in a variety of cancers, so increasing the levels of miR-34a in the body may be an effective therapeutic strategy for cancer treatment. By providing a DNA sequence form of miR-34a supplement (i.e., miRNA analog), the intracellular level of miR-34a can be increased, thereby inhibiting the proliferation and survival of tumor cells.

Therefore, in the application, the term miRNA is broadly defined to include various forms, such as the aforementioned pri-miRNA, pre-miRNA, mature miRNA, and miRNA analogs (DNA form, which, upon transcription, can form oligonucleotides with the same sequence and function as mature miRNA).In this application, miRNA analogs include, but are not limited to, one or more of miR-34, miR-542, miR-126, and miR-122.

Although many types of drug deliverycarriers, including nucleic acid carriers, have been developed in the prior art to improve drug delivery efficiency, such carriers still fail to address the problem of limitations in clinical applications. Moreover, combination drug therapy is a common clinical treatment approach, it generally involves the concurrent administration of separate single-agent formulations. The incorporation of multiple effector molecules into a single drug product remains technically challenging. To address these issues, the present invention provides a small molecule chemical drug, which includes a targeted nucleic acid carrier and a small molecule chemical drug conjugated to the targeted nucleic acid carrier. The targeted nucleic acid carrier includes a nucleic acid carrier and a targeting molecule connected to the nucleic acid carrier; wherein the nucleic acid carrier is a DNA carrier or an RNA carrier, and the nucleic acid carrier contains a self-assembling structure formed by a sequence a, a sequence b, and a sequence c. The sequence a is SEQ ID NO: 1: 5'-ACGAGCGTTCCG-3'; the sequence b is SEQ ID NO: 2: 5'-CGGTTCGCCG-3'; the sequence c is SEQ ID NO: 3: 5'-CGGCCATAGCCGT-3'; alternatively, the sequence a is SEQ ID NO: 7: 5'-ACGAGCGUUCCG-3'; the sequence b is SEQ ID NO: 8: 5'-CGGUUCGCCG-3'; the sequence c is SEQ ID NO: 9: 5'-CGGCCAUAGCCGU-3'; optionally, at least one base in the sequences a, b, and c undergoes substitution, insertion, or deletion.

The present invention employs a special nucleic acid carrier, wherein the DNA carrier retains the inherent resistance of DNA molecules to nucleases degradation, while overcoming the problem of the rigid structure of DNA that previously hindered self-assembly. This carrier is formed by the self-assembly of three DNA single strands, as described above. The RNA carrier also exhibits better self-assembly properties. The inventors further discovered that when the nucleic acid carrier is loaded with small molecule chemical drugs and connected to targeting molecules, it can insert covalently into the carrier's CG structure, or be linked to the carrier sequence in the form of base substitutions or extensions, or covalently connected to the carrier via a linker. This allows the small molecule chemical drugs to temporarily lose their activity, remaining primarily in the form attached to the carrier during the delivery process, and not falling off or existing in the free form, which significantly reduces toxicity.On the other hand, after the carrier is mediated by targeting molecules, especially those mediated by complex targeting aptamers, it can better target the desired tissues, tissue microenvironments, and cells. Through mechanisms such as endocytosis and pinocytosis, the targeted chemical drug is internalized intothe target cells and releases the small molecule chemical drugs under the combined action of the intracellular low pH environment, glutathione-mediated reduction, and enzymatic cleavage. This results in highly efficient targeted delivery of small molecule chemical drugs and greatly improves their bioavailability. Furthermore, when the carrier is concurrently loaded with other drugs (such as gene-regulatory or immune-regulatory drugs) and small molecule chemical drugs, the small molecule chemical drugs exhibits a synergistic effect with the other drug effector molecules, thereby significantly improving the efficiency of combination therapy and reducing toxicity.

In one preferred embodiment, the 5' and 3' ends of the sequence a, sequence b, and sequence c are each independently connected to an extended base segment, wherein the extended base segment includes 0 to 14 bases. Preferably, the nucleic acid carrier contains the following sequences that form the self-assembling structure:
The 1st set of sequences:

a-sequence: SEQ ID NO: 7: 5'-GCGGCGCCC**ACGAGCGTTCCG**GGAGC-3';

b-sequence: SEQ ID NO: 8: 5'-GCTCC**CGGTTCGCCG**CCAGCCGCC-3';

c-sequence: SEQ ID NO: 9: 5'-GGCGGCAGG**CGGCCATAGCCGT**GGGCGCCGC-3';

Alternatively,
The 2nd set of sequences:

a-sequence: SEQ ID NO: 10: 5'-GCGGCGCCC**ACGAGCGTTCCG**GGAGAGGAGC-3';

b-sequence: SEQ ID NO: 11: 5'-GCTCCTCTCC**CGGTTCGCCG**CGAGCCGCG-3';

c-sequence: SEQ ID NO: 12: 5'-CGCGGCT**CGCGGCCATAGCCGT**GGGCGCCGC-3';

Alternatively,
The 3rd set of sequences:

a-sequence: SEQ ID NO: 10: 5'-GCGGCGCCC**ACGAGCGTTCCG**GGAGAGGAGC-3';

b-sequence: SEQ ID NO: 13: 5'-GCTCCTCTCC**CGGTTCGCCG**CCAGCCGCGG-3';

c-sequence: SEQ ID NO: 14: 5'-GGCGGCTGG**CGGCCATAGCCGT**GGGCGCCGC-3';

Alternatively,
The 4th set of sequences:

a-sequence: SEQ ID NO: 15: 5'-GCGGCGCCC**ACGAGCGTTCCG**GGAGAGGCC-3';

b-sequence: SEQ ID NO: 16: 5'-GGCCTCTCC**CGGTTCGCCG**CCAGCCGCC-3';

c-sequence: SEQ ID NO: 14: 5'-GGCGGCTGG**CGGCCATAGCCGT**GGGCGCCGC-3';

Alternatively,
The 5th set of sequences:

a-sequence: SEQ ID NO: 17: 5'-GCGGCGCCC**ACGAGCGUUCCG**GGAGAGGCC-3';
b-sequence: SEQ ID NO: 18: 5'-GGCCUCUCC**CGGUUCGCCG**CCAGCCGCC-3';
c-sequence: SEQ ID NO: 19: 5'-GGCGGCUGG**CGGCCAUAGCCGU**GGGCGCCGC-3';

Alternatively,
The 6th set of sequences:

a-sequence: SEQ ID NO: 20: 5'- GCGGCGCCC**ACGAGCGTTCCG**GGAGAGGAGGCC-3';
b-sequence: SEQ ID NO: 21: 5'- GGCCTCCTCTCC**CGGTTCGCCG**CCAGCCGCC-3';
c-sequence: SEQ ID NO: 14: 5'- GGCGGCTGG**CGGCCATAGCCGT**GGGCGCCGC-3';

Alternatively,
The 7th set of sequences:

a-sequence: SEQ ID NO: 10: 5'-GCGGCGCCC**ACGAGCGTTCCG**GGAGAGGAGC-3';
b-sequence: SEQ ID NO: 22: 5'-GCTCCTCTCC**CGGTTCGCCG**CCAGCCGCC-3';
c-sequence: SEQ ID NO: 14: 5'-GGCGGCTGG**CGGCCATAGCCGT**GGGCGCCGC-3';

Alternatively,
The 8th set of sequences:

a-sequence: SEQ ID NO: 23: 5'-GCGACGCCC**ACGAGCGTTCCG**GGAGAGGAG-3';
b-sequence: SEQ ID NO: 24: 5'- CTCCTCTCC**CGGTTCGCCG**CGAGCCGCG-3';
c-sequence: SEQ ID NO: 25: 5'- CGCGGCACG**CGGCCATAGCCGT**GGGCGTCGC-3';

Alternatively,
The 9th set of sequences:

a-sequence: SEQ ID NO: 26: 5'-GCGACGCCC**ACGAGCGTTCCG**GGAGAGGAGC-3';
b-sequence: SEQ ID NO: 11: 5'-GCTCCTCTCC**CGGTTCGCCGC**GAGCCGCG-3';
c-sequence: SEQ ID NO: 27: 5'- CGCGGCTCG**CGGCCATAGCCGT**GGGCGTCGC-3';

Alternatively,
The 10th set of sequences:

a-sequence: SEQ ID NO: 28: 5'- GACGCCC**ACGAGCGTTCCG**GGAGAGG-3';

b-sequence: SEQ ID NO: 29: 5'- CCTCTCC**CGGTTCGCCG**CGAGCCT-3';

c-sequence: SEQ ID NO: 30:
5'-GGCTCG**CGGCCATAGCCGT**GGGCGTCTGCTGCTGCTGCTG -3';

Alternatively,
The 11th set of sequences:

a-sequence: SEQ ID NO: 31: 5'- GCCC**ACGAGCGTTCCG**GGAGA-3';

b-sequence: SEQ ID NO: 32: 5'- TCTCC**CGGTTCGCCG**CCAGCCGCC-3';

c-sequence: SEQ ID NO: 33: 5'- GGCGGCTGG**CGGCCATAGCCGT**GGGC-3'.

By employing nucleic acid carriers self-assembled from the foregoing sets of sequences, the above-described effects can be more fully realized.
As noted above, when the nucleic acid carrier provided by the present invention simultaneously carries other agents (e.g., gene-regulatory or immuno-regulatory drugs) together with a small-molecule chemical drug, the small-molecule chemical drug can act synergistically with the other effector molecules to produce a combined therapeutic effect, thereby markedly improving the efficiency of combination therapy within a single drug product and reducing toxicity. Specifically, the targeted chemical drug further includes an oligonucleotide effector molecule and/or an immunostimulant conjugated to the targeted nucleic acid carrier; Preferably, the oligonucleotide effector molecule includes one or more selected from the group consisting of an ASO, an siRNA, an miRNA, or a nucleic acid aptamer; More preferably, the siRNA includes one or more of ASAP1, ATAD2, CD24, CD47, EGFR, HBV, HSP, HS70, PD-L1, PAPP-1, Survivin, TAP, TIM-3, TGF-β1 (TGF-beta1), VEGF-C; Still more preferably, the siRNA includes one or more of ASAP1, CD47, PD-L1, TGF-β1; More preferably, the miRNA includes one or more of A-miR21, A-miR-10a, A-miR-30c, miR-34, miR-542, miR-126-3p, miR-122; Still more preferably, miRNA 为A-miR21; More preferably, the nucleic acid aptamer includes a DNA-form nucleic acid aptamer and/or an RNA-form nucleic acid aptamer, and the nucleic acid aptamer is preferably selected from one or more of A1, A15, AS1411, AFP, ATP, Act-12c, A18, BAF7-1, C-Met-SL1, CH6, CA2, C12, CRAC Orail, CEA, CEA-18, CEA-T84, CSC1, CSC13, CD40, CD16a, CD19, CD3-4, CD44, CD12 (HDLBP), CD20, CD24, CD33, CD38, CD105, CD117, CD63, CD123, EGFR, EpCAM, EcR, FAP, 抗 FAP, GPC-1, GSK836, GPC3 (APS63-1), Her2, Her3, HMGA2, H2, HbsAg, IFN-y (B4), IL-4Ra, IL-17, LZH8, MUC1, M5, M7, M1, N5, N-G-Dua, NKG2D (20-N-15), NSE, Np-A15, Np-A48, Np-A58, Np-A61, OX40, PSMA, PDGFRβ (PDGFRbeta), PDGF, PD-L1, PD-1, PTK-7, ProGRP-48, SF, TBA15, TBA29, TfRA4, TfRA3, TTA1, TLS9a, TGF-βII (S58) (TGF-betaII (S58)), TNF-a, TNF, T1, VEGF, VCAM-1, VCAM-12d, CH6, PL-45, EP66, AGC, Karpas299, SW620, MDA-MB-231, MCF-7, PC-3, BCMA, CTLA-4, CCL1, CD4-3, CD28, FGF2 (F2), FGF2, FGF5, LAG-3, MRP1, TIM3, TIMC-11, VEGF165, 4-1BB; And, in a further preferred embodiment, the nucleic acid aptamer includes one or more of A1, A15, AS1411, C12, CD40L, EGFR, GPC-1, IL-4Ra, MUC1, OX40, PD-L1, TTA1, TfRA3, TfRA4; Preferably, the immunostimulant includes one or more of CPG2006, CPG1826, CPG2216, CPG2395, CPG-ODNT7, CPG-ODN-PCIF1; More preferably, the immunostimulant is CPG2006.
Specifically, the sequence structures of the sense strand and the antisense strand of each siRNA, from the 5' end to the 3' end, are as follows:

ASAP1: Sense strand: SEQ ID NO: 34: 5'-UGAUAUUAUGGAAGCAAAUUU-3', antisense strand: SEQ ID NO: 35: 5'-AUUUGCUUCCAUAAUAUCAUU-3'; Alternatively, sense strand: SEQ ID NO: 36: 5'-UUAGGUUUGGGGUUGGAUCUU-3', antisense strand: SEQ ID NO: 37: 5'-GAUCCAACCCCAAACCUAAUU-3';

ATAD2: sense strand: SEQ ID NO: 38: 5'-AAUCCUACAACUUCGACGCUU -3', antisense strand: SEQ ID NO: 39: 5'-GCGUCGAAGUUGUAGGAUUUU-3';

CD24: sense strand: SEQ ID NO: 40: 5'-UGUUUACAUUGUUGAGGUAUU-3', antisense strand: SEQ ID NO: 41: 5'-UACCUCAACAAUGUAAACUUU-3';

CD47: sense strand: SEQ ID NO: 42: 5'-GGUGAUUACCCAGAGAUAUTT-3', antisense strand: SEQ ID NO: 43: 5'-AUAUCUCUGGGUAAUCACCTT-3'; Alternatively, sense strand: SEQ ID NO: 44: 5'-UGGUGAAAGAGGUCAUUCCUU-3', antisense strand: SEQ ID NO: 45: 5'-GGAAUGACCUCUUUCACCAUU-3'; Alternatively, sense strand: SEQ ID NO: 46: 5'-GGAAUGACCUCUUUCACCATT-3', antisense strand: SEQ ID NO: 47: 5'-UGGUGAAAGAGGUCAUUCCTT-3'; Alternatively, sense strand: SEQ ID NO: 48: 5'-GGUGAUUACCCAGAGAUAUUU-3', antisense

strand: SEQ ID NO: 49: 5'-AUAUCUCUGGGUAAUCACCUU-3'; Preferably, for **CD47,** phosphorothioate modifications are introduced at the internucleoside phosphate linkages (i) between adjacent nucleotides within positions 2-4 (inclusive) counted from the 5' terminus of the sense strand, (ii) between adjacent nucleotides within positions 1-3 (inclusive) counted from the 3' terminus of the sense strand, (iii) between adjacent nucleotides within positions 1-2 (inclusive) counted from the 5' terminus of the antisense strand, and (iv) between adjacent nucleotides within positions 1-3 (inclusive) counted from the 3' terminus of the antisense strand.It should be noted that the foregoing modifications are intended to further improve structural stability of the drug; unless otherwise specified, the modifications mentioned hereinafter serve this purpose and will not be repeated.

EGFR: sense strand: SEQ ID NO: 50: 5'-GGCUGGUUAUGUCCUCAUUUU-3', antisense strand: SEQ ID NO: 51: 5'-AAUGAGGACAUAACCAGCCUU-3'; Alternatively, sense strand: SEQ ID NO: 52: 5'-UUAGAUAAGACUGCUAAGGUU-3', antisense strand: SEQ ID NO: 53: 5'-UUAGAUAAGACUGCUAAGGUU-3'; Alternatively, sense strand: SEQ ID NO: 54: 5'-UGCCUUAGCAGUCUUAUCUAAUU -3', antisense strand: SEQ ID NO: 55: 5'-UUAGAUAAGACUGCUAAGGCAUU-3'; Alternatively, sense strand: SEQ ID NO: 78: 5'-UGCCUUAGCAGUCUUAUCUAAUUUU-3', antisense strand: SEQ ID NO: 79: 5'-AAUUAGAUAAGACUGCUAAGGCAUU-3';

HBV: sense strand: SEQ ID NO: 56: 5'-GGACUUCUCUCAAUUUUCUUU-3', antisense strand: SEQ ID NO: 57: 5'-AGAAAAUUGAGAGAAGUCCUU-3'; Preferably, in the HBV siRNA, cytidine (C) and uridine (U) are 2'-fluoro-modified;

HSP: sense strand: SEQ ID NO: 58: 5'-CGCAGAACACCGUGUUCGAUU-3', antisense strand: SEQ ID NO: 59: 5'-UCGAACACGGUGUUCUGCGUU-3'; Preferably, in the HSP siRNA, phosphorothioate modifications are introduced at internucleoside phosphate linkages between adjacent nucleotides within positions 1-3 from the 5' end of the sense strand and within positions 1-3 from the 3' end of the antisense strand;

HS70: sense strand: SEQ ID NO: 60: 5'-GGCCAACAAGAUCACCAUC-3', antisense strand: SEQ ID NO: 61: 5'-GAUGGUGAUCUUGUUGGCCUU-3'; Preferably, in the HS70 siRNA, phosphorothioate modifications are introduced at internucleoside phosphate linkages between adjacent nucleotides within positions 1-3 from the 5' end of the antisense strand and within positions 1-3 from the 3' end of the antisense strand;

PD-L1: sense strand: SEQ ID NO: 62: 5'-CCAGCACACUGAGAAUCAAUU-3', antisense strand: SEQ ID NO: 63: 5'-UUGAUUCUCAGUGUGCUGGUU-3'; Alternatively, sense strand: SEQ ID NO: 64: 5'-AGACGUAAGCAGUGUUGAATT-3', antisense strand: SEQ ID NO: 65: 5'-UUCAACACUGCUUACGUCUTT-3';

PAPP-1: sense strand: SEQ ID NO: 66: 5'-GAGGAAGGUAUCAACAAAUTT-3', antisense strand: SEQ ID NO: 67: 5'-AUUUGUUGAUACCUUCCUCTT-3';

Survivin:

Sense strand: SEQ ID NO: 70: 5'-GCAGGUUCCUUAUCUGUCACAUU-3', antisense strand: SEQ ID NO: 71: 5'-UGUGACAGAUAAGGAACCUGCAGUU-3'; Alternatively,

Sense strand: SEQ ID NO: 72: 5'-GGAAUUGGAAGGCUGGGAACCUU-3', antisense strand: SEQ ID NO: 73: 5'-GGUUCCCAGCCUUCCAAUUCCUU-3'; preferably, phosphorothioate modification is introduced at the internucleoside phosphate linkages (i) between adjacent nucleotides within positions 1-3 counted from the 5' terminus of the sense strand, (ii) between adjacent nucleotides within positions 1-3 counted from the 5' terminus of the antisense strand, and (iii) between nucleotides 1 and 2 counted from the 3' terminus of the antisense strand; Alternatively,

Sense strand: SEQ ID NO: 74: 5'-UGCAGGUUCCUUAUCUGUCATT-3', antisense strand: SEQ ID NO: 75: 5'-UGACAGAUAAGGAACCUGCTT-3'; Alternatively,

Sense strand: SEQ ID NO: 76: 5'-CUGCAGGUUCCUUAUCUGUCACAUU-3', antisense strand: SEQ ID NO: 77: 5'-UGUGACAGAUAAGGAACCUGCAGUU-3'; Preferably, phosphorothioate modification is introduced at the internucleoside phosphate linkages (i) between adjacent nucleotides within positions 1-3 counted from the 5' terminus of the sense strand, (ii) between adjacent nucleotides within positions 1-3 counted from the 3' terminus of the sense strand, (iii) between adjacent nucleotides within positions 1-3 counted from the 5' terminus of the antisense strand,

and (iv) between adjacent nucleotides within positions 1-3 counted from the 3' terminus of the antisense strand;

TAP:

Sense strand: SEQ ID NO: 80: 5'-GCUGCACACGGUUCAGAAUUU-3', antisense strand: SEQ ID NO: 81: 5'-AUUCUGAACCGUGUGCAGCUU-3'; Preferably, phosphorothioate modification is introduced at the internucleoside phosphate linkages (i) between adjacent nucleotides within positions 1-3 counted from the 5' terminus of the sense strand, (ii) between adjacent nucleotides within positions 1-3 counted from the 5' terminus of the antisense strand, and (iii) between adjacent nucleotides within positions 1-3 counted from the 3' terminus of the antisense strand; Alternatively,

Sense strand: SEQ ID NO: 82: 5'-CAGGAUGAGUUACUUGAAAUU -3', antisense strand: SEQ ID NO: 83: 5'-UUUCAAGUAACUCAUCCUGUU -3';

TIM-3: sense strand: SEQ ID NO: 86: 5'-GUGCUCAGGACUGAUGAAATT-3', antisense strand: SEQ ID NO: 87: 5'-UUUCAUCAGUCCUGAGCACTT-3';

TGF-β1: sense strand: SEQ ID NO: 88: 5'-GUCAACUGUGGAGCAACACUU-3', antisense strand: SEQ ID NO: 89: 5'-GUGUUGCUCCACAGUUGACUU-3'; Alternatively, sense strand: SEQ ID NO: 90: 5'-GCAACAACGCCAUCUAUGATT-3', antisense strand: SEQ ID NO: 91: 5'-UCAUAGAUGGCGUUGUUGCTT-3';

VEGF-C:

Sense strand: SEQ ID NO: 92: 5'-GCAAGACGUUGUUUGAAAUUAUU-3', antisense strand: SEQ ID NO: 93: 5'-UAAUUUCAAACAACGUCUUGCUU-3'; Preferably, phosphorothioate modification is present at the internucleoside phosphate linkages (i) between adjacent nucleotides within positions 1-3 (inclusive) counted from the 5' terminus of the sense strand, (ii) between adjacent nucleotides within positions 1-3 (inclusive) counted from the 5' terminus of the antisense strand, and (iii) between adjacent nucleotides within positions 1-3 (inclusive) counted from the 3' terminus of the antisense strand; Alternatively,

Sense strand: SEQ ID NO: 84: 5'-CAGCAAGACGUUGUUUGAAAUUAUU -3', antisense strand: SEQ ID NO: 85: 5'- UAAUUUCAAACAACGUCUUGCUGUU -3'; Alternatively,

Sense strand: SEQ ID NO: 94: 5'-CAGGAUGGUAAAGACUACAUU-3', antisense strand: SEQ ID NO: 95: 5'-UGUAGUCUUUACCAUCCUGUU-3'; Preferably, phosphorothioate modification is present at the internucleoside phosphate linkages (i) between adjacent nucleotides within positions 1-3 (inclusive) counted from the 5' terminus of the sense strand, (ii) between adjacent nucleotides within positions 1-3 (inclusive) counted from the 5' terminus of the antisense strand, and (iii) between adjacent nucleotides within positions 1-3 (inclusive) counted from the 3' terminus of the antisense strand;

Preferably, the miRNA includes one or more of miR-34, miR-542, miR-126-3p, and miR-122;

Preferably, the ASO includes one or more of A-miR21, A-miR-10a, A-miR-30c, and A-miR-1306;

The sequence structures of each miRNA and ASO from 5' to 3' are as follows:

A-miR21:

5'-GATAAGCT-3' ; Preferably, each nucleotide is locked nucleic acid-modified; Alternatively,

5'-GAUAAGCU-3' ; Preferably, each nucleotide is locked nucleic acid-modified; Alternatively,

SEQ ID NO: 96: 5'-GTCAACATCAGTCTGATAAGCTA-3'; Alternatively,

SEQ ID NO: 97: 5'-GUCAACAUCAGUCUGAUAAGCUA-3'; Alternatively,

SEQ ID NO: 98: 5'-TCAACATCAGTCTGATAAGCTA; Alternatively,

5'-GATAAGCT-3'; Preferably, phosphorothioate modification is present between adjacent nucleotides;

A-miR-10a: 5'-ACAGGGTA-3'; Preferably, each nucleotide is locked nucleic acid-modified;

A-miR-30c: SEQ ID NO: 99: 5'-GCTGAGAGTGTAGGATGTTTACA-3';

miR-34: 5'-TGTGACAG-3';

miR-542: 5'-TGGCAGTGT-3';

miR-126-3p: 5'-UCGUACC-3'; preferably, phosphorothioate modification is present between adjacent nucleotides;

miR-122: 5'-GGAAGTGT-3;

AmiR-1306: SEQ ID NO: 100: 5'-CATCACCACCAGAGCCAACGTC-3'; preferably, phosphorothioate modification is present at the internucleoside phosphate linkages between adjacent nucleotides within positions 1-5 (inclusive) counted from the 5' terminus;

The sequence structures of each nucleic acid aptamer from 5' to 3' are as follows:

A1: SEQ ID NO: 101: 5'-GGTTGCATGCCGTGGGGAGGGGGGTGGGTTTTATAGCGTACTCAG-3';

A15: SEQ ID NO: 102: 5'-CCCTCCTACATAGGG-3'; Alternatively, SEQ ID NO: 227: 5'-CCCUCCUACAUAGGG-3';

AS1411: SEQ ID NO: 103: 5'-GGTGGTGGTGGTTGTGGTGGTGGTGG-3'; Alternatively, SEQ **ID** NO: 228: 5'-GGUGGUGGUGGUUGUGGUGGUGGUGG-3'; Alternatively, SEQ ID NO: 229:

AUUCUGAACCGUGUGCAGCACCACGCUGCACACGGUUCAGAAUACACACGAGGCTATCTAG AATGTAC-3';

AFP: SEQ ID NO: 104:
5'-GGCAGGAAGACAAACAAGCTTGGCGGCGGGAAGGTGTTTAAATTCCCGGGTCTGCGTGGT CTGTGGTGCTGT-3'; Alternatively, SEQ ID NO: 105:
5'-ACCTGGGGAGTATTGGGGAGGAAGG-3';

ATP: SEQ ID NO: 106: 5'-ACCTGGGGAGTATTGGGGAGGAAGG-3'; Alternatively, SEQ ID NO: 107: 5'-GGGAG GACGATGCGGAGGAAGGGTAGG-3', preferably, phosphorothioate modification is present at the internucleoside phosphate linkages between adjacent nucleotides within positions 1-4 (inclusive) counted from the 5' terminus;

Act-12c: SEQ ID NO: 108:
5'-CGGGGAAAGTCACGGGGGGTTTCAGATGTTCTGATCGGTGTGGAG-3';

A18: SEQ ID NO: 109: 5'-CCAGATAGTCCCTGG-3';

BAF7-1: SEQ ID NO: 110: 5'-GATAACGGGCACGAATTCGGAGTG-3';

C-Met-SL1: SEQ ID NO: 111:
5'-ATCAGGCTGGATGGTAGCTCGGTCGGGGTGGGTGGGTTGGCAAGTCTGAT-3';

CH6: SEQ ID NO: 112: 5'-AGTCTGTTGGACCGAATCCCGTGGACGCACCCTTTGGACG-3';

CA2: SEQ ID NO: 113: 5'-CCCACGTCTGCGCTTAGCTCCTGGGCCTGGATGGGC-3';

C12: SEQ ID NO: 114: 5'-GTGGATTGTTGTGTTCTGTTGGTTTTTGTGTTGTC-3'; preferably, phosphorothioate modification is present at the internucleoside phosphate linkages between adjacent nucleotides within positions 1-3 (inclusive) counted from the 5' terminus;

CRAC Orail: SEQ ID NO: 115:
5'-CCAGTAGCCATACCGGTTTGTGGATGGGGTGTATGCGAGT-3';

CEA: SEQ ID NO: 116: 5'-CTAGGATCCCCACTCACCATCTCTCAGCTTGCTTCCTAGC-3'; Alternatively, SEQ ID NO: 234:

5'-CUAGGAUCCCCACUCACCAUCUCUCAGCUUGCUUCCUAGC-3'; CEA-18: SEQ ID NO: 117: 5'-TTAACTTATTCGACCATA-3';

CEA-T84: SEQ ID NO: 118:

5'-TCGCGCGAGTCGTCTGGGGAACCATCGAGTTACACCGACCTTCTATGTGCGGCCCCCCGCATCGTCCTCCC-3';

CSC1: SEQ ID NO: 119:

5'-ACCTTGGCTGTCGTGTTGTAGGTGGTTTGCTGCGGTGGGCTCAAGAAGAAAGCGCAAAGAGGTCAGTGGTCAGAGCGT-3';

CSC13: SEQ ID NO: 120:

5'-ACCTTGGCTGTCGTGTTGTGGGGTGTCGTATCTTTCGTGTCTTATTATTTTCTAGGTGGAGGTCAGTGGTCAGAGCGT-3';

CD40: SEQ ID NO: 121: 5'-CCAACGAGTAGGCGATAGCGCGTGG-3'; Alternatively, SEQ ID NO: 122: 5'-AGAGACGATGCGGCCAACGAGTAGGCGATAGCGCGTGGCAGAGCGTCGCT-3';

CD16a: SEQ ID NO: 123: 5'-CCACTGCGGGGGTCTATACGTGAGGAAGAAGTGG-3';

CD19: SEQ ID NO: 124: 5'-TGCGTGTGTAGTGTGTGTCGTTTCTCCTTTTTTTGGTTGCTGCTCTTAGGGATTTGGGCGG-3'; Alternatively, SEQ ID NO: 125: 5'-TGCGTGTGTAGTGTGTCTGTTCTCCTTTTTTTGGTTGCTGCTCTTAGGGATTTGGGCGG-3';

CD3-4: SEQ ID NO: 126:

5'-TCTCGGACGCGTGTGGTCGGCCGAGTGGCCCACGGTAGAAGGGTTAGAACTGCTGGTTGGTGAATCTCGCTGCCTGGCCCTAGAGTG-3';

CD44:

SEQ ID NO: 127: 5'-CCAAGGCCTGCAAGGGAACCAAGGACACAG-3'; Alternatively,

SEQ ID NO: 128: 5'-CCAAGGCCTGCAAGGGAACCAAGGACACAG-3'; preferably, phosphorothioate modification is introduced at the internucleoside phosphate linkages (i) between adjacent nucleotides within positions 3-5 (inclusive) counted from the 5' terminus; (ii) between adjacent nucleotides within positions 12-14 (inclusive) counted from the 5' terminus; (iii) between nucleotides 1 and 2 counted from the 3' terminus; (iv) between nucleotides 3 and 4 counted from the 3' terminus; (v) between nucleotides 5 and 6 counted from the 3' terminus; and (vi) between adjacent nucleotides within positions 12-14 (inclusive) counted from the 3' terminus; Alternatively,

SEQ ID NO: 237:

5'-GGGAUGGAUCCAAGCUUACUGGCAUCUGGAUUUGCGCGUGCCAGAAUAAAGAGUAUAACGUGUGAAUGGGAAGCUUCGAUAGGAAUUCGG-3';

CD12 (HDLBP): SEQ ID NO: 129: 5'-GTGGATTGTTGTGTTCTGTTGGTTTTTGTGTTGTC-3';

CD20: SEQ ID NO: 130:
5'-TGCGTGTGTAGTGTGTCTGTTTTTTATCTTCTTTTATCTACTCTTAGGGATTTGGGCGG-3';

CD24: SEQ ID NO: 131: 5'-TATGTGGGTGGGTGGGCGGTTATGCTGAGTCAGCCTTGCT-3'; Alternatively, SEQ ID NO: 132:

5'-ATCCAGAGTGACGCAGCATATGTGGGTGGGTGGGCGGTTATGCTGAGTCAGCCTTGCTTGGACACGGTGGCTTAGT-3';

CD33: SEQ ID NO: 133:
5'-TACCAGTGCGATGCTCAGCACGCTTATAGGGGCTGGACAAAATTCTACCCAGCCTTT-3';

CD38: SEQ ID NO: 134: 5'-TACGTGAATCTCGTACGATACTCTGTAAGCGT-3';

CD105: SEQ ID NO: 135:
5'-GATCAGTTTTCCATGCCAGTTGGTATTCCGCGACAGTTTGATCTC-3';

CD117: SEQ ID NO: 136: 5'-GGGGCCGGGGCAAGGGGGGGGTACCGTGGTAGGAC-3';

CD63: SEQ ID NO: 137: 5'-CACCCCACCTCGCTCCCGTGACACTAATGCTA-3';

CD123: SEQ ID NO: 138:
5'-TGCGTGTGTAGTGTGTCTGGGCTACATCGATGAGCTGCCTAGGGTCCCTCTTAGGGATTTG GGCGG-3';

EGFR: SEQ ID NO: 139: 5'-GCCTTAGTAACGTGCTTTGATGTCGATTCGACAGGAGGC-3'; Alternatively, SEQ ID NO: 239: 5'-GCCUUAGUAACGUGCUUUGAUGUCGAUUCGACAGGAGGC-3'; Alternatively, SEQ **ID** NO: 240: 5'-UGCCGCUAUAAUGCACGGAUUUAAUCGCCGUAGAAAAGCAUGUCAAAGCCGUU-3';

EpCAM: SEQ ID NO: 140: 5'-CACTACAGAGGTTGCGTCTGTCCCACGTTGTCATGGGGGGTTGGCCTG-3'; Alternatively, SEQ ID NO: 141: 5'-GACAAACGGGGGAAGATTTGACGTCGACGAC-3'; Alternatively, SEQ **ID** NO: 241: 5'-GCGACUGGUUACCCGGUCG-3';

EcR: SEQ ID NO: 142:
5'-GCAGGTCCACTGCGGGGGTCTATACGTGAGGAAGAAGTGGGCAGGTC-3';

FAP: SEQ ID NO: 143: 5'-TGGGGGTTGAGGCTAAGCCGA-3';

Anti-FAP: SEQ ID NO: 144: 5'-CCGCTCGAGCTAGTCTGACAAAGAGAAACAC-3';

GPC-1: SEQ ID NO: 145: 5'-AACGGAGTGTGGCTAACTCGA-3';

GSK836: SEQ ID NO: 147: 5'-GCAGAGGTGAAGCGAAGTCG-3'; preferably, phosphorothioate modification is present at the internucleoside phosphate linkages between adjacent nucleotides within positions 1-6 (inclusive) counted from the 5' terminus;

GPC3 (APS63-1): SEQ ID NO: 148:
5'-TAACGCTGACCTTAGCTGCATGGCTTTACATGTTCCA-3'; preferably, phosphorothioate modification is present at the internucleoside phosphate linkages between adjacent nucleotides within positions 1-4 (inclusive) counted from the 5' terminus and within positions 1-4 (inclusive) counted from the 3' terminus;

Her2: SEQ ID NO: 149: 5'-AGCCGCGAGGGGAGGGATAGGGTAGGGCGCGGCT-3'; Alternatively, SEQ ID NO: 150:
5'-GGGAGCTCAGAATAAACGCTCAAAGGGTCAAGCTGATTACACTTTGTCCACTATTGGGTCCT TCGACATGAGGCCCGGATC-3'; Alternatively, SEQ ID NO: 246: 5'-AGCCGCGAGGGGAGGGAUAGGGUAGGGCGCGGCU-3';

Her3: SEQ ID NO: 151: 5'-GGGAGCTCAGAATAAACGCTCAAGGCTAACAGCACGCAACGGGGGGGAGTAATCGTGTCT GTTCGACAT GAGGCCCGGATC-3'; Alternatively, SEQ ID NO: 247: 5'-CAGCGAAAGUUGCGUAUGGGUCACAUCGCAGGCACAUGUCAUCUGGGCG-3'; Alternatively, SEQ ID NO: 248:

5'-GAAUUCCGCGUGUGCCAGCGAAAGUUGCGUAUGGGCCACAUCGCAGGCACAUGUCAUC UGGGCGGUCCGUUCGGGAUCC-3';

HMGA2: SEQ ID NO: 152: 5'-GGAAAAAATTTTTTAAAAAACCC-3'; preferably, phosphorothioate modification is present at the internucleoside phosphate linkages between adjacent nucleotides;

H2: SEQ ID NO: 146: 5'-GGGCCGTCGAACACGAGCATGGTGCGTGGACCTAGGATGACCTGAGTACTGTCC-3';

HBsAg: SEQ ID NO: 153: 5'-CACAGCGAACAGCGGCGGACATAATAGTGCTTACTACGAC-3'; preferably, phosphorothioate modification is present at the internucleoside phosphate linkages between adjacent nucleotides within positions 1-4 (inclusive) counted from the 5' terminus;

IFN-y (B4): SEQ ID NO: 154:

5'-CCGCCCAAATCCCTAAGAGAAGACTGTAATGACATCAAACCAGACACACACACTACACACGC A-3';

IL-4Ra: SEQ ID NO: 155: 5'-GGAGGACGAUGCGGAAAAAGCAACAGGGUGCUCCAUGCGCAUGGAACCUGC GCGCAGA CGACUCGCUGAGGAUCCGAGA-3'; Alternatively, SEQ ID NO: 156: 5'-AAAAAGCAACAGGGUGCUCCAUGCGCAUGGAACCUGCGCG-3';

IL-17: SEQ ID NO: 157: 5'-CTTGGATCACCATAGTCGCTAGTCGAGGCT-3'; Alternatively, SEQ ID NO: 158: 5'-G CGGCATCCTATCACGCATTGACC-3';

LZH8: SEQ ID NO: 159: 5'-ATCCAGAGTGACGCAGCATATTAGTACGGCTTAACCCCATGGTGGACACGGTGG CTTAGT-3';

MUC1: SEQ ID NO: 160: 5'-GCAGTTGATCCTTTGGATACCCTGG-3'; Alternatively, SEQ ID NO: 161: 5'-GAAGT GAAAATGACAGAACACAACA-3'; Alternatively, SEQ ID NO: 162: 5'-AACCGCCCAAATCTCTAAGAGTCGGACT GCAACCTATGCTATCGTTGATGTCTGTCCAAGCA ACACAGACACACTACACACACGCACA-3'; Alternatively, SEQ ID NO: 163: 5'-AATGACAGAACACAACATT-3'; Alternatively, SEQ ID NO: 250: 5'-GCAGUUGAUCCUUUGGAUACCCUGG-3';

M5: SEQ ID NO: 164:

5'-AGCAGCACAGAGGTCAGATGCTTGGTTCCACCGTACTGACTGTAGTAAAATCTGATCACTCC TATGCGTGCTACCGTGAA-3';

M7: SEQ ID NO: 165:

5'-AGCAGCACAGAGGTCAGATGTAGTCGGTCTTCTTGTTTGAAACTGCTAATTTTGAAAAAACC TATGCGTGCTACCGTGAA-3';

M1: SEQ ID NO: 166:

5'-AGCAGCACAGAGGTCAGATGATATAACCTTAATAAATAAAATATAAATTATTTAATCTTACCTATG CGTGCTACCGTGAA-3';

N5: SEQ ID NO: 167: 5'-GATTGAGTAGATAGTGGTTCTGTACGTAGTGAAAGAGTGG-3';

N-G-Dua: SEQ ID NO: 168:

5'-CAAGTTGCTCGTCGCGATACGTTTGGTTGGTGTGGTTGGCAGTATCGCAGGTCCAAGTTGC TCGTCGCGATACAACGGAGTGTGGCTAACTCGA-3';

NKG2D (20-N-15): SEQ ID NO: 169: 5'-CAAGTTGCTCGTCGCGATACGTTTGGTTGGTGTGGTTGGCAGTATC-3';

NSE: SEQ ID NO: 170: 5'-TCACACGGACCTCTCTCTACATTAATTGCGCATTTCGTT-3';

Np-A15: SEQ ID NO: 171: 5'-GCTGGATGTTCATGCTGGCAAAATTCCTTAGGGGCACCGTTACTTTGACACATCCAGC-3';

Np-A48: SEQ ID NO: 172: 5'-GCTGGATGTCGCTTACGACAATATTCCTTAGGGGCACCGCTACATTGACACATCCAGC-3';

Np-A58: SEQ ID NO: 173: 5'-GCTGGATGTCACCGGATTGTCGGACATCGGATTGTCTGAGTCATATGACACATCCAGC-3';

Np-A61: SEQ ID NO: 174: 5'-GCTGGATGTTGACCTTTACAGATCGGATTCTGTGGGGCGTTAAACTGACACATCCAGC-3';

OX40: SEQ ID NO: 175: 5'-GGGAGGACGATGCGGCAGTCTGCATCGTAGGAATCGCCACCGTATACTTTCCCA CCAGACG ACTCGCTGAGGATCCGAGA-3'; Alternatively, SEQ ID NO: 176: 5'-CAGTCTGCATCGTAGGATTAGCCACCGUATCTTTCCCAC-3'; Alternatively, SEQ ID NO: 177: 5'-CCAACGAGTAGGCGATAGCGCGTGG-3'; Alternatively, SEQ ID NO: 252: 5'-GGGAGGACGAUGCGGCAGUCUGCAUCGUAGGAAUCGCCACCGUAUACUUUC CCACCAG ACGACUCGCUGAGGAUCCGAGA-3'; Alternatively, SEQ ID NO: 253: 5'-GGGAGGACGAUGCGGC AGUCUGCAUCGUAGGAAUCGCCACCGUAUACUUUCCCACCAG ACGACUCGCUG-3'; Alternatively, SEQ ID NO: 254: 5'-CAGUCUGCAUCGUAGGAAUCGCCACCGUAUACUUUCCCAC-3'; Alternatively, SEQ ID NO: 255: 5'-GGGAUGCGGAAAAAAGAACACUUCCGAUUAGGGCCCACCCUAACGGCCGCAGAC-3';

PSMA: SEQ ID NO: 179: 5'-GGGAGGACGATGCGGATCAGCCATGTTTACGTCACTCCT-3'; Alternatively, SEQ ID NO: 180: 5'-GCGTTTTCGCTTTTGCGTTTTGGGTCATCTGCTTACGATAGCAATGCT-3'; Alternatively, SEQ ID NO: 256: 5'-GGGACCGAAAAAGACCUGACUUCUAUACUAAGUCUACGUUCCC-3'; Alternatively, SEQ ID NO: 257: 5'-GGGAGGACGAUGCGGAUCAGCCAUGUUUACGUCACUCCU-3';

PDGFRβ: SEQ ID NO: 181: 5'-TGTCGTGGGGCATCGAGTAAATGCAATTCGACA-3'; Alternatively, SEQ ID NO: 258: 5'-UGUCGUGGGGCAUCGAGUAAAUGCAAUUCGACA-3';

PDGF: SEQ ID NO: 182: 5'-CAGGCTACGGCACGTAGAGCATCACCATGATCCTG-3';

PD-L1: SEQ ID NO: 183: 5'-AACAACGTATAACAATGCCCACGTCACCAGAGTACTATGG-3'; Alternatively, SEQ ID NO: 184: 5'-GCCCCAGTTATGCTTTCCCCCTCTGTCTCTTTG-3'; Alternatively, SEQ ID NO: 185: 5'-ATCGCCC GCAGCACCCATTTGTTTTTTTTTG-3'; Alternatively, SEQ ID NO: 69: ACGGGCCACATCAACTCATTGATAGACAATGCGTCCACTGCCCGT; Alternatively, SEQ ID NO: 186: 5'-TGCCCGCACATCAACTCATTGATAGACAATG CGTCCACTCGGGCA-3'; Alternatively, SEQ ID NO: 187: 5'-TGCCCGCACATCAACTCATTGATAGACAATGCGT CCACTACGGGC-3'; Alternatively, SEQ ID NO: 188: 5'-CGGGCACACATCAACTCATTGATAGACAATGCGTCCA CTGCCCGT-3'; Alternatively, SEQ ID NO: 189: 5'-GTTGGTCACATCAACTCATTGATAGACAATGCGTCCACTAC CAAC-3'; Alternatively, SEQ ID NO: 190: 5'-GGGCCACATCAACTCATTGATAGACAATGCGTCCACTGCCC -3'; Alternatively, SEQ ID NO: 191: 5'-TGGTTGCACATCAACTCATTGATAGACAATGCGTCCACTCAACCA-3'; Alternatively, SEQ ID NO: 200: 5'-TACAGGTTCTGGGGGGTGGGTGGGGAACCTGTT-3'; Alternatively, SEQ ID NO: 238: 5'-CTACGAGACGAACTTATGCGTAATAATGACTGTCGTAG-3';

PD-1: SEQ ID NO: 192: 5'-GACGATAGCGGTGACGGCACAGACGGTACAGTTCCCGTCCCTGCACTACACGTA TGCCGCT TCCGTCCGTCGCTC-3'; Alternatively, SEQ ID NO: 193: 5'-GAGCGACGGACGGAAGCGGCATACG

TGTAGTGCAGGGACGGGAACTGTACCGTCTGTGCC GTCACCGCTATCGTC-3'; Alternatively, SEQ ID NO: 194: 5'-GGATCCTAGACGCATTGACCCGCTGCCTCTACTGAGGCTGTGTCAGTGTGCGGCTCGGACT GTTGAATTC-3'; Alternatively, SEQ ID NO: 195: 5'-AGCGGTGACGGCACAGACGGTACAGTTCCCGTCCCTGC ACTACACGTATGCCGCTGAGAG AGAGGGAGGC-3'; Alternatively, SEQ ID NO: 196: 5'-ACCGACAGTGAAGG ACTCAGCGAACTCTCAGACTCGGTTC-3';

PTK-7: SEQ ID NO: 197: 5'-ATCTAACTGCTGCGCCGCCGGGAAAATACTGTACGGTTAGA-3';

ProGRP-48: SEQ ID NO: 198: 5'-CATGCGGAGTAGAGCGAGCCCAGATAGTCCCTGGTTATTTCCTTAGG-3';

SF: SEQ ID NO: 199: 5'-GATCTCTCTCTGCCCTAAGTCCGCACCCGTGCTTCCCTGT-3';

TBA15: SEQ ID NO: 201: 5'-GGTTGGTGTGGTTGG-3';

TBA29: SEQ ID NO: 202: 5'-AGTCCGTGGTAGGGCAGGTTGGGGTGACT-3';

TfRA4: SEQ ID NO: 203: 5'-GCGTGGTACCACGC-3'; Alternatively, SEQ ID NO: 259: 5'-GCGUGGUACCACGC-3';

TfRA3: SEQ ID NO: 204: 5'-GCGTGGTCACACGC-3'; Alternatively, SEQ ID NO: 205: 5'-GCGGCGCCCACGAG CGTTCGCGTGGTCACACGCGTTCCGCCCTCCTACATAGGGCGCATA GCCGTGGGCGCCGC-3'; Alternatively, SEQ ID NO: 260: 5'-GCGUGGUCACACGC-3';

TTA1: SEQ ID NO: 206: 5'-CCTGCACTTGGCTTGGATTTCAGAAGGGAGACCC-3'; Alternatively, SEQ ID NO: 286: 5'-CTGCACTTGGCTTGGATTTCAGAAGGGAGACCC-3'; Alternatively, SEQ ID NO: 261: 5'-CCUGCA-CUUGGCUUGGAUUUCAGAAGGGAGACCC-3'; TLS9a: SEQ ID NO: 207: 5'-AGTCCATTTTATTCCTGAATATTT GTTAACCTCATGGAC-3';

TGF-βII (S58): SEQ ID NO: 208:

5'-ACATTGCTGCGTGATCGCCTCACATGGGTTTGTCTGGTCGATTTGGAGGTGGTGGGTGGC-3';

TNF-a: SEQ ID NO: 209: 5'-GCGGCCGATAAGGTCTTTCCAAGCGAACGAAAA-3';

TNF: SEQ ID NO: 210: 5'-GCGCCACTACAGGGGAGCTGCCATTCGAATAGGTGGGCCGC-3';

T1: SEQ ID NO: 211:

5'-CGCTCGATAGATCGAGCTTCGCTCGATGTGGTGTTGTGGGGGCTTGTATTGGTCGATCACG CTCTAGAGCACTG-3';

VEGF: SEQ ID NO: 212: 5'-TGTGGGGGTGGACTGGGTGGGTACC-3'; Alternatively, SEQ ID NO: 213: 5'-TGTG GGGGTGGACGGGCCGGGTAGA-3'; Alternatively, SEQ ID NO: 214: 5'-GGTGGGGGTGGACGGGCCGGGTAG A-3'; Alternatively, SEQ ID NO: 266: 5'-AUGCAGUUUGAGAAGUCGCGCAU-3'; preferably, a phosphorothioate modification is present at the internucleoside phosphate linkage between nucleotides 6 and 7 counted from the 5' terminus;

VCAM-1: SEQ ID NO: 215: 5'-ATACCAGCTTATTCAATTGGACACGGCAAAGGGGTATAGCCTACCGGACCGTG AACATGGAA TGGTGTGCTGCGTGGAGATAGTAAGTGCAATCT-3'; Alternatively, SEQ ID NO: 216: 5'-GGACA CGGCAAAGGGGTATAGCCTACCGGACCGTGAACATGGAATGGTGTGCTGCGTGG-3' ;

VCAM-12d: SEQ ID NO: 217: 5'-AGGGAATCTTGCCTAGGGAGGGAGTAGCGAAAGGGCTCA-3';

CH6: SEQ ID NO: 218: 5'-AGTCTGTTGGACCGAATCCCGTGGACGCACCCTTTGGACG-3';

PL-45: SEQ ID NO: 219: 5'-ACTCATAGGGTTAGGGGCTGCTGGCCAGATACTCAGATGGTAGGGTTACTATGAG

C-3';

EP66: SEQ ID NO: 220: 5'-AACAGAGGGACAAACGGGGGAAGATTTGACGTCGACGACA-3';

AGC: SEQ ID NO: 221: 5'-CGACCCGGCACAAACCCAGAACCATATACACGATCATTCGTCTCCTGGGCCG-3';

Karpas299: SEQ ID NO: 222: 5'-ATCCAGATGACGCAGCACCACCACCGTACAATTTTTTCATTACCTACTCGGC-3';

SW620: SEQ ID NO: 223: 5'-CCCATCAATGTTACGACCCGCTAGGGCTGCTGTGCCATCGGGTAA-3';

MDA-MB-231: SEQ ID NO: 224:

5'-AGAATTCAGTCGGACAGCGAAGTAGTTTTCCTTCTAACCTAAGAACCCGCGGCAGTTTAATGTAGA-3';

MCF-7: SEQ ID NO: 225: 5'-GCATGGGGTTTCGGCGTTTCGTCTATCTTGTTTCTGTTAGCGTCT-3';

PC-3: SEQ ID NO: 226: 5'-TGCCACTACAGCTGGTTCGGTTTGGTGACTTCGTTCTTCGTTGTGGTGCTTAGTGGC-3';

BCMA: SEQ ID NO: 230: 5'-AGUGCAAGACGUUCGCAGAUUAGCGAAAAGAGGGUCUCAUUGACUAGUAC-3';

CTLA-4: SEQ ID NO: 231: 5'-GGUGGAAGAGGGAUGGGCCGACGUGCCGCAU-3'; Alternatively, SEQ ID NO: 232:

5'-TCCCTACGGCGCTAACGATGGTGAAAATGGGCCTAGGGTGGACGGTGCCACCGTGCTACAAC-3';

CCL1: SEQ ID NO: 233: 5'-UGACUCCUCUGACAGCCUAAUUUCUCCCGAUUACCCUG-3';

CD4-3: SEQ ID NO: 235: 5'-GGGAGGACGAUGCGGUUUGGGGUUUUCCCGUGCCCCAGACGACUCGCCCGA-3';

CD28: SEQ ID NO: 236:

5'-GGGAGAGAGGAAGAGGGAUGGGGAUUAGACCAUAGGCUCCCAACCCCCCCGGGAGAGAGGAAGAGGGAUGGGGAUUAGACCAUAGGCUCCCAACCCCCGGG-3';

FGF2 (F2): SEQ ID NO: 242: 5'-GGGAUACUAGGGCAUUAAUGUUACCAGUGUAGUCCC-3';

FGF2: SEQ ID NO: 243: 5'-GGGAAACUAGGGCGUUAACGUGACCAGUGUUUCUCGA-3'; Alternatively, SEQ ID NO: 244: 5'-GGGAAACUAGGGCGUUAACGUGACCAGUGUUUCCC-3';

FGF5: SEQ ID NO: 245: 5'-GGGCGACCUCUCCGUACUGACCUACAGAGCGACAUACUAGUGUAUCCAGAUCGCCC-3';

LAG-3: SEQ ID NO: 249:

5'-GGGAGAGAGAUAUAAGGGCCUCCUGAUACCCGCUGCUAUCUGGACCGAUCCCAUUACCAAAUUCUCUCCC-3';

MRP1: SEQ ID NO: 251: 5'-GGGAGAAUAGUCAACAAAUCGUUUGGGGCGACUUCUCCUUCCUUUCUCCC-3';

TIM3: SEQ ID NO: 262: 5'-GGGAGAGGACCAGUA-GCCACUAUGGUGUUGGAGCUAGCGG-CAGAGCGUCGCGGUCCC UCCC-3'; Alternatively, SEQ ID NO: 263: 5'-GGGAGAGGACCAGUA -CUGGUAGUUCUCUGUGCGACUCCUA-CAGAGCGUCGCGGUCCCUCCC-3';

TIMC-11: SEQ ID NO: 264: 5'-AAGCAACACUUAGUCGCGAUUGAUACGUGCGCAGUCAU-3'; Alternatively, SEQ ID NO: 265:

5'-GGAGGACGAUGCGGGGAAGCAACACUUAGUCGCGAUUGAUACGUGCGCAGUCAUCAGA CGACUCGCUGAGGAUCCGAGA-3';

VEGF165: SEQ ID NO: 267: 5'-CGGAAUCAGUGAAUGCUUA UACAUCCG-3';

4-1BB: SEQ ID NO: 268:

5'-GGGAGAGAGGAAGAGGGAUGGGCGACCGAACGUGCCCUUCAAAGCCGUUCACUAACCA GUGGCAUAACCCAGAGGUCGAUAGUACUGGAUCCCCCC-3';

The sequences of each immunostimulant from 5' to 3' are as follows:
CPG2006:

SEQ ID NO: 269: 5'-TCGTCGTTTTGTCGTTTTGTCGTT-3'; preferably, phosphorothioate modification is present at the internucleoside phosphate linkages between adjacent nucleotides; Alternatively,

SEQ ID NO: 270: 5'-UCGUCGUUUUGUCGUUUUGUCGUU-3';

CPG1826: SEQ ID NO: 271: TCCATGACGTTCCTGACG-3'; preferably, phosphorothioate modification is present at the internucleoside phosphate linkages between adjacent nucleotides;

CPG2216: SEQ ID NO: 272: 5'-GGGGGACGATCGTCGGGGGG-3';

CPG2395: SEQ ID NO: 273: TCGTCGTTTTCGGCGCGCGCCG-3';

CPG-ODNT7: SEQ ID NO: 274: 5'-TCGTCGTCGTCGTCGTCGTCG-3'; Alternatively, SEQ ID NO: 275: 5'-TCGT CGTCGTCGTCGTCGTCG-3';

CPG-ODN-PCIF1: 5'-AGCGAA-3'.

Preferably, when the targeted chemical drug includes a nucleic acid aptamer, the targeting moiety includes the nucleic acid aptamer and/or a small-molecule targeting ligand; when the targeted chemical drug does not include a nucleic acid aptamer, the targeting moiety is a small-molecule targeting ligand, wherein the small-molecule targeting ligand is selected from one or more of folic acid, biotin, vitamin B12, and mannose.

By selecting the foregoing types of oligonucleotide effector molecules, immunostimulants, and small-molecule targeting ligands and co-loading them together with the small-molecule chemical drug onto the above-specified nucleic acid carrier, synergistic effects can be further realized to achieve more favorable outcomes.For example, the nucleolin aptamer AS1411 can bind, with specificity and high affinity, to nucleolin on the surface of tumor cells, while exhibiting no affinity for normal cells.After AS1411 binds membrane nucleolin on tumor cells, it can induce macropinocytosis to enter cells rapidly and, by inducing the activities of EGFR, Akt, p38, and Rac1, further promote macropinocytosis; moreover, nucleolin, as a multifunctional shuttling protein, translocates among the plasma membrane, cytoplasm, and nucleus in this process, functioning in a chaperone-like "escort" capacity.As an auxiliary targeting ligand within a multi-ligand targeting system, the EGFR aptamer is highly expressed in tumor cells and can bind, with specificity and high affinity, to the EGFR receptor protein on the surface of tumor cells, while exhibiting no or low affinity for normal cells.CD133$^+$ tumor cells exhibit enhanced self-renewal, differentiation, and tumorigenic capacity; CD133 serves as a common marker of tumor stem-like cells and is highly expressed in a variety of solid tumors (e.g., glioma, breast cancer, gastric cancer, liver cancer, lung cancer, pancreatic cancer, prostate cancer, colorectal cancer, etc.).A15 aptamer, which specifically binds to CD133, exhibits in vivo targeting capacty and specific binding to CD133$^+$ tumor cells.

In the present invention, the A15 aptamer is modified, by sequence-extension, at one end of the three-branched DNA structure; preferably, together with AS1411 and EGFR aptamers modified at the other two termini of the nucleic acid

nanocarrier, it is assembled into a composite targeting configuration and loaded with epirubicin to obtain an enhanced targeted chemotherapeutic. Upon intravenous administration, the targeted, improved epirubicin formulation can multiply anchor to tumor cells in tumor tissues that overexpress nucleolin, EGFR, and CD133, thereby synergistically increasing the likelyhood and efficiency for specific binding of the targeted drug to tumor cells.Following cellular internalization, the nucleic acid nanocomplex undergoes reduction and enzymatic cleavage under intracellular conditions characeterized by low pH, high glutathione, and nuclease activity, thereby releasing the parent epirubicin drug.Once released into the nucleus, the tetracyclic anthracycline ring of epirubicin intercalates specifically into GC/CG sites of target-gene DNA, inhibiting DNA transcription and suppressing tumor proliferation and progression.In this confuguration, the A15 aptamer additionally mediates tracking of $CD133^+$ tumor stem-like cells, inhibiting the expansion of key tumor "seed" cells to achieve control or regression of tumor growth.miR-21 is associated with the development of drug resistance; employing its antisense sequence can suppress the emergence of resistance and enhance the cytotoxic effect of the drug, thereby more effectively killing tumor cells. The TfRA4 targeting ligand mediates passage of the nucleic acid nanoparticles across the blood-brain barrier, enabling epirubicin to more effectively kill intracranial tumor cells.In addition to AS1411, A15, EGFR, and TfRA4 aptamers, other targeting aptamers provided herein, when linked to the above DNA carrier, likewise exert corresponding functions to achieve improved delivery of epirubicin.

The small-molecule chemical drug employed in the present invention may be of types commonly used in the art, including but not limited to anthracycline chemotherapeutics, pyrimidine-class chemotherapeutics, platinum chemotherapeutics, glutamic-acid-derivative chemotherapeutics, flavonoid chemotherapeutics, plant-derived drugs and derivatives thereof, folate chemotherapeutics, salicylic-acid chemotherapeutics, or acridine-class chemotherapeutics.In view of improved binding stability with the nucleic acid carrier, in one preferred embodiment, the anthracycline chemotherapeutic is selected from doxorubicin, epirubicin, pirarubicin, daunorubicin, idarubicin, mitoxantrone, and valrubicin, or the free base or hydrochloride thereof; Preferably, the pyrimidine-class chemotherapeutic is selected from gemcitabine, 5-fluorouracil, cytarabine, or capecitabine; Preferably, the platinum chemotherapeutic is selected from cisplatin, oxaliplatin, carboplatin, nedaplatin, or lobaplatin; Preferably, the glutamic-acid-derivative chemotherapeutic is selected from lenalidomide, thalidomide, or pomalidomide; Preferably, the flavonoid chemotherapeutic is selected from flavones, flavonols, dihydroflavones, isoflavones, or chalcones; Preferably, the plant-derived drug or derivative is selected from vincristine, dihydroartemisinin, paclitaxel, maytansine, docetaxel, or 10-hydroxycamptothecin; Preferably, the folate chemotherapeutic is methotrexate; Preferably, the salicylic-acid chemotherapeutic is selected from aspirin or sodium salicylate; Preferably, the acridine-class chemotherapeutic is selected from tacrine or amsacrine; And, more preferably, the small-molecule chemical drug is selected from epirubicin or the free base or hydrochloride thereof, gemcitabine, 5-fluorouracil, paclitaxel, and maytansine (one or more).

Preferably, when the small-molecule targeting ligand, the oligonucleotide effector molecule, and the immunostimulant are linked to the nucleic acid carrier, they are either directly conjugated to terminal bases of the respective sequences of the nucleic acid carrier, or are linked thereto via a base linker including 1 to 10 nucleotides; preferably, when the small-molecule chemical drug is an anthracycline chemotherapeutic or an acridine-class chemotherapeutic, the small-molecule chemical drug intercalates specifically between adjacent GC base pairs of the targeted nucleic acid carrier; when the small-molecule chemical drug is a pyrimidine-class chemotherapeutic, the small-molecule targeting ligand, the oligonucleotide effector molecule, and the immunostimulant are linked to the terminal bases of the sequences of the nucleic acid carrier via a base linker including 1 to 10 nucleotides, and the small-molecule chemical drug is incorporated into the nucleic acid carrier in the form of at least partial substitution of bases in the base linker and/or as an extended base and/or as substitution of nucleotides of the carrier backbone; when the small-molecule chemical drug is a platinum chemotherapeutic, a glutamic-acid-derivative chemotherapeutic, a flavonoid chemotherapeutic, a plant-derived drug or a derivative thereof, a folate chemotherapeutic, or a salicylic-acid chemotherapeutic, the small-molecule chemical drug is covalently linked to the nucleic acid carrier via a linker; preferably, the linker is selected from long-chain primary amine compounds, more preferably long-chain primary amine compounds containing C2-C14, and further preferably 5-(bromomethyl)-6-bromohexan-1-amine (C7).After condensation with two equivalents of phosphate ester, a linker-containing base phosphoramidite monomer is further prepared (e.g., N4-benzoyl-5'-O-(4, 4'-dimethoxytrityl)-2'-deoxycytidine-3'-(6-aminohexyl-2-hydroxymethyl)-N, N'-diisopropyl phosphoramidite, or N2-benzoyl-5'-O-(4, 4'-dimethoxytrityl)-2'-deoxyguanosine-3'-(6-aminohexyl-2-hydroxymethyl)-N, N'-diisopropyl phosphoramidite).

The loading amount of each small-molecule chemical drug may range from 1 up to the theoretical loading amount, wherein the term "theoretical loading amount" refers to the maximum number of small-molecule chemical drug molecules that can theoretically be loaded based on the sequence structure of the nucleic acid carrier; for example, when the small-molecule chemical drug intercalates specifically between adjacent GC base pairs within the targeted nucleic acid carrier, the number of adjacent GC base pairs in the sequence is the theoretical loading amount, as will be readily understood by those skilled in the art.In practical applications, the molar ratio of epirubicin to the nucleic acid carrier is from 2: 1-300: 1, preferably from 2: 1-290: 1, more preferably from 2: 1-29: 1, further preferably from 10: 1-50: 1, and most preferably from 15: 1-25: 1.

For the purpose of further improving drug size and/or stability during delivery, and to optimize combination

pharmacodynamics with toxicity reduction and efficacy enhancement, in a preferred embodiment the present invention provides the following more reliable targeted chemical drugs:

(1)The targeted chemical drug includes a DNA carrier, a small-molecule chemical drug, and a nucleic acid aptamer EGFR and optional biotin linked to the DNA carrier; preferably, the small-molecule chemical drug is epirubicin hydrochloride or its free base. Preferably, the DNA carrier includes the first set of sequences; the nucleic acid aptamer EGFR is linked to the 5' end of the sequence b, and the molar ratio of the nucleic acid aptamer EGFR to the DNA carrier is 1: 1. Preferably, the nucleic acid aptamer EGFR is linked to the 5' end of the sequence b via a base linker "TTTTT".When the targeted chemical drug includes the DNA carrier, the small-molecule chemical drug, and the nucleic acid aptamer EGFR and biotin linked to the DNA carrier, 2 to 4 biotin moieties are linked per DNA carrier, each biotin being linked to the 5' or 3' end of the sequence a or the sequence c.Specifically, the targeted chemical drug includes a targeted nucleic acid carrier formed by self-assembly of the following sequences and epirubicin conjugated to the targeted nucleic acid carrier:

Drug 1: Apt EGFR-DNA-epirubicin

| Name | Sequence (sense, 5'-3') | Modification |
|---|---|---|
| Sequence a (SEQ ID NO: 7) | **5'-GCGGCGCCCACGAGCGTTCCGGGAGC-3'** | None |
| Sequence b (SEQ ID NO: | **5'-**GCCTTAGTAACGTGCTTTGATGTCGATTCGACAGGAGG CTTTTT**GCTCCCGGTTCGCCGCCAGCCGCC-3'** | None |
| 281) | | |
| Sequence c (SEQ ID NO: 9) | **5'-GGCGGCAGGCGGCCATAGCCGTGGGCGCCGC-3'** | None |
| Note: The **bold** portions denote the sequences a, b, or c of the nucleic-acid carrier (same hereinafter)."TTTTT" is the base linker. | | |

In Drug 1, the nucleic acid nanocarrier is modified by sequence-extension with an EGFR aptamer to confer active targeting capability, enabling specific binding to tumor cells overexpressing EGFR. The three double-stranded DNA arms, formed by self-assembly of the three core scaffold sequences are designed to including approximately 30 GC/CG sites serving as epirubicin loading positions.During the preparation, an excess ratio of 40: 1 (epirubicin : nucleic acid nanocarrier) isemployed and 4% paraformaldehyde is used as a coupling agent. The fused tetracyclic ring system of the epirubicin molecule specifically intercalates at GC/CG sites of the nanocarrier, and the amino group of its daunosamine moiety forms, under paraformaldehyde catalysis, a Schiff base (imine) with an exocyclic amine on guanine at the GC/CG binding site of the carrier, thereby establishing a stable loading mode including physical intercalation plus covalent attachment.In addition to the inherent passive-targeting "funneling" accumulation effect of nanoparticles in tumor tissues, the drug exhibits targeting affinity to tumors and cells with high EGFR expression; under the combined action of these targeting effects, high local accumulation at the tumor site is achieved.Upon binding of the EGFR aptamer to EGFR receptors overexpressed on the tumor cell surface, the nanoconstruct is internalized into the cytoplasm via endocytosis and related uptake pathways.Under the intracellular conditions of low pH and high glutathione (reducing environment), epirubicin dissociates from the nanocarrier and is released; the released free epirubicin then enters the nucleus and specifically intercalates into GC/CG base pair sites of nuclear double-stranded DNA, thereby blocking DNA transcription and inhibiting tumor-cell differentiation and proliferation, producing an antitumor effect that suppresses tumor occurrence and progression.Specifically, the mechanisms of delivery, intracellular trafficking, release, sustained release, and tumor inhibition for this drug are as follows:

**1.Delivery:** EGFR-DNA-epirubicin nanoparticles are introduced by targeted delivery and exhibit an intrinsic passive-targeting "funneling" accumulation effect in tumor tissue.

**2. Trafficking:** Upon binding of the EGFR aptamer to EGFR receptor proteins overexpressed on the tumor-cell surface, the nanoparticles are internalized via endocytosis and related pathways. This binding drives high local

accumulation at the tumor site and enhances therapeutic efficacy.

**3. Release:** Once internalized into tumor cells, epirubicin dissociates from the nucleic acid nanocarrier and is released under the catalytic conditions of low intracellular pH and a high-glutathione reducing environment.

**4. Sustained release:** Because epirubicin is stably loaded on the nucleic acid nanocarrier through a combination of physical intercalation and covalent linkage, controlled and sustained release is achieved, thereby reducing systemic toxicity and adverse effects.

**5. Mechanism of inhibition:** The released epirubicin translocates into the nucleus and specifically intercalates into GC/CG sites of nuclear double-stranded DNA, inhibitingDNA transcription and suppressing tumor-cell differentiation and proliferation to achieve an anti-tumor effect.

In summary, the coordinated mechanisms of delivery, trafficking, release, sustained release, and transcriptional inhibition render Drug 1 a promising targeted anti-tumor agent.By specifically targeting tumor tissues and cells with high EGFR expression, leveraging the passive accumulation of nanoparticles, achieving efficient yet controlled intracellular release, and specifically blocking DNA transcription in tumor cells, the present drug provides a novel approach for cancer therapy.

Drug 2: Apt EGFR-4*Bio-DNA-epirubicin

| **Name** | Sequence (sense, 5'→3') | Modification |
|---|---|---|
| Sequence a (SEQ ID NO: 7) | **5'-GCGGCGCCCACGAGCGTTCCGGGAGC-3'** | **Biotin (Bio)** at both 5' and 3' ends (one at each end) |
| Sequence b (SEQ ID NO: 281) | **5'-**GCCTTAGTAACGTGCTTTGATGTCGATTCGACAGGA GGCTTTTT**GCTCCCGGTTCGCCGCCAGCCGCC-3'** | None |
| Sequence c (SEQ ID NO: 9) | **5'-GGCGGCAGGCGGCCATAGCCGTGGGCGCCGC-3'** | **Biotin (Bio)** at both 5' and 3' ends (one at each end) |

In Drug 2, the nucleic acid nanocarrier is modified by sequence-extension with an EGFR aptamer, and four biotin moieties are conjugated via polyethylene glycol (PEG) modification at the termini of the other two arms so as to confer active targeting capability, enabling specific bindings to tumor cells overexpressing EGFR and biotin receptor proteins. The three double-stranded DNA arms formed by self-assembly of the three core scaffold sequences are designed to including approximately 30 GC/CG sites serving as epirubicin loading positions, thereby establishing a stable loading mode of physical intercalation plus covalent attachment. The mechanisms of delivery, intracellular trafficking, release, sustained release, and tumor inhibition are as follows:

**1.Delivery:** The nanocarrier achieves active targeting by EGFR-aptamer modification and biotin conjugation, enabling specific binding to EGFR and biotin receptor proteins highly expressed on tumor cells.

2. **Trafficking:** After the EGFR aptamer and biotin bind the target proteins on the tumor-cell surface, the nanoparticles are internalized via endocytosis and related pathways. This binding effect drives high local accumulation at the tumor site and enhances efficacy.

3. **Release:** Once internalized into tumor cells, epirubicin dissociates from the nucleic acid nanocarrier and is released under the catalytic conditions of low intracellular pH and a high-glutathione reducing environment.

4. **Sustained release:** Because epirubicin is stably loaded through physical intercalation and covalent linkage, controlled and sustained release is achieved, thereby reducing systemic toxicity and adverse effects.

5. **Mechanism of inhibition:** The released epirubicin translocates into the nucleus and specifically intercalates into

GC/CG sites of nuclear double-stranded DNA, thereby inhibiting DNA transcription and suppressing tumor-cell differentiation and proliferation to acheive an antitumor effect.

In summary, the coordinated mechanisms of delivery, trafficking, release, sustained release, and transcriptional inhibition render Drug 2 a promising targeted antitumor agent. By specifically targeting tumor tissues and cells with high EGFR and biotin-receptor expression, leveraging the passive "funneling" accumulation of nanoparticles, achieving efficient yet controlled intracellular release, and specifically blocking DNA transcription in tumor cells, the present drug provides a novel approach for cancer therapy.

(2)The targeted chemical drug includes a DNA carrier, a small-molecule chemical drug, and the DNA-form nucleic acid aptamer AS1411, the nucleic acid aptamer EGFR, the DNA-form nucleic acid aptamer A15, and optional biotin linked to the DNA carrier; preferably, the small-molecule chemical drug is epirubicin hydrochloride or its free base. Preferably, the DNA carrier includes the first set of sequences; the DNA-form nucleic acid aptamer AS1411 is present in a molar ratio of 1: 1 relative to the DNA carrier and is linked to the 5' terminus of the sequence a; preferably, AS1411 is linked to the 5' terminus of the sequence a via a base linker "TTTTT" (five thymidines). The EGFR nucleic acid aptamer is present in a molar ratio of 1: 1 relative to the DNA carrier and is linked to the 5' terminus of the sequence b; preferably, the EGFR aptamer is linked to the 5' terminus of the sequence b via a base linker "TTTTT". The DNA-form nucleic acid aptamer A15 is present in a molar ratio of 1: 1 relative to the DNA carrier and is linked to the 5' terminus of the sequence c; preferably, the A15 aptamer is linked to the 5' terminus of the sequence c via a base linker "TTTTT".When the targeted chemical drug includes the DNA carrier, the small-molecule chemical drug, and the DNA-form nucleic acid aptamer AS1411, the EGFR nucleic acid aptamer, the DNA-form nucleic acid aptamer A15, and biotin linked to the DNA carrier, two biotin moieties are linked per DNA carrier, the biotin moieties being respectively linked to the 3' terminus of the sequence a and the 3' terminus of the sequence c.Specifically, the targeted chemical drug includes a targeted nucleic acid carrier formed by self-assembly of the following sequences and epirubicin conjugated to the targeted nucleic acid carrier:

Drug 3: Apt AS1411-Apt EGFR-Apt A15-2*Bio-DNA-epirubicin:

| Name | Sequence **(sense, 5'→3')** | Modification |
|---|---|---|
| Sequence a (SEQ ID NO: 282) | **5'-**GGTGGTGGTGGTTGTGGTGGTGGTGGTTTTT**GCGG CGCCCACGAGCGTTCCGGGAGC-3'** | One biotin (Bio) at the 3' end |
| Sequence b (SEQ ID NO: 283) | **5'-**GCCTTAGTAACGTGCTTTGATGTCGATTCGACAGGAG GCTTTTT**GCTCCCGGTTCGCCGCCAGCCGCC-3'** | None |
| Sequence c (SEQ ID NO: 284) | **5'-**CCCTCCTACATAGGGTTTTT**GGCGGCAGGCGGCCAT AGCCGTGGGCGCCGC-3'** | One biotin (Bio) at the 3' end |

Drug 4: Apt AS1411-Apt EGFR-Apt A15-DNA-epirubicin:

| **Name** | Sequence (sense, 5'→3') | Modification |
|---|---|---|
| Sequence a (SEQ ID NO: 282) | **5'-**GGTGGTGGTGGTTGTGGTGGTGGTGGTTTTT**GCGGCG CCCACGAGCGTTCCGGGAGC-3'** | None |
| Sequence b (SEQ ID NO: 283) | **5'-**GCCTTAGTAACGTGCTTTGATGTCGATTCGACAGGAGG CTTTTT**GCTCCCGGTTCGCCGCCAGCCGCC-3'** | None |
| Sequence c (SEQ ID NO: 284) | **5'-**CCCTCCTACATAGGGTTTTT**GGCGGCAGGCGGCCATAG CCGTGGGCGCCGC-3'** | None |

In Drugs 3 and 4, the nucleic acid nanocarrier is prepared by sequence-extension modification, whereby the AS1411 aptamer, the EGFR aptamer, and the A15 aptamer are respectively attached to the 5' termini of the three oligonucleotide sequences that constitute the carrier backbone; optionally, biotin (Bio) is attached via PEG linkage to the 3' termini of the sequences a and c.In this way, a composite-targeting nucleic acid nanocarrier is formed that specifically binds nucleolin, EGFR, CD133 (tumor stem-like) receptors, and biotin receptors, with epirubicin being covalently loaded onto the carrier. This drug is a nucleic-acid nanocarrier that achieves composite targeting through the AS1411, EGFR, and A15 aptamers and biotin (Bio). The mechanisms of delivery, intracellular trafficking, release, sustained release, and inhibition are as follows:

1.**Delivery:** Composite targeting is realized by modification with the AS1411, EGFR, and A15 aptamers and optional biotin (Bio). This enables the drug to bind specifically to different classes of tumor cells by exploiting the affinity of the aptamers for their respective surface receptors.

2. **Trafficking:** Upon binding of the aptamer to the target receptors on the tumor-cell surface, the construct is internalized into the cell via endocytic mechanisms (including ER trafficking pathways).Such binding drives high local accumulation of nanoparticles at the tumor site and enhances efficacy.

3. **Release:** Once internalized into tumor cells, epirubicin dissociates from the nucleic acid nanocarrier and is released under the catalytic conditions of low intracellular pH and high glutathione concentration (reducing environment).

4. **Sustained release:** Because epirubicin is stably loaded through a combination of physical intercalation and covalent linkage, controlled and sustained release is achieved, thereby reducing systemic toxicity and adverse effects.

5. **Mechanism of inhibition:** The released epirubicin translocates into the nucleus and specifically intercalates into GC/CG sites of nuclear double-stranded DNA, thereby inhibiting DNA transcription and suppressing tumor-cell differentiation and proliferation to achieve an antitumor effect.

In summary, the coordinated mechanisms of delivery, trafficking, release, sustained release, and transcriptional inhibition render Drug 3 a promising composite-targeting antitumor agent. By specifically targeting different receptors on tumor tissues and cells, leveraging the passive (funneling) accumulation of nanoparticles, achieving efficient yet controlled intracellular release, and specifically blocking DNA transcription in tumor cells, the present drug provides a novel approach for cancer therapy.

(3)The targeted chemical drug includes a DNA carrier, a small-molecule chemical drug, and the DNA-form nucleic acid aptamer AS1411 and biotin linked to the DNA carrier; preferably, the small-molecule chemical drug is epirubicin hydrochloride or its free base. Preferably, the DNA carrier includes the first set of sequences; the DNA-form nucleic acid aptamer AS1411 is linked to the 5' terminus of the sequence b, with a molar ratio of AS1411 to the DNA carrier of 1: 1; preferably, AS1411 is linked to the 5' terminus of the sequence b via a base linker "TTTTT" (five thymidines).From 2 to 4 biotin moieties are linked per DNA carrier, each biotin being linked to the 5' or 3' terminus of the sequence a or the sequence c. Specifically, the targeted chemical drug includes a targeted nucleic acid carrier formed by self-assembly of the following sequences and epirubicin conjugated to the targeted nucleic acid carrier:

Drug 5: Apt AS1411-4*Bio-DNA-epirubicin:

| Name | Sequence (sense, 5'→3') | Modification |
|---|---|---|
| Sequence a (SEQ ID NO: 7) | **5'-GCGGCGCCCACGAGCGTTCCGGGAGC-3'** | **Biotin (Bio)** at both 5' and 3' ends (one at each end) |
| Sequence b (SEQ ID NO: 285) | **5'-**GGTGGTGGTGGTTGTGGTGGTGGTGGTTTTT**GCTCCCGGTTCGCCGCCAGCCGCC-3'** | None |
| Sequence c (SEQ ID NO: 9) | **5'-GGCGGCAGGCGGCCATAGCCGTGGGCGCCGC-3'** | **Biotin (Bio)** at both 5' and 3' ends (one at each end) |

In Drug 5, the nucleic-acid nanocarrier achieves active, specific targeting by modification with the AS1411 aptamer and, optionally, by conjugation of biotin. The mechanisms of delivery, trafficking, release, sustained release, and inhibition are as follows:

**1.Delivery:** Active targeting is achieved by AS1411 aptamer modification and optional biotin conjugation, enabling the drug to bind specifically to tumor cells that overexpress nucleolin (NCL) and biotin-receptor proteins.

2. **Trafficking:** Upon binding of AS1411 and biotin to the target proteins on the tumor-cell surface, the nanoparticles are internalized via endocytosis and related pathways. This interaction drives high local accumulation of the nanoparticles at the tumor site, enhancing therapeutic efficacy.

3. **Release:** Once internalized into tumor cells, epirubicin dissociates from the nucleic-acid nanocarrier and is released under the catalytic conditions of low intracellular pH and a high-glutathione reducing environment.

4. **Sustained release:** Because epirubicin is stably loaded on the nanocarrier through physical intercalation together with covalent linkage, controlled and sustained release is achieved, thereby reducing systemic toxicity and adverse effects.

5. **Mechanism of inhibition:** The released epirubicin translocates into the nucleus and specifically intercalates into GC/CG sites of nuclear double-stranded DNA, thereby inhibiting DNA transcription and suppressing tumor-cell differentiation and proliferation to achieve an antitumor effect.

In summary, the coordinated mechanisms of delivery, trafficking, release, sustained release, and transcriptional inhibition render Drug 5 a promising targeted antitumor agent. By specifically targeting tumor tissues and cells that overexpress nucleolin (NCL) and biotin-receptor proteins, leveraging the passive accumulation of nanoparticles, achieving efficient yet controlled intracellular release, and specifically blocking DNA transcription in tumor cells, the present drug provides a novel approach for cancer therapy.

(4)The targeted chemical drug includes a DNA carrier, a small-molecule chemical drug, and the nucleic acid aptamer A1 and the DNA-form nucleic acid aptamer A15 linked to the DNA carrier; preferably, the small-molecule chemical drug is epirubicin hydrochloride or its free base. Preferably, the DNA carrier includes the first set of sequences; the A1 aptamer is present in a molar ratio of 1: 1 relative to the DNA carrier and is linked to the 5' terminus of the sequence b; preferably, the A1 aptamer is linked to the 5' terminus of the sequence b via a base linker "TTTTT". The DNA-form nucleic acid aptamer A15 is present in a molar ratio of 1: 1 relative to the DNA carrier and is linked to the 5' terminus of the sequence c; preferably, the A15 aptamer is linked to the 5' terminus of the sequence b via a base linker "TTTTT".Specifically, the targeted chemical drug includes a targeted nucleic acid carrier formed by self-assembly of the following sequences and epirubicin conjugated to the targeted nucleic acid carrier:

Drug 6: Apt A1-AptA15-DNA-epirubicin:

| Name | Sequence (sense, 5'→3') | Modification |
|---|---|---|
| Sequence a (SEQ ID NO: 7) | **5'-GCGGCGCCCACGAGCGTTCCGGGAGC-3'** | None |
| Sequence b (SEQ ID NO: 287) | **5'-**GGTTGCATGCCGTGGGGAGGGGGGTGGGTTTTATAGC GTACTCAGTTTTT**GCTCCCGGTTCGCCGCCAGCCGCC-3'** | None |
| Sequence c (SEQ ID NO: 288) | **5'-**CCCTCCTACATAGGGTTTTT**GGCGGCAGGCGGCCATAG CCGTGGGCGCCGC-3'** | None |

In Drug 6, the nucleic-acid nanocarrier achieves active, specific targeting through modification with the A1 and A15 aptamers. The mechanisms of delivery, trafficking, release, sustained release, and inhibition are as follows:

**1.Delivery:** Active targeting is realized by modification with the A1 and A15 aptamers. This configuration enables the drug to bind specifically to tumor cells by exploiting the affinity of A1 and A15 for the corresponding CD133 receptors

on the tumor-cell surface.

2. **Trafficking:** Upon the binding of A1 and A15 aptamers to the target receptors on the tumor-cell surface, the nanoparticles are internalized via endocytosis and related pathways.Such binding drives high local accumulation at the tumor site and enhances therapeutic efficacy.

3. **Release:** Once internalized into tumor cells, epirubicin dissociates from the nucleic-acid nanocarrier and is released under the catalytic conditions of low intracellular pH and a high-glutathione reducing environment.

4. **Sustained release:** Because epirubicin is stably loaded through physical intercalation together with covalent linkage, controlled sustained release is achieved, thereby reducing systemic toxicity and adverse effects.

5. **Mechanism of inhibition:** The released epirubicin translocates into the nucleus and specifically intercalates into GC/CG sites of nuclear double-stranded DNA, thereby inhibiting DNA transcription and suppressing tumor-cell differentiation and proliferation to achieve an antitumor effect.

In summary, the coordinated mechanisms of delivery, trafficking, release, sustained release, and transcriptional inhibition described above render Drug 6 a promising targeted anti-tumor agent. By specifically targeting tumor tissues and cells, leveraging the passive (funneling) accumulation effect of nanoparticles, achieving efficient yet controlled intracellular release, and specifically blocking DNA transcription in tumor cells, the present drug provides a novel approach for cancer therapy.

(5)The targeted chemical drug includes a DNA carrier, a small-molecule chemical drug, and the DNA-form nucleic acid aptamer AS1411, the nucleic acid aptamer EGFR, the DNA-form nucleic acid aptamer A15, optional biotin, and the oligonucleotide A-miR-21, all linked to the DNA carrier; preferably, the small-molecule chemical drug is epirubicin hydrochloride or its free base. Preferably, the DNA carrier includes the first set of sequences; the DNA-form nucleic acid aptamer AS1411 is present at a 1: 1 molar ratio relative to the DNA carrier and is linked to the 5' terminus of the sequence a; preferably, AS1411 is linked to the 5' terminus of the sequence a via a base linker "TTTTT" (five thymidines). The EGFR aptamer is present at a 1: 1 molar ratio relative to the DNA carrier and is linked to the 5' terminus of the sequence b; preferably, the EGFR aptamer is linked to the 5' terminus of the sequence b via a base linker "TTTTT." The DNA-form nucleic acid aptamer A15 is present at a 1: 1 molar ratio relative to the DNA carrier and is linked to the 5' terminus of the sequence c; preferably, the A15 aptamer is linked to the 5' terminus of the sequence c via a base linker "TTTTT." The oligonucleotide A-miR-21 is present at a 1: 1 molar ratio relative to the DNA carrier and is linked to the 3' terminus of the sequence c.Optionally, two biotin moieties are linked per DNA carrier, the biotin moieties being respectively linked to the 3' terminus of the sequence a and to the end of A-miR-21 distal from the sequence c.Specifically, the targeted chemical drug includes a targeted nucleic acid carrier formed by self-assembly of the following sequences and epirubicin conjugated to the targeted nucleic acid carrier:

Drug 7: Apt AS1411-Apt EGFR-Apt A15-2*Bio-AmiR-21-DNA-epirubicin

| Name | Sequence (sense, 5'→3') | Modification |
|---|---|---|
| Sequence a (SEQ ID NO: 289) | **5'-**GGTGGTGGTGGTTGTGGTGGTGGTGGTTTTT**GCG GCGCCCACGAGCGTTCCGGGAGC-3'** | One biotin (Bio) at the 3' end |
| Sequence b (SEQ ID NO: 290) | **5'-**GCCTTAGTAACGTGCTTTGATGTCGATTCGACAGGA GGCTTTTT**GCTCCCGGTTCGCCGCCAGCCGCC-3'** | None |
| Sequence c (SEQ ID NO: 291) | **5'-**CCCTCCTACATAGGGTTTTT**GGCGGCAGGCGGCCA TAGCCGTGGGCGCCGC**GATAAGCT**-3'** | Eight nucleotides immediately upstream of the 3' terminus LNA-modified; one biotin (Bio) at the 3' end |

Drug 8: Apt AS1411-Apt EGFR-Apt A15-AmiR-21-DNA-epirubicin

| Name | Sequence (sense, 5'→3') | Modification |
|---|---|---|
| Sequence a (SEQ ID NO: 289) | **5'-**GGTGGTGGTGGTTGTGGTGGTGGTGGTTTTT**GCGGCGCCCACGAGCGTTCCGGGAGC-3'** | None |
| Sequence b (SEQ ID NO: 290) | **5'-**GCCTTAGTAACGTGCTTTGATGTCGATTCGACAGGAGGCTTTTT**GCTCCCGGTTCGCCGCCAGCCGCC-3'** | None |
| Sequence c (SEQ ID NO: 291) | **5'-**CCCTCCTACATAGGGTTTTT**GGCGGCAGGCGGCCATAGCCGTGGGCGCCGC**GATAAGCT**-3'** | Eight nucleotides immediately upstream of the 3' terminus LNA-modified |

In Drugs 7 and 8, the nucleic-acid nanocarrier achieves a three-tier targeting-mediated effect via the AS1411, EGFR, and A15 aptamers and optionally biotin. The functions of each moiety and the three-tier targeting-mediated effect are analyzed as follows:

**1. AS1411 aptamer:** AS1411 is an oligonucleotide that specifically binds nucleolin on the surface of tumor cells.Such binding promotes cellular uptake of the drug and enables targeted anti-tumor activity.

2. **EGFR aptamer:** EGFR (epidermal growth factor receptor) is overexpressed in many malignant tumors.Via the EGFR aptamer, the drug specifically binds tumor cells with EGFR overexpression, thereby increasing the local drug concentration within tumor tissue.

3. **A15 aptamer:** A15 is an aptamer that specifically binds the CD133 receptor present on tumor stem-like cells.Since tumor stem cells are key drivers of recurrence and drug resistance, incorporation of the A15 aptamer enables targeting of tumor stem cells.

4. **A-miR-21:** A-miR-21 is an anti-tumor antisense oligonucleotide (ASO) directed against microRNA-21.Since microRNA-21 is overexpressed in many cancers and promotes tumorigenesis and progression, A-miR-21 suppresses the function of microRNA-21 and thereby inhibits tumor development.

5. **Epirubicin:** Epirubicin inhibits differentiation and proliferation of tumor cells by blocking DNA transcription.

Through this three-tier targeting-mediated effect, the above drugs accomplish effective delivery, intracellular trafficking, release, sustained/controlled release, and inhibitory action.Emphasis is placed on targeting tumor stem cells to suppress tumor initiation and progression. This composite targeting strategy is expected to enhance therapeutic efficacy, reduce adverse effects, and provide a new therapeutic approach for the treatment of multiple malignancies.

(6)The targeted chemical drug includes a DNA carrier, a small-molecule chemical drug, and the DNA-form nucleic acid aptamers TfRA4, AS1411, and A15 linked to the DNA carrier; preferably, the small-molecule chemical drug is epirubicin hydrochloride or its free base. Preferably, the DNA carrier includes the first set of sequences; the DNA-form nucleic acid aptamer TfRA4 is present at a 1: 1 molar ratio relative to the DNA carrier and is linked to the 5' terminus of the sequence a; preferably, TfRA4 is linked to the 5' terminus of the sequence a via a base linker "TTTTT" (five thymidines). The DNA-form nucleic acid aptamer AS1411 is present at a 1: 1 molar ratio relative to the DNA carrier and is linked to the 5' terminus of the sequence b; preferably, AS1411 is linked to the 5' terminus of the sequence b via a base linker "TTTTT." The DNA-form nucleic acid aptamer A15 is present at a 1: 1 molar ratio relative to the DNA carrier and is linked to the 5' terminus of the sequence c; preferably, A15 is linked to the 5' terminus of the sequence c via a base linker "TTTTT.":

Drug 9: Apt TfRA4-Apt AS1411-Apt A15-DNA-epirubicin (1):

| **Name** | Sequence (sense, 5'→3') | Modification |
|---|---|---|
| Sequence a (SEQ ID NO: 292) | **5'-**GCGTGGTACCACGCTTTTT**GCGGCGAGCGGCGCCC ACGAGCGTTCCGGGAGAGGAGC-3'** | None |
| Sequence b (SEQ ID NO: 293) | **5'-**GGTGGTGGTGGTTGTGGTGGTGGTGGTTTTT**GCTCC TCTCCCGGTTCGCCGCCAGCCGCC-3'** | None |
| Sequence c (SEQ ID NO: 294) | **5'-**CCCTCCTACATAGGGTTTTT**GGCGGCTGGCGGCCAT AGCCGTGGGCGCCGCTCGCCGC-3'** | None |

In Drug 9, the AS1411, TfRA4, and A15 aptamers are respectively attached to the 5' termini of the three oligonucleotide sequences that constitute the scaffold of the nucleic-acid carrier, thereby forming a composite-targeting nucleic-acid nanocarrier that specifically binds nucleolin, the transferrin receptor on the blood-brain barrier (BBB), CD133 on tumor stem-like cells, and (optionally) biotin receptors, with epirubicin covalently loaded to yield a targeted epirubicin drug. This nanocarrier can bind the BBB transferrin receptor and, with AS1411 acting in concert, traverse the BBB to the brain parenchyma, where it binds nucleolin highly expressed on glioma cells; notably, it further binds CD133-high tumor stem-like cells, enabling cellular entry and suppression of tumor initiation and progression. Through a three-tier targeting-mediated effect, the drug accomplishes delivery, trafficking, release, sustained release, and inhibition. Details are as follows:

1.Delivery: Incorporation of the TfRA4 aptamer enables specific binding to the transferrin receptor (TfR) on the BBB, thereby facilitating transport across the BBB into the brain.

2. Trafficking: After BBB transit, the AS1411 aptamer promotes specific binding within the brain to nucleolin overexpressed on the surface of glioma cells, achieving targeted delivery to glioma cells.

3. Release and sustained release: Once internalized, the covalently loaded epirubicin is gradually released from the nanocarrier under low intracellular pH and high glutathione conditions, affording a sustained-release profile that reduces systemic toxicity while improving efficacy.

4. Mechanism of inhibition: The A15 aptamer confers specific binding to CD133-positive tumor stem-like cells, promoting cellular uptake and subsequent inhibitory action. This is critical for suppressing gliomas, as tumor stem cells are key drivers of recurrence and drug resistance.

Summary for Drug 9. Through a three-tier targeting-mediated effect, Drug 9 specifically engages the transferrin receptor on the blood-brain barrier (BBB), nucleolin receptors, and the CD133 receptor on tumor stem-like cells, thereby accomplishing efficient delivery, intracellular trafficking, release, sustained/controlled release, and inhibitory action. Emphasis is placed on targeting tumor stem cells to suppress the onset and progression of glioblastoma. This composite targeting strategy is expected to enhance therapeutic efficacy, minmize adverse effects, and provide a novel therapeutic approachfor glioblastoma and other malignant tumors.

(7)The targeted chemical drug includes a DNA carrier, a small-molecule chemical drug, and the DNA-form nucleic acid aptamers TfRA4, AS1411, and A15 linked to the DNA carrier; the small-molecule chemical drug is epirubicin hydrochloride or the free base. The DNA carrier includes the eleventh group of sequences. The DNA-form nucleic acid aptamer TfRA4 is present at a 1: 1 molar ratio relative to the DNA carrier and is linked to the 5' terminus of the sequence a; preferably, TfRA4 is linked to the 5' terminus of the sequence a via a transition base segment (SEQ ID NO: 276: 5'-TTGCGGCGAGCGGCGA-3'), and the targeted chemical drug further includes a complementary sequence (SEQ ID NO: 277: 5'-TCGCCGCTCGCCGCTT-3') that complementarily hybridizes with the transition base segment, the 3' terminus of the complementary sequence being joined to the 5' portion of the TfRA4 aptamer. The DNA-form nucleic acid aptamer AS1411 is present at a 1: 1 molar ratio relative to the DNA carrier and is linked to the 5' terminus of the sequence b; preferably, AS1411 is linked to the 5' terminus of the sequence b via a base linker "TTTTT." The DNA-form nucleic acid aptamer A15 is present at a 1: 1 molar ratio relative to the DNA carrier and is linked to the 5' terminus of the sequence c; preferably, A15 is linked to the 5' terminus of the sequence c via a base linker "TTTTT." Specifically, the targeted chemical drug includes a targeted nucleic acid carrier formed by self-assembly of the following sequences and epirubicin conjugated to the targeted nucleic acid carrier:

Drug 10: Apt TfRA4-Apt AS1411-AptA15-DNA + epirubicin (2):

| Name | Sequence (sense, 5'→3') | Modification |
|---|---|---|
| Sequence a (SEQ ID NO: 295) | **5'-**TCGCCGCTCGCCGCTTGCGTGGTACCACGCTTGC GGCGAGCGGCGA**GCCCACGAGCGTTCCGGGAGA-3'** | None |
| Sequence a(SEQ ID NO: 296) | **5'-**GGTGGTGGTGGTTGTGGTGGTGGTGGTTTTT**TCTC CCGGTTCGCCGCCAGCCGCC-3'** | None |
| Sequence c (SEQ ID NO: 297) | **5'-**CCCTCCTACATAGGGTTTTT**GGCGGCTGGCGGCCA TAGCCGTGGGC-3'** | None |

Drug 10 is a variant of Drug 9, and differs from Drug 8 in that the 14-nucleotide sequences flanking the TfRA4 aptamer-the transition base segment 5'-TTGCGGCGAGCGGCGA-3' and the complementary sequence 5'-TCGCCG CTCGCCGCTT-3'-are perfectly complementary.Upon self-assembly, these segments hybridize through an intervening TT/TT spacer and an extended "neck" (stem) region, thereby further improving the structural stability of the construct and resolving the propensity of TfRA4 to form multimers via pairwise self-assembly.

(8)The targeted chemical drug includes a DNA carrier, a small-molecule chemical drug, and the DNA-form nucleic acid aptamers AS1411, TfRA3, and A15 linked to the DNA carrier; preferably, the small-molecule chemical drug is epirubicin hydrochloride or its free base. Preferably, the DNA carrier includes the first set of sequences; the DNA-form nucleic acid aptamer AS1411 is present at a 1: 1 molar ratio relative to the DNA carrier and is linked to the 5' terminus of the sequence a; preferably, AS1411 is linked to the 5' terminus of the sequence a via a base linker "TTTTT" (five thymidines). The DNA-form nucleic acid aptamer TfRA3 is present at a 1: 1 molar ratio relative to the DNA carrier and is linked to the 5' terminus of the sequence b; preferably, TfRA3 is linked to the 5' terminus of the sequence b via a base linker "TTTTT." The DNA-form nucleic acid aptamer A15 is present at a 1: 1 molar ratio relative to the DNA carrier and is linked to the 5' terminus of the sequence c; preferably, the A15 aptamer is linked to the 5' terminus of the sequence b via a base linker "TTTTT." Specifically, the targeted chemical drug includes a targeted nucleic acid carrier formed by self-assembly of the following sequences and epirubicin conjugated to the targeted nucleic acid carrier:

Drug 11: Apt TfRA3-Apt AS1411-Apt A15-DNA-epirubicin

| Name | Sequence (sense, 5'→3') | Modification |
|---|---|---|
| Sequence a (SEQ ID NO: 298) | **5'-**GGTGGTGGTGGTTGTGGTGGTGGTGGTTTTT**GCG GCGCCCACGAGCGTTCCGGGAGC-3'** | None |
| Sequence b (SEQ ID NO: 299) | **5'-**GCGTGGTCACACGCTTTTT**GCTCCCGGTTCGCCGC CAGCCGCC-3'** | None |
| Sequence c (SEQ ID NO: 300) | **5'-**CCCTCCTACATAGGGTTTTT**GGCGGCAGGCGGCCA TAGCCGTGGGCGCCGC-3'** | None |

In Drug 11, the nucleic-acid nanocarrier is prepared by sequence-extension so that the AS1411, TfRA3, and A15 aptamers are respectively attached to the 5' termini of the three oligonucleotide strands forming the carrier backbone, thereby constituting a composite-targeting nucleic-acid nanocarrier that specifically binds nucleolin, the transferrin receptor on the blood-brain barrier (BBB), CD133 on tumor stem-like cells, and (optionally) biotin receptors; epirubicin is covalently loaded to yield a targeted epirubicin drug. The nanocarrier can bind the BBB transferrin receptor and, in concert with AS1411, traverse the BBB to the brain parenchyma, where it binds nucleolin highly expressed on the surface of glioma cells; notably, it also binds CD133-high tumor stem-like cells, enabling cellular entry and inhibition of tumor

initiation and progression.Specifically, Drug 11 is a nucleic-acid nanocarrier that achieves composite targeting through modification with the AS1411, TfRA3, and A15 aptamers. The mechanisms of delivery, trafficking, release, sustained release, and inhibition are as follows:

1.Delivery: By virtue of the AS1411, TfRA3, and A15 aptamers, the nanocarrier specifically engages nucleolin, the BBB transferrin receptor (TfR), and CD133 receptors, thereby enabling binding to TfR on the BBB, traversal of the BBB, and access to the brain parenchyma.

2. Three-tier targeting-mediated effect: a) First tier: binding to TfR on the BBB via the TfRA3 aptamer, with BBB transit aided by AS1411 acting in concert; b) Second tier: after reaching the brain parenchyma, binding to nucleolin overexpressed on glioma cells via AS1411; c) Third tier: specific binding to CD133-positive tumor stem-like cells via A15.

3. Trafficking: Following receptor engagement on tumor-cell surfaces, the construct is internalized via endocytosis and related mechanisms.

4. Release: Once internalized into tumor cells, epirubicin dissociates from the nucleic-acid nanocarrier and is released under conditions of low intracellular pH and high glutathione.

5. Sustained release: Because epirubicin is stably loaded through physical intercalation together with covalent linkage, controlled and sustained release is achieved, thereby reducing systemic toxic side effects.

6. Mechanism of inhibition: The released epirubicin translocates into the nucleus and specifically intercalates into GC/CG sites of nuclear double-stranded DNA, inhibiting DNA transcription and thereby suppressing tumor-cell differentiation and proliferation to achieve an antitumor effect. In particular, by binding CD133-high tumor stem-like cells via the A15 aptamer, the drug enters and suppresses tumor stem cells-an effect that is critical for controlling and eliminating glioblastoma.

Tumor stem cells possess self-renewal capacity and multipotent differentiation potential and play a pivotal role in tumor initiation, recurrence, and drug resistance. By targeting the CD133 receptor highly expressed on tumor stem-like cells, Drug 11 can more effectively recognize and eliminate these cells, thereby addressing the root cause of tumor development and suppressing tumor relapse.

Accordingly, the delivery, trafficking, release, sustained-release, and inhibitory mechanisms of Drug 11 are realized through a three-tier targeting-mediated effect, with emphasis on targeting tumor stem cells to inhibit the initiation and progression of glioblastoma. This composite-targeting strategy is expected to enhance therapeutic efficacy, reduce adverse effects, and provide a novel therapeutic approach for glioblastoma and other malignancies.

(9)The targeted chemical drug includes a DNA carrier, a small-molecule chemical drug, and CPG2006 (immunostimulant), the CD40 nucleic acid aptamer, and the PD-L1 DNA-form nucleic acid aptamer linked to the DNA carrier; preferably, the small-molecule chemical drug is epirubicin hydrochloride or its free base. Preferably, the DNA carrier includes the ninth set of sequences; CPG2006 is present at a 1: 1 molar ratio relative to the DNA carrier and is linked to the 5' terminus of the sequence a; preferably, CPG2006 is linked to the 5' terminus of the sequence a via a base linker "TTTTT." The CD40 aptamer is present at a 1: 1 molar ratio relative to the DNA carrier and is linked to the 5' terminus of the sequence b; preferably, the CD40 aptamer is linked to the 5' terminus of the sequence b via a base linker "TTTTT." The PD-L1 DNA-form aptamer is present at a 1: 1 molar ratio relative to the DNA carrier and is linked to the 5' terminus of the sequence c; preferably, the PD-L1 aptamer is linked to the 5' terminus of the sequence c via a base linker "TTTTTT" (six thymidines).Specifically, the targeted chemical drug includes a targeted nucleic acid carrier formed by self-assembly of the following sequences and epirubicin conjugated to the targeted nucleic acid carrier:

Drug 12: CPG2006 (DNA) - Apt CD40 - Apt PD-L1 - DNA - epirubicin

| Name | Sequence (sense, 5'→3') | Modification |
|---|---|---|
| Sequence a (SEQ ID NO: 301) | **5'-**TCGTCGTTTTGTCGTTTTGTCGTTTTTTT**GCGACGCCCACGA GCGTTCCGGGAGAGGAGC-3'** | See modification notes below |

(continued)

| Name | Sequence (sense, 5'→3') | Modification |
|---|---|---|
| Sequence b (SEQ ID NO: 302) | **5'-**CCAACGAGTAGGCGATAGCGCGTGGTTTTT**GCTCCTCTCCC GGTTCGCCGCGAGCCGCG-3'** | See modification notes below |
| Sequence c (SEQ ID NO: 303) | **5'-**ACGGGCCACATCAACTCATTGATAGACAATGCGTCCACTGC CCGTTTTTT**CGCGGCTCGCGGCCATAGCCGTGGGCGTCGC-3'** | See modification notes below |
| Note: For the sequence a, phosphorothioate modification is introduced at the internucleoside phosphate linkages between each pair of adjacent nucleotides at positions 1-24 from the 5' terminus, and between each pair of adjacent nucleotides at positions 1-3 from the 3' terminus.For the Sequence b and sequence c, phosphorothioate modification is introduced between each pair of adjacent nucleotides at positions 1-3 from the 5' terminus and between each pair of adjacent nucleotides at positions 1-3 from the 3' terminus. | | |

In Drug 12, the nucleic-acid nanocarrier integrates three aptamers (CPG2006, CD40, and PD-L1) to achieve active, combined immunotherapy together with chemotherapy targeted to the tumor microenvironment (TME). The respective functions of each effector and the synergistic antitumor effect arising from integrated TME targeting are as follows:

1.CPG2006 aptamer (CpG oligonucleotide): Acts as an immunostimulant that activates the immune system via TLR9 recognition.Within the TME, CPG2006 activates dendritic cells (DCs) and B cells, thereby promoting T-cell activation and expansion and strengthening antitumor immune responses.

2. CD40 aptamer: Engagement of the CD40 pathway (via CD40/CD40L interaction) activates dendritic cells and B cells, helps remodel the TME, and augments antitumor immunity.

3. PD-L1 aptamer: PD-L1 is an immunosuppressive ligand exploited by tumors to inhibit T-cell activity. The PD-L1 aptamer blocks the PD-1/PD-L1 interaction, restores T-cell function, and enhances antitumor immune responses.

4. Epirubicin: A cytotoxic chemotherapeutic that, upon release, blocks DNA transcription, thereby inhibiting tumor-cell differentiation and proliferation.

By loading epirubicin onto a single nanocarrier that simultaneously presents CPG2006, CD40, and PD-L1 aptamers, this drug realizes combined immunotherapy plus classic chemotherapy targeted to the TME. In the TME, CPG2006 and CD40 activate DCs and B cells and promote T-cell priming/expansion; the PD-L1 aptamer relieves checkpoint inhibition by blocking PD-1/PD-L1 and restores T-cell activity. The three aptamers act synergistically to amplify antitumor immune responsed.Meanwhile, epirubicin exerts a direct cytotoxic effect on tumor cells by blocking DNA transcription, further facilitating immune-mediated clearance.

The tumor microenvironment is a complex cellular and molecular network including tumor cells, immune cells, stromal/supporting cells, and extracellular matrix. By integrated targeting of the TME, this drug achieves a synergistic antitumor effect from the combination of immunotherapy and chemotherapy: (i) activation of immune cells (e.g., DCs, B cells, and T cells) to condition the TME in favor of antitumor responses; (ii) checkpoint blockade to enhance T-cell cytotoxicity; and (iii) direct tumor-cell kill by epirubicin.

In summary, by integrating immunotherapy with chemotherapy on a single targeted platform, the drug produces a cooperative, TME-targeted antitumor effect, with the potential to improve therapeutic efficacy and patient outcomes while reducing adverse effects.

(10)The targeted chemical drug includes a DNA carrier, a small-molecule chemical drug, and, linked to the DNA carrier, an immunostimulant CPG2006, a CD40 nucleic acid aptamer, a PD-L1 DNA-form nucleic acid aptamer, and a C12 nucleic acid aptamer; wherein the DNA carrier further includes complementary sequence d and sequence e, the sequence d being SEQ ID NO: 278: 5'-GCCACCGTGCTACA-3' and the sequence e being SEQ ID NO: 279: 5'-CAGCAGCAGCAGCA-3'. The 3' terminus of the sequence b of the DNA carrier is joined to a transition base segment SEQ ID NO: 280: 5'-GTAGCACGGTGGC-3'; the sequence d is complementarily hybridized to the transition base segment SEQ ID NO: 280: 5'-GTAGCACGGTGGC-3', and the sequence e is complementarily hybridized to the sequence c of the DNA carrier. Preferably, the small-molecule chemical drug is epirubicin hydrochloride or its free base. Preferably, the DNA carrier includes the tenth set of sequences; CPG2006 is present at a 2: 1 molar ratio relative to the DNA carrier and is linked to the 5' termini of the sequence a and the sequence c, respectively; preferably, CPG2006 is linked to the 5' terminus of the

sequence a via a base linker "TTTTTT" (six thymidines) and to the 5' terminus of the sequence c via a base linker "TTTTT" (five thymidines). The CD40 aptamer is present at a 1: 1 molar ratio relative to the DNA carrier and is linked to the 5' terminus of the sequence b; preferably, the CD40 aptamer is linked to the 5' terminus of the sequence b via a base linker "TTTTT." The PD-L1 DNA-form aptamer is present at a 1: 1 molar ratio relative to the DNA carrier and is linked to the 5' terminus of the sequence d; preferably, the PD-L1 aptamer is linked to the 5' terminus of the d sequence via a base linker "TTTTTT." The C12 aptamer is present at a 1: 1 molar ratio relative to the DNA carrier and is linked to the 5' terminus of the sequence e; preferably, the C12 aptamer is linked to the 5' terminus of the sequence e via a base linker "TTTTT." Specifically, the targeted chemical drug includes a targeted nucleic-acid carrier formed by self-assembly of the following sequences and epirubicin conjugated to the targeted nucleic-acid carrier:

Drug 13: 2*CPG2006 (DNA) - Apt CD40 - Apt PD-L1 - Apt C12 - DNA - epirubicin

| Name | Sequence (sense, 5'-3') |
|---|---|
| Sequence a (SEQ ID NO: 304) | **5'-**TCGTCGTTTTGTCGTTTTGTCGTTTTTTT**GACGCCCACGAGCGTTCCGGGAGAGG-3'** |
| Sequence b (SEQ ID NO: 305) | **5'-**CCAACGAGTAGGCGATAGCGCGTGGTTTTT**CCTCTCCCGGTTCGCCGCGAGCCTGTAGCACGGTGGC-3'** |
| Sequence c (SEQ ID NO: 306) | **5'-**TCGTCGTTTTGTCGTTTTGTCGTTTTTT**GGCTCGCGGCCATAGCCGTGGGCGTCTGCTGCTGCTGCTG-3'** |
| Sequence d (SEQ ID NO: 307) | **5'-**ACGGGCCACATCAACTCATTGATAGACAATGCGTCCACTGCCCGTTTTTTGCCACCGTGCTACA**-3'** |
| Sequence e (SEQ ID NO: 308) | **5'-**GTGGATTGTTGTGTTCTGTTGGTTTTTGTGTTGTCTTTTTCAGCAGCAGCAGCA**-3'** |
| Note.For the sequences a and c, phosphorothioate modification is introduced at the internucleoside phosphate linkages between each pair of adjacent nucleotides at positions 1-24 from the 5' terminus.For the sequences b, d, and e, phosphorothioate modification is introduced between each pair of adjacent nucleotides at positions 1-3 from the 5' terminus. | |

In Drug 13, the nucleic-acid nanocarrier integrates three aptamers (CPG2006, CD40, and PD-L1) and targets the C12 receptor protein highly expressed on the surface of small cell lung cancer (SCLC) cells, thereby achieving combined immunotherapy and classical chemotherapy co-targeted to the tumor microenvironment (TME). The drug can efficiently suppress recurrent, refractory, and SCLC tumor cells.

The CPG2006 aptamer is a CpG oligonucleotide mimic that activates the Toll-like receptor 9 (TLR9) pathway, thereby stimulating activation and proliferation of dendritic cells and B cells and enhancing the antitumor immune response. The CD40 aptamer, by binding to the CD40 receptor, activates dendritic cells, B cells, and other antitumor immune effector cells, thereby promoting an antitumor immune response. The PD-L1 aptamer blocks the interaction between PD-L1 and PD-1, restoring T-cell activity and strengthening T-cell cytotoxicity against tumor cells. The C12 aptamer specifically targets a receptor protein highly expressed on the surface of SCLC cells; through this targeting, the drug can accurately recognize and bind tumor cells, improving treatment precision.Epirubicin, as a classical chemotherapeutic, inhibits tumor-cell growth and dissemination by blocking DNA replication and transcription.

In this integrated nucleic-acid nanocarrier, the aptamers and epirubicin act in concert to achieve combined immunotherapy and chemotherapy co-targeted to the tumor microenvironment (TME). The CPG2006-CD40-PD-L1 aptamers activate immune cells and enhance antitumor immune responses; the C12 aptamer confers specific targeting to SCLC cells; and epirubicin exerts direct cytotoxicity on tumor cells. This synergistic antitumor effect can markedly improve therapeutic effectiveness.In particular, the integrated nanocarrier functions in SCLC therapy as follows:

1. TME remodeling: The CPG2006-CD40-PD-L1 aptamers act within the TME to reverse immunosuppression,

strengthening antitumor immune responses and improving clearance of tumor cells.

2. Targeted delivery: The C12 aptamer specifically binds a receptor protein highly expressed on SCLC cells, enabling precise delivery of the drug, reducing distribution to normal tissues, and lowering adverse effects.

3. Restoration of immune surveillance: The PD-L1 aptamer blocks the PD-1/PD-L1 interaction, restoring T-cell activity and enhancing immune surveillance to prevent relapse and metastasis.

4. Formation of immune memory: The CD40 aptamer activates dendritic cells and B cells, promoting immunologic memory to tumor antigens, prolonging the benefit of immunotherapy, and helping to prevent recurrence.

5. Formation of immune memory: The CD40 aptamer activates dendritic cells and B cells, promoting immunologic memory to tumor antigens, extending the durability of immunotherapy effects, and helping to prevent recurrence.

In summary, Drug 13 exhibits a synergistic antitumor effect in the treatment of SCLC. By jointly remodeling the TME, augmenting antitumor immunity, achieving precise delivery, inducing tumor-cell apoptosis, restoring immune surveillance, and establishing immune memory, this integrated nucleic-acid nanocarrier offers a promising therapeutic approach for relapsed/refractory SCLC and related malignancies.

(11)The targeted chemical drug includes a DNA carrier, a small-molecule chemical drug, and-linked to the DNA carrier-an immunostimulant CPG2006, a C12 nucleic acid aptamer, a CD47 siRNA oligonucleotide, a PD-L1 DNA-form nucleic acid aptamer, and a PD-L1 siRNA oligonucleotide; preferably, the small-molecule chemical drug is epirubicin hydrochloride or its free base. Preferably, the DNA carrier includes the ninth set of sequences; the PD-L1 siRNA oligonucleotide is present at a 1: 1 molar ratio relative to the DNA carrier, with its sense strand serving as a transition base segment linked to the 5' terminus of the sequence c, and its antisense strand serving as a complementary sequence complementarily hybridized to the sense strand; the PD-L1 DNA-form aptamer is linked to the 5' end of the antisense strand of the PD-L1 siRNA oligonucleotide; preferably, the PD-L1 aptamer is linked to said 5' end via a base linker "TTTTT." The CPG2006 immunostimulant is present at a 1: 1 molar ratio relative to the DNA carrier and is linked to the 5' terminus of the sequence a; preferably, CPG2006 is linked to the 5' terminus of the sequence a via a base linker "TTTTT." The CD47 siRNA oligonucleotide is present at a 1: 1 molar ratio relative to the DNA carrier, its sense strand serving as a transition base segment linked to the 5' terminus of the sequence b, and its antisense strand serving as a complementary sequence complementarily hybridized to the sense strand. The C12 aptamer is present at a 1: 1 molar ratio relative to the DNA carrier and is linked to the 5' end of the antisense strand of the CD47 siRNA; preferably, the C12 aptamer is linked to said 5' end via a base linker "TTTTT." Specifically, the targeted chemical drug includes a targeted nucleic-acid carrier formed by self-assembly of the following sequences and epirubicin conjugated to the targeted nucleic-acid carrier:

Drug 14: CPG2006 (DNA) - Apt C12 - CD47 siRNA - Apt PD-L1 - PD-L1 siRNA - DNA - epirubicin

| **Name** | Sequence (sense, 5'→3') |
|---|---|
| Sequence a (SEQ ID NO: 309) | **5'**-TCGTCGTTTTGTCGTTTTGTCGTTTTTT**GCGACGCCCACGAGCGTTCCGGGAGAGGAGC-3'** |
| Sequence b (SEQ ID NO: 310) | **5'-**GGUGAUUACCCAGAGAUAUUU**GCTCCTCTCCCGGTTCGCCGCGAGCCGCG-3'** |
| Sequence c (SEQ ID NO: 311) | **5'-**CCAGCACACUGAGAAUCAAUU**CGCGGCTCGCGGCCATAGCCGTGGGCGTCGC-3'** |
| Sequence d (SEQ ID NO: 312) | **5'-**GTGGATTGTTGTGTTCTGTTGGTTTTTGTGTTGTCTTTTTAUAUCUCUGGGUAAUCACCUU**-3'** |

(continued)

| Name | Sequence (sense, 5'→3') |
|---|---|
| Sequence e (SEQ ID NO: 313) | **5'**-GCCCCAGTTATGCTTTCCCCCTCTGTCTCTTTGTTTTTUUGAUUCUCAGUGUGCUGGUU-**3'** |
| **Note:** For sequence a, the internucleotide phosphate linkages between each pair of adjacent nucleotides at positions 1-24 counted from the 5' end are phosphorothioate (PS) linkages, and the linkages between each pair of adjacent nucleotides within the 3'-terminal positions 1-3 are also PS; for sequence b, the linkages between each pair of adjacent nucleotides within the 5'-terminal positions 1-3 and within the 3'-terminal positions 1-3 are PS, and the linkages between the 19th-20th and 20th-21st nucleotides counted from the 5' end are PS; for sequence c, the linkages between each pair of adjacent nucleotides within the 5'-terminal positions 1-3 and within the 3'-terminal positions 1-3 are PS, and the linkages between the 19th-20th and 20th-21st nucleotides counted from the 5' end are PS; for sequence d, the linkages between each pair of adjacent nucleotides within the 5'-terminal positions 1-3 and within the 3'-terminal positions 1-3 are PS, and the linkages between the 19th-20th and 20th-21st nucleotides counted from the 3' end are PS; for sequence e, the linkages between each pair of adjacent nucleotides within the 5'-terminal positions 1-3 and within the 3'-terminal positions 1-3 are PS, and the linkages between the 19th-20th and 20th-21st nucleotides counted from the 3' end are PS.In the RNA sequences indicated by underlining, cytidine and uridine residues bear 2'-fluoro (2'-F) modifications. | |

In Drug 14, the nanonucleic-acid carrier is modified, by sequence extension, with CpG 2006, a C12 aptamer and a PD-L1 aptamer to provide active targeting, thereby mediating specific binding to HDLBP (high-density lipoprotein-binding protein) and PD-L1 that are overexpressed on the surface of tumor cells; the carrier further bears CD47 siRNA and PD-L1 siRNA as gene-silencing agents.In addition, approximately 30 GC/CG doxorubicin-loading sites are engineered on the three double-stranded DNA arms formed by self-assembly of the three core backbone sequences.During preparation, a 40: 1 molar excess of doxorubicin relative to the nanonucleic-acid carrier is preferably employed, and 4% paraformaldehyde is used as a crosslinking (coupling) agent. The anthracycline tetracyclic aglycone of doxorubicin specifically intercalates at GC/CG sites on the carrier, and the amino group of the daunosamine moiety undergoes a paraformaldehyde-catalyzed Schiff-base reaction with the exocyclic amine of a guanine residue at such GC/CG sites, thereby establishing a hybrid loading mode that combines physical intercalation with covalent attachment for stable loading.Besides the intrinsic passive targeting and enhanced permeability and retention (EPR) effect that drive nanoparticle accumulation in tumors, the formulation exhibits active affinity for tumor tissues and cells overexpressing HDLBP and PD-L1, such that, under the combined passive and active targeting effects, the nanoparticles accumulate at high levels at tumor sites.After the C12 aptamer binds HDLBP on tumor cells, the particles are internalized (e.g., by endocytosis) into the cytosol; under the acidic pH and high-glutathione (GSH) reducing intracellular conditions of tumor cells, doxorubicin dissociates and is released from the carrier. The released free doxorubicin enters the nucleus and intercalates at GC/CG sites of nuclear double-stranded DNA, thereby blocking DNA transcription and inhibiting tumor-cell differentiation and proliferation, to achieve an antitumor effect that suppresses tumor initiation and progression.

Drug 14 integrates multiple therapeutic modalities-including immunotherapy, gene knockdown, and chemotherapy-to jointly inhibit advanced, recurrent, and metastatic refractory SCLC. The mechanisms of the respective components are as follows:

1. CpG 2006 (CpG ODN): Activates TLR9 to modulate immune responses; enhances antitumor immunity by stimulating dendritic-cell and B-cell activation, promoting Th1-type cytokine production, and augmenting CTL and NK-cell activity.

2. C12 aptamer (anti-HDLBP): An aptamer targeting HDLBP overexpressed on SCLC cells; confers tumor-cell-specific targeting of the nanoparticles, improving therapeutic efficacy while reducing toxicity to normal tissues.

3. CD47 siRNA: A small interfering RNA that knocks down CD47 expression to counter tumor immune evasion.CD47 delivers a "don't-eat-me" signal via SIRPα to inhibit macrophage phagocytosis, lowering CD47 restores macrophage phagocytic function and thereby enhances antitumor immunity.

4. PD-L1 aptamer: Binds PD-L1 and blocks its interaction with PD-1, thereby releasing T cells from checkpoint inhibition and reactivating antitumor immune responses.

5. PD-L1 siRNA: Reduces PD-L1 gene expression, further enhancing immune-cell attack on tumor cells. In

combination with the PD-L1 aptamer, it provides more effective blockade of the PD-1/PD-L1 pathway and amplifies immune activation.

6. Nanonucleic-acid carrier: Formed by self-assembly of three DNA oligonucleotides having partially contiguous sequences that pair complementarily. The carrier tethers the foregoing therapeutic nucleic-acid moieties via sequence extension, thereby increasing their stability and targeting, and enabling synergistic combination therapy.

7. Doxorubicin: A topoisomerase II inhibitor that blocks DNA replication and transcription to suppress tumor-cell growth. Targeted delivery by the nanoparticles and slow/controlled release under low-pH and high-GSH tumor conditions increase intratumoral drug levels and reduce systemic toxicity.

In summary, Drug 14 exerts its activity by: immune activation via CpG 2006; C12-aptamer-mediated tumor targeting; CD47 siRNA and PD-L1 siRNA-mediated suppression of immune evasion; PD-L1 aptamer checkpoint blockade; and doxorubicin-mediated inhibition of tumor-cell growth-thereby integrating multiple modalities to co-inhibit advanced, recurrent, and metastatic refractory cancers such as SCLC.

(12)The targeted chemical drug includes a DNA carrier; a small-molecule chemotherapeutic; and, linked to the DNA carrier, an miRNA oligonucleotide A-miR-21, an siRNA oligonucleotide targeting TGF-β1, a nucleic-acid aptamer TfRA4, and a nucleic-acid aptamer PD-L1. Preferably, the small-molecule chemotherapeutic is epirubicin, its free base, or its hydrochloride salt. Preferably, the DNA carrier includes a second set of sequences; the molar ratio of the TGF-β1 siRNA oligonucleotide to the DNA carrier is 1: 1, the antisense strand is linked as a nucleotide linker segment to the 3' end of sequence c, and the sense strand serves as a complementary sequence that is complementarily hybridized to the antisense strand. The molar ratio of the TfRA4 aptamer to the DNA carrier is 1: 1, and the aptamer is linked to the 5' end of sequence a; preferably, TfRA4 is linked via a thymidine base linker (TTTTT). The molar ratio of the PD-L1 aptamer to the DNA carrier is 1: 1, and the aptamer is linked to the 5' end of sequence b; preferably, PD-L1 is linked via a thymidine base linker (TTTTT). The molar ratio of A-miR-21 to the DNA carrier is 2: 1, and two A-miR-21 units are sequentially (in tandem) linked to the 5' end of the sense strand of the TGF-β1 siRNA oligonucleotide. Specifically, the targeted chemical drug includes a targeted nucleic-acid carrier formed by self-assembly of the following sequences, and epirubicin loaded on the targeted nucleic-acid carrier:

Drug 15: A-miR-21 - TGF-β1 siRNA - Apt TfRA4 - Apt PD-L1 - DNA - Epirubicin

| Name | Sequence (sense, 5'-3') |
|---|---|
| Sequence a (SEQ ID NO: 314) | **5'-**GCGTGGTACCACGCTTTTT**GCGGCGCCCACGAGCGTTCCGGGAGC-3'** |
| Sequence b (SEQ ID NO: 315) | **5'-**ACGGGCCACATCAACTCATTGATAGACAATGCGTCCACTGCCCGTTTTTT**GCTCCTCTCCCGGTTCGCCGCGAGCCGCG-3'** |
| Sequence c (SEQ ID NO: 316) | **5'-CGCGGCTCGCGGCCATAGCCGTGGGCGTCGC**AA<u>UCAUAGAUGGCGUUGUUGCTT</u>**-3'** |
| Sequence d (SEQ ID NO: 317) | 5'-GATAAGCTGATAAGCTAAGCAACAACGCCAUCUAUGATT-3' |
| Note: For sequence b, the internucleotide phosphate linkages between each pair of adjacent nucleotides at 5' positions 1-3 are phosphorothioate (PS) linkages.For sequence c, the internucleotide linkages between each pair of adjacent nucleotides at 5' positions 29-31 are PS, and the linkages between each pair of adjacent nucleotides at the 3'-terminal positions 1-3 are PS.For sequence d, the internucleotide linkages between each pair of adjacent nucleotides at the 3'-terminal positions 1-3 are PS, and the linkages at positions 19-21 counted from the 3' end are PS.In the underlined RNA segments, cytidine and uridine residues bear 2'-fluoro (2'-F) modifications. | |

Drug 15 is an integrated nanonucleic-acid, aptamer-directed gene-immune combination therapeutic, configured to co-target the tumor microenvironment with an immunotherapeutic component and a classical chemotherapeutic, thereby effectively inhibiting recurrent, treatment-resistant tumor cells, including gliomas and brain-metastatic tumors.

The functions of the constituent effector molecules and their synergistic antitumor actions are as follows:

1. A-miR-21 (ASO against microRNA-21): A-miR-21 is an antisense oligonucleotide directed to miR-21, a widely expressed microRNA implicated in tumor initiation, progression, invasion, and metastasis. By hybridizing to miR-21 and inhibiting its regulation of target genes, A-miR-21 reduces tumor-cell proliferation, invasion, and metastatic potential.

2. TGF-β1 siRNA: A small interfering RNA that specifically knocks down TGF-β1. As TGF-β1 is typically overexpressed within the tumor microenvironment and promotes tumor growth, invasion, and metastasis, lowering TGF-β1 expression suppresses tumor-cell growth and dissemination.

3. Apt TfRA4 (anti-transferrin receptor aptamer): TfRA4 binds the transferrin receptor (TfR), which is highly expressed on tumor cells and at the blood-brain barrier (BBB).By engaging TfR, the aptamer actively targets the formulation to tumor cells and the BBB, increasing intratumoral drug concentration while reducing off-target toxicity.

4. Apt PD-L1 (anti-PD-L1 aptamer): A nucleic-acid aptamer that binds PD-L1 on tumor cells and blocks its interaction with PD-1 on immune cells, thereby reversing immune suppression, restoring immune-cell activity, and enhancing antitumor immunity.

5. Nanonucleic-acid carrier scaffold. The DNA nanoscaffold integrates A-miR-21, TGF-β1 siRNA, the TfRA4 aptamer, and the PD-L1 aptamer into a unified targeted construct (e.g., via sequence-extension linkages), stabilizes the overall assembly, and improves biostability and duration of action.

6. Epirubicin.A widely used antineoplastic that inhibits DNA synthesis and cell division. When covalently loaded onto the nanonucleic-acid scaffold, epirubicin enables combination therapy with the immuno-/gene-modulatory components, thereby improving antitumor efficacy.

Specifically, Drug 15 achieves high therapeutic efficacy through the following synergistic antitumor effects:

1. Combined targeting: A-miR-21, TGF-β1 siRNA, the TfRA4 aptamer, and the PD-L1 aptamer respectively target miR-21, TGF-β1, TfR, and PD-L1 in tumor cells, thereby providing multi-pathway tumor suppression.Such multiplexed targeting improves efficacy while reducing adverse effects.

2. Combination therapy: Epirubicin, as a classical chemotherapeutic, is used in combination with gene-modulatory agents (A-miR-21, TGF-β1 siRNA) and the PD-L1 aptamer immunotherapeutic, delivering a comprehensive treatment that enhances antitumor activity.

3. Immunotherapy synergy: By releasing immune suppression in the tumor microenvironment, the PD-L1 aptamer reactivates immune cells and acts synergistically with epirubicin and other components to produce enhanced antitumor effects.

4. Knockdown of key signaling pathways: A-miR-21 and TGF-β1 siRNA knock down critical oncogenic signaling axes in tumor cells, inhibiting growth, invasion, and metastasis, thereby potentiating the overall antitumor efficacy.

Accordingly, Drug 15 employs multiple targeting strategies and combination modalities to act synergistically in tumor suppression, as evidenced by the following:

5. Improved tissue penetration / BBB transit: By binding TfR, the TfRA4 aptamer enables the nanosystem to more readily traverse the blood-brain barrier (BBB) and increase drug concentrations within tumor tissue-of particular relevance for gliomas and brain metastases.

6. Reduced toxicity and adverse effects: The multi-pathway targeting and combination-therapy approach permits dose reduction, thereby decreasing toxicity to normal cells and tissues. Integration on the nanonucleic-acid scaffold enhances biostability and duration of action, further mitigating side effects.

7. Overcoming drug resistance: The combination strategy mitigates resistance that may arise with monotherapy by concurrently engaging multiple tumor pathways and mechanisms, thereby lowering tumor tolerance and improving therapeutic response.

8. Prolonged survival and improved quality of life: Through multi-modal synergistic inhibition of tumor growth, invasion, and metastasis, Drug 15 can extend patient survival; reduced toxicity and adverse effects are expected to improve quality of life.

In sum, Drug 15 is an integrated, aptamer-directed nanonucleic-acid gene-immunotherapy configured for co-targeting of the tumor microenvironment together with a classical chemotherapeutic, and is of significant value for the effective suppression of recurrent, refractory tumors, including gliomas and brain-metastatic lesions.

(13)The targeted chemical drug includes a DNA carrier; a small-molecule chemotherapeutic; and, linked to the DNA carrier, a nucleic-acid aptamer IL-4Rα, an siRNA oligonucleotide targeting CD47, a nucleic-acid aptamer TfRA4 in DNA form, an siRNA oligonucleotide targeting PD-L1, and a nucleic-acid aptamer GPC-1. Preferably, the small-molecule chemotherapeutic is epirubicin hydrochloride or its free base. The DNA carrier includes a third set of sequences. The molar ratio of the TfRA4 aptamer to the DNA carrier is 1: 1, and the aptamer is linked to the 5' end of sequence a; preferably, TfRA4 is linked via a thymidine base linker (TTTTT). The molar ratio of the GPC-1 aptamer to the DNA carrier is 1: 1, and the aptamer is linked to the 5' end of sequence c. The molar ratio of the PD-L1 siRNA oligonucleotide to the DNA carrier is 1: 1; its sense strand serves as a bridging nucleotide segment linked to the 5' end of sequence b, and its antisense strand serves as the complementary sequence hybridized to the sense strand and is linked to the 3' end of sequence a. The molar ratio of the CD47 siRNA oligonucleotide to the DNA carrier is 1: 1; its sense strand serves as a bridging nucleotide segment linked to the 3' end of sequence c, and its antisense strand serves as the complementary sequence hybridized to the sense strand. The IL-4Rα aptamer is present at a 1: 1 molar ratio relative to the DNA carrier and is linked to the 5' end of the antisense strand of the CD47 siRNA; preferably, the IL-4Rα aptamer is linked via an adenosine base linker (AAAA) to the 5' end of the CD47 siRNA antisense strand.Specifically, the targeted chemical drug includes a targeted nucleic-acid carrier formed by self-assembly of the following sequences, together with epirubicin loaded on the targeted nucleic-acid carrier:

Drug 16: Apt IL-4Rα-CD47 siRNA-Apt TfRA4-PD-L1 siRNA-Apt GPC-1-DNA-Epirubicin

| **Name** | Sequence (sense, 5'→3') |
|---|---|
| Sequence a (SEQ ID NO: 318) | **5'-**GCGTGGTACCACGCTTTTT**GCGGCGCCCACGAGCGTTCCGGGAGAGGAGC**UUUGAUUCUCAGUGUGCUGGUU**-3'** |
| Sequence b (SEQ ID NO: 319) | **5'-**CCAGCACACUGAGAAUCAAUU**GCTCCTCTCCCGGTTCGCCGCCAGCCGCGG-3'** |
| Sequence c (SEQ ID NO: 320) | **5'-AACGGAGTGTGGCTAACTCGATTTTTGGCGGCTGGCGGCCATAGCCGTGGGCGCCGC**UGGUGAUUACCCAGAGAUAUUU**-3'** |
| sequence d (SEQ ID NO: 321) | **5'-**AAAAAGCAACAGGGUGCUCCAUGCGCAUGGAACCUGCGCGAAAAUAUCUCUGGGUAAUCACCUU**-3'** |
| Note: For sequence a, the internucleotide phosphate linkages between each pair of adjacent nucleotides at the 3'-terminal positions 1-3 are phosphorothioate (PS) linkages, and the linkages at positions 19-21 counted from the 3' end are also PS; for sequence b, the internucleotide linkages between each pair of adjacent nucleotides at the 5'-terminal positions 1-3 are PS, and those at the 5'-terminal positions 19-21 are PS; for sequence c, the internucleotide linkages between each pair of adjacent nucleotides at the 3'-terminal positions 1-3 are PS, and those at positions 19-21 counted from the 3' end are PS; for Sequence D, the internucleotide linkages between each pair of adjacent nucleotides at the 5'-terminal positions 1-4, at the 3'-terminal positions 1-3, and at positions 19-21 counted from the 3' end are PS.In sequences a-c, within the underlined RNA segments, cytidine (C) and uridine (U) residues bear 2'-fluoro (2'-F) modifications; in sequence d, nucleotides 1-44 are sugar-modified such that C/U are 2'-F and A/G are 2'-O-methyl (2'-OMe), and nucleotides 45-64 bear 2'-F substitutions on C/U. | |

Drug 16 is an integrated nanonucleic-acid, aptamer-directed, actively targeted gene-immunotherapy in combination with chemotherapy, which combines immunotherapy, targeted therapy, gene therapy and a classical chemotherapeutic to achieve co-targeting of the tumor microenvironment (TME). The integrated regimen exhibits high efficacy for suppressing recurrent and refractory tumors, including gliomas and brain metastases. The functions of the constituent

effector moieties and their contributions to synergistic antitumor activity are as follows:

1. IL-4Rα (aptamer): IL-4Rα is the α-subunit of the interleukin-4 receptor and serves as an immunotherapeutic target. The IL-4Rα aptamer is configured to modulate polarization of tumor-associated macrophages (TAMs), thereby remodeling the TME and inhibiting tumor growth and metastasis.

2. CD47 siRNA: CD47 functions as an immune-checkpoint "don't-eat-me" signal that impedes macrophage phagocytosis of tumor cells.CD47-targeting siRNA specifically knocks down CD47 expression, abolishing the "don't-eat-me" effect and enhancing macrophage-mediated phagocytosis of tumor cells.

3. TfRA4 aptamer: The TfRA4 aptamer specifically binds the transferrin receptor (TfR), which is enriched on tumor cells and at the blood-brain barrier (BBB), thereby facilitating receptor-mediated transcytosis across the BBB and increasing drug accumulation in brain tumor tissue.

4. PD-L1 siRNA: PD-L1 engages PD-1 on Tcells to suppress antitumor immunity.PD-L1-targeting siRNA silences PD-L1 expression, thereby relieving T-cell inhibition and improving immune-mediated tumor clearance.

5. GPC-1 aptamer: The glypican-1 (GPC-1) aptamer specifically binds the GPC-1 receptor overexpressed on glioma and brain-metastatic tumor cells and functionally inhibits its activity, providing targeted suppression of tumor growth and dissemination.

6. Nanonucleic-acid carrier: The DNA nanoscaffold co-assembles the foregoing aptamers and siRNAs into a single integrated nanodrug, thereby enhancing targeting performance and bioactivity and improving structural stability.

7. Epirubicin: A classical antineoplastic that inhibits DNA replication and cell division. Co-delivery on the nanocarrier together with the immune- and gene-modulatory components affords combination therapy, resulting in enhanced antitumor efficacy.

Through the coordinated action of the foregoing effector moieties, the drug-formulated as an integrated nanonucleic-acid, aptamer-directed, actively targeted gene-immunotherapeutic-exhibits marked advantages in suppressing recurrent and treatment-refractory tumor cells, including gliomas and brain-metastatic tumors. This combination strategy enables synergy among multiple therapeutic modalities by modulating the tumor microenvironment, enhancing immune-mediated tumor clearance, facilitating drug transit across the blood-brain barrier (BBB), and exerting tumor-cell-specific inhibitory activity, thereby collectively achieving potent inhibition of recurrent and refractory tumor cells, including gliomas and brain metastases.

(14)The targeted chemical drug includes a DNA carrier; a small-molecule chemotherapeutic; and, linked to the DNA carrier, nucleic-acid aptamers AS1411 (in DNA form), EGFR (in DNA form), and A15 (in DNA form). Preferably, the small-molecule chemotherapeutic is gemcitabine. Preferably, the DNA carrier includes a fourth set of sequences; the molar ratio of the AS1411 aptamer (DNA form) to the DNA carrier is 1: 1, with AS1411 linked to the 5' end of sequence a; the molar ratio of the EGFR aptamer (DNA form) to the DNA carrier is 1: 1, with the EGFR aptamer linked to the 5' end of sequence b; and the molar ratio of the A15 aptamer (DNA form) to the DNA carrier is 1: 1, with the A15 aptamer linked to the 5' end of sequence c. Preferably, gemcitabine is linked in the form of insertion of at least one nucleotide at one or more of the following positions: between the AS1411 (DNA form) aptamer and sequence a; at the 3' end of sequence a; between the EGFR (DNA form) aptamer and sequence b; at the 3' end of sequence b; between the A15 (DNA form) aptamer and sequence c; and at the 3' end of sequence c.Specifically, the targeted chemical drug is a gemcitabine-targeted chemotherapeutic formed by self-assembly of the following sequences:

Drug 17: Apt AS1411 - Apt EGFR - Apt A15 - DNA + Gemcitabine

| **Name** | Sequence (sense, 5'→3') |
|---|---|
| Sequence a | **5'-** SEQ ID NO: 103 - JJJJJJ - **SEQ ID NO: 15** - JJJJJJ-**3'** |
| Sequence b | **5'-** SEQ ID NO: 139 - JJJJJJ - **SEQ ID NO: 16-** JJJJJJ-**3'** |

(continued)

| **Name** | Sequence (sense, 5'→3') |
|---|---|
| Sequence c | **5'-** SEQ ID NO: 102- JJJJJJ - **SEQ ID NO: 14-** JJJJJJ-**3'** |
| Note: In the foregoing sequences, J denotes gemcitabine (2', 2'-difluorodeoxycytidine, dFdC), which is directly incorporated into the oligonucleotide during synthesis as a nucleobase-replacement and chain-extension unit; in aggregate, a single DNA targeting carrier bears thirty-six gemcitabine residues.<br>SEQ ID NO: 103: **5'**-GGTGGTGGTGGTTGTGGTGGTGGTGG-**3';**<br>SEQ ID NO: 15: **5'**-GCGGCGCCCACGAGCGTTCCGGGAGAGGCC-**3'**;<br>SEQ ID NO: 139: **5'**-GCCTTAGTAACGTGCTTTGATGTCGATTCGACAGGAGGC-**3'**;<br>SEQ ID NO: 16: **5'**-GGCCTCTCCCGGTTCGCCGCCAGCCGCC-**3'**;<br>SEQ ID NO: 102: **5'**-CCCTCCTACATAGGG-3';<br>SEQ ID NO: 14: **5'**-GGCGGCTGGCGGCCATAGCCGTGGGCGCCGC-**3'**. | |

In Drug 17, a nanonucleic-acid carrier integrating multiple DNA-form aptamers affords highly specific targeting of tumor cells.Gemcitabine may be directly incorporated in-line during solid-phase oligonucleotide synthesis of the carrier/-aptamer sequences (via an oligonucleotide synthesizer), i.e., introduced as nucleotide units during chain assembly. The functions of the constituent aptamers and their roles in the synergistic antitumor effect are as follows:

1. AS1411 (nucleolin aptamer).Specifically binds cell-surface nucleolin on tumor cells, thereby conferring tumor-cell targeting.

2. EGFR aptamer.Specifically binds the epidermal growth factor receptor (EGFR), which is overexpressed in numerous tumor types, thereby enhancing targeting of the nanonucleic-acid carrier to tumor cells.

3. A15 aptamer.Specifically binds the CD133 (prominin-1) stem-cell marker, enabling preferential targeting of tumor stem cells and helping to mitigate recurrence and drug resistance.

4. Nanonucleic-acid carrier (scaffold).Serves as the structural backbone that co-presents the aptamers together with the drug payload, thereby improving biostability, bioactivity, and targeting performance.

5. Gemcitabine.A widely used antineoplastic that inhibits DNA synthesis and cell division; when combined on the nanocarrier with the foregoing aptamers, it enables highly specific delivery to tumor cells.

In summary, Drug 17 employs multi-aptamer targeting of nucleolin, EGFR, and CD133, yielding potent tumor-cell inhibition-particularly against tumor stem cells. Direct incorporation of gemcitabine into the oligonucleotide framework further augments antitumor activity, indicating promising clinical applicability of this nanonucleic-acid targeted chemotherapeutic.

(15)The targeted chemical drug includes an RNA carrier; a small-molecule chemotherapeutic; and, linked to the RNA carrier, nucleic-acid aptamers AS1411 (DNA form), EGFR (DNA form), and A15 (DNA form). Preferably, the small-molecule chemotherapeutic is 5-fluorouracil (5-FU). Preferably, the RNA carrier includes a fifth set of sequences; the molar ratio of the AS1411 aptamer (DNA form) to the RNA carrier is 1: 1, with AS1411 linked to the 5' end of sequence a; the molar ratio of the EGFR aptamer (DNA form) to the RNA carrier is 1: 1, with the EGFR aptamer linked to the 5' end of sequence b; and the molar ratio of the A15 aptamer (DNA form) to the RNA carrier is 1: 1, with the A15 aptamer linked to the 5' end of sequence c. Preferably, 5-fluorouracil is incorporated as at least one nucleobase-insertion unit at one or more of the following positions: between the AS1411 (DNA form) aptamer and sequence a; at the 3' end of sequence a; between the EGFR (DNA form) aptamer and sequence b; at the 3' end of sequence b; between the A15 (DNA form) aptamer and sequence c; and at the 3' end of sequence c.Specifically, the targeted chemical drug is a 5-fluorouracil-targeted chemotherapeutic formed by self-assembly of the following sequences:

Drug 18: Apt AS1411 - Apt EGFR - Apt A15 - RNA - 5-Fluorouracil

| Name | Sequence (sense, 5'→3') |
|---|---|
| Sequence a (SEQ ID NO: 322) | **5'-**GGTGGTGGTGGTTGTGGTGGTGGTGGNNNNNN**GCGGCGCCCACGAGCGUUCCGGGAGAGGCC**NNNNNN**-3'** |
| Sequence b (SEQ ID NO: 323) | **5'-**GCCUUAGUAACGUGCUUUGAUGUCGAUUCGACAGGAGGCNNNNNN**GGCCUCUCCCGGUUCGCCGCCAGCCGCC**NNNNNN**-3'** |
| Sequence c (SEQ ID NO: 324) | **5'-**CCCUCCUACAUAGGGNNNNNN**GGCGGCUGGCGGCCAUAGCCGUGGGCGCCGC**NNNNNN**-3'** |
| Note: In the foregoing sequences, N denotes 5-fluorouracil (5-FU), which is directly incorporated during oligonucleotide synthesis as a nucleobase-replacement and chain-extension unit; the internucleotide phosphate linkages joining each pair of adjacent N (5-FU) residues are phosphorothioate (PS) linkages; in aggregate, a single DNA targeting carrier bears thirty-six 5-FU residues.For sequence a, the internucleotide linkages between each pair of adjacent nucleotides at the 5'-terminal positions 1-2 are PS; for sequence b, the internucleotide linkages between each pair of adjacent nucleotides at the 5'-terminal positions 1-2 and at the 3'-terminal positions 1-2 are PS; for sequence c, the internucleotide linkages between each pair of adjacent nucleotides at the 5'-terminal positions 1-2 are PS.For RNA segments, cytidine (C) and uridine (U) residues are 2'-fluoro (2'-F)-modified, and adenosine (A) and guanosine (G) residues are 2'-O-methyl (2'-OMe)-modified; moreover, the internucleotide linkages between each pair of adjacent nucleotides at the 5'- and 3'-terminal positions 1-2 of the RNA are PS.In the underlined RNA sequences, C/U bear 2'-F and A/G bear 2'-OMe modifications. | |

In Drug 18, a nanonucleic-acid carrier integrating multiple DNA-form aptamers affords highly specific targeting of tumor cells.5-fluorouridine (FUr)-the nucleoside form of 5-fluorouracil-can be directly incorporated, in a single pass, during solid-phase oligonucleotide synthesis of the carrier/aptamer sequences. The functions of the respective components are as follows:

1. 5-Fluorouridine (FUr).A widely used antineoplastic that inhibits DNA and RNA synthesis.As the nucleoside analogue of 5-FU, FUr more efficiently enters cells and releases the active 5-FU intracellularly.

2. AS1411 (nucleolin aptamer).Specifically binds cell-surface nucleolin on tumor cells to confer tumor-cell targeting.

3. EGFR aptamer. Specifically binds the epidermal growth factor receptor (EGFR), which is overexpressed in many tumor types, thereby enhancing targeting by the nanonucleic-acid carrier.

4. A15 aptamer. Specifically binds the CD133 (prominin-1) stem-cell marker, enabling preferential targeting of tumor stem cells and helping to counter recurrence and drug resistance.

5. Nanonucleic-acid carrier (scaffold).Serves as the structural backbone that integrates the aptamers with the drug moiety, thereby improving biostability, bioactivity, and targeting performance.

In summary, Drug 18 employs multi-aptamer targeting (nucleolin, EGFR, and CD133) to achieve potent, tumor-cell-specific inhibition-particularly against tumor stem cells. In-line incorporation of 5-fluorouridine further enhances antitumor activity, indicating promising clinical applicability of this nanonucleic-acid targeted chemotherapeutic.

(16)The targeted chemical drug includes a DNA carrier; a small-molecule chemotherapeutic; and, linked to the DNA carrier, nucleic-acid aptamers AS1411 (DNA form), EGFR (DNA form), and A15 (DNA form). Preferably, the small-molecule chemotherapeutic is paclitaxel. Preferably, the DNA carrier includes a sixth set of sequences. The molar ratio of the AS1411 aptamer (DNA form) to the DNA carrier is 1: 1, and AS1411 is linked to the 5' end of sequence a; preferably, AS1411 is linked via a thymidine base linker (TTTTT). The molar ratio of the EGFR aptamer (DNA form) to the DNA carrier is 1: 1, and the EGFR aptamer is linked to the 5' end of sequence b; preferably, via a thymidine base linker (TTTTT). The molar ratio of the A15 aptamer (DNA form) to the DNA carrier is 1: 1, and the A15 aptamer is linked to the 5' end of sequence c; preferably, via a thymidine base linker (TTTTT).Specifically, the targeted chemical drug includes a targeted nucleic-acid carrier formed by self-assembly of the following sequences, together with paclitaxel loaded on the targeted nucleic-acid carrier:

Drug 19: Apt AS1411 - Apt EGFR - Apt A15 - DNA - Paclitaxel

| Name | Sequence (sense, 5'→3') |
|---|---|
| Sequence a (SEQ ID NO: 325) | **5'-**GGTGGTGGTGGTTGTGGTGGTGGTGGTTTTT**GCGGCGCCCACGAGCGTTCCGGGAGAGGAGGCC-3'** |
| Sequence b (SEQ ID NO: 326) | **5'-**GCCTTAGTAACGTGCTTTGATGTCGATTCGACAGGAGGCTTTTT**GGCCTCCTCTCCCGGTTCGCCGCCAGCCGCC-3'** |
| Sequence c (SEQ ID NO: 327) | **5'-**CCCTCCTACATAGGGTTTTT**GGCGGCTGGCGGCCATAGCCGTGGGCGCCGC-3'** |
| Note: For sequences a, b, and c, the internucleotide phosphate linkages between the nucleotides at the 5'-terminal positions 1-2 and at the 3'-terminal positions 1-2 are phosphorothioate (PS) linkages. In sequence a, the nucleotides counted from the 5' end at positions 35, 39, 54, and 59 bear a primary amine ($-NH_2$) modification; in sequence b, the nucleotides at positions 50, 55, 66, and 70 (from the 5' end) bear a primary amine ($-NH_2$) modification; and in sequence c, the nucleotides at positions 24 and 28 (from the 5' end) bear a primary amine ($-NH_2$) modification. | |

In Drug 19, during solid-phase synthesis of the three DNA oligonucleotide/aptamer sequences that constitute the nanonucleic-acid carrier, multiple amino-terminated linkers ($-NH_2$) are co-synthesized at sequence-defined positions. These pendant amino linkers are then covalently coupled, in the presence of a coupling reagent, to a paclitaxel derivative bearing a disulfide-containing linker installed at the 2'-hydroxyl (2'-OH) of paclitaxel. The resulting nanoparticle carries about 8-12 paclitaxel molecules per construct and, under composite tri-aptamer targeting (AS1411/EGFR/A15), efficiently accumulates within the tumor microenvironment, enters target cells, and releases paclitaxel. This targeted paclitaxel nanoconstruct can effectively inhibit a variety of paclitaxel-sensitive tumors and offers advantages such as high targeting specificity, controlled release, and reduced toxicity, thereby improving therapeutic efficacy and diminishing side effects. Specifically, the drug has the following properties:

1. High targeting specificity. Through the combined use of the AS1411, EGFR, and A15 aptamers, the nanoparticles display high target selectivity: the AS1411 aptamer primarily targets cell-surface nucleolin on tumor cells; the EGFR aptamer targets the epidermal growth factor receptor on tumor cells; and the A15 aptamer recognizes and binds stem-like tumor-cell markers. The synergy of these three aptamers enables efficient accumulation in the tumor microenvironment (TME) and entry into target cells.

2. Controlled release. Paclitaxel is covalently attached via a disulfide-containing linker. After nanoparticle internalization, the reducing intracellular milieu (e.g., elevated glutathione) cleaves the disulfide bond, thereby releasing paclitaxel. This controlled-release mechanism reduces nonspecific deposition of paclitaxel in normal tissues and lowers toxicity.

3. Low toxicity.Owing to high target selectivity and controlled release, nonspecific accumulation of paclitaxel in normal tissues is diminished, thereby reducing off-target toxicity and improving the therapeutic index.

4. Antitumor activity. Upon release, paclitaxel disrupts microtubule polymerization-depolymerization dynamics, causing mitotic arrest and apoptosis of tumor cells. Paclitaxel further inhibits tumor-cell migration, invasion, and angiogenesis, thereby suppressing tumor progression and dissemination.

5. Addressing drug resistance. Multi-target engagement via the three aptamers reduces the likelihood of paclitaxel resistance. In addition, high intratumoral concentrations achieved by targeted delivery and controlled release enhance cytotoxicity against drug-resistant tumor cells.

6. Combination therapy. The AS1411-EGFR-A15-DNA-paclitaxel nanocarrier can be used in combination with other anticancer agents, radiotherapy, or immunotherapy to produce synergistic antitumor effects-for example, by remodeling the TME to enhance immune-cell activity or by acting as a radiosensitizer to increase tumor sensitivity to radiation.

7. Personalized treatment. The aptamer composition can be adjusted based on a patient's tumor-surface target expression profile, enabling customized nanonucleic-acid carrier designs for individualized therapy.

In sum, Drug 19 leverages high target specificity, controlled release, and reduced toxicity to deliver strong antitumor efficacy. Through combination use, resistance management, and personalization, the nanonucleic-acid carrier holds promise as an effective treatment for multiple tumor types.

(17)The targeted chemical drug includes a DNA carrier; a small-molecule chemotherapeutic; and, linked to the DNA carrier, nucleic-acid aptamers AS1411 (DNA form), EGFR (DNA form), and A15 (DNA form). Preferably, the small-molecule chemotherapeutic is maytansine. Preferably, the DNA carrier includes a seventh set of sequences; the molar ratio of the AS1411 aptamer (DNA form) to the DNA carrier is 1: 1, and AS1411 is linked to the 5' end of sequence a; preferably, AS1411 is linked via a thymidine base linker (TTTTT). The molar ratio of the EGFR aptamer (DNA form) to the DNA carrier is 1: 1, and the EGFR aptamer is linked to the 5' end of sequence b; preferably, via a thymidine base linker (TTTTT). The molar ratio of the A15 aptamer (DNA form) to the DNA carrier is 1: 1, and the A15 aptamer is linked to the 5' end of sequence c; preferably, via a thymidine base linker (TTTTT).Specifically, the targeted chemical drug includes a targeted nucleic-acid carrier formed by self-assembly of the following sequences, together with maytansine loaded on the targeted nucleic-acid carrier:

Drug 20: Apt AS1411 - Apt EGFR - Apt A15 - DNA + Maytansine

| Name | Sequence (sense, 5'→3') |
|---|---|
| Sequence a (SEQ ID NO: 328) | **5'-**GGTGGTGGTGGTTGTGGTGGTGGTGGTTTTT**GCGGCGCCCACGAGCGTTCCGGGAGAGGAGC-3'** |
| Sequence b (SEQ ID NO: 178) | **5'-**GCCTTAGTAACGTGCTTTGATGTCGATTCGACAGGAGGCTTTTT**GCTCCTCTCCCGGTTCGCCGCCAGCCGCC-3'** |
| Sequence c (SEQ ID NO: 68) | **5'-**CCCTCCTACATAGGGTTTTT**GGCGGCTGGCGGCCATAGCCGTGGGCGCCGC-3'** |
| Note: For sequences a, b, and c, the internucleotide phosphate linkages between each pair of adjacent nucleotides at the 5'-terminal positions 1-2 and at the 3'-terminal positions 1-2 are phosphorothioate (PS) linkages.In sequence a, the nucleotides at positions 43 and 61 (counted from the 5' end) bear a primary amine ($-NH_2$) modification; in sequence b, the nucleotides at positions 48 and 63 (from the 5' end) bear a primary amine ($-NH_2$) modification; and in sequence c, the nucleotides at positions 24 and 43 (from the 5' end) bear a primary amine ($-NH_2$) modification. | |

In Drug 20, during solid-phase synthesis of the three DNA oligonucleotide/aptamer base sequences that constitute the nanonucleic-acid carrier, multiple amino-terminated linkers ($-NH_2$) are co-synthesized at sequence-designated sites. These pendant amino linkers are then covalently conjugated, in the presence of a coupling reagent, to a maytansine derivative bearing a linker segment installed at the 2'-hydroxyl (2'-OH) of maytansine. The resulting nanoparticles carry six (6) maytansine molecules per construct and, under tri-aptamer composite targeting, efficiently accumulate within the tumor microenvironment, enter target cells, and release the highly cytotoxic maytansine payload. This targeted maytansine nanoconstruct can effectively inhibit a variety of maytansine-sensitive tumors.

The foregoing drug, via tri-aptamer co-targeting, likewise accumulates efficiently in the tumor microenvironment, enters target cells, and releases highly cytotoxic maytansine, thereby effectively suppressing multiple maytansine-responsive cancers.A detailed analysis of the nanoparticle's mechanism of action is provided below:

1. Targeting specificity. The AS1411-EGFR-A15-DNA-maytansine nanonucleic-acid carrier achieves highly specific tumor targeting via three aptamers (AS1411, EGFR, and A15). The AS1411 aptamer binds cell-surface nucleolin, the EGFR aptamer binds the epidermal growth factor receptor, and the A15 aptamer binds the CD133 receptor protein. Through this tri-aptamer combination, the nanocarrier accurately recognizes and targets tumor cells.

2. Drug loading.In the nanocarrier design, amino linkers are used to covalently conjugate maytansine to the DNAh oligonucleotide/aptamer base sequences.Each nanoparticle carries six (6) maytansine molecules, thereby increasing payload capacity.

3. Drug release. After successful cellular uptake, maytansine is released from the linker under appropriate intracellular conditions, exerting cytotoxic effects on tumor cells. Maytansine is described herein as a DNA-synthesis inhibitor, which blocks tumor-cell DNA synthesis and thereby induces apoptosis.

4. Antitumor activity. By virtue of its targeting, payload, and controlled-release characteristics, the maytansine nanocarrier efficiently suppresses tumor cells.In addition, maytansine can further inhibit tumor-cell migration, invasion, and angiogenesis, thereby limiting tumor progression and dissemination.

5. Addressing drug resistance. Multi-target engagement via the three aptamers reduces the likelihood that tumor cells develop resistance to maytansine. High tumor-site concentrations achieved by targeted delivery and controlled release also limit toxicity to normal cells, improving overall therapeutic outcome and patient tolerability.

6. Biodistribution and pharmacokinetics. The nanocarrier design favors tumor-directed distribution in vivo. The nanoparticles exhibit enhanced stability in circulation, helping to avoid rapid clearance by non-target organs such as the liver and kidneys. The carrier further enables high intratumoral accumulation via the enhanced permeability and retention (EPR) effect.

7.Safety and biocompatibility. The materials and synthesis process provide good biocompatibility, reducing the probability of adverse reactions during treatment. High target selectivity and controlled release decrease off-target toxicity, enhancing safety.

Accordingly, the present drug-via tri-aptamer composite targeting-efficiently accumulates in the tumor microenvironment, enters target cells, and releases highly cytotoxic maytansine, thereby effectively suppressing multiple maytansine-sensitive tumors by leveraging its targeting specificity, loading capacity, controlled release, antitumor efficacy, resistance management, favorable biodistribution/pharmacokinetics, and safety/biocompatibility.

(18)The targeted chemical drug includes a DNA carrier; a small-molecule chemotherapeutic; and, linked to the DNA carrier, nucleic-acid aptamers MUC1, AS1411, and GPC-1. Preferably, the small-molecule chemotherapeutic is gemcitabine. Preferably, the DNA carrier includes a ninth set of sequences; the molar ratio of the MUC1 aptamer to the DNA carrier is 1: 1, and the MUC1 aptamer is linked to the 5' end of sequence a; the molar ratio of the AS1411 aptamer to the DNA carrier is 1: 1, and the AS1411 aptamer is linked to the 5' end of sequence b; the molar ratio of the GPC-1 aptamer to the DNA carrier is 1: 1, and the GPC-1 aptamer is linked to the 5' end of sequence c.Specifically, the targeted chemical drug includes a targeted nucleic-acid carrier formed by self-assembly of the following sequences, together with epirubicin loaded on the targeted nucleic-acid carrier:

Drug 21: Apt MUC1 - Apt AS1411 - Apt GPC-1 + Gemcitabine - DNA - Epirubicin

| **Name** | Sequence (sense, 5'→3') |
|---|---|
| Sequence a | **5'-** SEQ ID NO: 160 - JJJJJJ - **SEQ ID NO: 26** - JJJJJJ-**3'** |
| Sequence b | **5'-** SEQ ID NO: 103 - JJJJJJ - **SEQ ID NO: 11** - JJJJJJ-**3'** |

(continued)

| Name | Sequence (sense, 5'→3') |
|---|---|
| Sequence c | **5'-** SEQ ID NO: 145- JJJJJJ - **SEQ ID NO: 27** - JJJJJJ-**3'** |
| Note: In the foregoing sequences, J denotes gemcitabine (2', 2'-difluorodeoxycytidine, dFdC), which is directly incorporated during oligonucleotide synthesis as a nucleobase-replacement and chain-extension unit; the internucleotide phosphate linkages between each pair of adjacent J (gemcitabine) residues are phosphorothioate (PS) linkages.In aggregate, a single DNA targeting carrier bears thirty-six gemcitabine residues.For sequences a, b, and c, the internucleotide phosphate linkages between the nucleotides at the 5'-terminal positions 1-2 are phosphorothioate (PS).<br>SEQ ID NO: 160: **5**'-GCAGTTGATCCTTTGGATACCCTGG-**3**';<br>SEQ ID NO: 26: **5**'-GCGACGCCCACGAGCGTTCCGGGAGAGGAGC-**3'**;<br>SEQ ID NO: 103: **5**'-GGTGGTGGTGGTTGTGGTGGTGGTGG-**3**';<br>SEQ ID NO: 11: **5**'-GCTCCTCTCCCGGTTCGCCGCGAGCCGCG-**3**';<br>SEQ ID NO: 145: **5**'-AACGGAGTGTGGCTAACTCGA-**3**';<br>SEQ ID NO: 27: **5**'-CGCGGCTCGCGGCCATAGCCGTGGGCGTCGC-**3**'. | |

In Drug 21, the nanonucleic-acid carrier is constructed by sequence extension on three core DNA carrier backbone oligonucleotides, in which the three aptamer sequences and contiguously arranged gemcitabine residues are co-synthesized in one pass on an oligonucleotide synthesizer onto three single strands having partially complementary regions, followed by base-pairing self-assembly to yield a MUC1/AS1411/GPC-1-gemcitabine-DNAh scaffold; epirubicin is then installed at thirty (30) pre-designated GC/CG loading sites on the nanocarrier by a hybrid mode of physical intercalation and amine crosslinking, thereby affording a MUC1/AS1411/GPC-1-gemcitabine-DNAh-epirubicin targeted nanoparticulate drug. The composite (tri-aptamer) targeting ensures specific binding to-and accumulation within-tumor microenvironments and tumor cells that overexpress MUC1, nucleolin, and GPC-1. In the acidic, glutathione-rich intracellular milieu of tumor cells, epirubicin is reductively cleaved/dissociated and released; the released epirubicin together with enzymatically liberated gemcitabine enters the nucleus and, by selectively engaging GC/CG sites of chromosomal double-stranded DNA, blocks DNA transcription (epirubicin) and alternatively blocks DNA synthesis (gemcitabine), thereby preventing tumor-cell division and expansion and suppressing tumor initiation and progression. The MUC1/AS1411/GPC-1-gemcitabine-DNAh3.3-epirubicin integrated tri-aptamer nanonucleic-acid chemotherapeutic thus exhibits a distinct mechanism of action and advantages; the following analysis sets forth its mechanistic basis, cooperative multi-targeted delivery, epirubicin active-drug contribution, gemcitabine sustained-release effect, and comparative benefits.

1. **Mechanism of action.** The nanoparticulate drug incorporates three aptamers-MUC1, AS1411, and GPC-1-that respectively target tumor-associated biomarkers.MUC1 is a membrane-associated mucin overexpressed in multiple cancers; AS1411 is an oligonucleotide aptamer that binds the nucleolin receptor; and GPC-1 is a cell-surface glycoprotein associated with the growth and invasiveness of pancreatic and other cancers.Acting in concert, the three aptamers promote accumulation in target cells, thereby enhancing therapeutic efficacy.

2. **Cooperative multi-targeted delivery**. Synergy among the three aptamers confers high specificity and selectivity, enabling the nanoparticles to bind and accumulate within the tumor microenvironment (TME) and tumor cells, which markedly increases intratumoral drug concentration, improves efficacy, and reduces injury to normal tissues.

3. **Epirubicin active drug / gemcitabine sustained-release effect.** Under the acidic, glutathione-rich intracellular conditions of tumor cells, epirubicin is released and enters the nucleus, where it selectively intercalates at GC/CG sites of chromosomal double-stranded DNA to block DNA transcription, thereby inhibiting tumor-cell division and expansion. Gemcitabine, upon enzymatic cleavage-mediated liberation, alternatively blocks DNA synthesis. This sustained-release behavior supports prolonged drug action within tumor tissue and lowers systemic adverse effects.

4. **Comparative advantages.**(1) High specificity: tri-aptamer synergy affords high specificity and reduces damage to normal tissue.(2) Enhanced efficacy: cooperative targeting raises intratumoral drug levels; combined action of epirubicin and gemcitabine-via transcriptional blockade and antimetabolite-mediated synthesis blockade, respectively-suppresses tumor-cell division and proliferation, augmenting antitumor activity.(3) Reduced side effects: sustained release of epirubicin and gemcitabine within the TME diminishes systemic toxicity.(4) Lower propensity for resistance: differing mechanisms of epirubicin and gemcitabine reduce resistance arising from monotherapy.(5) Broad applicability: the MUC1, AS1411 (nucleolin), and GPC-1 aptamers address biomarkers overexpressed across

multiple cancers, rendering the nanoparticles suitable for diverse tumor types.

Accordingly, Drug 21 exhibits highly specific targeting, cooperative multi-targeted delivery, and sustained intratumoral release of epirubicin and gemcitabine, conferring substantial potential and utility for anticancer therapy.

The targeted chemical-drug architectures described herein improve the reliability of targeted delivery of small-molecule chemotherapeutics and confer superior target specificity.At the same time, use of these architectures enhances loading stability and loading capacity (drug-to-carrier ratio) of the small-molecule payloads.By virtue of the foregoing linkage schemes, the particle size is tuned to a favorable hydrodynamic diameter that permits receptor-mediated endocytosis while reducing nonspecific membrane permeation and rapid renal filtration; accordingly, the advantageous size improves pharmacokinetics, pharmacodynamics, biodistribution, and toxicological profiles.

According to a second aspect of the invention, there is provided a pharmaceutical composition including any of the foregoing targeted chemical drugs and a pharmaceutically acceptable carrier and/or excipient.

The optional pharmaceutically acceptable excipients may vary with the intended dosage form and/or route of administration and may be selected from known pharmaceutical excipients, including but not limited to pharmaceutically acceptable carriers, fillers (excipients), or adjuvants. The targeted chemical drug may be formulated in different dosage forms to accommodate oral, parenteral (injectable), or transdermal administration. Dosage forms include, without limitation, capsules, tablets, oral solutions, injectables, and transdermal delivery preparations.

Specifically, the targeted chemical drug of the present application may be formulated into capsules, tablets, oral solutions, injectables, or transdermal preparations by methods known in the pharmaceutical industry. Preferably, the injectable is an intravenous injection preparation. In preparing capsules, tablets, or oral solutions suitable for oral administration, sucrose, lactose, galactose, corn starch, gelatin, microcrystalline cellulose, carboxymethyl cellulose, and the like may be used as carriers or excipients. In addition, by using methods and auxiliary ingredients known in the pharmaceutical art, the drug of the present application may be formulated into solutions or suspensions suitable for oral administration. For solutions or suspensions suitable for parenteral administration, distilled water, Water for Injection, isotonic sodium chloride solution or glucose solution, or a low-concentration (e.g., 1-100 mM) phosphate-buffered saline (PBS) may be used as the carrier or diluent. One or more additional auxiliaries or additives may be incorporated into these parenteral formulations, for example, ascorbic acid as an antioxidant and sodium benzoate or the like as a preservative. These dosage forms may further contain other solubilizers, disintegrants, colorants, dispersants, or surfactants.

According to a third aspect of the present invention, there is provided the use of the above targeted chemical drug in the manufacture of a medicament for treating tumors. Further, the tumor is selected from andrological tumors, gynecological tumors, respiratory system tumors, digestive system tumors, hematologic tumors, urinary system tumors, bone-marrow tumors, neurological tumors, dermatological tumors, general surgical tumors, or otorhinolaryngological tumors; preferably, the andrological tumors are selected from prostate cancer, penile cancer, testicular tumors, or urethral carcinoma in males; the gynecological tumors are selected from ovarian cancer, cervical cancer, endometrial carcinoma, uterine fibroids (leiomyoma), vulvar cancer, or malignant hydatidiform mole; the respiratory system tumors are selected from lung cancer, non-small-cell lung cancer, small-cell lung cancer, nasopharyngeal carcinoma, tracheal tumors, metastasis of lung cancer, pulmonary inflammatory pseudotumor, or radiation-induced lung cancer; the digestive system tumors are selected from liver cancer, gastric cancer, colorectal cancer, gallbladder cancer, esophageal cancer, rectal cancer, pancreatic cancer, or colon cancer; the hematologic tumors are selected from leukemia, lymphoma, lymphosarcoma, or multiple myeloma; the urinary system tumors are selected from renal cancer, bladder cancer, or urothelial carcinoma; the bone-marrow tumors are selected from giant cell tumor of bone, osteochondroma, or osteosarcoma; the neurological tumors are selected from brain tumor, meningioma, cerebral tuberculoma, pituitary adenoma, neuroblastoma, glioblastoma, or cerebral glioma; the dermatological tumors are selected from skin cancer or melanoma; the general surgical tumors are selected from breast cancer, lipoma, thyroid cancer, or thyroid tumor; and the otorhinolaryngological tumors are selected from oral cancer, tongue cancer, laryngeal cancer, middle-ear carcinoma, gingival cancer, or intraorbital tumor. Preferably, the route of administration in use is intratumoral administration, intravenous administration, or intraperitoneal administration; more preferably, the daily dosage during use is from 0.1 $\mu$g/kg to 100 mg/kg.

According to a fourth aspect of the present invention, there is further provided a method for preventing and/or treating cancer, the method including: providing the above-described targeted chemical drug or pharmaceutical composition; and administering to a tumor patient an effective amount of the targeted chemical drug or the pharmaceutical composition. Preferably, the tumor is selected from andrological tumors, gynecological tumors, respiratory system tumors, digestive system tumors, hematologic tumors, urinary system tumors, bone-marrow tumors, neurological tumors, dermatological tumors, general surgical tumors, or otorhinolaryngological tumors. Preferably, the andrological tumors are selected from prostate cancer, penile cancer, testicular tumors, or urethral carcinoma in males; the gynecological tumors are selected from ovarian cancer, cervical cancer, endometrial carcinoma, uterine fibroids (leiomyoma), vulvar cancer, or malignant hydatidiform mole; the respiratory system tumors are selected from lung cancer, non-small-cell lung cancer, small-cell lung cancer, nasopharyngeal carcinoma, tracheal tumors, metastasis of lung cancer, pulmonary inflammatory pseudotumor, or radiation-induced lung cancer; the digestive system tumors are selected from liver cancer,

gastric cancer, colorectal cancer, gallbladder cancer, esophageal cancer, rectal cancer, pancreatic cancer, or colon cancer; the hematologic tumors are selected from leukemia, lymphoma, lymphosarcoma, or multiple myeloma; the urinary system tumors are selected from renal cancer, bladder cancer, or urothelial carcinoma; the bone-marrow tumors are selected from giant cell tumor of bone, osteochondroma, or osteosarcoma; the neurological tumors are selected from brain tumor, meningioma, cerebral tuberculoma, pituitary adenoma, neuroblastoma, glioblastoma, or cerebral glioma; the dermatological tumors are selected from skin cancer or melanoma; the general surgical tumors are selected from breast cancer, lipoma, thyroid cancer, or thyroid tumor; and the otorhinolaryngological tumors are selected from oral cancer, tongue cancer, laryngeal cancer, carcinoma of the middle ear, gingival cancer, or intraorbital tumor. Preferably, the route of administration during use is intratumoral administration, intravenous administration, or intraperitoneal administration.

As used herein, "effective amount" includes a prophylactically effective amount and/or a therapeutically effective amount.A "therapeutically effective amount" refers to an amount and duration sufficient to achieve the desired therapeutic outcome, such as a reduction in the burden of gastric cancer or lung cancer. In a particular embodiment, the dose may be titrated to provide an optimal therapeutic response, and the therapeutically effective amount may vary depending on factors such as the subject's disease status, age, sex, body weight, and the ability of the formulation to elicit the desired response in the individual. The term "therapeutically effective amount" further encompasses an amount at which the therapeutic benefit outweighs any toxic or deleterious effects. A "prophylactically effective amount" refers to an amount and duration sufficient to achieve the desired preventive outcome, such as preventing or inhibiting the occurrence of acute leukemia, breast cancer, and/or ovarian cancer.A prophylactically effective amount may be determined analogously from the foregoing description of the therapeutically effective amount.For any particular subject, the specific dosage may be adjusted over time based on individual needs and the professional judgment of the administering practitioner.

According to a fifth aspect of the present invention, there is provided a method for preparing a targeted chemical drug, characterized in that it includes the following steps: forming, by sequence self-assembly, a targeting nucleic acid carrier bearing optional oligonucleotide effector molecule(s) and optional immunostimulant(s); and linking a chemical small-molecule drug to the targeting nucleic acid carrier to obtain the targeted chemical drug.As described above, the targeted chemical drug includes a targeting nucleic acid carrier and a chemical small-molecule drug loaded on the targeting nucleic acid carrier, wherein the targeting nucleic acid carrier includes a nucleic acid carrier and a targeting moiety linked to the nucleic acid carrier; optionally, the targeting nucleic acid carrier further carries oligonucleotide effector molecule(s) and/or an immunostimulant.During sequence self-assembly, the targeting nucleic acid carrier may be provided as single-stranded sequences directly generated at the stage of sequence design and synthesis and then formed by self-assembly. The chemical small-molecule drug may be linked to the targeting nucleic acid carrier either during sequence design and synthesis or by loading onto the targeting nucleic acid carrier; this will be understood by those skilled in the art.

In the practical synthesis of sequence self-assembly, the desired targeting nucleic acid carrier may, based on the base (nucleotide) distribution of the target design, be apportioned into a plurality of single-stranded sequences, which are then self-assembled to yield the targeting nucleic acid carrier.In a preferred embodiment, the step of forming, by sequence self-assembly, the targeting nucleic acid carrier bearing optional oligonucleotide effector molecule(s) and optional immunostimulant(s) includes: dissolving at least three sequences-respectively bearing the optional oligonucleotide effector molecule(s), the optional immunostimulant(s), and the targeting moiety-in an assembly solution and subjecting them to a denaturation treatment to form a crude self-assembly product; and successively purifying, eluting, and evaporating to dryness the crude self-assembly product to obtain the targeting nucleic acid carrier bearing the optional oligonucleotide effector molecule(s) and/or the optional immunostimulant(s).Self-assembly from at least three sequences reduces assembly difficulty and affords more suitable individual sequence lengths.

To enhance the efficiency of self-assembly, it is preferred that the at least three sequences include at least the following three sequences: sequence a: a sequence whose terminus is directly connected, or connected via a nucleotide linker (bridge), to one or more of optional oligonucleotide effector molecule(s), optional immunostimulant(s), and optional targeting moiety(ies); sequence b: a sequence whose terminus is directly connected, or connected via a nucleotide linker (bridge), to one or more of optional oligonucleotide effector molecule(s), optional immunostimulant(s), and optional targeting moiety(ies); and sequence c: a sequence whose terminus is directly connected, or connected via a nucleotide linker (bridge), to one or more of optional oligonucleotide effector molecule(s), optional immunostimulant(s), and optional targeting moiety(ies). The molar ratio among sequence a, sequence b, and sequence c is 0.90-1.10: 0.90-1.10: 0.90-1.10, more preferably 1: 1: 1. Where the at least three sequences include sequences in addition to the a, b, and c sequences, the molar ratio between each sequence and sequence a is 0.90-1.10: 0.90-1.10, more preferably 1: 1. Controlling the sequence molar ratios within these ranges provides higher assembly efficiency.

Preferably, when the at least three sequences include sequence(s) other than sequence a, sequence b, and sequence c (designated herein as "complementary sequence(s)"), any one of the 5' or 3' ends of each of the sequences a, b, and c is independently connected to a transitional nucleotide segment (i.e., at least one of the 5' or 3' ends of the a-sequence, the 5' or 3' ends of the sequence b, and the 5' or 3' ends of the sequence c is linked to the transitional nucleotide segment; one transitional nucleotide segment corresponds to one complementary sequence). The complementary

sequence(s) and the transitional nucleotide segment(s) are in a base-complementary relationship, such that, during self-assembly, the complementary sequence(s) complementarily hybridize with the transitional nucleotide segment(s).More preferably, the complementary sequence itself is the sense or antisense strand of an siRNA oligonucleotide effector molecule or a miRNA oligonucleotide effector molecule (i.e., the sense or antisense strand of an siRNA oligonucleotide effector molecule, or the sense or antisense strand of a miRNA oligonucleotide effector molecule), and/or the complementary sequence provides a loading site for a small-molecule payload (as "warhead"), a targeting aptamer, or an immunostimulant.Use of the foregoing complementary sequence(s) is advantageous in shortening the length of individual strands in the self-assembly process, thereby improving assembly efficiency and increasing the stability of the final drug.

Further preferably, when the at least three sequences include complementary sequence(s), complementary sequence(s) carrying optional small-molecule payload(s), optional targeting aptamer(s), and/or optional immunostimulant(s) are simultaneously added to the assembly solution and subjected to denaturation.In this case, the complementary sequence(s) also serve as carrier sequences for the small-molecule payload(s), targeting aptamer(s), and immunostimulant(s), such that these are loaded into the final targeted chemical drug during the self-assembly process.

In a preferred embodiment, the assembly solution is a TMS aqueous solution, a sodium chloride solution, a magnesium chloride solution, or purified water. Preferably, the temperature for the denaturation treatment is 80-99 °C, more preferably 85-99 °C, and still more preferably 90-99 °C. Preferably, upon completion of the denaturation treatment, the reaction system is cooled to a holding (annealing) temperature for an incubation, and is then cooled to obtain the assembled product; the holding temperature is 50-70 °C, more preferably 55-65 °C, and still more preferably 57-63 °C. The duration of the holding incubation is 3~15 min, more preferably 3-10 min, and still more preferably 3-5 min. Preferably, during cooling of the reaction system to the holding temperature, the cooling rate is 2~10 °C/min, more preferably 2~6 °C/min, and still more preferably 2~3 °C/min. Preferably, the final cooling (endpoint) temperature is 0~25 °C, more preferably 0-15 °C, and still more preferably 0~4 °C. Preferably, the pH during the denaturation treatment is 5.4-8.8.Conducting the self-assembly/renaturation of the respective sequences under the foregoing process conditions yields a higher assembly efficiency and improved reaction stability.

Depending on the structural class of the chemical small-molecule drug, the loading may be carried out as follows:

When the chemical small-molecule drug is an anthracycline chemotherapeutic or an acridine chemotherapeutic, the step of loading the chemical small-molecule drug onto the targeting nucleic acid carrier includes: dissolving the chemical small molecule, a coupling agent, and the targeting nucleic acid carrier bearing optional oligonucleotide effector molecule(s) and optional immunostimulant(s) in a first solvent and reacting under light-protection, so as to allow the chemical small molecule to specifically intercalate between adjacent GC base pairs of the targeting nucleic acid carrier, thereby affording a crude reaction product; and successively subjecting the crude reaction product to crystallization, washing, and evaporation to dryness to obtain the targeted chemical drug. Preferably, the coupling agent is formaldehyde and/or paraformaldehyde. Preferably, the reaction temperature is 0-37 °C, more preferably 4-20 °C, and still more preferably 4~8 °C. Preferably, the reaction pH is 6-8. Preferably, the reaction time is 24~96 h, more preferably 48-72 h, and still more preferably 72 h. Preferably, the first solvent is purified water and/or a PBS buffer. Preferably, the washing agent used in the washing step is a halogenated alkane solvent, an ether solvent, or an arene solvent, more preferably chloroform, dichloromethane, diethyl ether, toluene, or xylene. Preferably, the solvent employed for crystallization is a lower alkanol, more preferably methanol, ethanol, or isopropanol. Preferably, the average number of chemical small-molecule drugs loaded per targeting nucleic acid carrier (bearing the optional oligonucleotide effector molecule(s) and optional immunostimulant(s)) is 1~50, more preferably 10-40, and still more preferably 20~35;

When the chemical small-molecule drug is a pyrimidine-class chemotherapeutic, the small-molecule payload (as "warhead"), the oligonucleotide effector molecule(s), and the immunostimulant(s) are linked to terminal nucleotide(s) of the respective sequences of the nucleic acid carrier via a nucleotide linker including 1 to 10 nucleotides. The step of attaching the chemical small-molecule drug to the targeting nucleic acid carrier bearing the optional oligonucleotide effector molecule(s) and optional immunostimulant(s) includes: during the step of forming, by sequence self-assembly, the targeting nucleic acid carrier bearing the optional oligonucleotide effector molecule(s) and optional immunostimulant(s), connecting the chemical small-molecule drug to at least one sequence of the nucleic acid carrier in such a manner that the drug at least partially replaces nucleotide(s) within the nucleotide linker and/or serves as an extension nucleotide and/or replaces a scaffold nucleotide of the carrier, and thereafter completing attachment of the chemical small-molecule drug by sequence self-assembly. Preferably, the average number of chemical small-molecule drugs loaded per targeting nucleic acid carrier molecule is 1~30, more preferably 10-20, and still more preferably 12-18;

When the chemical small-molecule drug is a platinum-class chemotherapeutic, a glutamic-acid-derivative chemotherapeutic, a flavonoid chemotherapeutic, a botanical drug or a derivative thereof, a folate-class chemotherapeutic, or a salicylic-acid-class chemotherapeutic, the step of connecting the chemical small-molecule drug to the targeting nucleic acid carrier bearing the optional oligonucleotide effector molecule(s) and optional immunostimulant(s) includes: in the course of synthesizing the respective sequences of the nucleic acid carrier to which the targeting moiety is linked, incorporating a linker into at least one sequence of the nucleic acid carrier bearing the optional oligonucleotide effector molecule(s), optional immunostimulant(s), and targeting moiety to obtain a targeting nucleic acid carrier having a linker; dissolving the linker-bearing targeting nucleic acid carrier and the chemical small-molecule drug in a second solvent to react, and then cooling, purifying, washing, and evaporating to dryness to obtain the targeted chemical drug;

Preferably, where the chemical small-molecule drug itself carries a succinimidyl ester (NHS) group, the reaction is carried out by dissolving the linker-bearing targeting nucleic acid carrier and the chemical small-molecule drug in the second solvent.Where the chemical small-molecule drug does not carry a succinimidyl ester group, prior to reacting in the second solvent, the method further includes sequentially reacting the chemical small-molecule drug with bis(2-hydroxyethyl) disulfide and N, N-diisopropylethylamine so as to introduce a succinimidyl ester group onto the chemical small-molecule drug. Preferably, the second solvent is purified water and/or a PBS buffer solution.

In order to allow the targeting aptamer to extend freely, maintain its three-dimensional conformation without perturbation, and preserve its specificity and affinity, the targeting aptamer may be linked to one end of a single-stranded sequence via a nucleotide linker (bridge) including several nucleotides, e.g., a segment of 3~6 bases.For example, the EGFR aptamer may be synthesized and linked to the 5' end of the b-sequence via the nucleotide linker "TTTTT" (see the sequence architectures of the respective drugs described above). This approach will be readily understood by those skilled in the art and is therefore not further elaborated herein.Where the targeting moiety is instead provided as another small-molecule structure, it may likewise be directly incorporated at one end of the single strand by chemical modification, which is routine in the art.

The present application is further described in detail below with reference to specific embodiments, which are not to be construed as limiting the scope of protection sought herein.

Example 1

In this example, the targeted epirubicin drug Apt EGFR-DNA-epirubicin is synthesized.

**Step 1:** Synthesize the Apt EGFR-DNA targeting carrier. The sequences of the single strands are shown in **Table 1** below:

Table 1

| Name | Sequence (sense, 5'-3') | Modification |
|---|---|---|
| Sequence a (SEQ ID NO: 7) | **5'-GCGGCGCCCACGAGCGTTCCGGGAGC-3'** | Unmodified |
| Sequence b (SEQ ID NO: 281) | **5'-**GCCTTAGTAACGTGCTTTGATGTCGATTCGACAGGAGG CTTTTT**GCTCCCGGTTCGCCGCCAGCCGCC-3'** | Unmodified |
| Sequence c (SEQ ID NO: 9) | **5'-GGCGGCAGGCGGCCATAGCCGTGGGCGCCGC-3'** | Unmodified |
| **Note:** The bolded portions denote the a-, b-, or c-sequence within the nucleic acid carrier; the same applies hereinafter. | | |

1. Materials and Reagents:

(1)Sequence a;

(2)Sequence b;

(3)Sequence c;

(4)TMS buffer (Tris-hydrochloride, sodium chloride, magnesium chloride);

(5)TBM (Tris-borate-magnesium) buffer

2. Experimental Method:

Dissolve the sequences a, b, and b separately in TMS buffer at a molar ratio of 1: 1: 1; after mixing, adjust the reaction mixture to pH 6, heat to 80 °C and maintain for 5 min, then cool at 10 °C/min to 70 °C and hold (anneal) for 15 min, and subsequently cool at 10 °C/min to 4 °C to obtain a crude product.

Load the product onto an 8% (m/v) native PAGE gel and perform electrophoretic purification of the carrier in TBM buffer at 4 °C and 100 V.Excise the target band and elute in DNA elution buffer at 37 °C, then precipitate with ethanol overnight and evaporate to dryness under reduced pressure at low temperature to afford the self-assembled product-the EGFR-DNA targeting carrier.

Step 2: Loading of Epirubicin

1. Materials and Reagents

(1)Apt EGFR-DNA targeting carrier prepared in Step 1;

(2)DEPC water (diethyl pyrocarbonate-treated water);

(3)PBS buffer (pH 7.4);

(4)4% formaldehyde (aq.);

(5)Epirubicin hydrochloride;

(6)Chloroform;

(7)Anhydrous ethanol.

2. Experimental Method:

(1)Accurately weigh epirubicin hydrochloride (0.79 mg, 1.354 μmol) and dissolve in DEPC water (1.0 mL) and PBS buffer (1.25 mL) in an ice-water bath.Add an aqueous formaldehyde solution (0.25 mL) with mixing.Combine the entire mixture with the EGFR-DNA targeting carrier (1 mg), mix thoroughly, and react for 24 h at 25 °C under light-protection, maintaining the reaction pH at 6.

(2)Wash the reaction mixture with chloroform (10 mL × 3). To the aqueous phase add anhydrous ethanol (25 mL), mix, and keep at 4 °C protected from light for 4 h to allow complete precipitation. Centrifuge (4, 000 rpm), decant the supernatant, wash the solid again with ethanol (50 mL), remove the supernatant, and dry the residue under reduced pressure at low temperature to afford a dark-red solid-namely, the targeted epirubicin drug Apt EGFR-DNA-epirubicin.

(4)Determination of Loading (Drug-to-Carrier Ratio):

1. Prepare epirubicin-PBS standard solutions at 2 μM, 4 μM, 6 μM, 8 μM, and 10 μM, 100 μL each;

2. Dissolve **Apt EGFR-DNA-epirubicin** in 100 μL PBS;

3. Place the reference standard solutions and the sample in a PCR plate, heat at 85 °C for 5 min, then cool to room temperature;

4. Using a microplate reader, measure epirubicin absorbance at 492 nm, generate a standard curve, and calculate the molar concentration of epirubicin in the loaded product;

5. Using a spectrophotometer, measure DNA absorbance at 260 nm to obtain the mass concentration of the DNA carrier in each sample;

6. Based on the measured epirubicin molar concentration and DNA carrier mass concentration, calculate the loading value. For the above **Apt EGFR-DNA-epirubicin,** the maximum theoretical loading number per carrier molecule is approximately **23,** and the measured average loading number is **20.**

Example 2

In this example, the targeted epirubicin drug **Apt EGFR-4*Bio-DNA-epirubicin** is synthesized.

**Step 1:** Synthesis of the **Apt EGFR-4*Bio-DNA** targeting carrier. The single-strand sequences are shown in **Table 2:**

Table 2

| Name | Sequence (sense, 5'-3') | Modification(s) |
|---|---|---|
| Sequence a (SEQ ID NO: 7) | **5'-GCGGCGCCCACGAGCGTTCCGGGAGC-3'** | Biotin at both 5' and 3' termini (one biotin per end) |
| Sequence b (SEQ ID NO: 281) | **5'-**GCCTTAGTAACGTGCTTTGATGTCGATTCGACAG GAGGCTTTTT**GCTCCCGGTTCGCCGCCAGCCGCC-3'** | Unmodified |
| Sequence c (SEQ ID NO: 9) | **5'-GGCGGCAGGCGGCCATAGCCGTGGGCGCCGC-3'** | Biotin at both 5' and 3' termini (one biotin per end) |

1. Materials and Reagents:

(1)Sequence a;

(2)Sequence b;

(3)Sequence c;

(4)Aqueous sodium chloride solution;

(5)TBM buffer (Tris-borate-magnesium).

2. Experimental Method:

Dissolve the Sequences a, b and c separately in the aqueous sodium chloride solution at a molar ratio of 1.05: 1: 1.05.After mixing, adjust the reaction mixture to pH 7.Heat the mixture to 85 °C and maintain for 5 min; then cool at 4 °C/min to 50 °C and hold (anneal) for 10 min; subsequently cool at 4 °C/min to 10 °C to obtain a crude product.

Load the product onto an 8% (m/v) native PAGE gel and purify the carrier by electrophoresis in TBM buffer at 4 °C and 100 V.Excise the desired band and elute in DNA elution buffer at 37 °C, then precipitate with ethanol overnight and dry under reduced pressure at low temperature to afford the self-assembled product-**the Apt EGFR-4*Bio-DNA targeting carrier.**

Step 2: Loading of Epirubicin

1. Materials and Reagents:

(1)Apt EGFR-4*Bio-DNA targeting carrier prepared in Step 1;

(2)DEPC water (diethyl-pyrocarbonate-treated water);

(3)PBS buffer (pH 5.8);

(4)4% paraformaldehyde (aq.);

(5)Epirubicin hydrochloride;

(6)Chloroform;

(7)Anhydrous ethanol.

2. Experimental Method:

(1)Accurately weigh epirubicin hydrochloride (0.79 mg, 1.354 μmol) and dissolve in DEPC water (1.0 mL) and PBS buffer (1.25 mL) in an ice-water bath.Add 4% aqueous paraformaldehyde (0.25 mL) with mixing.Combine the entire mixture with the EGFR-4Bio-DNA targeting carrier (1 mg), mix thoroughly, and react for 48 h at 20 °C under light-protection, maintaining the reaction pH at 7.

(2)Wash the reaction mixture with chloroform (10 mL × 3). To the aqueous phase add anhydrous ethanol (25 mL), mix, and keep at 4 °C protected from light for 4 h to allow complete precipitation.Centrifuge (4, 000 rpm), decant the supernatant, wash the solid again with ethanol (50 mL), remove the supernatant, and dry the residue under reduced pressure at low temperature to afford a dark-red solid-namely, the targeted epirubicin drug **EGFR-4Bio-DNA-epirubicin**.

(4)Determination of Loading (Drug-to-Carrier Ratio):

1. Prepare epirubicin-PBS standards at 2 μM, 4 μM, 6 μM, 8 μM, and 10 μM, 100 μL each;

2. Dissolve **Apt EGFR-4*Bio-DNA-epirubicin** in 100 μL PBS;

3. Place the reference standard solutions and the sample in a PCR plate, heat at 85 °C for 5 min, then cool to room temperature;

4. Using a microplate reader, measure the absorbance of epirubicin at 492 nm, generate a standard curve, and calculate the molar concentration of epirubicin in the loaded product;

5. Using a spectrophotometer, measure the absorbance of DNA at 260 nm to obtain the mass concentration of the DNA carrier in each sample;

6. Calculate the loading value based on the measured epirubicin molar concentration and DNA-carrier mass concentration. For **Apt EGFR-4*Bio-DNA-epirubicin,** the maximum theoretical loading number per carrier molecule is approximately **23,** and the measured average loading number is **20.**

Example 3

In this example, the targeted epirubicin drug **Apt AS1411-4*Bio-DNA-epirubicin** is synthesized.

**Step 1:** Synthesis of the **Apt AS1411-4*Bio-DNA** targeting carrier. The single-strand sequences are shown in **Table 3:**

Table 3

| Name | Sequence (sense, 5'→3') | Modification(s) |
|---|---|---|
| Sequence a (SEQ ID NO: 7) | **5'-GCGGCGCCCACGAGCGTTCCGGGAGC-3'** | Biotin at both 5' and 3' termini (one biotin per end) |

(continued)

| Name | Sequence (sense, 5'→3') | Modification(s) |
|---|---|---|
| Sequence b (SEQ ID NO: 285) | **5'-**GGTGGTGGTGGTTGTGGTGGTGGTGGTTTTT**GC TCCCGGTTCGCCGCCAGCCGCC-3'** | Unmodified |
| Sequence c (SEQ ID NO: 9) | **5'-GGCGGCAGGCGGCCATAGCCGTGGGCGCCGC-3'** | Biotin at both 5' and 3' termini (one biotin per end) |

1. Materials and Reagents:

(1)Sequence a;

(2)Sequence b;

(3)Sequence c;

(4)Aqueous magnesium chloride solution;

(5)TBM buffer (Tris-borate-magnesium).

2. Experimental Method:

Dissolve the sequences a, b, and c separately in the aqueous magnesium chloride solution at a molar ratio of 1.05: 1.05: 1. After mixing, adjust the reaction mixture to pH 8.Heat to 90 °C and maintain for 5 min; cool at 2 °C/min to 65 °C and hold (anneal) for 5 min; then cool at 2 °C/min to 5 °C to obtain a crude product.

Load the product onto an 8% (m/v) native PAGE gel and purify the carrier by electrophoresis in TBM buffer at 4 °C and 100 V.Excise the desired band and elute in DNA elution buffer at 37 °C, then precipitate with ethanol overnight and dry under reduced pressure at low temperature to afford the self-assembled product-**the Apt AS1411-4*Bio-DNA targeting carrier.**

Step 2: Loading of Epirubicin

1. Materials and Reagents:

(1)Apt AS1411-4*Bio-DNA targeting carrier prepared in Step 1;

(2)DEPC water (diethyl-pyrocarbonate-treated water);

(3)PBS buffer (pH 6.5);

(4)4% paraformaldehyde (aq.);

(5)Epirubicin;

(6)Toluene;

(7)Anhydrous ethanol.

2. Experimental Method:

(1)Accurately weigh epirubicin hydrochloride (0.79 mg, 1.354 μmol) and dissolve in DEPC water (1.0 mL) and PBS buffer (1.25 mL) in an ice-water bath.Add 4% aqueous paraformaldehyde (0.25 mL) with mixing.Combine the entire mixture with the AS1411-4Bio-DNA targeting carrier (1 mg), mix thoroughly, and react for 72 h at 15 °C under light-protection, maintaining the reaction pH at 8.

(2)Wash the reaction mixture with toluene (10 mL × 3). To the aqueous phase add anhydrous ethanol (25 mL), mix, and keep at 4 °C protected from light for 4 h to allow complete precipitation.Centrifuge (4, 000 rpm), decant the supernatant, wash the solid again with ethanol (50 mL), remove the supernatant, and dry the residue under reduced pressure at low temperature to afford a dark-red solid-namely, the targeted epirubicin drug **AS1411-4Bio-DNA-epirubicin**.

(4)Determination of Loading (Drug-to-Carrier Ratio):

1. Prepare epirubicin-PBS standard solutions of known concentration: 2 μM, 4 μM, 6 μM, 8 μM, and 10 μM, 100 μL each;

2. Dissolve **Apt AS1411-4*Bio-DNA-epirubicin** in 100 μL PBS;

3. Place the reference standard solutions and the sample in a PCR plate, heat at 85 °C for 5 min, then cool to room temperature;

4. Using a microplate reader, measure epirubicin absorbance at 492 nm, plot the standard curve, and calculate the molar concentration of epirubicin in the loaded product;

5. Using a spectrophotometer, measure DNA absorbance at 260 nm to determine the mass concentration of the DNA carrier in each sample;

6. Calculate the loading value based on the measured epirubicin molar concentration and DNA-carrier mass concentration.For **Apt AS1411-4*Bio-DNA-epirubicin,** the maximum theoretical loading number per carrier molecule is approximately **23,** and the measured average loading number is **20.**

Example 4

In this example, the targeted epirubicin drug **Apt A1-Apt A15-DNA-epirubicin** is synthesized.

**Step 1:** Synthesis of the **Apt A1-Apt A15-DNA** targeting carrier. The single-strand sequences are shown in **Table 4:**

Table 4

| Name | Sequence (sense, 5'→3') | Modification(s) |
|---|---|---|
| Sequence a (SEQ ID NO:7) | **5'-GCGGCGCCCACGAGCGTTCCGGGAGC-3'** | Unmodified |
| Sequence b (SEQ ID NO:287) | **5'-**GGTTGCATGCCGTGGGGAGGGGGGTGGGTTTTATAGC GTACTCAGTTTTT**GCTCCCGGTTCGCCGCCAGCCGCC-3'** | Unmodified |
| Sequence c | **5'-**CCCTCCTACATAGGGTTTTT**GGCGGCAGGCGGCCATAG** | Unmodified |
| (SEQ ID NO:288) | **CCGTGGGCGCCGC-3'** | |

1. Materials and Reagents:

(1)Sequence a;

(2)Sequence b;

(3)Sequence c;

(4)DEPC water (diethyl-pyrocarbonate-treated water);

(5)TBM buffer (Tris-borate-magnesium).

2. Experimental Method:

Dissolve the sequences a, b, and c separately in DEPC water at a molar ratio of 1.1: 1: 1. After mixing, adjust the reaction mixture to pH 5.4.Heat the mixture to 95 °C and maintain for 5 min; then cool at 2 °C/min to 55 °C and hold (anneal) for 10 min; subsequently cool at 2 °C/min to 8 °C.

Load the product onto an 8% (m/v) native PAGE gel and purify the carrier by electrophoresis in TBM buffer at 4 °C and 100 V.Excise the desired band and elute in DNA elution buffer at 37 °C, then precipitate with ethanol overnight and dry under reduced pressure at low temperature to afford the self-assembled product-**the Apt A1-Apt A15-DNA targeting carrier.**

Step 2: Loading of Epirubicin

1. Materials and Reagents:

(1)Apt A1-Apt A15-DNA targeting carrier prepared in Step 1;

(2)DEPC water;

(3)PBS buffer (pH 6.6);

(4)4% paraformaldehyde (aq.);

(5)Epirubicin;

(6)Diethyl ether;

(7)Anhydrous ethanol.

2. Experimental Method:

(1)Accurately weigh epirubicin hydrochloride (0.79 mg, 1.354 μmol) and dissolve in DEPC water (1.0 mL) and PBS buffer (1.25 mL) in an ice-water bath.Add 4% aqueous paraformaldehyde (0.25 mL) with mixing.Combine the entire mixture with the **Apt A1-Apt A15-DNA** targeting carrier (1 mg), mix thoroughly, and react for 96 h at 5 °C under light-protection, maintaining the reaction pH at 6-8.

(2)Wash the reaction mixture with diethyl ether (10 mL × 3). To the aqueous phase add anhydrous ethanol (25 mL), mix, and keep at 4 °C protected from light for 4 h to allow complete precipitation.Centrifuge (4, 000 rpm), decant the supernatant, wash the solid again with ethanol (50 mL), remove the supernatant, and dry the residue under reduced pressure at low temperature to afford a dark-red solid-namely, the targeted epirubicin drug **Apt A1-Apt A15-DNA-epirubicin.**

(4)Determination of Loading (Drug-to-Carrier Ratio):

1. Prepare epirubicin-PBS standard solutions of known concentration: 2 μM, 4 μM, 6 μM, 8 μM, and 10 μM, 100 μL each;

2. Dissolve **Apt A1-Apt A15-DNA-epirubicin** in 100 μL PBS;

3. Place the reference standard solutions and the sample in a PCR plate, heat at 85 °C for 5 min, then cool to room temperature;

4. Using a microplate reader, measure the absorbance of epirubicin at 492 nm, plot the standard curve, and calculate the molar concentration of epirubicin in the loaded product;

5. Using a spectrophotometer, measure DNA absorbance at 260 nm to determine the mass concentration of the DNA carrier in each sample;

6. Calculate the loading value based on the measured epirubicin molar concentration and DNA-carrier mass concentration.For **Apt A1-Apt A15-DNA-epirubicin,** the maximum theoretical loading number per carrier molecule is approximately **20,** and the measured average loading number is **18.**

Example 5

In this example, the targeted epirubicin drug **Apt AS1411-Apt EGFR-Apt A15-2*Bio-DNA-epirubicin** is synthesized.

**Step 1:** Synthesize the **Apt AS1411-Apt EGFR-Apt A15-2*Bio-DNA** targeting carrier. The single-strand sequences are shown in **Table 5:**

Table 5

| Name | Sequence (sense, 5'→3') | Modification(s) |
|---|---|---|
| Sequence a (SEQ ID NO:282) | **5'**-GGTGGTGGTGGTTGTGGTGGTGGTGGTTTTT**GCGGCGCCCACGAGCGTTCCGGGAGC**-**3'** | One biotin at the 3' terminus |
| Sequence b (SEQ ID NO:283) | **5'**-GCCTTAGTAACGTGCTTTGATGTCGATTCGACAGGAGGCTTTTT**GCTCCCGGTTCGCCGCCAGCCGCC-3'** | Unmodified |
| Sequence c (SEQ ID NO:284) | **5'**-CCCTCCTACATAGGGTTTTT**GGCGGCAGGCGGCCATAGCCGTGGGCGCCGC-3'** | One biotin at the 3' terminus |

1. Materials and Reagents:

(1)Sequence a;

(2)Sequence b;

(3)Sequence c;

(4)TMS aqueous solution;

(5)TBM buffer (Tris-borate-magnesium).

2. Experimental Method:

Dissolve the sequences a, b, and c separately in the TMS aqueous solution at a molar ratio of 1: 1: 1. After mixing, adjust the reaction mixture to pH 8.8.Heat to 99 °C and maintain for 5 min; then cool at 2 °C/min to 60 °C and hold (anneal) for 5 min; subsequently cool at 2 °C/min to 2 °C to obtain a crude product.

Load the product onto size-exclusion chromatography (SEC) column(s) (in series), collect the main fraction, perform column desalting, and evaporate to dryness under reduced pressure at low temperature to afford the self-assembled product-**the Apt AS1411-Apt EGFR-Apt A15-2*Bio-DNA targeting carrier.**

Step 2: Loading of Epirubicin

1. Materials and Reagents:

(1)Apt AS1411-Apt EGFR-Apt A15-2*Bio-DNA targeting carrier prepared in Step 1;

(2)DEPC water (diethyl-pyrocarbonate-treated water);

(3)PBS buffer (pH 6.8);

(4)4% paraformaldehyde (aq.);

(5)Epirubicin hydrochloride;

(6)Dichloromethane;

(7)Anhydrous ethanol.

2. Experimental Method:

(1)Accurately weigh epirubicin hydrochloride (0.79 mg, 1.354 μmol) and dissolve in DEPC water (1.0 mL) and PBS buffer (1.25 mL) in an ice-water bath. Add 4% aqueous paraformaldehyde (0.25 mL) with mixing. Combine the entire mixture with the **Apt AS1411-Apt EGFR-Apt A15-2*Bio-DNA** targeting carrier (1 mg), mix thoroughly, and react for 72 h at 4 °C under light-protection, maintaining the reaction pH at 7.

(2)Wash the reaction mixture with dichloromethane (10 mL × 3). To the aqueous phase add anhydrous ethanol (25 mL), mix, and keep at 4 °C protected from light for 4 h to allow complete precipitation. Centrifuge (4, 000 rpm), decant the supernatant, wash the solid again with ethanol (50 mL), remove the supernatant, and dry the residue under reduced pressure at low temperature to afford a dark-red solid-namely, the targeted epirubicin drug **Apt AS1411-Apt EGFR-Apt A15-2*Bio-DNA-epirubicin.**

(4)Determination of Loading (Drug-to-Carrier Ratio):

1. Prepare epirubicin-PBS standard solutions of known concentration: 2 μM, 4 μM, 6 μM, 8 μM, and 10 μM, 100 μL each;

2. Dissolve **Apt AS1411-Apt EGFR-Apt A15-2*Bio-DNA-epirubicin** in 100 μL PBS;

3. Place the reference standard solutions and the sample in a PCR plate, heat at 85 °C for 5 min, then cool to room temperature;

4. Using a microplate reader, measure the absorbance of epirubicin at 492 nm, plot a standard curve, and calculate the molar concentration of epirubicin in the loaded product;

5. Using a spectrophotometer, measure DNA absorbance at 260 nm to determine the mass concentration of the DNA carrier in each sample;

6. Calculate the loading value based on the measured epirubicin molar concentration and DNA-carrier mass concentration. For **Apt AS1411-Apt EGFR-Apt A15-2*Bio-DNA-epirubicin,** the maximum theoretical loading number per carrier molecule is approximately **30,** and the measured average loading number is **25.**

Example 6

In this example, the targeted epirubicin drug **Apt AS1411-Apt EGFR-Apt A15-DNA-epirubicin** is synthesized.

**Step 1:** Synthesize the **Apt AS1411-Apt EGFR-Apt A15-DNA** targeting carrier. The single-strand sequences are shown in **Table 6:**

Table 6

| Name | Sequence (sense, 5'→3') | Modification(s) |
|---|---|---|
| Sequence a (SEQ ID NO:282) | **5'-**GGTGGTGGTGGTTGTGGTGGTGGTGGTTTTT**GCGG CGCCCACGAGCGTTCCGGGAGC-3'** | Unmodified |
| Sequence b (SEQ ID NO:283) | **5'-**GCCTTAGTAACGTGCTTTGATGTCGATTCGACAGGAG GCTTTTT**GCTCCCGGTTCGCCGCCAGCCGCC-3'** | Unmodified |
| Sequence c (SEQ ID NO:284) | **5'-**CCCTCCTACATAGGGTTTTT**GGCGGCAGGCGGCCAT AGCCGTGGGCGCCGC-3'** | Unmodified |

1. Materials and Reagents:

(1)Sequence a;

(2)Sequence b;

(3)Sequence c;

(4)TMS aqueous solution;

(5)TBM buffer (Tris-borate-magnesium).

2. Experimental Method:

Dissolve the sequences a, b, and c separately in the TMS aqueous solution at a molar ratio of 1: 1: 1. After mixing, adjust the reaction mixture to pH 8.8.Heat to 99 °C and maintain for 5 min; then cool at 5 °C/min to 65 °C and hold (anneal) for 3 min; subsequently cool at 5 °C/min to 2 °C to obtain a crude product. The SEC chromatographic profile of the crude targeting nucleic acid carrier after sequence assembly is shown in **Fig.1** (analysis conditions: column, Biosep SEC-S2000, 5 μm, 7.8 × 300 mm, two columns in series; mobile phase, water [50 mM Tris-HCl, 100 mM NaCl, pH 8]; detection wavelength, 260 nm; flow rate, 0.6 mL/min; column temperature, 35 °C; sample preparation, dissolve at 2 mg/mL in the mobile phase, gently shake at 37 °C for 30 min, filter through a 0.22 μm nylon membrane; injection volume, 100 μL).

Load the product onto SEC column(s) in series, collect the main fraction, desalt by ultrafiltration, and dry under reduced pressure at low temperature. The HPLC (SEC) chromatogram of the purified targeting nucleic acid carrier is shown in **Fig.2.**

Step 2: Loading of Epirubicin

1. Materials and Reagents:

(1)Apt AS1411-Apt EGFR-Apt A15-DNA targeting carrier prepared in Step 1;

(2)DEPC water (diethyl-pyrocarbonate-treated water);

(3)PBS buffer (pH 7.0);

(4)4% paraformaldehyde (aq.);

(5)Epirubicin hydrochloride;

(6)Chloroform;

(7)Anhydrous ethanol.

2. Experimental Method:

(1)Accurately weigh epirubicin hydrochloride (0.79 mg, 1.354 μmol) and dissolve in DEPC water (1.0 mL) and PBS buffer (1.25 mL) in an ice-water bath.Add 4% aqueous paraformaldehyde (0.25 mL) with mixing.Combine the entire mixture with the **Apt AS1411-Apt EGFR-AptA15-DNA** targeting carrier (1 mg), mix thoroughly, and react for 72 h at 4 °C under light-protection, maintaining the reaction pH at 7.

(2)Wash the reaction mixture with chloroform (10 mL × 3). To the aqueous phase add anhydrous ethanol (25 mL), mix, and keep at 4 °C protected from light for 4 h to allow complete precipitation.Centrifuge (4, 000 rpm), decant the supernatant, wash the solid again with ethanol (50 mL), remove the supernatant, and dry the residue under reduced pressure at low temperature to afford a dark-red solid-namely, the targeted epirubicin drug **Apt AS1411-Apt EGFR-AptA15-DNA-epirubicin.** The RP-HPLC chromatogram of the crude targeted chemical drug after epirubicin loading is shown in **Fig.3,** and the RP-HPLC chromatogram of the product after purification on an RP-HPLC column is shown in **Fig.4** (test conditions: column, Aeris widepore XB-C18, 3.6 μm, 200 Å, 4.6 × 250 mm; mobile phase A, water [1% HFIP, pH adjusted to 8 with TEA]; mobile phase B, acetonitrile; flow rate, 0.8 mL/min; detection wavelength, 260 nm; column temperature, 40 °C):

(4)Determination of Loading (Drug-to-Carrier Ratio):

1. Prepare epirubicin-PBS standard solutions of known concentration: 2 μM, 4 μM, 6 μM, 8 μM, and 10 μM, 100 μL each;

2. Dissolve **Apt AS1411-Apt EGFR-AptA15-DNA-epirubicin** in 100 μL PBS;

3. Place the reference standard solutions and the sample in a PCR plate, heat at 85 °C for 5 min, then cool to room temperature;

4. Using a microplate reader, measure epirubicin absorbance at 492 nm, construct a standard curve, and calculate the molar concentration of epirubicin in the loaded product;

5. Using a spectrophotometer, measure DNA absorbance at 260 nm to determine the mass concentration of the DNA carrier in each sample;

6. Calculate the loading value based on the measured epirubicin molar concentration and DNA-carrier mass concentration. For **Apt AS1411-Apt EGFR-AptA15-DNA-epirubicin,** the maximum theoretical loading number per carrier molecule is approximately **30,** and the measured average loading number is **25.**

In this example, the effect of varying excess epirubicin on the final loading of **Apt AS1411-Apt EGFR-AptA15-DNA-epirubicin** was also evaluated.Specifically, **Figs.5-7** show the RP-HPLC chromatograms of products obtained at epirubicin-to-carrier molar ratios of **10: 1, 20: 1,** and **30: 1,** respectively. From these figures it can be seen that: (1) the epirubicin loading exhibits a distribution over a range; and (2) as the amount of epirubicin added increases, the area of the characteristic peaks in the RP-HPLC chromatograms increases markedly, indicating that the DAR approaches the theoretical loading value.

Example 7

In this example, the targeted epirubicin drug **Apt AS1411-Apt EGFR-Apt A15-2*Bio-AmiR-21-DNA-epirubicin** is synthesized.

**Step 1:** Synthesize the **Apt AS1411-Apt EGFR-Apt A15-2*Bio-AmiR-21-DNA** targeting carrier. The sequences of the single strands are shown in **Table 7** below:

Table 7

| Name | Sequence (sense, 5'→3') | Modification(s) |
|---|---|---|
| Sequence a (SEQ ID NO:289) | **5'-**GGTGGTGGTGGTTGTGGTGGTGGTGGTTTTT**GC GGCGCCCACGAGCGTTCCGGGAGC-3'** | One biotin at the 3' terminus |
| Sequence b (SEQ ID NO: | **5'-**GCCTTAGTAACGTGCTTTGATGTCGATTCGACAG GAGGCTTTTT**GCTCCCGGTTCGCCGCCAGCCGCC-** | Unmodified |
| 290) | **3'** | |
| Sequence c (SEQ ID NO:291) | **5'-**CCCTCCTACATAGGGTTTTT**GGCGGCAGGCGGC CATAGCCGTGGGCGCCGC**GATAAGCT**-3'** | The eight nucleotides immediately upstream of the 3' terminus are LNA-modified; additionally, one biotin is installed at the 3' terminus. |

1. Materials and Reagents:

(1)Sequence a;

(2)Sequence b;

(3)Sequence c;

(4)DEPC water (diethyl-pyrocarbonate-treated water);

(5)TBM buffer (Tris-borate-magnesium).

2. Experimental Method:

Dissolve the sequences a, b, and c separately in DEPC water at a molar ratio of 1: 1: 1. After mixing, adjust the reaction mixture to pH 7.Heat to 85 °C and maintain for 5 min; then cool at 2 °C/min to 60 °C and hold (anneal) for 5 min; subsequently cool at 2 °C/min to 4 °C.

Load the product onto an 8% (m/v) native PAGE gel and purify the carrier by electrophoresis in TBM buffer at 4 °C and 100 V.Excise the desired band and elute in DNA elution buffer at 37 °C, then precipitate with ethanol overnight and dry under reduced pressure at low temperature to afford the self-assembled product-**the Apt AS1411-Apt EGFR-Apt A15-2*Bio-AmiR-21-DNA targeting carrier.**

Step 2: Loading of Epirubicin

1. Materials and Reagents:

(1)Targeting carrier **Apt AS1411-Apt EGFR-Apt A15-2*Bio-AmiR-21-DNA** prepared in Step 1;

(2)DEPC water;

(3)PBS buffer (pH 6.8);

(4)4% paraformaldehyde (aq.);

(5)Epirubicin hydrochloride;

(6)Chloroform;

(7)Methanol.

2. Experimental Method:

(1)Accurately weigh epirubicin hydrochloride (0.79 mg, 1.354 μmol) and dissolve in DEPC water (1.0 mL) and PBS buffer (1.25 mL) in an ice-water bath.Add 4% aqueous paraformaldehyde (0.25 mL) with mixing.Combine the entire mixture with the **Apt AS1411-Apt EGFR-Apt A15-2*Bio-AmiR-21-DNA** targeting carrier (1 mg), mix thoroughly, and react for 72 h at 4 °C under light-protection, maintaining the reaction pH at 6.

(2)Wash the reaction mixture with chloroform (10 mL × 3). To the aqueous phase add methanol (25 mL), mix, and keep at 4 °C protected from light for 4 h to allow complete precipitation.Centrifuge (4, 000 rpm), decant the supernatant, wash the solid again with methanol (50 mL), remove the supernatant, and dry the residue under reduced pressure at low temperature to afford a dark-red solid-namely, the targeted epirubicin drug **Apt AS1411-Apt EGFR-Apt A15-2*Bio-AmiR-21-DNA-epirubicin.**

(4)Determination of Loading (Drug-to-Carrier Ratio):

1. Prepare epirubicin-PBS standard solutions of known concentration: 2 μM, 4 μM, 6 μM, 8 μM, and 10 μM, 100 μL each;

2. Dissolve **Apt AS1411-Apt EGFR-Apt A15-2*Bio-AmiR-21-DNA-epirubicin** in 100 μL PBS;

3. Place the reference standard solutions and the sample in a PCR plate, heat at 85 °C for 5 min, then cool to room temperature;

4. Using a microplate reader, measure the absorbance of epirubicin at 492 nm, construct a standard curve, and calculate the molar concentration of epirubicin in the loaded product;

5. Using a spectrophotometer, measure DNA absorbance at 260 nm to determine the mass concentration of the DNA carrier in each sample;

6. Calculate the loading value based on the measured epirubicin molar concentration and DNA-carrier mass concentration. For **Apt AS1411-Apt EGFR-Apt A15-2*Bio-AmiR-21-DNA-epirubicin,** the maximum theoretical loading number per carrier molecule is approximately **27,** and the measured average loading number is **23.**

Example 8

In this example, the targeted epirubicin drug **Apt TfRA4-Apt AS1411-Apt A15-DNA-epirubicin (1)** is synthesized.

**Step 1:** Synthesize the **Apt TfRA4-Apt AS1411-Apt A15-DNA** targeting carrier. The sequences of the single strands are shown in **Table 8** below:

Table 8

| Name | Sequence (sense, 5'→3') | Modification(s) |
|---|---|---|
| Sequence a (SEQ ID NO:292) | **5'**-GCGTGGTACCACGCTTTTT**GCGGCGAGCGGCGCCCACGAGCGTTCCGGGAGAGGAGC-3'** | Unmodified |
| Sequence b (SEQ ID NO:293) | **5'**-GGTGGTGGTGGTTGTGGTGGTGGTGGTTTTT**GCTCCTCTCCCGGTTCGCCGCCAGCCGCC-3'** | Unmodified |

(continued)

| Name | Sequence (sense, 5'→3') | Modification(s) |
|---|---|---|
| Sequence c (SEQ ID NO:294) | **5'**-CCCTCCTACATAGGGTTTTT**GGCGGCTGGCGG CCATAGCCGTGGGCGCCGCTCGCCGC**-**3'** | Unmodified |

1. Materials and Reagents:

(1)Sequence a;

(2)Sequence b;

(3)Sequence c;

(4)DEPC water (diethyl-pyrocarbonate-treated water);

(5)TBM buffer (Tris-borate-magnesium).

2. Experimental Method:

Dissolve the sequences a, b, and c separately in DEPC water at a molar ratio of 1: 1: 1. After mixing, adjust the reaction mixture to pH 7.Heat to 99 °C and maintain for 5 min; then cool at 2 °C/min to 65 °C and hold (anneal) for 15 min; subsequently cool at 2 °C/min to 4 °C.

Load the product onto an 8% (m/v) native PAGE gel and purify the carrier by electrophoresis in TBM buffer at 4 °C and 100 V.Excise the desired band and elute in DNA elution buffer at 37 °C, then precipitate with ethanol overnight and dry under reduced pressure at low temperature to afford the self-assembled product-**the Apt TfRA4-Apt AS1411-Apt A15-DNA targeting carrier (1).**

Step 2: Loading of Epirubicin

1. Materials and Reagents:

(1)Targeting carrier **Apt TfRA4-Apt AS1411-Apt A15-DNA (1)** prepared in Step 1;

(2)DEPC water;

(3)PBS buffer (pH 6.6);

(4)4% paraformaldehyde (aq.);

(5)Epirubicin hydrochloride;

(6)Chloroform;

(7)Isopropanol.

2. Experimental Method:

(1)Accurately weigh epirubicin hydrochloride (0.79 mg, 1.354 μmol) and dissolve in DEPC water (1.0 mL) and PBS buffer (1.25 mL) in an ice-water bath. Add 4% aqueous paraformaldehyde (0.25 mL) with mixing. Combine the entire mixture with the **Apt TfRA4-Apt AS1411-Apt A15-DNA** targeting carrier (1 mg), mix thoroughly, and react for 72 h at 4

°C under light-protection, maintaining the reaction pH at 7.

(2)Wash the reaction mixture with chloroform (10 mL × 3). To the aqueous phase add isopropanol (25 mL), mix, and keep at 4 °C protected from light for 4 h to allow complete precipitation. Centrifuge (4, 000 rpm), decant the supernatant, wash the solid again with isopropanol (50 mL), remove the supernatant, and dry the residue under reduced pressure at low temperature to afford a dark-red solid-namely, the targeted epirubicin drug **Apt TfRA4-Apt AS1411-Apt A15-DNA-epirubicin (1).**

(4)Determination of Loading (Drug-to-Carrier Ratio):

1. Prepare epirubicin-PBS standard solutions of known concentration: 2 μM, 4 μM, 6 μM, 8 μM, and 10 μM, 100 μL each;

2. Dissolve **Apt TfRA4-Apt AS1411-Apt A15-DNA-epirubicin (1)** in 100 μL PBS;

3. Place the reference standard solutions and the sample in a PCR plate, heat at 85 °C for 5 min, then cool to room temperature;

4. Using a microplate reader, measure the absorbance of epirubicin at 492 nm, construct a standard curve, and calculate the molar concentration of epirubicin in the loaded product;

5. Using a spectrophotometer, measure DNA absorbance at 260 nm to determine the mass concentration of the DNA carrier in each sample;

6. Calculate the loading value based on the measured epirubicin molar concentration and DNA-carrier mass concentration. For **Apt TfRA4-Apt AS1411-Apt A15-DNA-epirubicin (1),** the maximum theoretical loading number per carrier molecule is approximately **36,** and the measured average loading number is **30.**

Example 9

In this example, the targeted epirubicin drug **Apt TfRA3-Apt AS1411-Apt A15-DNA-epirubicin** is synthesized.

**Step 1:** Synthesize the **Apt TfRA3-Apt AS1411-Apt A15-DNA** targeting carrier. The sequences of the single strands are shown in **Table 9** below:

Table 9

| Name | Sequence (sense, 5'→3') | Modification(s) |
|---|---|---|
| Sequence a (SEQ ID NO: 298) | **5'**-GGTGGTGGTGGTTGTGGTGGTGGTGGTTTTT**GCGGCGCCCACGAGCGTTCCGGGAGC-3'** | Unmodified |
| Sequence b (SEQ ID NO: 299) | **5'**-GCGTGGTCACACGCTTTTT**GCTCCCGGTTCGCCGCCAGCCGCC-3'** | Unmodified |
| Sequence c (SEQ ID NO: 300) | **5'**-CCCTCCTACATAGGGTTTTT**GGCGGCAGGCGGCCATAGCCGTGGGCGCCGC-3'** | Unmodified |

1. Materials and Reagents:

(1)Sequence a;

(2)Sequence b;

(3)Sequence c;

(4)DEPC water (diethyl-pyrocarbonate-treated water);

(5)TBM buffer (Tris-borate-magnesium).

2. Experimental Method:

Dissolve the Sequences a, b and c separately in DEPC water at a molar ratio of 1: 1: 1. After mixing, adjust the reaction mixture to pH 7.Heat to 99 °C and maintain for 5 min; then cool at 2 °C/min to 65 °C and hold (anneal) for 15 min; subsequently cool at 2 °C/min to 4 °C.

Load the product onto an 8% (m/v) native PAGE gel and purify the carrier by electrophoresis in TBM buffer at 4 °C and 100 V.Excise the desired band and elute in DNA elution buffer at 37 °C, then precipitate with ethanol overnight and dry under reduced pressure at low temperature to afford the self-assembled product-**the Apt TfRA3-Apt AS1411-Apt A15-DNA targeting carrier.**

Step 2: Loading of Epirubicin

1. Materials and Reagents:

(1)Targeting carrier **Apt TfRA3-Apt AS1411-Apt A15-DNA** prepared in Step 1;

(2)DEPC water;

(3)PBS buffer (pH 6.6);

(4)4% paraformaldehyde (aq.);

(5)Epirubicin hydrochloride;

(6)Chloroform;

(7)Isopropanol.

2. Experimental Method:

(1)Accurately weigh epirubicin hydrochloride (0.79 mg, 1.354 μmol) and dissolve in DEPC water (1.0 mL) and PBS buffer (1.25 mL) in an ice-water bath.Add 4% aqueous paraformaldehyde (0.25 mL) with mixing.Combine the entire mixture with the **Apt TfRA3-Apt AS1411-Apt A15-DNA** targeting carrier (1 mg), mix thoroughly, and react for 72 h at 4 °C under light-protection, maintaining the reaction pH at 7.

(2)Wash the reaction mixture with chloroform (10 mL × 3). To the aqueous phase add isopropanol (25 mL), mix, and keep at 4 °C protected from light for 4 h to allow complete precipitation. Centrifuge (4, 000 rpm), decant the supernatant, wash the solid again with isopropanol (50 mL), remove the supernatant, and dry the residue under reduced pressure at low temperature to afford a dark-red solid-namely, the targeted epirubicin drug **Apt TfRA3-Apt AS1411-Apt A15-DNA-epirubicin.**

(4)Determination of Loading (Drug-to-Carrier Ratio):

1. Prepare epirubicin-PBS standard solutions of known concentration: 2 μM, 4 μM, 6 μM, 8 μM, and 10 μM, 100 μL each;

2. Dissolve **Apt TfRA3-Apt AS1411-Apt A15-DNA-epirubicin** in 100 μL PBS;

3. Place the reference standard solutions and the sample in a PCR plate, heat at 85 °C for 5 min, then cool to room temperature;

4. Using a microplate reader, measure the absorbance of epirubicin at 492 nm, construct a standard curve, and calculate the molar concentration of epirubicin in the loaded product;

5. Using a spectrophotometer, measure DNA absorbance at 260 nm to determine the mass concentration of the DNA carrier in each sample;

6. Calculate the loading value based on the measured epirubicin molar concentration and DNA-carrier mass concentration. For **Apt TfRA3-Apt AS1411-Apt A15-DNA-epirubicin,** the maximum theoretical loading number per carrier molecule is approximately **27,** and the measured average loading number is **25.**

Example 10

In this example, the targeted epirubicin drug **CPG2006 (DNA)-Apt CD40-Apt PD-L1-DNA-epirubicin** is synthesized.

**Step 1:** Synthesize the **CPG2006 (DNA)-Apt CD40-Apt PD-L1-DNA** targeting carrier. The sequences of the single strands are shown in **Table 10** below:

Table 10

| Name | Sequence (sense, 5'→3') | Modification(s) |
|---|---|---|
| Sequence a (SEQ ID NO:301) | **5'-**TCGTCGTTTTGTCGTTTTGTCGTTTTTTT**GCGACGCCCACG AGCGTTCCGGGAGAGGAGC-3'** | See notes under Methods below |
| Sequence b (SEQ ID NO:302) | **5'-**CCAACGAGTAGGCGATAGCGCGTGGTTTTT**GCTCCTCTCC CGGTTCGCCGCGAGCCGCG-3'** | See notes under Methods below |
| Sequence c (SEQ ID NO:303) | **5'-**ACGGGCCACATCAACTCATTGATAGACAATGCGTCCACTGC CCGTTTTTT**CGCGGCTCGCGGCCATAGCCGTGGGCGTCGC-3 '** | See notes under Methods |
| | | below |
| **Note:** For sequence a, every internucleotide linkage between adjacent nucleotides in the region spanning positions 1-24 from the 5' terminus, and every internucleotide linkage between adjacent nucleotides at positions 1-3 from the 3' terminus, is phosphorothioate-modified (PS).Forsequences b and c, every internucleotide linkage between adjacent nucleotides at the first three positions from both the 5' and 3' termini is phosphorothioate-modified (PS). | | |

1. Materials and Reagents:

(1)Sequence a;

(2)Sequence b;

(3)Sequence c;

(4)DEPC water (diethyl-pyrocarbonate-treated water);

(5)TBM buffer (Tris-borate-magnesium).

2. Experimental Method:

Dissolve the sequences a, b and c separately in DEPC water at a molar ratio of 1: 1: 1. After mixing, adjust the reaction mixture to pH 7.Heat to 99 °C and maintain for 5 min; then cool at 2 °C/min to 65 °C and hold (anneal) for 15 min; subsequently cool at 2 °C/min to 4 °C.

Load the product onto an 8% (m/v) native PAGE gel and purify the carrier by electrophoresis in TBM buffer at 4 °C

and 100 V.Excise the desired band and elute in DNA elution buffer at 37 °C, then precipitate with ethanol overnight and dry under reduced pressure at low temperature to afford the self-assembled product-the CPG2006 (DNA)-Apt CD40-Apt PD-L1-DNA targeting carrier.

Step 2: Loading of Epirubicin

1. Materials and Reagents:

(1)Targeting carrier **CPG2006 (DNA)-Apt CD40-Apt PD-L1-DNA** prepared in Step 1;

(2)DEPC water;

(3)PBS buffer (pH 6.6);

(4)4% paraformaldehyde (aq.);

(5)Epirubicin hydrochloride;

(6)Chloroform;

(7)Isopropanol.

2. Experimental Method:

(1)Accurately weigh epirubicin hydrochloride (0.79 mg, 1.354 μmol) and dissolve in DEPC water (1.0 mL) and PBS buffer (1.25 mL) in an ice-water bath.Add 4% aqueous paraformaldehyde (0.25 mL) with mixing.Combine the entire mixture with the **CPG2006 (DNA)-Apt CD40-Apt PD-L1-DNA** targeting carrier (1 mg), mix thoroughly, and react for 72 h at 4 °C under light-protection, maintaining the reaction pH at 7.

(2)Wash the reaction mixture with chloroform (10 mL × 3). To the aqueous phase add isopropanol (25 mL), mix, and keep at 4 °C protected from light for 4 h to allow complete precipitation.Centrifuge (4, 000 rpm), decant the supernatant, wash the solid again with isopropanol (50 mL), remove the supernatant, and dry the residue under reduced pressure at low temperature to afford a dark-red solid-namely, the targeted epirubicin drug **CPG2006 (DNA)-Apt CD40-Apt PD-L1-DNA-epirubicin.**

(4)Determination of Loading (Drug-to-Carrier Ratio):

1. Prepare epirubicin-PBS standard solutions of known concentration: 2 μM, 4 μM, 6 μM, 8 μM, and 10 μM, 100 μL each;

2. Dissolve **CPG2006 (DNA)-Apt CD40-Apt PD-L1-DNA-epirubicin** in 100 μL PBS;

3. Place the reference standard solutions and the sample in a PCR plate, heat at 85 °C for 5 min, then cool to room temperature;

4. Using a microplate reader, measure epirubicin absorbance at 492 nm, construct a standard curve, and calculate the molar concentration of epirubicin in the loaded product;

5. Using a spectrophotometer, measure DNA absorbance at 260 nm to determine the mass concentration of the DNA carrier in each sample;

6. Calculate the loading value based on the measured epirubicin molar concentration and DNA-carrier mass concentration. For **CPG2006 (DNA)-Apt CD40-Apt PD-L1-DNA-epirubicin,** the maximum theoretical loading number per carrier molecule is approximately **25,** and the measured average loading number is **21.**

Example 11

In this example, the targeted epirubicin drug **2*CPG2006 (DNA)-Apt CD40-Apt PD-L1-Apt C12-DNA-epirubicin** is synthesized.

**Step 1:** Synthesize the **2*CPG2006 (DNA)-Apt CD40-Apt PD-L1-Apt C12-DNA** targeting carrier. The sequences of the single strands are shown in **Table 11** below:

Table 11

| Name | Sequence (sense, 5'-3') |
|---|---|
| Sequence a (SEQ ID NO: 304) | **5'**-TCGTCGTTTTGTCGTTTTGTCGTTTTTT**GACGCCCACGAGCGTTCCGGGAGAGG-3'** |
| Sequence b (SEQ ID NO: 305) | **5'**-CCAACGAGTAGGCGATAGCGCGTGGTTTTT**CCTCTCCCGGTTCGCCGCGAGCCTGTAGCACGGTGGC**-**3'** |
| Sequence c (SEQ ID NO: 306) | **5'**-TCGTCGTTTTGTCGTTTTGTCGTTTTTT**GGCTCGCGGCCATAGCCGTGGGCGTCTGCTGCTGCTGCTG-3'** |
| Sequence d (SEQ ID NO: 307) | **5'**-ACGGGCCACATCAACTCATTGATAGACAATGCGTCCACTGCCCGTTTTTTGCCACCGTGCTACA-**3'** |
| Sequence e (SEQ ID NO: 308) | **5'**-GTGGATTGTTGTGTTCTGTTGGTTTTTGTGTTGTCTTTTTCAGCAGCAGCAGCA-**3'** |
| **Note:** For sequences a and c, every internucleotide linkage between adjacent nucleotides at positions 1-24 from the 5' terminus is phosphorothioate-modified (PS).For sequences b, d, and e, every internucleotide linkage between adjacent nucleotides at positions 1-3 from the 5' terminus is phosphorothioate-modified (PS). | |

1. Materials and Reagents:

(1)Sequence a;

(2)Sequence b;

(3)Sequence c;

(4)Sequence d;

(5)DEPC water (diethyl-pyrocarbonate-treated water);

(6)Sequence e;

(7)TBM buffer (Tris-borate-magnesium).

2. Experimental Method:

Dissolve the sequences a, b, c, d, and e separately in DEPC water at a molar ratio of 1: 1: 1: 1: 1. After mixing, adjust the reaction mixture to pH 7.Heat to 99 °C and maintain for 5 min; then cool at 2 °C/min to 65 °C and hold (anneal) for 15 min; subsequently cool at 2 °C/min to 4 °C.

Load the product onto an 8% (m/v) native PAGE gel and purify the carrier by electrophoresis in TBM buffer at 4 °C and 100 V.Excise the desired band and elute in DNA elution buffer at 37 °C, then precipitate with ethanol overnight and dry under reduced pressure at low temperature to afford the self-assembled product-the 2*CPG2006 (DNA)-Apt CD40L-Apt PD-L1-Apt C12-DNA targeting carrier.

Step 2: Loading of Epirubicin

1. Materials and Reagents:

(1)Targeting carrier **2*CPG2006 (DNA)-Apt CD40-Apt PD-L1-Apt C12-DNA** prepared in Step 1;

(2)DEPC water;

(3)PBS buffer (pH 6.6);

(4)4% paraformaldehyde (aq.);

(5)Epirubicin hydrochloride;

(6)Chloroform;

(7)Isopropanol.

2. Experimental Method:

(1)Accurately weigh epirubicin hydrochloride (0.79 mg, 1.354 μmol) and dissolve in DEPC water (1.0 mL) and PBS buffer (1.25 mL) in an ice-water bath.Add 4% aqueous paraformaldehyde (0.25 mL) with mixing.Combine the entire mixture with the **2*CPG2006 (DNA)-Apt CD40-Apt PD-L1-Apt C12-DNA** targeting carrier (1 mg), mix thoroughly, and react for 72 h at 4 °C under light-protection, maintaining the reaction pH at 7.

(2)Wash the reaction mixture with chloroform (10 mL × 3). To the aqueous phase add isopropanol (25 mL), mix, and keep at 4 °C protected from light for 4 h to allow complete precipitation.Centrifuge (4, 000 rpm), decant the supernatant, wash the solid again with isopropanol (50 mL), remove the supernatant, and dry the residue under reduced pressure at low temperature to afford a dark-red solid-namely, the targeted epirubicin drug **2*CPG2006 (DNA)-Apt CD40-Apt PD-L1-Apt C12-DNA-epirubicin.**

(4)Determination of Loading (Drug-to-Carrier Ratio):

1. Prepare epirubicin-PBS standard solutions of known concentration: 2 μM, 4 μM, 6 μM, 8 μM, and 10 μM, 100 μL each;

2. Dissolve **2*CPG2006 (DNA)-Apt CD40-Apt PD-L1-Apt C12-DNA-epirubicin** in 100 μL PBS;

3. Place the reference standard solutions and the sample in a PCR plate, heat at 85 °C for 5 min, then cool to room temperature;

4. Using a microplate reader, measure the absorbance of epirubicin at 492 nm, construct a standard curve, and calculate the molar concentration of epirubicin in the loaded product;

5. Using a spectrophotometer, measure DNA absorbance at 260 nm to determine the mass concentration of the DNA carrier in each sample;

6. Calculate the loading value based on the measured epirubicin molar concentration and DNA-carrier mass concentration. For **2*CPG2006 (DNA)-Apt CD40-Apt PD-L1-Apt C12-DNA-epirubicin,** the maximum theoretical loading number per carrier molecule is approximately **36,** and the measured average loading number is **30.**

Example 12

In this example, the targeted epirubicin drug **CPG2006 (DNA)-Apt C12-CD47 siRNA-Apt PD-L1-PD-L1 siRNA-**

**DNA-epirubicin** is synthesized.

**Step 1:** Synthesize the **CPG2006 (DNA)-Apt C12-CD47 siRNA-Apt PD-L1-PD-L1 siRNA-DNA** targeting carrier. The sequences of the single strands are shown in **Table 12** below:

Table 12

| Name | Sequence (sense, 5'-3') |
|---|---|
| Sequence a (SEQ ID NO: 309) | **5'**-TCGTCGTTTTGTCGTTTTGTCGTTTTTTT**GCGACGCCCACGAGCGTTCCGGGAGAGGAGC**-3' |
| Sequence b (SEQ ID NO: 310) | **5'**-GGUGAUUACCCAGAGAUAUUU**GCTCCTCTCCCGGTTCGCCGCGAGCCGCG-3'** |
| Sequence c (SEQ ID NO: 311) | **5'**-CCAGCACACUGAGAAUCAAUU**CGCGGCTCGCGGCCATAGCCGTGGGCGTCGC**-**3'** |
| Sequence d (SEQ ID NO: 312) | **5'**-GTGGATTGTTGTGTTCTGTTGGTTTTTGTGTTGTCTTTTTAUAUCUCUGGGUAAUCACCUU-**3'** |
| Sequence e (SEQ ID NO: 313) | **5'**-GCCCCAGTTATGCTTTCCCCCTCTGTCTCTTTGTTTTTUUGAUUCUCAGUGUGCUGGUU-**3'** |
| Note: For sequence a, phosphorothioate (PS) modifications are introduced at the internucleotide linkages between adjacent nucleotides at positions 1-24 from the 5' terminus, and at positions 1-3 from the 3' terminus; for sequence b, PS modifications are introduced at the internucleotide linkages between adjacent nucleotides at positions 1-3 from both the 5' and 3' termini, and additionally at positions 19-21 from the 5' terminus; for sequence c, PS modifications are introduced at the internucleotide linkages between adjacent nucleotides at positions 1-3 from both the 5' and 3' termini, and additionally at positions 19-21 from the 5' terminus; for sequence d, PS modifications are introduced at the internucleotide linkages between adjacent nucleotides at positions 1-3 from both the 5' and 3' termini, and additionally at positions 19-21 from the 3' terminus; for sequence e, PS modifications are introduced at the internucleotide linkages between adjacent nucleotides at positions 1-3 from both the 5' and 3' termini, and additionally at positions 19-21 from the 3' terminus.In the underlined RNA sequences, cytidine and uridine residues bear 2'-fluoro (2'-F) modifications. | |

1. Materials and Reagents:

(1)Sequence a;

(2)Sequence b;

(3)Sequence c;

(4)Sequence d;

(5)Sequence e;

(6)DEPC water;

(7)TBM (Tris-borate-magnesium) buffer.

2. Experimental Method:

Accurately weigh epirubicin hydrochloride (0.79 mg, 1.354 μmol) and dissolve in DEPC-treated water (1.0 mL)

and PBS buffer (1.25 mL) in an ice-water bath.Add 4% paraformaldehyde (0.25 mL) and mix thoroughly.Combine the entire mixture with the CPG2006 (DNA)-Apt C12-CD47 siRNA-Apt PD-L1-PD-L1 siRNA-DNA targeted carrier (1 mg), mix well, and allow to react at 4 °C for 72 h protected from light, maintaining the reaction at pH 7.

Wash the reaction mixture with chloroform (10 mL × 3). To the aqueous phase add 25 mL isopropanol, mix, and allow the product to precipitate fully at 4 °C protected from light (4 h).Centrifuge (4, 000 rpm), decant the supernatant, wash the solid product again with isopropanol (50 mL), remove the supernatant, and evaporate the residue to dryness under reduced pressure at low temperature to obtain a dark-red solid, i.e., the targeted epirubicin conjugate **CPG2006 (DNA)-Apt C12-CD47 siRNA-Apt PD-L1-PD-L1 siRNA-DNA-epirubicin.**

Step 2: Loading of Epirubicin

1. Materials and Reagents:

(1)The CPG2006 (DNA)-Apt C12-CD47 siRNA-Apt PD-L1-PD-L1 siRNA-DNA targeted carrier prepared in Step 1;

(2)DEPC-treated water; .

(3)PBS buffer (pH 6.6);

(4)4% paraformaldehyde;

(5)Epirubicin hydrochloride;

(6)Chloroform;

(7)Isopropanol.

2. Experimental Method:

(1)Accurately weigh epirubicin hydrochloride (0.79 mg, 1.354 μmol) and dissolve in DEPC-treated water (1.0 mL) and PBS buffer (1.25 mL) in an ice-water bath.Add 4% paraformaldehyde (0.25 mL) and mix thoroughly. Combine the entire mixture with the **CPG2006 (DNA)-Apt C12-CD47 siRNA-Apt PD-L1-PD-L1 siRNA-DNA** targeted carrier (1 mg), mix well, and allow the reaction to proceed at 4 °C for 72 h protected from light, maintaining the reaction pH at 7.

(2)Wash the reaction mixture with chloroform (10 mL × 3).Add 25 mL isopropanol to the aqueous phase, mix, and allow the product to precipitate completely at 4 °C protected from light (4 h).Centrifuge (4, 000 rpm), decant the supernatant, wash the solid product again with isopropanol (50 mL), remove the supernatant, and evaporate the residue to dryness under reduced pressure at low temperature to obtain a dark-red solid, i.e., the targeted epirubicin product **CPG2006 (DNA)-Apt C12-CD47 siRNA-Apt PD-L1-PD-L1 siRNA-DNA-epirubicin.**

(4)Determination of Loading Ratio:

1. Prepare epirubicin-PBS standard solutions at known concentrations: 2 μM, 4 μM, 6 μM, 8 μM, and 10 μM, 100 μL each;

2. Dissolve CPG2006 (DNA)-Apt C12-CD47 siRNA-Apt PD-L1-PD-L1 siRNA-DNA-epirubicin in 100 μL PBS;

3. Place the reference standard solutions and the product solution in a PCR plate, heat at 85 °C for 5 min, then cool to room temperature;

4. Measure the absorbance of epirubicin at 492 nm using a microplate reader, plot a standard curve, and calculate the molar concentration of epirubicin in the loaded product;

5. Measure the absorbance of DNA at 260 nm with a spectrophotometer to obtain the mass concentration of the

DNA carrier in each sample;

6. Calculate the loading ratio based on the measured molar concentration of epirubicin and the mass concentration of the DNA carrier. The maximum theoretical loading number of epirubicin is approximately 30 (per DNA carrier), and the measured average loading number is 26.

Example 13

In this example, the targeted epirubicin product **A-miR-21-TGF-β1 siRNA-Apt TfRA4-Apt PD-L1-DNA-epirubicin** is synthesized.

Step 1: Synthesis of the A-miR-21-TGF-β1 siRNA-Apt TfRA4-Apt PD-L1-DNA targeted carrier. The sequences of the individual single strands are set forth in Table 13:

Table 13

| Name | Sequence (sense, 5'→3') |
|---|---|
| Sequence a (SEQ ID NO: 314) | **5'-**GCGTGGTACCACGCTTTTT**GCGGCGCCCACGAGCGTTCCGGGAGC-3'** |
| Sequence b (SEQ ID NO: 315) | **5'-**ACGGGCCACATCAACTCATTGATAGACAATGCGTCCACTGCCCGTTTTTT**GCTCCTCTCCCGGTTCGCCGCGAGCCGCG-3'** |
| Sequence c (SEQ ID NO: 316) | **5'-CGCGGCTCGCGGCCATAGCCGTGGGCGTCGC**AAUCAUAGAUGGCGUUGUUGCTT**-3'** |
| Sequence d (SEQ ID NO: 317) | 5'-GATAAGCTGATAAGCTAAGCAACAACGCCAUCUAUGATT-3' |
| **Note:** For sequence b, phosphorothioate (PS) modifications are introduced at the internucleoside phosphate linkages between the adjacent nucleotides at positions 1-3 counted from the 5' terminus; for sequence c, PS modifications are introduced at the internucleoside phosphate linkages between the adjacent nucleotides at positions 29-31 counted from the 5' terminus, and at positions 1-3 counted from the 3' terminus; for sequence d, PS modifications are introduced at the internucleoside phosphate linkages between the adjacent nucleotides at positions 1-3 counted from the 3' terminus, and at positions 19-21 counted from the 3' terminus.In the underlined RNA segments, cytidine and uridine residues bear 2'-fluoro (2'-F) modifications. | |

1. Materials and Reagents:

(1)Sequence a;

(2)Sequence b;

(3)Sequence c;

(4)Sequence d;

(5)DEPC-treated water;

(6)TBM (Tris-borate-magnesium) buffer.

2. Experimental Method:

Dissolve sequences a, b, c, and d separately in DEPC-treated water at a molar ratio of 1: 1: 1: 1; after mixing, adjust the reaction solution to pH 7.Heat the mixture to 99 °C and maintain for 5 min; cool at 2 °C/min to 65 °C and hold for 15 min, then continue cooling at 2 °C/min to 4 °C.

Load the product onto an 8% (w/v) native PAGE gel and purify the carrier by electrophoresis at 100 V in TBM buffer at 4 °C.Excise the target band and elute in DNA elution buffer at 37 °C, then precipitate with ethanol overnight. Remove solvent under reduced pressure at low temperature to afford the self-assembled product, namely **A-miR-21-TGF-β1 siRNA-Apt TfRA4-Apt PD-L1-DNA targeted carrier.**

Step 2: Loading of Epirubicin

1. Materials and Reagents:

(1)The **A-miR-21-TGF-β1 siRNA-Apt TfRA4-Apt PD-L1-DNA** targeted carrier prepared in Step 1;

(2)DEPC-treated water;

(3)PBS buffer (pH 6.6);

(4)4% paraformaldehyde;

(5)Epirubicin hydrochloride;

(6)Chloroform;

(7)Isopropanol.

2. Experimental Method:

(1)Accurately weigh epirubicin hydrochloride (0.79 mg, 1.354 μmol) and dissolve in DEPC-treated water (1.0 mL) and PBS buffer (1.25 mL).While cooling in an ice-water bath, add 4% paraformaldehyde (0.25 mL) and mix thoroughly. Combine the entire mixture with the **A-miR-21-TGF-β1 siRNA-Apt TfRA4-Apt PD-L1-DNA** targeted carrier (1 mg), mix well, and allow the reaction to proceed at 4 °C for 72 h protected from light, maintaining the reaction at pH 7.

(2)Wash the reaction mixture with chloroform (10 mL × 3).Add 25 mL isopropanol to the aqueous phase, mix, and allow the product to precipitate completely at 4 °C protected from light (4 h).Centrifuge (4, 000 rpm), decant the supernatant, wash the solid again with isopropanol (50 mL), remove the supernatant, and evaporate the residue to dryness under reduced pressure at low temperature to obtain a dark-red solid, i.e., the targeted epirubicin product **A-miR-21-TGF-β1 siRNA-Apt TfRA4-Apt PD-L1-DNA-epirubicin.**

(4)Determination of Loading Ratio:

1. Prepare epirubicin-PBS standard solutions at 2 μM, 4 μM, 6 μM, 8 μM, and 10 μM, 100 μL each;

2. Dissolve **A-miR-21-TGF-β1 siRNA-Apt TfRA4-Apt PD-L1-DNA-epirubicin** in 100 μL PBS;

3. Place the reference standard solutions and the sample in a PCR plate, heat at 85 °C for 5 min, then cool to room temperature;

4. Measure the absorbance of epirubicin at 492 nm using a microplate reader, plot a standard curve, and calculate the molar concentration of epirubicin in the loaded product;

5. Measure DNA absorbance at 260 nm with a spectrophotometer to obtain the mass concentration of the DNA carrier in each sample;

6. Calculate the loading ratio from the measured molar concentration of epirubicin and the mass concentration of the DNA carrier. The maximum theoretical loading number of epirubicin for the above targeted product A-miR-21-TGF-β siRNA-Apt TfRA4-Apt PD-L1-DNA-epirubicin is approximately 33 (drug molecules per DNA carrier), and

the measured average loading number is 28.

Example 14

In this example, the targeted epirubicin product Apt IL-4Rα-CD47 siRNA-Apt TfRA4-PD-L1 siRNA-Apt GPC-1-DNA-epirubicin is synthesized.

Step 1: Synthesis of the Apt IL-4Rα-CD47 siRNA-Apt TfRA4-PD-L1 siRNA-Apt GPC-1-DNA targeted carrier. The sequences of the individual single strands are set forth in Table 14:

Table 14

| Name | Sequence (sense, 5'-3') |
|---|---|
| Sequence a (SEQ ID NO: 318) | **5'-**GCGTGGTACCACGCTTTTT**GCGGCGCCCACGAGCGTTCCGGGAGAGGAGC**UUUGAUUCUCAGUGUGCUGGUU**-3'** |
| Sequence b (SEQ ID NO: 319) | **5'-**CCAGCACACUGAGAAUCAAUU**GCTCCTCTCCCGGTTCGCCGCCAGCCGCGG-3'** |
| Sequence c (SEQ ID NO: 320) | **5'-AACGGAGTGTGGCTAACTCGATTTTTGGCGGCTGGCGGCCATAGCCGTGGGCGCCGC**UGGUGAUUACCCAGAGAUAUUU**-3'** |
| Sequence d (SEQ ID NO: 321) | **5'-**AAAAAGCAACAGGGUGCUCCAUGCGCAUGGAACCUGCGCGAAAAUAUCUCUGGGUAAUCACCUU**-3'** |
| **Note:** In sequence a, the internucleoside phosphate linkages between adjacent nucleotides at positions 1-3 from the 3' terminus and at positions 19-21 from the 3' terminus are phosphorothioate (PS) linkages; in sequence b, the internucleoside phosphate linkages between adjacent nucleotides at positions 1-3 from the 5' terminus and at positions 19-21 from the 5' terminus are PS linkages; in sequence c, the internucleoside phosphate linkages between adjacent nucleotides at positions 1-3 from the 3' terminus and at positions 19-21 from the 3' terminus are PS linkages; in sequence d, the internucleoside phosphate linkages between adjacent nucleotides at positions 1-4 from the 5' terminus, and at positions 1-3 and 19-21 from the 3' terminus, are PS linkages.In sequences a, b, and c, the underlined RNA segments contain 2'-fluoro (2'-F) substitutions on cytidine and uridine residues.In sequence d, nucleotides 1-44 incorporate 2'-fluoro substitutions on cytidine and uridine residues and 2'-O-methyl (2'-OMe) substitutions on adenosine and guanosine residues; nucleotides 45-64 incorporate 2'-fluoro substitutions on cytidine and uridine residues. | |

1. Materials and Reagents:

(1)Sequence A;

(2)Sequence B;

(3)Sequence C;

(4)Sequence D;

(5)DEPC-treated water;

(6)TBM (Tris-borate-magnesium) buffer.

2. Experimental Method:

Dissolve sequences a, b, c, and d separately in DEPC-treated water at a molar ratio of 1: 1: 1: 1; after mixing, adjust the reaction solution to pH 7.Heat the mixture to 99 °C and maintain for 5 min; cool at 2 °C/min to 65 °C and hold for 15

min, then continue cooling at 2 °C/min to 4 °C.

Load the product onto an 8% (w/v) native PAGE gel and purify the carrier by electrophoresis at 100 V in TBM buffer at 4 °C.Excise the target band and elute in DNA elution buffer at 37 °C, then precipitate with ethanol overnight.Evaporate to dryness under reduced pressure at low temperature to obtain the self-assembled product, namely Apt IL-4Rα-CD47 siRNA-Apt TfRA4-PD-L1 siRNA-Apt GPC-1-DNA targeted carrier.

Step 2: Loading of Epirubicin

1. Materials and Reagents:

(1)The **Apt IL-4Rα-CD47 siRNA-Apt TfRA4-PD-L1 siRNA-Apt GPC-1-DNA** targeted carrier prepared in Step 1;

(2)DEPC-treated water;

(3)PBS buffer (pH 6.6);

(4)4% (w/v) paraformaldehyde;

(5)Epirubicin hydrochloride;

(6)Chloroform;

(7)Isopropanol.

2. Experimental Method:

(1)Accurately weigh epirubicin hydrochloride (0.79 mg, 1.354 μmol) and dissolve in DEPC-treated water (1.0 mL) and PBS buffer (1.25 mL).While cooling in an ice-water bath, add 4% paraformaldehyde (0.25 mL) and mix thoroughly. Combine the entire mixture with the Apt IL-4Rα-CD47 siRNA-Apt TfRA4-PD-L1 siRNA-Apt GPC-1-DNA targeted carrier (1 mg), mix well, and allow the reaction to proceed at 4 °C for 72 h protected from light, maintaining the reaction pH at 7.

(2)Wash the reaction mixture with chloroform (10 mL × 3).Add 25 mL isopropanol to the aqueous phase, mix, and allow the product to precipitate completely at 4 °C protected from light (4 h).Centrifuge (4, 000 rpm), decant the supernatant, wash the solid again with isopropanol (50 mL), remove the supernatant, and evaporate the residue to dryness under reduced pressure at low temperature to obtain a dark-red solid, i.e., the targeted epirubicin product Apt IL-4Rα-CD47 siRNA-Apt TfRA4-PD-L1 siRNA-Apt GPC-1-DNA-epirubicin.

(4)Determination of Loading Ratio:

1. Prepare epirubicin-PBS standard solutions at 2 μM, 4 μM, 6 μM, 8 μM, and 10 μM, 100 μL each;

2. Dissolve Apt IL-4Rα-CD47 siRNA-Apt TfRA4-PD-L1 siRNA-Apt GPC-1-DNA-epirubicin in 100 μL PBS;

3. Place the reference standard solutions and the sample in a PCR plate, heat at 85 °C for 5 min, then cool to room temperature;

4. Measure the absorbance of epirubicin at 492 nm using a microplate reader, plot a standard curve, and calculate the molar concentration of epirubicin in the loaded product;

5. Measure the absorbance of DNA at 260 nm with a spectrophotometer to obtain the mass concentration of the DNA carrier in each sample;

6. Calculate the loading ratio from the measured molar concentration of epirubicin and the mass concentration of

the DNA carrier. The maximum theoretical loading number of epirubicin for the above targeted product is approximately 32 (epirubicin molecules per DNA carrier), and the measured average loading number is 27.

Example 15

In this example, the targeted gemcitabine product **Apt AS1411-Apt EGFR-Apt A15-DNA-gemcitabine** is synthesized.

Synthesis of Apt AS1411-Apt EGFR-Apt A15-DNA-gemcitabine. The sequences of the individual single strands are set forth in Table 15:

Table 15

| Name | Sequence (sense, 5'-3') |
|---|---|
| Sequence a | **5'-** SEQ ID NO: 103 - JJJJJJ - **SEQ ID NO: 15** - JJJJJJ-**3**' |
| Sequence b | **5'-** SEQ ID NO: 139 - JJJJJJ - **SEQ ID NO: 16-** JJJJJJ-**3**' |
| Sequence c | **5'-** SEQ ID NO: 102- JJJJJJ - **SEQ ID NO: 14-** JJJJJJ-**3**' |
| Note: In the foregoing, "J" denotes gemcitabine.During oligonucleotide sequence synthesis, J is directly incorporated into the sequence by nucleobase substitution and nucleobase extension (appendage), such that a single DNA targeted carrier bears 36 covalently attached gemcitabine moieties. | |

1. Materials and Reagents:

(1)Sequence A;

(2)Sequence B;

(3)Sequence C;

(4)DEPC-treated water;

(5)TBM (Tris-borate-magnesium) buffer.

2. Experimental Method:

Dissolve sequences a, b, and c separately in DEPC-treated water at a molar ratio of 1: 1: 1; after mixing, adjust the reaction solution to pH 7.Heat the mixture to 99 °C and maintain for 5 min; cool at 2 °C/min to 65 °C and hold for 15 min, then continue cooling at 2 °C/min to 4 °C.

Load the product onto an 8% (w/v) native PAGE gel and purify the carrier by electrophoresis at 100 V in TBM buffer at 4 °C.Excise the target band and elute in DNA elution buffer at 37 °C, then precipitate with ethanol overnight.Evaporate to dryness under reduced pressure at low temperature to obtain the self-assembled product, namely the targeted gemcitabine product Apt AS1411-Apt EGFR-Apt A15-DNA-gemcitabine.

Example 16

In this example, a dual-drug targeted product, Apt MUC1-Apt AS1411-Apt GPC1-gemcitabine-DNA-epirubicin, is synthesized.

Step 1: Synthesis of the Apt MUC1-Apt AS1411-Apt GPC1-gemcitabine-DNA targeted carrier. The sequences of the individual single strands are set forth in Table 16:

Table 16

| Name | Sequence (sense, 5'-3') |
|---|---|
| Sequence a | **5'-** SEQ ID NO: 160 - JJJJJJ - **SEQ ID NO: 26** - JJJJJJ-**3**' |
| Sequence b | **5'-** SEQ ID NO: 103 - JJJJJJ - **SEQ ID NO: 11** - JJJJJJ-**3**' |

(continued)

| Name | Sequence (sense, 5'-3') |
|---|---|
| Sequence c | **5'-** SEQ ID NO: 145- JJJJJJ - **SEQ ID NO: 27** - JJJJJJ-**3**' |

Note: In the foregoing, "J" denotes gemcitabine.During oligonucleotide synthesis, J is directly incorporated into the sequence by nucleobase substitution and nucleobase extension, and the internucleoside phosphate linkages flanking each incorporated gemcitabine (J) unit are phosphorothioate (PS) linkages.Accordingly, a single DNA targeted carrier bears 36 gemcitabine moieties.In sequences a, b, and c, the internucleoside phosphate linkages between adjacent nucleotides at positions 1-2 counted from the 5' terminus are PS linkages.

1. Materials and Reagents:

(1)Sequence A;

(2)Sequence B;

(3)Sequence C;

(4)DEPC-treated water;

(5)TBM (Tris-borate-magnesium) buffer.

2. Experimental Method:

Dissolve sequences a, b, and c separately in DEPC-treated water at a molar ratio of 1: 1: 1; after mixing, adjust the reaction solution to pH 7.Heat the mixture to 99 °C and maintain for 5 min; cool at 2 °C/min to 65 °C and hold for 15 min, then continue cooling at 2 °C/min to 4 °C.

Load the product onto an 8% (w/v) native PAGE gel and purify the carrier by electrophoresis at 100 V in TBM buffer at 4 °C.Excise the target band and elute in DNA elution buffer at 37 °C, then precipitate with ethanol overnight.Evaporate to dryness under reduced pressure at low temperature to obtain the self-assembled product, namely the Apt MUC1-Apt AS1411-Apt GPC1-gemcitabine-DNA targeted carrier.

Step 2: Loading of Epirubicin

1. Materials and Reagents:

(1)The Apt MUC1-Apt AS1411-Apt GPC1-gemcitabine-DNA targeted carrier prepared in Step 1;

(2)DEPC-treated water;

(3)PBS buffer (pH 6.6);

(4)4% (w/v) paraformaldehyde;

(5)Epirubicin hydrochloride;

(6)Chloroform;

(7)Isopropanol.

2. Experimental Method:

(1)Accurately weigh epirubicin hydrochloride (0.79 mg, 1.354 μmol) and dissolve in DEPC-treated water (1.0 mL) and PBS buffer (1.25 mL).While cooling in an ice-water bath, add 4% paraformaldehyde (0.25 mL) and mix thoroughly. Combine the entire mixture with the Apt MUC1-Apt AS1411-Apt GPC1-gemcitabine-DNA targeted carrier (1 mg), mix well, and allow the reaction to proceed at 4 °C for 72 h protected from light, maintaining the reaction pH at 7.

(2)Wash the reaction mixture with chloroform (10 mL × 3).Add 25 mL isopropanol to the aqueous phase, mix, and allow the product to precipitate completely at 4 °C protected from light (4 h).Centrifuge (4, 000 rpm), decant the supernatant, wash the solid again with isopropanol (50 mL), remove the supernatant, and evaporate the residue to dryness under reduced pressure at low temperature to obtain a dark-red solid, i.e., the targeted product Apt MUC1-Apt AS1411-Apt GPC1-gemcitabine-DNA-epirubicin.

(4)Determination of Loading Ratios:

1. Prepare epirubicin-PBS standard solutions at 2 μM, 4 μM, 6 μM, 8 μM, and 10 μM, 100 μL each;

2. Dissolve Apt MUC1-Apt AS1411-Apt GPC1-gemcitabine-DNA-epirubicin in 100 μL PBS;

3. Place the reference standard solutions and the sample in a PCR plate, heat at 85 °C for 5 min, then cool to room temperature;

4. Measure the absorbance of epirubicin at 492 nm using a microplate reader, plot a standard curve, and calculate the molar concentration of epirubicin in the loaded product;

5. Measure DNA absorbance at 260 nm with a spectrophotometer to obtain the mass concentration of the DNA carrier in each sample;

6. Calculate the loading ratios from the measured molar concentration of epirubicin and the mass concentration of the DNA carrier. For the above targeted product, the maximum theoretical loading number of epirubicin is approximately 36 (epirubicin molecules per DNA carrier), and the maximum theoretical loading number of gemcitabine is 28; the measured average loading numbers are 30 for epirubicin and 28 for gemcitabine.

Example 17

In this example, the targeted 5-fluorouracil product Apt AS1411-Apt EGFR-Apt A15-RNA-5'-fluorouracil is synthesized.

Synthesis of Apt AS1411-Apt EGFR-Apt A15-RNA-5'-fluorouracil (targeted product). The sequences of the individual single strands are set forth in Table 17:

Table 17

| Name | Sequence (sense, 5'-3') |
|---|---|
| Sequence a (SEQ ID NO: 322) | **5'-**GGTGGTGGTGGTTGTGGTGGTGGTGGNNNNNN**GCGGCGCCCACGAGCGUUCCGGGAGAGGCC**NNNNNN**-3'** |
| Sequence b (SEQ ID NO: 323) | **5'-**<u>GCCUUAGUAACGUGCUUUGAUGUCGAUUCGACAGGAGGC</u>NNNNNN**GGCCUCUCCCGGUUCGCCGCCAGCCGCC**NNNNNN**-3'** |

(continued)

| Name | Sequence (sense, 5'-3') |
|---|---|
| Sequence c (SEQ ID NO: 324) | **5'-**CCCUCCUACAUAGGGNNNNNN**GGCGGCUGGCGGCCAUAGCC GUGGGCGCCGC**NNNNNN**-3'** |
| Note: In the foregoing, "N" denotes 5-fluorouracil (5-FU).During oligonucleotide synthesis, N is directly incorporated into the sequence by nucleobase substitution and nucleobase extension; the internucleoside phosphate linkages between adjacent 5-fluorouracil (N) residues are phosphorothioate (PS) linkages; thus, a single DNA targeted carrier bears 36 5-fluorouracil residues.In sequence a, the internucleoside phosphate linkage between nucleotides 1-2 counted from the 5' terminus is a PS linkage; in sequence b, the internucleoside phosphate linkages between nucleotides 1-2 at each of the 5' and 3' termini are PS linkages; in sequence c, the internucleoside phosphate linkage between nucleotides 1-2 counted from the 5' terminus is a PS linkage.In the RNA segments, cytidine and uridine bear 2'-fluoro (2'-F) substitutions, and adenosine and guanosine bear 2'-O-methyl (2'-OMe) substitutions; PS linkages are introduced between nucleotides 1-2 at both the 5' and 3' termini. The underlined RNA sequences indicate regions bearing the C/U-2'-F and A/G-2'-OMe substitutions as specified. | |

1. Materials and Reagents:

(1)Sequence a;

(2)Sequence b;

(3)Sequence c;

(4)DEPC-treated water;

(5)TBM (Tris-borate-magnesium) buffer.

2. Experimental Method:

Dissolve sequences a, b, and c separately in DEPC-treated water at a molar ratio of 1: 1: 1; after mixing, adjust the reaction solution to pH 7.Heat the mixture to 99 °C and maintain for 5 min; cool at 2 °C/min to 65 °C and hold for 15 min, then continue cooling at 2 °C/min to 4 °C.

Load the product onto an 8% (w/v) native PAGE gel and purify the carrier by electrophoresis at 100 V in TBM buffer at 4 °C.Excise the target band and elute in DNA elution buffer at 37 °C, then perform ethanol precipitation overnight. Remove solvent under reduced pressure at low temperature to obtain the self-assembled product, namely the targeted 5'-fluorouracil product Apt AS1411-Apt EGFR-Apt A15-RNA-5'-fluorouracil.

Example 18

In this example, the targeted paclitaxel product Apt AS1411-Apt EGFR-Apt A15-DNA-paclitaxel is synthesized.

Step 1: Synthesis of the Apt AS1411-Apt EGFR-Apt A15-DNA targeted carrier. The sequences of the individual single strands are set forth in Table 18:

Table 18

| Name | Sequence (sense, 5'-3') |
|---|---|
| Sequence a (SEQ ID NO: 325) | **5'-**GGTGGTGGTGGTTGTGGTGGTGGTGGTTTTT**GCGGCGCCCAC GAGCGTTCCGGGAGAGGAGGCC-3'** |
| Sequence b (SEQ ID NO: 326) | **5'-**GCCTTAGTAACGTGCTTTGATGTCGATTCGACAGGAGGCTTTTT **GGCCTCCTCTCCCGGTTCGCCGCCAGCCGCC-3'** |

(continued)

| Name | Sequence (sense, 5'-3') |
|---|---|
| Sequence c (SEQ ID NO: 327) | **5'-**CCCTCCTACATAGGGTTTTT**GGCGGCTGGCGGCCATAGCCGTGGGCGCCGC-3'** |
| Note: During synthesis on an automated oligonucleotide synthesizer, at the designated positions-i.e., between the nucleotides indicated by underlines in the table (twelve theoretical attachment sites)-a nucleoside phosphoramidite monomer bearing a long-chain primary-amine linker, namely N4-benzoyl-5'-O-(4, 4'-dimethoxytrityl)-2'-deoxycytidine-3'-(6-aminohexyl-2-hydroxymethyl)-N, N'-diisopropyl phosphoramidite, is introduced for solid-phase coupling.Upon completion of the coupling, the programmed cycles are continued to complete the full-length single-strand synthesis and derivatization.In sequences a, b, and c, the internucleoside phosphate linkages between nucleotides 1-2 at each of the 5' and 3' termini are phosphorothioate (PS) linkages.In sequence a, the nucleotides at positions 35, 39, 54, and 59 (counted from the 5' terminus) are modified with primary amine (-$NH_2$) groups; in sequence b, the nucleotides at positions 50, 55, 66, and 70 are modified with -$NH_2$; and in sequence c, the nucleotides at positions 24 and 28 are modified with -$NH_2$. | |

1. Materials and Reagents:

(1)Sequence a;

(2)Sequence b;

(3)Sequence c;

(4)DEPC-treated water;

(5)TBM (Tris-borate-magnesium) buffer.

2. Experimental Method:

Dissolve sequences a, b, and c separately in DEPC-treated water at a molar ratio of 1: 1: 1; after mixing, adjust the reaction solution to pH 7.Heat the mixture to 99 °C and maintain for 5 min; cool at 2 °C/min to 65 °C and hold for 15 min, then continue cooling at 2 °C/min to 4 °C.

Load the product onto an 8% (w/v) native PAGE gel and purify the carrier by electrophoresis at 100 V in TBM buffer at 4 °C.Excise the target band and elute in DNA elution buffer at 37 °C, then precipitate with ethanol overnight. Remove solvent under reduced pressure at low temperature to obtain the self-assembled product, namely the Apt AS1411-Apt EGFR-Apt A15-DNA targeted carrier.

Step 2: Paclitaxel Derivatization

Dissolve triphosgene (1.2 g, 0.004 mol) in dichloromethane (DCM) (25 mL).With stirring at room temperature, add dropwise over ~30 min a solution of paclitaxel (8.5 g, 0.01 mol) and 4-dimethylaminopyridine (DMAP) (2.45 g, 0.02 mol) in DCM (100 mL).After the addition is complete, continue the reaction for 10 min, then add a solution of bis(2-hydroxyethyl) disulfide (1.53 g, 0.01 mol) in DCM (10 mL) and continue stirring at room temperature for 30 min. Evaporate the solvent, add ethyl acetate (40 mL), and wash successively with 10% $KHSO_4$ (aq), 5% $NaHCO_3$ (aq), and saturated NaCl solution. Dry the organic phase over anhydrous $MgSO_4$, evaporate to dryness to afford paclitaxel-hydroxyethyl disulfide (9.1 g, 88% yield). Recrystallize from methanol to give 8.2 g of a white crystalline solid.

Dissolve paclitaxel-hydroxyethyl disulfide (8.2 g, 0.008 mol) in 1, 2-dichloroethane (DCE) (50 mL).With stirring at room temperature, add dropwise a solution of N, N-diisopropylethylamine (DIPEA) (1.5 g, 0.012 mol) in DCE (10 mL).After the addition, reflux for 3 h. Cool to room temperature, add water (40 mL) and stir for 5 min; transfer to a separatory funnel and allow the layers to separate. Collect the lower organic phase, evaporate the solvent to obtain paclitaxel disulfide succinimidyl ester (7.7 g, 81% yield).

Step 3: Loading of Paclitaxel

1. Materials and Reagents:

(1)The **Apt AS1411-Apt EGFR-Apt A15-DNA** targeted carrier prepared in Step 1;

(2)DEPC-treated water;

(3)PBS buffer (pH 6.6);

(4)4% (w/v) paraformaldehyde;

**(5)Paclitaxel disulfide N-hydroxysuccinimidyl (NHS) ester** prepared in Step 2;

(6)Chloroform;

(7)Isopropanol.

2. Experimental Method:

The Apt AS1411-Apt EGFR-Apt A15-DNA targeted carrier and the paclitaxel disulfide NHS ester are added into water at a molar ratio of 1: 12, the volume of water being 30 times that of the paclitaxel reagent, and allow the reaction to proceed at room temperature for 48 h.Cool, concentrate under reduced pressure to near dryness, add ethanol and stir to disperse, then filter and wash the filter cake with ethanol.Dry the filter cake under reduced pressure to afford Apt AS1411-Apt EGFR-Apt A15-DNA-paclitaxel.

Example 19

In this example, the targeted mertansine (DM1) product Apt AS1411-Apt EGFR-Apt A15-DNA-mertansine (DM1) is synthesized.
Step 1: Synthesis of the Apt AS1411-Apt EGFR-Apt A15-DNA targeted carrier. The sequences of the individual single strands are set forth in Table 19:

Table 19

| Name | Sequence (sense, 5'-3') |
|---|---|
| Sequence a (SEQ ID NO: 328) | **5'-**GGTGGTGGTGGTTGTGGTGGTGGTGGTTTTT**GCGGCGCCCACGAGCGTTCCGGGAGAGGAGC-3'** |
| Sequence b (SEQ ID NO: 178) | **5'-**GCCTTAGTAACGTGCTTTGATGTCGATTCGACAGGAGGCTTTTT**GCTCCTCTCCCGGTTCGCCGCCAGCCGCC-3'** |
| Sequence c (SEQ ID NO: 68) | **5'-**CCCTCCTACATAGGGTTTTT**GGCGGCTGGCGGCCATAGCCGTGGGCGCCGC-3'** |
| Note: During synthesis of each sequence on an automated oligonucleotide synthesizer under programmed control, at the designated positions-i.e., between the nucleotides indicated by underlines in the table (six theoretical attachment sites)-a nucleoside phosphoramidite monomer bearing a long-chain primary-amine linker, namely N4-benzoyl-5'-O-(4, 4'-dimethoxytrityl)-2'-deoxycytidine-3'-(6-aminohexyl-2-hydroxymethyl)-N, N'-diisopropyl phosphoramidite, is introduced for solid-phase coupling.After the coupling step, the programmed cycles are continued to complete the full-length single-strand synthesis and derivatization.In sequences a, b, and c, the internucleoside phosphate linkages between nucleotides 1-2 at each of the 5' and 3' termini are phosphorothioate (PS) linkages.In sequence a, the nucleotides at positions 43 and 61 (counted from the 5' terminus) are modified with primary amine ($-NH_2$) groups; in sequence b, the nucleotides at positions 48 and 63 are modified with $-NH_2$; and in sequence c, the nucleotides at positions 24 and 43 are modified with $-NH_2$. | |

1. Materials and Reagents:

(1)Sequence a;

(2)Sequence b;

(3)Sequence c;

(4)DEPC-treated water;

(5)TBM (Tris-borate-magnesium) buffer.

2. Experimental Method:

Dissolve sequences a, b, and c separately in DEPC-treated water at a molar ratio of 1: 1: 1; after mixing, adjust the reaction solution to pH 7.Heat the mixture to 99 °C and maintain for 5 min; cool at 2 °C/min to 65 °C and hold for 15 min, then continue cooling at 2 °C/min to 4 °C.

Load the product onto an 8% (w/v) native PAGE gel and purify the carrier by electrophoresis at 100 V in TBM buffer at 4 °C.Excise the target band and elute in DNA elution buffer at 37 °C, then perform ethanol precipitation overnight. Remove solvent under reduced pressure at low temperature to obtain the self-assembled product, namely the Apt AS1411-Apt EGFR-Apt A15-DNA targeted carrier.

Step 2: Loading of Mertansine

1. Materials and Reagents:

(1)The **Apt AS1411-Apt EGFR-Apt A15-DNA** targeted carrier prepared in Step 1;

(2)DEPC-treated water;

(3)PBS buffer (pH 6.6);

(4)4% (w/v) paraformaldehyde;

**(5)Mertansine SMCC-DM1** ($C_{51}H_{66}ClN_5O_{16}S$; M_r = 1072.61; CAS No.1228105-51-8);

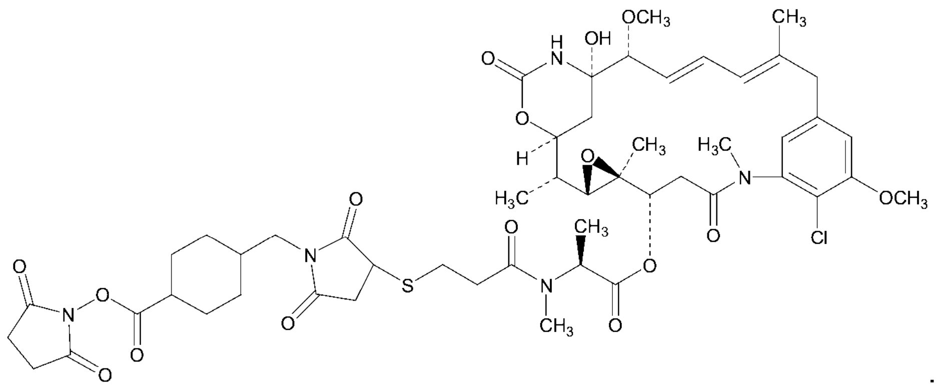

.

(6)Chloroform;

(7)Isopropanol.

2. Experimental Method:

The Apt AS1411-Apt EGFR-Apt A15-DNA targeted carrier and SMCC-DM1 are added into water at a molar ratio of 1: 6, the volume of water being 30 times that of the SMCC-DM1, and allow the reaction to proceed at room temperature for 48 h.Cool, concentrate under reduced pressure to near dryness, add ethanol and stir to disperse, then filter and wash the filter cake with ethanol.Dry the filter cake under reduced pressure to obtain Apt AS1411-Apt EGFR-Apt A15-DNA-mertansine.

Example 20

In this example, the targeted epirubicin product Apt AS1411-Apt EGFR-Apt A15-AmiR-21-DNA-epirubicin is synthesized.

Step 1: Synthesis of the Apt AS1411-Apt EGFR-Apt A15-AmiR-21-DNA targeted carrier. The sequences of the individual single strands are set forth in Table 20:

Table 20

| Name | Sequence (sense, 5'-3') | Modification(s) |
|---|---|---|
| Sequence a (SEQ ID NO: 289) | **5'-**GGTGGTGGTGGTTGTGGTGGTGGTGGTTTTT**GCG GCGCCCACGAGCGTTCCGGGAGC-3'** | Unmodified |
| Sequence b (SEQ ID NO: 290) | **5'-**GCCTTAGTAACGTGCTTTGATGTCGATTCGACAGGA GGCTTTTT**GCTCCCGGTTCGCCGCCAGCCGCC-3'** | Unmodified |
| Sequence c (SEQ ID NO: 291) | **5'-**CCCTCCTACATAGGGTTTTT**GGCGGCAGGCGGCCA TAGCCGTGGGCGCCGC**GATAAGCT**-3'** | The eight nucleotides immediately upstream of the 3' terminus are locked nucleic acid (LNA)-modified |

1. Materials and Reagents:

(1)Sequence a;

(2)Sequence b;

(3)Sequence c;

(4)DEPC-treated water;

(5)TBM (Tris-borate-magnesium) buffer.

2. Experimental Method:

Dissolve sequences a, b, and c separately in DEPC-treated water at a molar ratio of 1: 1: 1; after mixing, adjust the reaction solution to pH 7.Heat the mixture to 85 °C and maintain for 5 min; cool at 2 °C/min to 60 °C and hold for 5 min, then continue cooling at 2 °C/min to 4 °C.

Load the product onto an 8% (w/v) native PAGE gel and purify the carrier by electrophoresis at 100 V in TBM buffer at 4 °C. Excise the target band and elute in DNA elution buffer at 37 °C, then perform ethanol precipitation overnight. Remove solvent under reduced pressure at low temperature to obtain the self-assembled product, namely the Apt AS1411-Apt EGFR-Apt A15-2*Bio-AmiR-21-DNA targeted carrier.

Step 2: Loading of Epirubicin

1. Materials and Reagents:

**(1)Apt AS1411-Apt EGFR-Apt A15-AmiR-21-DNA** targeted carrier prepared in Step 1;

(2)DEPC-treated water;

(3)PBS buffer (pH 6.8);

(4)4% (w/v) paraformaldehyde solution;

(5)Epirubicin hydrochloride;

(6)Chloroform;

(7)Methanol.

2. Experimental Method:

(1)Accurately weigh epirubicin hydrochloride (0.79 mg, 1.354 μmol) and dissolve in DEPC-treated water (1.0 mL) and PBS buffer (1.25 mL).While cooling in an ice-water bath, add 4% paraformaldehyde solution (0.25 mL) and mix thoroughly. Combine the entire mixture with the Apt AS1411-Apt EGFR-Apt A15-AmiR-21-DNA targeted carrier (1 mg), mix well, and allow the reaction to proceed at 4 °C for 72 h protected from light, maintaining the reaction pH at 6.

(2)Wash the reaction mixture with chloroform (10 mL × 3). To the aqueous phase add methanol (25 mL), mix, and keep protected from light at 4 °C to allow complete precipitation (4 h).Centrifuge (4, 000 rpm), decant the supernatant, wash the solid product again with methanol (50 mL), remove the supernatant, and evaporate the residue to dryness under reduced pressure at low temperature to obtain a dark-red solid, namely the targeted epirubicin product Apt AS1411-Apt EGFR-Apt A15-AmiR-21-DNA-epirubicin.

(4)Determination of Loading Ratio:

1. Prepare epirubicin-PBS standard solutions at known concentrations of 2 μM, 4 μM, 6 μM, 8 μM, and 10 μM, 100 μL each;

2. Dissolve **Apt AS1411-Apt EGFR-Apt A15-AmiR-21-DNA-epirubicin** in 100 μL PBS;

3. Place the reference standard solutions and the **Apt AS1411-Apt EGFR-Apt A15-AmiR-21-DNA-epirubicin** sample in a PCR plate, heat at 85 °C for 5 min, then cool to room temperature;

4. Measure the absorbance of epirubicin at 492 nm using a microplate reader, plot a standard curve, and calculate the molar concentration of epirubicin in the loaded product;

5. Measure the absorbance of DNA at 260 nm with a spectrophotometer to obtain the mass concentration of the DNA carrier in each sample;

6. Calculate the loading ratio from the measured molar concentration of epirubicin and the mass concentration of the DNA carrier. For the above targeted epirubicin product Apt AS1411-Apt EGFR-Apt A15-AmiR-21-DNA-epirubicin, the maximum theoretical loading number (average value) of epirubicin is approximately 27 (molecules per DNA carrier), and the measured average loading number is 23.

Example 21

In this example, the targeted epirubicin product Apt TfRA4-AptAS1411-Apt A15-DNA-epirubicin (2) is synthesized.

Step 1: Synthesis of the Apt TfRA4-Apt AS1411-Apt A15-DNA targeted carrier (2). The sequences of the individual single strands are set forth in Table 21.:

Table 21

| Name | Sequence (sense, 5'-3') | Modification(s) |
|---|---|---|
| Sequence a (SEQ ID NO: 295) | **5'**-TCGCCGCTCGCCGCTTGCGTGGTACCACGCTTGCGGCGAGCGGCGA**GCCCACGAGCGTTCCGGGAGA-3'** | Unmodified |
| Sequence b (SEQ ID NO: 296) | **5'**-GGTGGTGGTGGTTGTGGTGGTGGTGGTTTTT**TCTCCCGGTTCGCCGCCAGCCGCC**-**3'** | Unmodified |
| Sequence c (SEQ ID | **5'**-CCCTCCTACATAGGGTTTTT**GGCGGCTGGCGGCCATAGCCGTGGGC**-**3'** | Unmodified |
| NO: 297) | | |

1. Materials and Reagents:

(1)Sequence a;

(2)Sequence b;

(3)Sequence c;

(4)DEPC-treated water;

(5)TBM (Tris-borate-magnesium) buffer.

2. Experimental Method:

Dissolve sequences a, b, and c separately in DEPC-treated water at a molar ratio of 1: 1: 1; after mixing, adjust the reaction solution to pH 7.Heat the mixture to 99 °C and maintain for 5 min; cool at 2 °C/min to 65 °C and hold for 15 min, then continue cooling at 2 °C/min to 4 °C.

Load the product onto an 8% (w/v) native PAGE gel and purify the carrier by electrophoresis at 100 V in TBM buffer at 4 °C.Excise the target band and elute in DNA elution buffer at 37 °C, then perform ethanol precipitation overnight. Remove solvent under reduced pressure at low temperature to obtain the self-assembled product, namely the **Apt TfRA4-Apt AS1411-Apt A15-DNA targeted carrier (2).**

Step 2: Loading of Epirubicin

1. Materials and Reagents:

(1)Apt TfRA4-Apt AS1411-Apt A15-DNA targeted carrier (2) prepared in Step 1;

(2)DEPC-treated water;

(3)PBS buffer (pH 6.6);

(4)4% (w/v) paraformaldehyde;

(5)Epirubicin hydrochloride;

(6)Chloroform;

(7)Isopropanol.

2. Experimental Method:

(1)Accurately weigh epirubicin hydrochloride (0.79 mg, 1.354 μmol) and dissolve in DEPC-treated water (1.0 mL) and PBS buffer (1.25 mL).While cooling in an ice-water bath, add 4% (w/v) paraformaldehyde (0.25 mL) and mix thoroughly.Combine the entire mixture with the targeted carrier prepared in Step 1 (i.e., Apt TfRA4-Apt AS1411-Apt A15-DNA targeted carrier (2); 1 mg), mix well, and allow the reaction to proceed at 4 °C for 72 h, protected from light, maintaining the reaction pH at 7.

(2)Wash the reaction mixture with chloroform (10 mL × 3). To the aqueous phase add isopropanol (25 mL), mix, and keep protected from light at 4 °C to allow complete precipitation (4 h).Centrifuge (4, 000 rpm), decant the supernatant, wash the solid again with isopropanol (50 mL), remove the supernatant, and evaporate the residue to dryness under reduced pressure at low temperature to obtain a dark-red solid, namely the targeted epirubicin product Apt TfRA4-Apt AS1411-Apt A15-DNA-epirubicin (2).

(4)Determination of Loading Ratio:

1. Prepare epirubicin-PBS standard solutions at known concentrations: 2 μM, 4 μM, 6 μM, 8 μM, 10 μM, 100 μL each;

2. Dissolve Apt TfRA4-Apt AS1411-Apt A15-DNA-epirubicin (2) in 100 μL PBS;

3. Place the reference standard solutions and the Apt TfRA4-Apt AS1411-Apt A15-DNA-epirubicin (2) sample in a PCR plate, heat at 85 °C for 5 min, then cool to room temperature;

4. Measure the absorbance at 492 nm of epirubicin using a microplate reader, plot a standard curve, and calculate the molar concentration of epirubicin in the loaded product;

5. Measure the absorbance at 260 nm of DNA with a spectrophotometer to obtain the mass concentration of the DNA carrier in each sample;

6. Calculate the loading ratio (loading number) from the measured molar concentration of epirubicin and the mass concentration of the DNA carrier. For the above targeted epirubicin product, the maximum theoretical loading number of epirubicin is approximately 36 (molecules per DNA carrier), and the measured average loading number is 31.

Performance Characterization:

1. In vivo antitumor efficacy of Apt EGFR-DNA-epirubicin prepared in Example 1 in an N87 gastric cancer mouse model:

**Test animals**

**Species/strain:** BALB/c nude (nu/nu)

**Age:** 6-8 weeks

**Sex:** Male or female

Body weight: 18-22 g

Experimental procedure

(1)..Cell culture: Tumor cells were cultured in RPMI-1640 medium supplemented with heat-inactivated 10% fetal bovine serum, 100 U/mL penicillin, 100 μg/mL streptomycin, and 2 mM L-glutamine, in a humidified incubator at 37 °C with 5% $CO_2$.Subculture was performed every 3-4 days after confluence; tumor cells in logarithmic growth phase were used for in vivo inoculation.

(2).Tumor cell inoculation and grouping:

Tumor cells ($1 \times 10^7$) resuspended in PBS: Matrigel were inoculated subcutaneously into the right flank of each mouse.When tumors reached approximately 80-100 $mm^3$, animals were randomized into groups for dosing. The specific dosing regimens are shown in the table below (with PBS and epirubicin API serving as comparators).

**(3).Dosing Regimen:**

Table 22

| Group | Number of animals | Test article | dose per administration | Route of administration | Dosing Regimen |
|---|---|---|---|---|---|
| 1 | 10 | PBS | -- | Intravenous (tail vein) | Twice per week, for 5 weeks |
| 2 | 10 | Epirubicin (API) | 0.06mg | Intravenous (tail vein) | Twice per week, for 5 weeks |
| 3 | 10 | Apt EGFR-DNA-epirubicin | 0.12mg | Intravenous (tail vein) | Twice per week, for 5 weeks |
| 4 | 10 | Apt EGFR-DNA-epirubicin | 0.06mg | Intravenous (tail vein) | Twice per week, for 5 weeks |
| 5 | 10 | Apt EGFR-DNA-epirubicin | 0.03mg | Intravenous (tail vein) | Twice per week, for 5 weeks |
| **Note:** If an animal's body weight decreases by 20% during the study, dosing is discontinued. Dosing is resumed after body weight recovers. | | | | | |

The dosing volume is 200 μL per 20 g mouse, with the administered dose calculated on a body-weight basis; the observation period is 60 days, adjustable according to the condition of the animals and tumor status.

**Animal Husbandry and Management**

Housing: Animals were maintained in SPF-grade, temperature- and humidity-controlled laminar-flow clean rooms, using individually ventilated cages (IVC) at 3 mice per cage.

Temperature / relative humidity: maintained at (23 ± 3) °C and 40-70% RH.

Cages: Polycarbonate cages, 370 mm × 155 mm × 135 mm; autoclaved soft corncob bedding, changed twice weekly.Each cage was labeled with animal number, sex, strain, date/time of receipt, group assignment, and study start date.

Diet and water: SPF-grade rodent chow sterilized by Co-60 irradiation; drinking water ultrafiltered and autoclaved.Food and water provided ad libitum under sterile conditions.

Animal identification: Ear punch.

**Endpoints and Assessments**

### 1. Assessment Parameters

a. Tumor volume: Measured twice weekly using vernier calipers. The long and short diameters were recorded; tumor volume was calculated as: Volume = 0.5 × length × $(width)^2$.

b. Post-administration reactions in animals: At each tumor-measurement time point, body weight was recorded.Changes in body weight relative to dosing time were documented.Survival and general health (e.g., activity, feeding) were monitored throughout the dosing period.

c. Tumor excision, weighing, and photography: at the end of the study, mice were euthanized; tumors were excised, photographed and collected; specimens were fixed or snap-frozen as required.

a) Photograph each animal post-euthanasia with tumor in situ;

b) Dissect and weigh each subcutaneous tumor;

c) Arrange all tumors on a white board according to group codes and photograph; then bisect longitudinally, realign by group code, and photograph again.

d) Tumors were snap-frozen or formalin-fixed per requirements.

d. Necropsy: organ collection, weighing, and imaging:

a) For each animal, collect heart, liver, spleen, lungs, and kidneys, record organ weights, and calculate organ-to-body-weight ratios; mark and photograph any gross metastatic lesions.Organs were fixed or snap-frozen, and observations recorded.
b) From each treatment group, one mouse's organs (heart, liver, spleen, lungs, kidneys) and tumor were subjected to fluorescence imaging at the excitation/emission wavelengths of epirubicin to assess residual drug.

2. Efficacy Evaluation Metrics:

a. Tumor Growth Inhibition (TGI, %)

$$TGI (\%) = (1 - T/C) \times 100\%.$$

Here, T/C (relative tumor growth ratio) = $RTV_{_treatment\ group}$ / $RTV_{_control\ group}$, where RTV denotes relative tumor volume.A result is deemed effective when TGI ≥ 60% and $p < 0.05$ (statistical significance).
b. Treatment group/Control group Tumor Volume Ratio, T/C (%)

$$T/C (\%) = (RTV_{treatment\ group}/ RTV_{control\ group}) \times 100\%.$$

Efficacy assessment criterion: T/C (%) > 40% = ineffective; T/C (%) ≤ 40% with $p < 0.05$ = effective.
c. Inhibition Rate of Tumor Weight ($IR_{TW}$, %)

$$IR_{TW} (\%) = (W_{control\ group} - W_{treatment\ group})/ W_{control\ group} \times 100,$$

where W is tumor weight.$IR_{TW}$ > 60% is considered effective.

Test results are shown in the table below:

Table 23

| Group | Tumor Growth Inhibition Rate at Different Days Post-Inoculation (%) | | | | | | | | | | Body-weight difference between Day 37 and Day 6 (g) |
|---|---|---|---|---|---|---|---|---|---|---|---|
| | 6 | 9 | 13 | 17 | 20 | 23 | 27 | 30 | 34 | 37 | |
| 1 | 0.00 | 0.00 | 0.00 | 0.00 | 0.00 | 0.00 | 0.00 | 0.00 | 0.00 | 0.00 | 3.98 |
| 2 | | 8.44 | 0.40 | 6.68 | 7.83 | 12.46 | 23.34 | 25.87 | 31.16 | 36.26 | 0.91 |
| 3 | | 12.75 | 16.10 | 25.58 | 28.35 | 32.23 | 45.97 | 58.57 | 67.74 | 71.71 | 0.02 |
| 4 | | 8.36 | 8.76 | 12.47 | 9.38 | 12.00 | 19.60 | 28.35 | 40.15 | 44.52 | 1.33 |
| 5 | | 1.68 | -2.87 | -0.13 | -0.12 | 0.34 | 5.62 | 11.09 | 18.86 | 22.08 | 2.43 |

2. In vivo antitumor efficacy of **Apt AS1411-Apt EGFR-Apt A15-2*Bio-DNA-epirubicin** (prepared in Example 5) in an **N87 gastric cancer mouse model:**

**Species/strain:** BALB/c nude

**Age:** 5 weeks

**Sex:** Male

**Body weight:** ~16 g

Experimental procedure

1. Cell culture and preparation: Thaw cells and seed into the corresponding medium (RPMI-1640 basal medium + 10% FBS + 1% penicillin/streptomycin [P/S]).

Passaging method:

a) Discard the culture medium and gently rinse the cells with PBS twice.

b) Add an appropriate volume of 0.25% trypsin, digest in a 37 °C incubator for about 1-2 min until cells visibly round up; then add complete medium (RPMI-1640 + 10% FBS + 1% P/S) to neutralize the trypsin.Gently pipette to detach cells and subculture at the desired split ratio.

c) Split ratio: 1: 2-1: 4; the split ratio used in this experiment is 1: 3.

d) Passage frequency: Every 2-3 days, depending on seeding density and growth.

Collection:

a) Harvest cells in logarithmic growth phase (~80-90% confluence recommended);

b) After trypsinization, add complete medium to terminate digestion; collect the suspension into centrifuge tubes and centrifuge at 1, 000 rpm for 5 min;

c) Wash the cell pellet twice with pre-chilled PBS and centrifuge each time;

d) Resuspend the pellet in PBS or serum-free medium to a final concentration of 2 × 10^7 cells/mL.

2. Inoculation:

Injection site: Subcutaneous, right axillary-dorsal region.

Inoculum: 3 × $10^6$ cells/mouse; 30 mice inoculated.

Injection volume: 0.1 mL.

Method: Tumor cell inoculation.

Thaw and expand N87 cells to the required quantity; adjust the cell concentration to 3 × $10^7$ cells/mL with sterile PBS and keep on ice until use. Using a single-use sterile 1 mL syringe, draw 0.1 mL of the cell suspension and inject subcutaneously into the right axillary-dorsal region of each mouse. The day of inoculation is designated Day 0 (D0).

Inoculation procedure:

a)Immediately before injection, thoroughly disperse the tumor cell suspension by pipetting to prevent cell clumping, which can reduce viability;

b)Restrain the mouse by grasping the dorsal neck skin with the thumb and forefinger while holding the tail with the little finger;

c)Using a disposable 1 mL syringe, inject the suspension subcutaneously; advance the needle slightly deeper under the skin (approximately 1 cm) to minimize leakage of the suspension from the needle tract;

**3. Dosing regimen:**

Table 24

| Group | Number of Animals | Test Article | Dose per Administration | Route of Administration | Dosing Regimen |
|---|---|---|---|---|---|
| 1 | 8 | PBS | -- | Intravenous (tail vein) | Once every 5 days, for a total of 5 administrations |
| 2 | 8 | Epirubicin drug substance (API) | 0.12mg | Intravenous (tail vein) | Once every 5 days, for a total of 5 administrations |
| 3 | 8 | Apt AS1411-Apt EGFR-Apt A15-2*Bio-DNA-Epirubicin | 0.12mg | Intravenous (tail vein) | Once every 5 days, for a total of 5 administrations |

**Assessment Parameters**

1. General clinical observation:

Animals are observed once daily throughout the study for clinical signs, including ulceration of tumor nodules, general condition/mentation, coat condition, behavior, and food intake and excretion.

2. Body weight:

Body weight is measured three times per week.Data are recorded and changes analyzed. The relative body-weight growth curves for each group are shown in **Fig.8.**

3. Tumor volume (V):

Tumor size is measured three times per week using a vernier caliper. The long diameter (a) and the short diameter (b) are recorded. Tumor volume is calculated as: $V=0.5\times a\times b^2$.

4. Relative tumor volume (RTV):

RTV is calculated as: $RTV = V_t/V_0$,
where $V_0$ is the tumor volume at grouping and initial dosing (D0), and $V_t$ is the tumor volume at each measurement time point.

5. Tumor growth inhibition rate (TGI):

$$TGI(\%) = (1- T/C)\times 100\%, \text{ where } T/C = RTV_{\text{treatment group}}/ RTV_{\text{control group}}$$

Efficacy is concluded when TGI≥60% and, after statistical analysis, the tumor volume of the treatment group is significantly lower than that of the vehicle control group (P<0.05), indicating a significant inhibitory effect on tumor growth.

6. Inhibition rate of tumor weight ($IR_{TW}$):

$$IR_{TW}(\%)= (W_{\text{control group}} - W_{\text{treatment group}})/ W_{\text{control group}} \times 100,$$

where W denotes tumor weight.$IR_{TW}\%>60\%$ is considered effective.

The test results are presented in **Table 25,** and the tumor growth curves for each group are shown in **Fig.9:**

Table 25

| Group | Inhibition rate (%) at different post-inoculation days | | | | | | | | Body-weight difference (g) between Day 23 and Day 5 |
|---|---|---|---|---|---|---|---|---|---|
| | 5 | 9 | 11 | 13 | 16 | 18 | 20 | 23 | |
| 1 | 0.00 | 0.00 | 0.00 | 0.00 | 0.00 | 0.00 | 0.00 | 0.00 | 1.72 |
| 2 | | 17.5 | 3.5 | 10.2 | 24.1 | 32.9 | 36.4 | 34.6 | -3.84 |
| 3 | | 38.6 | 38.3 | 46.9 | 56.8 | 69.6 | 67.6 | 73.1 | -3.68 |

The tumor weight results and the inhibition rate of tumor weight ($IR_{TW}\%$) are presented in Table 26 below; the changes in tumor volume for each group are shown in Figure 10:

Table 26

| Group | Number of animals | Tumor weight (g) | $IR_{TW}\%$ |
|---|---|---|---|
| 1 | 6 | 0.6251±0.11 | -- |
| 2 | 6 | 0.1751±0.13 | 71.987 |
| 3 | 6 | 0.1583±0.008 | 74.670 |

3. Comparative in-vivo efficacy test in the N87 mouse gastric carcinoma model between the epirubicin-targeted drug prepared in Example 5 (Apt AS1411-Apt EGFR-Apt A15-2Bio-DNA-epirubicin) and the epirubicin-targeted drug prepared in Example 7 (Apt AS1411-Apt EGFR-Apt A15-2Bio-AmiR-21-DNA-epirubicin).:

Experimental Animals

Species/strain: BALB/c nude (BALB/c-nu)

Age: 6-8 weeks

Sex: Male

Body weight: 17-19 g

Housing conditions: Animals were maintained in an SPF-grade barrier facility of the Institute of Materia Medica, Chinese Academy of Medical Sciences. Husbandry was performed in isolators in accordance with the facility standards, six animals per cage, with adequate illumination, good ventilation and air-conditioning; room temperature 18-25 °C and relative humidity 50-70%.

Experimental Methods

1).Inoculation and grouping: For the N87 human gastric carcinoma model, male BALB/c-nu mice (17.0-19.0 g) were used.Under aseptic conditions, N87 tumor tissue was excised and cut into 2.0 mm × 2.0 mm × 2.0 mm tumor blocks, which were implanted subcutaneously into the left axillary dorsal region of each nude mouse. The day of implantation was designated as Day 0 (D0).When tumor volume reached approximately 250.0 $mm^3$ (D6), animals with poor tumor take were excluded, and the remaining animals were randomized into groups according to tumor volume; dosing commenced on the day of randomization.
**2).Dosing regimen:**

Table 27

| Group | Number of animals | Test article | Dose per administration | Route of administration | Dosing regimen |
|---|---|---|---|---|---|
| 1 | 8 | PBS | -- | Intravenous (tail vein) | Once weekly, for 6 weeks |
| 2 | 8 | Epirubicin drug substance (API) | 0.12mg | Intravenous (tail vein) | Once weekly, for 6 weeks |
| 3 | 8 | Apt AS1411-Apt EGFR-Apt A15-2*Bio-DNA-Epirubicin | 0.12mg | Intravenous (tail vein) | Once weekly, for 6 weeks |
| 4 | 8 | Apt AS1411-Apt EGFR-Apt A15-2*Bio-AmiR-21-DNA-Epirubicin | 0.12mg | Intravenous (tail vein) | Once weekly, for 6 weeks |

**Assessment Parameters**1).General clinical observation:
Mice are observed once daily throughout the study for clinical signs, including ulceration of tumor nodules, general condition/mentation, coat condition, behavior, and food intake and excretion.

2).Body weight:

Body weight is measured three times per week.Data are recorded and changes analyzed; body-weight change curves are shown in **Fig.11.**

3).Tumor volume (V):

Tumor size is measured three times per week using a vernier caliper. The long diameter (a) and short diameter (b) are recorded. Tumor volume is calculated as: $V=0.5\times a\times b^2$

4).Relative tumor volume (RTV):

$$RTV = V_t / V_0,$$

where $V_0$ is the tumor volume at grouping/first dosing (D0) and $V_t$ is the tumor volume at each measurement time point.

5).Tumor growth inhibition rate (TGI):

$$TGI(\%) = (1- T/C)\times 100\%$$

$$T/C = RTV_{\text{Treatment group}} / RTV_{\text{Controll group}}$$

Efficacy is concluded when TGI ≥60% and, after statistical analysis, the tumor volume in the treatment group is significantly lower than that in the vehicle control group (P<0.05), indicating a significant inhibitory effect on tumor growth.

6).Inhibition rate of tumor weight ($IR_{TW}$):

$$IR_{TW}\ (\%) = (W_{\text{control group}} - W_{\text{treatment group}}) / W_{\text{control group}}\times 100,$$

where W is tumor weight.$IR_{TW}$ > 60% is considered effective.All animals are closely monitored for tumor growth during the study; tumors are measured twice weekly, results recorded, and TGI calculated. The TGI test results are presented in **Table 28,** the tumor-volume change curves are shown in **Fig.12,** and the relative tumor-volume (RTV) curves are shown in **Fig.13.**

Table 28

| Group | Tumor growth inhibition rate (TGI, %) at different post-inoculation days | | | | | | | | | | | Body-weight difference (g) between Day 41 and Day 6 |
|---|---|---|---|---|---|---|---|---|---|---|---|---|
| | 6 | 10 | 13 | 17 | 20 | 24 | 27 | 31 | 34 | 38 | 41 | |
| 1 | 0.00 | 0.00 | 0.00 | 0.00 | 0.00 | 0.00 | 0.00 | 0.00 | 0.00 | 0.00 | 0.00 | 0.26 |
| 2 | | 41.17 | 34.62 | 37.16 | 35.17 | 43.86 | 43.86 | 44.19 | 42.97 | 40.97 | 51.83 | 3.29 |
| 3 | | 47.33 | 55.51 | 47.23 | 55.96 | 60.68 | 68.24 | 69.87 | 68.58 | 68.45 | 70.23 | 1.94 |
| 4 | | 47.87 | 63.76 | 54.65 | 65.00 | 67.03 | 75.17 | 69.99 | 61.94 | 65.58 | 66.52 | 4.21 |

The test results for the treatment group/control group tumor-volume ratio (T/C) are presented in the table below:

Table 29

| Group | Treatment/control tumor volume ratio (T/C, %) at different post-inoculation days | | | | | | | | | | |
|---|---|---|---|---|---|---|---|---|---|---|---|
| | 6 | 10 | 13 | 17 | 20 | 24 | 27 | 31 | 34 | 38 | 41 |
| 1 | / | / | / | / | / | / | / | / | / | / | / |
| 2 | | 58.97 | 63.48 | 62.83 | 64.61 | 55.39 | 55.22 | 54.72 | 56.16 | 59.25 | 46.98 |
| 3 | | 51.99 | 42.99 | 50.75 | 42.57 | 37.73 | 30.92 | 29.16 | 30.57 | 30.04 | 28.81 |

(continued)

| Group | Treatment/control tumor volume ratio (T/C, %) at different post-inoculation days | | | | | | | | | |
|---|---|---|---|---|---|---|---|---|---|---|
| | 6 | 10 | 13 | 17 | 20 | 24 | 27 | 31 | 34 | 38 | 41 |
| 4 | | 52.69 | 36.80 | 45.85 | 34.63 | 33.19 | 24.61 | 28.42 | 36.83 | 34.09 | 32.52 |

At 41 days post-N87 inoculation, all animals were euthanized by cervical dislocation; tumors were excised and weighed, and the inhibition rate of tumor weight ($IR_{TW}$%) data are shown in the table below:

Table 30

| Group | Tumor weight (g) | Tumor-weight inhibition rate, $IR_{TW}$ (%) |
|---|---|---|
| 1 | 1.29±0.45 | / |
| 2 | 0.62±0.55 | 51.74 |
| 3 | 0.40±0.24 | 69.02 |
| 4 | 0.45±0.21 | 65.38 |

From the above data, for tail-vein injection of epirubicin drug substance (API), the tumor growth inhibition rate-calculated from tumor weight and tumor volume-was < 60% at the end of the study (D41), and the T/C ratio was > 40%.In contrast, for tail-vein injection of the targeted drugs Apt AS1411-Apt EGFR-Apt A15-2Bio-DNA-epirubicin and Apt AS1411-Apt EGFR-Apt A15-2Bio-AmiR-21-DNA-epirubicin, the tumor growth inhibition rate at D41 (based on tumor weight and tumor volume) exceeded 60% and the T/C ratio was < 40% for both, indicating a significant inhibitory effect on N87 tumor growth.

4. In-vivo efficacy study of the epirubicin-targeted drug prepared in Example 3 (Apt AS1411-4×Bio-DNA-epirubicin) in the A549 mouse lung carcinoma model:

**Experimental animals**

**Species/strain:** BALB/c nude (BALB/c-nu)

**Age:** 6-7 weeks

**Sex:** Male

**Body weight:** 16-18 g

**Experimental methods**

1).Inoculation and grouping: For the A549 mouse lung carcinoma model, male BALB/c-nu mice weighing 16.0-18.0 g were used.Under aseptic conditions, A549 tumor cells were harvested and the cell concentration was adjusted with sterile normal saline to $1\times10^7$ cells/mL.A 0.2 mL aliquot was inoculated subcutaneously into the axillary dorsal region of nude mice.After tumors had grown to a volume of 1, 000-1, 500 $mm^3$, tumors were aseptically excised, cut into 2.0mm×2.0mm×2.0mm fragments, and uniformly implanted subcutaneously into the axillary dorsal region of nude mice. The day of implantation was designated as Day 0 (D0).When the tumor volume reached approximately 100.0 $mm^3$ (D5), animals with poor tumor take were excluded, the remaining animals were randomized by tumor volume, and dosing commenced on the day of randomization.

**2).Dosing regimen:**

Table 31

| **Group** | Number of animals | Test article | Dose per administration | Route of administration | Dosing regimen |
|---|---|---|---|---|---|
| 1 | 8 | PBS | -- | Intravenous (tail vein) | Twice weekly, for 5 weeks |
| 2 | 8 | Epirubicin drug substance (API) | 0.14mg | Intravenous (tail vein) | Twice weekly, for 5 weeks |
| 3 | 8 | Apt AS1411-4*Bio-DNA-Epirubicin | 0.14mg | Intravenous (tail vein) | Twice weekly, for 5 weeks |
| 4 | 8 | Epirubicin drug substance (API) | 0.12mg | Intravenous (tail vein) | Twice weekly, for 5 weeks |
| 5 | 8 | Apt AS1411-4*Bio-DNA-Epirubicin | 0.12mg | Intravenous (tail vein) | Twice weekly, for 5 weeks |
| 6 | 8 | Epirubicin drug substance (API) | 0.08mg | Intravenous (tail vein) | Twice weekly, for 5 weeks |
| 7 | 8 | Apt AS1411-4*Bio-DNA-Epirubicin | 0.08mg | Intravenous (tail vein) | Twice weekly, for 5 weeks |

**Assessment Parameters**

1).General clinical observation:

Mice are observed once daily throughout the study for clinical signs, including ulceration of tumor nodules, general condition/mentation, coat condition, behavior, and food intake and excretion.

2).Body weight:

Body weight is measured twice per week.Data are recorded and changes analyzed.

3).Tumor volume (V):

Tumor size is measured twice per week using a vernier caliper. The long diameter (a) and short diameter (b) are recorded. Tumor volume is calculated as: $V=0.5\times a\times b^2$.

4).Relative tumor volume (RTV):

$$RTV = V_t / V_0 ,$$

where $V_0$ is the tumor volume at grouping/first dosing (D0), and $V_t$ is the tumor volume at each measurement time point.

5).Tumor growth inhibition rate (TGI):

$$TGI\ (\%) = (1 - T/C) \times 100\%$$

$$T/C = RTV_{\text{treatment group}} / RTV_{\text{control group}}$$

Efficacy is concluded when TGI ≥60% and, after statistical analysis, the tumor volume of the treatment group is significantly lower than that of the vehicle control group ($P<0.05$), indicating a significant inhibitory effect on tumor growth.

All animals are closely monitored for tumor growth during the study; tumors are measured twice weekly, results

recorded, and TGI calculated. The TGI test results are presented in **Table 32,** the tumor-volume change curves are shown in **Fig.14,** and the relative tumor-volume (RTV) curves are shown in **Fig.15:**

Table 32

| Group | Tumor growth inhibition rate (TGI, %) at different days post-inoculation | | | | | | | | | Body-weight difference (g) between Day 32 and Day 5 |
|---|---|---|---|---|---|---|---|---|---|---|
| | 5 | 8 | 11 | 14 | 17 | 22 | 25 | 29 | 32 | |
| 1 | 0.00 | 0.00 | 0.00 | 0.00 | 0.00 | 0.00 | 0.00 | 0.00 | 0.00 | 5.8 |
| 2 | | 17.47 | 28.43 | 6.41 | 31.81 | 12.37 | 44.37 | 31.80 | 24.63 | 0.2 |
| 3 | | 40.79 | 45.88 | 53.35 | 69.39 | 59.40 | 70.24 | 61.83 | 68.33 | 6 |
| 4 | | 17.42 | 15.55 | -4.64 | 28.37 | 8.93 | 41.13 | 34.27 | 33.27 | 6.1 |
| 5 | | 24.34 | 31.10 | 20.48 | 47.33 | 52.83 | 62.20 | / | / | 6.2 |
| 6 | | 19.90 | 17.02 | -12.27 | 10.74 | 10.11 | 28.23 | 2.07 | 13.19 | 6.3 |
| 7 | | 26.92 | 25.33 | 5.78 | 26.31 | 27.23 | 52.98 | 36.53 | 53.63 | 6.4 |

The test results for the treatment/control tumor-volume ratio (T/C) are presented in table 33 below:

Table 33

| Group | Treatment/control tumor volume ratio (T/C, %) at different days post-inoculation | | | | | | | | |
|---|---|---|---|---|---|---|---|---|---|
| | 5 | 8 | 11 | 14 | 17 | 22 | 25 | 29 | 32 |
| 1 | / | / | / | / | / | / | / | / | / |
| 2 | | 77.89 | 69.20 | 89.92 | 65.45 | 79.55 | 51.14 | 63.37 | 72.00 |
| 3 | | 59.56 | 53.87 | 46.26 | 29.30 | 40.49 | 29.43 | 39.55 | 34.89 |
| 4 | | 87.00 | 86.67 | 104.81 | 71.74 | 87.86 | 59.16 | 67.00 | 66.79 |
| 5 | | 80.04 | 68.79 | 80.43 | 54.70 | 48.35 | 30.67 | / | / |
| 6 | | 82.24 | 90.04 | 111.15 | 91.62 | 95.11 | 75.96 | 104.80 | 93.49 |
| 7 | | 74.61 | 77.28 | 95.10 | 73.64 | 73.16 | 46.71 | 63.93 | 52.29 |

From the foregoing data it is apparent that intravenous tail-vein administration of epirubicin drug substance (API) yielded, at the end of the study (D32), a tumor growth inhibition rate (calculated on the basis of tumor weight and tumor volume) of less than 60%, with T/C greater than 40%.By contrast, for high- and medium-dose intravenous tail-vein administration of the targeted drug Apt AS1411-4Bio-DNA-epirubicin, the tumor growth inhibition rate at the end of the study (D32 or D25), as calculated from tumor weight and tumor volume, exceeded 60% and T/C was less than 40% in both cases. For low-dose intravenous tail-vein administration of Apt AS1411-4Bio-DNA-epirubicin, the tumor growth inhibition rate at the end of the study (D32) was also markedly higher than that of the epirubicin drug substance at the same dose. These results indicate that intravenous tail-vein administration of Apt AS1411-4*Bio-DNA-epirubicin exerts a significant inhibitory effect on the growth of A549 lung tumors.

5. In-vivo efficacy study in the A549 mouse lung carcinoma model of the epirubicin-targeted drug prepared in Example 6 (Apt AS1411-Apt EGFR-Apt A15-DNA-epirubicin) and the epirubicin-targeted drug prepared in Example 20 (Apt AS1411-Apt EGFR-Apt A15-AmiR-21-DNA-epirubicin):

**Experimental Animals**

**Species/strain:** BALB/cAnSlacNifdc-nu

**Sex:** Male

Body weight: 17-21 g

Experimental Methods

1).Inoculation and grouping: For the A549 mouse lung carcinoma model, male BALB/cAnSlacNifdc-nu mice weighing 17.0-21.0 g were used. Under aseptic conditions, A549 tumor tissue was excised and cut into 2.0 mm × 2.0 mm × 2.0 mm tumor blocks, which were implanted subcutaneously into the left axillary dorsal region of nude mice. The day of implantation was designated as Day 0 (D0).When the tumor volume reached approximately 150.0 $mm^3$ (D7), animals were randomized by tumor volume into four groups: A (vehicle control), B, C, and D (drug), with 8 animals per group. On the day of randomization, all groups began intravenous (tail-vein) dosing. Groups A, B, C, and subgroup D1 were dosed once weekly for a total of 3 administrations; subgroup D2 was dosed twice weekly for a total of 6 administrations. Details of inoculation and grouping are shown in the table below.

2).Dosing regimen:

Table 34

| Group | Number of animals | Test article | Dose per administration | Route of administration | Dosing Regimen |
|---|---|---|---|---|---|
| A | 8 | PBS | -- | Intravenous (tail vein) | Once weekly, total of 3 administrations |
| B | 8 | Epirubicin drug substance (API) | 0.12mg/20g | Intravenous (tail vein) | Once weekly, total of 3 administrations |
| C | 8 | Apt AS 1411-Apt EGFR-AptA15-DNA-Epirubicin | 0.12mg/20g | Intravenous (tail vein) | Once weekly, total of 3 administrations |
| D | 8 | Apt AS 1411-Apt EGFR-AptA15-DNA-Epirubicin | 0.12mg/20g | Intravenous (tail vein) | Once weekly, total of 3 administrations |
| | | Apt AS 1411-Apt EGFR-Apt A15-AmiR-21-DNA-Epirubicin | 0.18μg/animal | Intravenous (tail vein) | Once weekly, total of 3 administrations |

**Assessment Parameters**

1).General clinical observation:

Mice are observed once daily throughout the study for clinical signs, including ulceration of tumor nodules, general condition/mentation, coat condition, behavior, and food intake and excretion.

2).Body weight:

Body weight is measured twice per week.Data are recorded and changes analyzed.

3).Tumor volume (V):

Tumor size is measured twice per week using a vernier caliper. The long diameter (a) and short diameter (b) are recorded. Tumor volume is calculated as: $V=0.5\times a\times b^2$.

4).Relative tumor volume (RTV):

$$RTV = V_t / V_0,$$

where $V_0$ is the tumor volume at grouping/first dosing (D0) and $V_t$ is the tumor volume at each measurement time point.

5).Tumor growth inhibition rate (TGI):

$$TGI\ (\%) = (1 - T/C) \times 100\%$$

$$T/C = RTV_{\text{treatment group}} / RTV_{\text{control group}}$$

Efficacy is concluded when TGI ≥60% and, after statistical analysis, the tumor volume of the treatment group is significantly lower than that of the vehicle control group (P<0.05), indicating a significant inhibitory effect on tumor growth.

All animals are closely monitored for tumor growth during the study; tumors are measured twice weekly, results recorded, and TGI calculated. The TGI test results are presented in Table 35, the tumor-volume change curves are shown in **Fig.16,** and the relative tumor-volume (RTV) curves are shown in **Fig.17:**

Table 35

| Group | Tumor growth inhibition rate (TGI, %) at different days post-inoculation | | | | | | |
|---|---|---|---|---|---|---|---|
| | 7 | 11 | 14 | 18 | 21 | 25 | 29 |
| A | 0.00 | 0.00 | 0.00 | 0.00 | 0.00 | 0.00 | 0.00 |
| B | | 27.01 | 45.82 | 43.61 | 44.97 | 46.31 | 49.64 |
| C | | 43.82 | 63.80 | 67.13 | 75.23 | 70.34 | 70.60 |
| D | | 41.57 | 61.48 | 64.36 | 69.99 | 69.55 | 71.07 |

The test results for the treatment/control tumor-volume ratio (T/C) are presented in table 36 below:

Table 36

| Group | Treatment/control tumor volume ratio (T/C, %) at different days post-inoculation | | | | | | |
|---|---|---|---|---|---|---|---|
| | 7 | 11 | 14 | 18 | 21 | 25 | 29 |
| A | / | / | / | / | / | / | / |
| B | | 71.0 | 54.68 | 56.91 | 55.52 | 53.29 | 50.10 |
| C | | 56.37 | 36.05 | 33.28 | 24.77 | 29.45 | 29.54 |
| D | | 56.79 | 36.75 | 33.76 | 28.18 | 28.14 | 26.71 |

From the data above, it can be seen that intravenous administration of epirubicin drug substance (API) resulted in a tumor growth inhibition rate (calculated based on tumor volume) of less than 60% at the end of the study (D29), with a T/C greater than 40%.In contrast, intravenous administration of the targeted drug Apt AS1411-Apt EGFR-Apt A15-DNA-epirubicin resulted in a tumor growth inhibition rate (calculated based on tumor volume) greater than 60% at the end of the study (D29), with a T/C less than 40%, indicating that intravenous administration of Apt AS1411-Apt EGFR-Apt A15-DNA-epirubicin significantly inhibits the growth of A549 lung cancer.

6. In-vivo efficacy study of the epirubicin-targeted drug Apt AS1411-Apt EGFR-Apt A15-DNA-epirubicin, prepared in Example 6, in the HepG2 mouse liver cancer model:

Experimental Animals

Species/strain: BALB/cAnSlacNifdc-nu

Sex: Male

Body weight: 16-18 g

Experimental Methods

1).Inoculation and grouping: For the HepG2 mouse liver cancer model, male BALB/cAnSlacNifdc-nu nude mice, weighing 16.0-18.0 g, were used.Under aseptic conditions, HepG2 tumor tissue was excised and cut into 2.0mm×2.0mm×2.0mm tumor blocks, which were uniformly implanted subcutaneously into the left axillary dorsal region of nude mice. The day of implantation was designated as Day 0 (D0).When the tumor volume reached approximately 250.0 $mm^3$ (D7), animals were randomized by tumor volume into three groups: A (control group), B, and C (treatment groups), with 8 animals per group.On the day of randomization, all animals began intravenous (tail-vein) dosing once weekly for a total of 3 administrations.
**2.Dosing regimen:**

Table 37

| Group | Number of animals | Test article | Dose per administration | Route of administration | Dosing regimen |
|---|---|---|---|---|---|
| 1 | 8 | PBS | PBS | Intravenous (tail vein) | Once weekly, for a total of 3 administrations |
| 2 | 8 | Epirubicin drug substance (API) | 6 mg/kg (0.12 mg/mouse) | Intravenous (tail vein) | Once weekly, for a total of 3 administrations |
| 3 | 8 | Apt AS 1411-Apt EGFR-AptA15-DNA-Epirubicin | 6 mg/kg (0.12 mg/mouse) | Intravenous (tail vein) | Once weekly, for a total of 3 administrations |

**Assessment Parameters**

1).General clinical observation:

Mice are observed once daily throughout the study for clinical signs, including ulceration of tumor nodules, general condition/mentation, coat condition, behavior, and food intake and excretion.

2).Body weight:

Body weight is measured twice per week.Data are recorded and changes analyzed.

3).Tumor volume (V):

Tumor size is measured twice per week using a vernier caliper. The long diameter (a) and short diameter (b) are recorded. Tumor volume is calculated as: $V=0.5\times a\times b^2$.

4).Relative tumor volume (RTV):

$$RTV = V_t / V_0 ,$$

where $V_0$ is the tumor volume at grouping/first dosing (D0), and $V_t$ is the tumor volume at each measurement time point.

5).Tumor growth inhibition rate (TGI):

$$TGI\ (\%) = (1 - T/C) \times 100\%$$

$$T/C = RTV_{\text{treatment group}} / RTV_{\text{control group}}$$

Efficacy is concluded when TGI ≥60% and, after statistical analysis, the tumor volume of the treatment group is significantly lower than that of the vehicle control group (P<0.05), indicating a significant inhibitory effect on tumor growth.

6).Tumor weight: At the end of the study (D23), the remaining animals were euthanized by cervical dislocation. Tumors were excised, weighed, and photographed. Tumor weight inhibition rate ($IR_{TW}$%) was calculated with:

$$IRTW(\%) = \frac{W\ \text{control group} - W\ \text{treatment group}}{W\ \text{control group}} \times 100,$$

where W refers to tumor weight.An $IR_{TW}$% greater than 60% was considered indicative of efficacy.

All animals were closely monitored for tumor growth during the study; tumors were measured twice weekly, results recorded, and TGI calculated. The TGI test results are presented in **Table 38,** the tumor volume change curves are shown in **Fig.18,** and the relative tumor volume (RTV) curves are shown in **Fig.19:**

Table 38

| Group | Tumor growth inhibition rate (TGI, %) at different days post-inoculation | | | | | |
|---|---|---|---|---|---|---|
| | 7 | 10 | 14 | 17 | 21 | 23 |
| 1 | 0.00 | 0.00 | 0.00 | 0.00 | 0.00 | 0.00 |
| 2 | | 16.33 | 36.16 | 24.38 | 24.72 | 22.08 |
| 3 | | 24.36 | 44.38 | 33.90 | 53.50 | 75.34 |

The test results for the treatment/control tumor volume ratio (T/C) are presented in table 39 below:

Table 39

| Group | Treatment/control tumor volume ratio (T/C, %) at different days post-inoculation | | | | | |
|---|---|---|---|---|---|---|
| | 7 | 10 | 14 | 17 | 21 | 23 |
| 1 | 0.00 | 0.00 | 0.00 | 0.00 | 0.00 | 0.00 |
| 2 | | 83.46 | 64.33 | 75.67 | 76.79 | 79.36 |
| 3 | | 74.12 | 54.11 | 65.38 | 46.07 | 25.75 |

At 23 days post-HepG2 inoculation, all animals were euthanized by cervical dislocation. Tumors were excised and weighed, and the tumor weight inhibition rate ($IR_{TW}$%) data are shown in table 40 below:

Table 40

| Group | Tumor weight (g) | Tumor weight inhibition rate, $IR_{TW}$ (%) |
|---|---|---|
| 1 | 1.98±0.48 | / |
| 2 | 1.48±0.52 | 25.20 |
| 3 | 0.47±0.19 | 76.48 |

From the data above, intravenous injection of epirubicin drug substance (API) resulted in a tumor growth inhibition rate (calculated based on tumor weight and tumor volume) of less than 60%, with a T/C ratio greater than 40%.However, intravenous injection of Apt AS1411-Apt EGFR-Apt A15-DNA-epirubicin resulted in a tumor growth inhibition rate (calculated based on tumor weight and tumor volume) exceeding 60%, with a T/C ratio less than 40% at the end of the study (D23). This demonstrates that intravenous injection of Apt AS1411-Apt EGFR-Apt A15-DNA-epirubicin has a significant inhibitory effect on the growth of HepG2 liver cancer.

7.In-vivo efficacy study of the epirubicin-targeted drug Apt TFRA3-Apt AS1411-Apt A15-DNA-epirubicin against

U251 glioblastoma xenograft models:

Cell Information

U251-RFP, a human glioma cell line expressing red fluorescent protein, sourced from Antai Kang Biotechnology (Beijing) Co., Ltd.

Experimental Animals

Species/strain: BALB/c nude (immunodeficient mice)

Age: 4 weeks

Sex: Female

Body weight: 15-18 g

Housing conditions: The animals were housed in an SPF-grade barrier facility at Antai Kang Biotechnology (Beijing) Co., Ltd., using a laminar flow cabinet in accordance with the facility's standards. The animals were kept three per cage in an environment with adequate lighting, good ventilation, and air-conditioning. Room temperature was maintained at 18-25°C, with a relative humidity of 50-70%.

Experimental Methods

1).Inoculation and grouping: Female 4-week-old BALB/c-nu mice (32 mice), purchased from Beijing Vito Li Hua Laboratory Animal Technology Co., Ltd.(Certificate number: SCXK Beijing 2016-0006), were used.Using a surgical orthotopic implantation technique, U251-RFP tumor tissue sourced from the subcutaneous tumors of genetically identical mice was excised, and necrotic tissue was removed under a stereomicroscope. The tumor tissue was cut into uniform 1 $mm^3$ blocks.After anesthetizing the mice with isoflurane, tumor tissue blocks were implanted into the brains of the mice under sterile conditions using the surgical orthotopic implantation method. The skull was repositioned with the implant method and sutured with 6-0 surgical sutures (ETHICON.INC) to close the scalp, establishing the U251-RFP human glioma orthotopic mouse model. The day of implantation was designated as Day 0 (D0).On Day 3 (D3), all mice underwent non-invasive real-time imaging of their brain tumors using a FluorVivo Model-100 fluorescence imager (INDEC BioSystems, CA, USA). The mice were randomly assigned to groups, and dosing began on the day of grouping.
2.Dosing regimen:

Table 41

| Gro up | Number of animals | Test article | Dose per administration | Dose volume | Route of administration | Dosing Regimen |
|---|---|---|---|---|---|---|
| 1 | 3 | PBS | -- | 200 μL | Intravenous (tail vein) | Twice weekly, for a total of 8 administrations |
| 2 | 3 | Positive control drug - Temozolomide | 15mg/Kg | 200 μL | Intraperiton | Daily, for 4 weeks |
| 3 | 3 | Epirubicin drug substance (API) | 6mg/Kg | 200 μL | Intravenous (tail vein) | Twice weekly, for a total of 8 administrations |
| 4 | 3 | Apt TfRA3-Apt AS 1411- Apt A15-DNA-epirubicin | 6 mg/Kg | 200 μL | Intravenous (tail vein) | Once weekly, for a total of 4 administrations |

All test articles are administered at a volume of 200 μL/20 g of body weight for mice.Actual doses need to be

calculated based on the mouse's body weight when determining the dosage.

**Assessment Parameters**

a) **Tumor Area:** Fluorescent area measurement is performed using a fluorescence imaging system, twice per week. Tumor growth inhibition rate (TGI, %) is calculated using the formula:

$$TGI\ (\%) = (1 - T/C) \times 100\%,\ T/C = RTV_{\text{treatment group}} / RTV_{\text{control group}},$$

where volume data are derived from the fluorescent area.

b) **Response after Drug Administration:** Body weight is measured alongside tumor volume measurements, twice per week. The relationship between body weight change and the dosing regimen is recorded.Survival and health status are also monitored, including activity levels and food intake during the dosing period.

c) **Tumor and Organ Imaging:** At the end of the dosing period, mice are euthanized, and their brain, heart, liver, spleen, lungs, and kidneys are excised. These organs, along with the corresponding in-situ tumor, are arranged and photographed. Tumor and organ imaging is then performed using the tumor's fluorescence wavelength and the epirubicin wavelength.

d) **Tumor Imaging:** In-situ tumors are excised, weighed, and photographed (using a camera).Imaging is performed with both the tumor's spontaneous fluorescence wavelength and the epirubicin wavelength.For tumor and organ imaging: One mouse from each group is selected, and the tumor (already excised), heart, liver, spleen, lungs, kidneys, and large intestine are harvested for imaging using both tumor spontaneous fluorescence and epirubicin wavelengths.After the experiment, the mouse carcasses and organs are discarded in a -20°C freezer.

$$IR_{TW}\ (\%) = (W_{\text{control group}} - W_{\text{treatment group}}) / W_{\text{control group}} \times 100,$$

where W denotes tumor weight.$IR_{TW}\% > 60\%$ is considered effective.

**Results**

During the dosing period, all animals maintained good general health, including activity and food intake. Around D10 post-dosing, the body weight of the mice in Group 3 started to decrease, and their spontaneous activity decreased, possibly due to drug toxicity.

Tumor growth was closely monitored throughout the study, with tumor size measured twice weekly. The tumor growth inhibition rate results are presented in table 42 below:

Table 42

| Group | Tumor growth inhibition rate (TGI, %) at different days post-inoculation | | | | | | | | Body weight change on Day 27 compared to pre-dosing |
|---|---|---|---|---|---|---|---|---|---|
| | 3 | 6 | 10 | 13 | 17 | 20 | 24 | 27 | |
| 1 | 0.00 | 0.00 | 0.00 | 0.00 | 0.00 | 0.00 | 0.00 | 0.00 | +15% |
| 2 | | 9.9 | 13.2 | 39.6 | 65.6 | 73.7 | 73.7 | 74.2 | +12% |
| 3 | | 35.8 | -3.9 | -7.1 | 19.4 | -- | -- | -- | -- |
| 4 | | 11.3 | 23.9 | 7.1 | 25.3 | 31.5 | 40.7 | 46.4 | -10% |

Tumor weight results and tumor weight inhibition rate (IRTW%) are shown in Table 43. Tumor photographs for Groups 1, 2, and 4 are shown in Figure 20:

Table 43

| Group | Number of animals | Tumor weight (g) | $IR_{TW}$% |
|---|---|---|---|
| 1 | 3 | 0.129±0.026 | -- |
| 2 | 3 | 0.002±0.001 | 98.34 |
| 3 | 3 | -- | -- |
| 4 | 3 | 0.012±0.014 | 90.63 |

From the table above, it can be seen that, compared to the epirubicin drug substance (API), the epirubicin-targeted drug Apt TfRA3-Apt AS1411-Apt A15-DNA-epirubicin provided in this invention exhibits a more significant inhibitory effect on U251-RFP tumor growth. Furthermore, compared to the positive control-temozolomide, this invention achieves a higher tumor growth inhibition rate at a lower dose.

8.In-vivo efficacy study of the epirubicin-targeted drug Apt TfRA4-Apt AS1411-Apt A15-DNA-epirubicin (1), prepared in Example 8, in the GL261-Luc mouse glioma model:

**Cell Culture**

GL261-Luc cells were purchased from Ningbo Mingzhou Biotechnology Co., Ltd. The species identification confirmed that they are mouse cell lines with no contamination from rat or human cells. The cells were cultured in DMEM medium containing 10% fetal bovine serum, 1% glutamine, and 1% HEPES, in a 37°C, 5% $CO_2$ cell culture incubator. The cells were passaged every 3 to 4 days once they reached confluence. Tumor cells in the logarithmic growth phase were used for in vivo tumor inoculation.

**Experimental Animals**

**Species/strain:** C57BL/6

**Grade:** SPF grade

**Age:** 6-8 weeks

**Sex:** Female

**Body weight:** 18-22 g

**Animal Housing:** The experimental animals were housed at Youji (Tianjin) Pharmaceutical Technology Co., Ltd.All animals were kept in barrier-controlled IVC systems.After receipt, the animals underwent at least 3 days of quarantine, and only animals that passed the quarantine were included in the formal study.During the study, the animal housing temperature was maintained at 20.5-24.5°C, humidity at 40-70%, and a 12-hour light/12-hour dark cycle.Each cage housed 3-5 animals, with access to sufficient water and feed throughout the experiment.

Experimental Methods

1).Inoculation and Grouping: GL261-Luc cells were resuspended in PBS to a concentration of $1 \times 10^9$ cells/mL and injected intracranially at a dose of 5 μL per mouse.After 7 days of tumor cell inoculation, the mice were randomly assigned to two groups, with 6 mice per group. The specific dosing regimen is as follows:
2).Dosing Regimen:

Table 44

| Group | Number of animals | Test article | Dose per administration | Route of administration | Dosing regimen |
|---|---|---|---|---|---|
| 1 | 6 | Physiological saline | -- | Intravenous (tail vein) | Once daily for 7 days |
| 2 | 6 | Apt TfRA4-Apt AS 1411-Apt A15-DNA-epirubicin (1) | 19mg/kg (380μg/20g) | Intravenous (tail vein) | Once every 7 days |

**Assessment Parameters**

**1)Imaging and Body Weight Monitoring:** After grouping, small animal in vivo imaging was performed on all mice in each group using a bioluminescence imaging (BLI) system. The BLI values were recorded, and the body weight of the mice was measured twice per week. The relationship between body weight changes and dosing was documented.Additionally, survival and general health conditions of the mice, including activity levels and food intake during the dosing period, were observed.

**2)Survival and Morbidity Monitoring:** The survival status of the tumor-bearing mice in each group was checked and recorded daily.When mice showed signs of morbidity or were near death, they were euthanized, and their survival time was recorded. The longest observation period was 138 days post-tumor cell inoculation.At the end of the experiment, the median survival time (MST) for tumor-bearing mice in each group was calculated. The survival prolongation rate (ILS%) for the treatment group was calculated using the following formula:
Increase in life span (ILS%) of tumor-bearing mice in the treatment group = [(median survival days of the treatment group ÷ median survival days of the vehicle control group) - 1] × 100%.3)The median survival time and survival prolongation rate for each group were calculated and subjected to statistical analysis.A significance level of $P<0.05$ was considered to indicate a significant difference. The BLI value curves for each group were plotted and analyzed, with $P<0.05$ considered statistically significant.

4)Upon the death of any mouse, brain tissue was fixed in formalin, embedded in paraffin, and pathological sections were prepared for hematoxylin and eosin (H&E) staining, followed by microscopic observation and photography.

5)At the end of the observation period, surviving mice were anesthetized, and blood was collected by ocular exsanguination. The mice were then euthanized, and brain tissue was harvested. The left and right femurs were also collected, and bone marrow cells were flushed from the marrow cavity using PBS. The brain tissue was fixed in formalin, embedded in paraffin, and pathological sections were prepared for H&E staining, followed by microscopic observation and photography. Peripheral blood and bone marrow cells were analyzed by flow cytometry to determine the proportions of CD4+ T cells, CD8+ T cells, and Treg cells.

**Data Processing and Statistical Analysis**

IBM SPSS Statistics 21.0 software was used for statistical analysis.One-Way ANOVA or Kruskal-Wallis tests were applied for inter-group statistical analysis of BLI values. Kaplan-Meier tests were used to analyze the survival times of tumor-bearing mice in each group.A significance level of $P<0.05$ was considered to indicate a statistically significant difference.

**Results**

**(1)Response to Drug Administration and Body Weight Changes**

After drug administration, the body weight change curves for each group are shown in **Fig.21,** and the percentage of body weight change is shown in **Fig.22.**
From the figures, it can be observed that the control group had a weight decrease of more than 15% by Day 39, while the Apt TfRA4-Apt AS1411-Apt A15-DNA-epirubicin treatment group exhibited normal general condition, no significant weight loss, no interruption of dosing, and no other abnormal signs or deaths.

**(2)In vivo Imaging Results**

On Day 5 post-tumor inoculation, bioluminescence imaging (BLI) was performed on the animals using a small animal in vivo imaging system, and the animals were then randomly assigned to the treatment groups. Bioluminescent imaging was performed twice per week, and the BLI curves were plotted and analyzed. The bioluminescent imaging results for each group of mice post-tumor inoculation are shown in **Fig.23** to **Fig.26** (Fig.23: dorsal view of the in vivo imaging, Fig.24: left-side view, Fig.25: right-side view, Fig.26: ventral view). The BLI value change curves for each group of mice are shown in **Fig.27** (Fig.27a: dorsal BLI value change curve, Fig.27b: left-side BLI value change curve, Fig.27c: right-side BLI value change curve, Fig.27d: ventral BLI value change curve).Statistical analysis of BLI for the GL261-Luc orthotopic xenograft tumor model is shown in **Table 45.**

Table 45

| BLI Imaging Site | Time Point (Days) | *P* |
|---|---|---|
| Dorsal | D8 | >0.05 |
| | D11 | >0.05 |
| | D15 | 0.017 |
| | D18 | 0.001 |
| | D21 | 0.006 |
| | D25 | 0.024 |
| | D28 | 0.014 |
| Left Side | D18 | 0.004 |
| | D21 | 0.007 |
| | D25 | 0.044 |
| | D28 | 0.038 |
| Right Side | D18 | 0.007 |
| | D21 | 0.009 |
| | D25 | 0.022 |
| | D28 | 0.017 |
| Ventral | D18 | 0.004 |
| | D21 | 0.007 |
| | D25 | 0.030 |
| | D28 | 0.024 |

From the above data, it can be observed that there is a significant difference between the Apt TfRA4-Apt AS1411-Apt A15-DNA-epirubicin treatment group and the control group.After 28 days, the number of surviving mice in the control group was insufficient for statistical analysis.In contrast, the average BLI of the treatment group showed an increase.After 102 days, the BLI of the treatment group mice remained at an extremely low level.

**(3)Survival Observation**

The survival curve is shown in **Figure 28,** and the statistical analysis results for the survival of the GL261-Luc orthotopic xenograft tumor model are presented in **Table 46.**

Table 46

| Group | Number of animals | MST (days) | ILS (%) | *P* |
|---|---|---|---|---|
| 1 | 6 | 32 | - | - |
| 2 | 6 | 66 | 106.25 | 0.001 |

The survival observation period lasted up to 138 days post-tumor cell inoculation. From the data, it is evident that the median survival time (MST) of the control group was 32 days, while the MST for the **Apt TfRA4-Apt AS1411-Apt A15-DNA-epirubicin (1)** treatment group was 66 days. The survival prolongation rate (ILS%) for the treatment group was 106.25%, which showed a significant difference compared to the control group (P=0.001).

**(4)Histopathological Examination of Brain Tissue in Model Group**

For the control group mice on D32, D39, and D41, under low magnification, it was observed that the majority of the brain tissue near the rostral edge was invaded by tumor cells, with tumors compressing and infiltrating the brain parenchyma near the caudal edge. Hemorrhages were seen around the tumor and within the brain ventricles.At high magnification, dense tumor cells were visible in the brain tissue, with varying nuclear sizes, significant atypia, and abundant mitotic figures. Necrotic foci in the tumor cells were also visible.

In the **Apt TfRA4-Apt AS1411-Apt A15-DNA-epirubicin (1)** treatment group, brain tissue from the 3 surviving mice on D138 showed intact structure without any significant abnormalities.

**(5)Flow Cytometry Detection of CD4+ T Cells, CD8+ T Cells, and Treg Cells in Peripheral Blood and Bone Marrow**

At Day 138, peripheral blood and bone marrow cells from the surviving mice treated with **Apt TfRA4-Apt AS1411-Apt A15-DNA-epirubicin (1)** were collected and analyzed by flow cytometry.Healthy 6-8 week-old C57BL/6 mice were used as the normal control group for comparison. The proportions of CD4+ T cells, CD8+ T cells, and Treg cells in peripheral blood and bone marrow are shown in **Table 47,** and the statistical analysis results for these cell populations in peripheral blood and bone marrow are presented in **Table 48:**

Table 47

| Group | Subgroup | CD4+ T Cell Proportion (%) | CD8+ T Cell Proportion (%) | Treg Cell Proportion (%) |
|---|---|---|---|---|
| Peripheral Blood | 1 | 58.46 ± 1.71 | 35.63 ± 1.31 | 0.34 ± 0.06 |
| | 2 | 49.46 ± 4.75 | 39.33 ± 1.75 | 3.27 ± 0.18 |
| Bone Marrow | 1 | 8.87 ± 1.27 | 24.08 ± 3.41 | 2.57 ± 0.30 |
| | 2 | 24.47 ± 3.15 | 48.73 ± 1.75 | 12.24 ± 1.14 |

Table 48

| **Group** | Subgroup | $P^a$ | $P^c$ | $P^c$ | $P^d$ | $P^e$ | $P^f$ |
|---|---|---|---|---|---|---|---|
| Peripheral Blood | 2 | >0.05 | >0.05 | >0.05 | - | - | - |
| Bone Marrow | 2 | - | - | - | 0.005 | 0.001 | 1.53E-4 |

Note: a: CD4+ T cells in peripheral blood, comparison between the treatment group and the normal control group; b: CD8+ T cells in peripheral blood, comparison between the treatment group and the normal control group; c: Treg cells in peripheral blood, comparison between the treatment group and the normal control group; d: CD4+ T cells in bone marrow, comparison between the treatment group and the normal control group; e: CD8+ T cells in bone marrow, comparison between the treatment group and the normal control group; f: Treg cells in bone marrow, comparison between the treatment group and the normal control group.

From the data above, it can be concluded that the proportions of CD4+ T cells, CD8+ T cells, and Treg cells in peripheral blood of the treatment group did not show a significant difference compared to the normal control group. However, the proportions of CD4+ T cells, CD8+ T cells, and Treg cells in bone marrow were significantly higher in the treatment group compared to the normal control group.

**Conclusion:**

1. The Apt TfRA4-Apt AS1411-Apt A15-DNA-epirubicin (1) drug exhibits significant tumor growth inhibition in the GL261-Luc orthotopic xenograft tumor model.

2. The Apt TfRA4-Apt AS1411-Apt A15-DNA-epirubicin (1) drug significantly increases the survival rate of mice in the GL261-Luc orthotopic xenograft tumor model.

3. Based on imaging and tissue pathology results, tumor complete regression was observed in the brain tissue of the 3 surviving mice in the Apt TfRA4-Apt AS1411-Apt A15-DNA-epirubicin (1) treatment group at D138.

4. In the Apt TfRA4-Apt AS1411-Apt A15-DNA-epirubicin (1) treatment group, the proportion of CD4+ T cells, CD8+ T cells, and Treg cells in peripheral blood was not significantly different from the normal control group.However, the proportions of CD4+ T cells, CD8+ T cells, and Treg cells in bone marrow were significantly higher compared to the normal control group.

5. Tumor-bearing mice demonstrated good tolerance to the **Apt TfRA4-Apt AS1411-Apt A15-DNA-epirubicin (1)** drug at the experimental dose.

The above is merely the preferred embodiment of the present invention and should not be construed as limiting. To those skilled in the art, various modifications and changes can be made to the present invention.Any modification, equivalent replacement, or improvement made within the spirit and scope of the present invention should be included within the scope of protection of the invention.

## Claims

**1.** A targeted chemical drug, wherein the targeted chemical drug comprises a targeted nucleic acid carrier and a small molecule chemical drug conjugated to the targeted nucleic acid carrier, wherein the targeted nucleic acid carrier comprises a nucleic acid carrier and a targeting molecule linked thereto; and wherein the nucleic acid carrier is a DNA carrier or RNA carrier, the nucleic acid carrier comprises sequence a, sequence b, and sequence c, which together form an assembly structure,

The sequence a is SEQ ID NO: 1: 5'-ACGAGCGTTCCG-3';
The sequence b is SEQ ID NO: 2: 5'-CGGTTCGCCG-3';
The sequence c is SEQ ID NO: 3: 5'-CGGCCATAGCCGT-3';
Alternatively,
The sequence a is SEQ ID NO: 4: 5'-ACGAGCGUUCCG-3';
The sequence b is SEQ ID NO: 5: 5'-CGGUUCGCCG-3';
The sequence c is SEQ ID NO: 6: 5'-CGGCCAUAGCCGU-3';
Optionally, at least one base substitution, insertion, or deletion occurs in sequence a, sequence b, or sequence c.

**2.** The targeted chemical drug according to claim 1, wherein the 5' and 3' ends of sequence a, sequence b, and sequence c are independently connected to an extended base segment, the extended base segment comprising 0 to 14 bases; Preferably, the nucleic acid carrier comprises the following sequences which together form an assembly structure:
First group of sequences:

sequence a: SEQ ID NO: 7: 5'-GCGGCGCCC**ACGAGCGTTCCG**GGAGC-3';
sequence b: SEQ ID NO: 8: 5'-GCTCC**CGGTTCGCCG**CCAGCCGCC-3';
sequence c: SEQ ID NO: 9: 5'-GGCGGCAGG**CGGCCATAGCCGT**GGGCGCCGC-3';
Alternatively,
Second group of sequences:

sequence a: SEQ ID NO: 10: 5'-GCGGCGCCC**ACGAGCGTTCCG**GGAGAGGAGC-3';
sequence b: SEQ ID NO: 11: 5'-GCTCCTCTCC**CGGTTCGCCG**CGAGCCGCG-3';
sequence c: SEQ ID NO: 12: 5'-CGCGGCTCG**CGGCCATAGCCGT**GGGCGCCGC-3';

Alternatively,
Third group of sequences:

sequence a: SEQ ID NO: 10: 5'-GCGGCGCCC**ACGAGCGTTCCG**GGAGAGGAGC-3';
sequence b: SEQ ID NO: 13: 5'-GCTCCTCTCC**CGGTTCGCCG**CCAGCCGCGG-3';
sequence c: SEQ ID NO: 14: 5'-GGCGGCTGG**CGGCCATAGCCGT**GGGCGCCGC-3';

Alternatively,
Fourth group of sequences:

sequence a: SEQ ID NO: 15: 5'-GCGGCGCCC**ACGAGCGTTCCG**GGAGAGGCC-3';
sequence b: SEQ ID NO: 16: 5'-GGCCTCTCC**CGGTTCGCCG**CCAGCCGCC-3';
sequence c: SEQ ID NO: 14: 5'-GGCGGCTGG**CGGCCATAGCCGT**GGGCGCCGC-3';

Alternatively,
Fifth group of sequences:

sequence a: SEQ ID NO: 17: 5'-GCGGCGCCC**ACGAGCGUUCCG**GGAGAGGCC-3';
sequence b: SEQ ID NO: 18: 5'-GGCCUCUCC**CGGUUCGCCG**CCAGCCGCC-3';
sequence c: SEQ ID NO: 19: 5'-GGCGGCUGG**CGGCCAUAGCCGU**GGGCGCCGC-3';

Alternatively,
Sixth group of sequences:

sequence a: SEQ ID NO: 20: 5'-GCGGCGCCC**ACGAGCGTTCCG**GGAGAGGAGGCC-3';
sequence b: SEQ ID NO: 21: 5'- GGCCTCCTCTCC**CGGTTCGCCG**CCAGCCGCC-3';
sequence c: SEQ ID NO: 14: 5'- GGCGGCTGGCGG**CCATAGCCGT**GGGCGCCGC-3';

Alternatively,
Seventh group of sequences:

sequence a: SEQ ID NO: 10: 5'-GCGGCGCCC**ACGAGCGTTCCG**GGAGAGGAGC-3';
sequence b: SEQ ID NO: 22: 5'-GCTCCTCTCC**CGGTTCGCCG**CCAGCCGCC-3';
sequence c: SEQ ID NO: 14: 5'-GGCGGCTGG**CGGCCATAGCCGT**GGGCGCCGC-3';

Alternatively
Eighth group of sequences:

sequence a: SEQ ID NO: 23: 5'-GCGACGCCC**ACGAGCGTTCCG**GGAGAGGAG-3';
sequence b: SEQ ID NO: 24: 5'- CTCCTCTCC**CGGTTCGCCG**CGAGCCGCG-3';
sequence c: SEQ ID NO: 25: 5'- CGCGGCACG**CGGCCATAGCCGT**GGGCGTCGC-3';

Alternatively,
Ninth group of sequences:

sequence a: SEQ ID NO: 26: 5'-GCGACGCCC**ACGAGCGTTCCG**GGAGAGGAGC-3';
sequence b: SEQ ID NO: 11: 5'-GCTCCTCTCC**CGGTTCGCCG**CGAGCCGCG-3';
sequence c: SEQ ID NO: 27: 5'- CGCGGCTCG**CGGCCATAGCCGT**GGGCGTCGC-3';

Alternatively,
Tenth group of sequences:

sequence a: SEQ ID NO: 28: 5'- GACGCCC**ACGAGCGTTCCG**GGAGAGG-3';
sequence b: SEQ ID NO: 29: 5'- CCTCTCC**CGGTTCGCCG**CGAGCCT-3';
sequence c: SEQ ID NO: 30: 5'-GGCTCG**CGGCCATAGCCGT**GGGCGTCTGCTGCTGCTGCTG -3';

Alternatively,
Eleventh group of sequences:

sequence a: SEQ ID NO: 31: 5'- GCCC**ACGAGCGTTCCG**GGAGA-3';
sequence b: SEQ ID NO: 32: 5'- TCTCC**CGGTTCGCCG**CCAGCCGCC-3';
sequence c: SEQ ID NO: 33: 5'- GGCGGCTGG**CGGCCATAGCCGT**GGGC-3'.

**3.** The targeted chemical drug according to claim 2, wherein the targeted chemical drug further comprises an

oligonucleotide effector molecule and/or an immune stimulant conjugated to the targeted nucleic acid carrier;

Preferably, the oligonucleotide effector molecule is selected from one or more of ASO, siRNA, miRNA, or nucleic acid aptamers;
More preferably, the siRNA is selected from one or more of ASAP1, ATAD2, CD24, CD47, EGFR, HBV, HSP, HS70, PD-L1, PAPP-1, Survivin, TAP, TIM-3, TGF-β1, VEGF-C; Further preferably, the siRNA is selected from one or more of ASAP1, CD47, PD-L1, TGF-β1; ;
More preferably, the miRNA is selected from one or more of A-miR21, A-miR-10a, A-miR-30c, miR-34, miR-542, miR-126-3p, miR-122; Further preferably, the miRNA is A-miR21;
More preferably, the nucleic acid aptamer is selected from DNA-based nucleic acid aptamers and/or RNA-based nucleic acid aptamers, with the nucleic acid aptamer selected from one or more of A1, A15, AS1411, AFP, ATP, Act-12c, A18, BAF7-1, C-Met-SL1, CH6, CA2, C12, CRAC Orail, CEA, CEA-18, CEA-T84, CSC1, CSC13, CD40, CD16a, CD19, CD3-4, CD44, CD12 (HDLBP), CD20, CD24, CD33, CD38, CD105, CD117, CD63, CD123, EGFR, EpCAM, EcR, FAP, GPC-1, GSK836, GPC3 (APS63-1), Her2, Her3, HMGA2, H2, HbsAg, IFN-y (B4), IL-4Ra, IL-17, LZH8, MUC1, M5, M7, M1, N5, N-G-Dua, NKG2D (20-N-15), NSE, Np-A15, Np-A48, Np-A58, Np-A61, OX40, PSMA, PDGFRβ, PDGF, PD-L1, PD-1, PTK-7, ProGRP-48, SF, TBA15, TBA29, TfRA4, TfRA3, TTA1, TLS9a, TGF-βII (S58), TNF-a, TNF, T1, VEGF , VCAM-1, VCAM-12d, CH6, PL-45, EP66, AGC, Karpas299, SW620, MDA-MB-231, MCF-7, PC-3, BCMA, CTLA-4, CCL1, CD4-3, CD28, FGF2 (F2), FGF2, FGF5, LAG-3, MRP1, TIM3, TIMC-11, VEGF165, 4-1BB; Further preferably, the nucleic acid aptamer includes one or more of: A1, A15, AS1411, C12, CD40L, EGFR, GPC-1, IL-4Ra, MUC1, OX40, PD-L1, TTA1, TfRA3, TfRA4;
Preferably, the immune stimulant is selected from one or more of CPG2006, CPG1826, CPG2216, CPG2395, CPG- ODNT7, CPG-ODN-PCIF1; More preferably, the immune stimulant is CPG2006.

**4.** The targeted chemical drug according to claim 3, wherein the sense and antisense strands of each of siRNAs have the following sequence structures from the 5' end to 3' end:

ASAP1: sense strand: SEQ ID NO: 34: 5'-UGAUAUUAUGGAAGCAAAUUU-3', antisense strand: SEQ ID NO: 35: 5'-AUUUGCUUCCAUAAUAUCAUU-3'; Alternatively, sense strand: SEQ ID NO: 36: 5'-UUAGGUUUGGGGUUGGAUCUU-3', antisense strand: SEQ ID NO: 37: 5'-GAUCCAACCCCAAACCUAAUU-3';
ATAD2: sense strand: SEQ ID NO: 38: 5'-AAUCCUACAACUUCGACGCUU -3', antisense strand: SEQ ID NO: 39: 5'-GCGUCGAAGUUGUAGGAUUUU-3';
CD24: sense strand: SEQ ID NO: 40: 5'-UGUUUACAUUGUUGAGGUAUU-3', antisense strand: SEQ ID NO: 41: 5'-UACCUCAACAAUGUAAACUUU-3';
CD47: sense strand: SEQ ID NO: 42: 5'-GGUGAUUACCCAGAGAUAUTT-3', antisense strand: SEQ ID NO: 43: 5'-AUAUCUCUGGGUAAUCACCTT-3'; Alternatively, sense strand: SEQ ID NO: 44: 5'-UGGUGAAAGAGGUCAUUCCUU-3', antisense strand: SEQ ID NO: 45: 5'-GGAAUGACCUCUUUCACCAUU-3'; Alternatively, sense strand: SEQ ID NO: 46: 5'-GGAAUGACCUCUUUCACCATT-3', antisense strand: SEQ ID NO: 47: 5'-UGGUGAAAGAGGUCAUUCCTT-3'; Alternatively, sense strand: SEQ ID NO: 48: 5'-GGUGAUUACCCAGA-GAUAUUU-3', antisense strand: 为 SEQ ID NO: 49: 5'-AUAUCUCUGGGUAAUCACCUU-3';
Preferably, in the siRNA targeting **CD47,** the bases are modified as follows: the two adjacent bases between the 2nd and 4th bases at the 5' end of the sense strand, the two adjacent bases between the 1st and 3rd bases at the 3' end of the sense strand, the two adjacent bases between the 1st and 2nd bases at the 5' end of the antisense strand, and the two adjacent bases between the 1st and 3rd bases at the 3' end of the antisense strand are sulfur-modified;
EGFR: sense strand: SEQ ID NO: 50: 5'-GGCUGGUUAUGUCCUCAUUUU-3', antisense strand: SEQ ID NO: 51: 5'-AAUGAGGACAUAACCAGCCUU-3'; Alternatively, sense strand: SEQ ID NO: 52: 5'-UUAGAUAAGACUGCUAAGGUU-3', antisense strand: SEQ ID NO: 53: 5'-UUAGAUAAGACUGCUAAGGUU-3'; Alternatively, sense strand: SEQ ID NO: 54: 5'-UGCCUUAGCAGUCUUAUCUAAUU-3', antisense strand: SEQ ID NO: 55: 5'-UUAGAUAAGACUGCUAAGGCAUU-3'; Alternatively, sense strand: SEQ ID NO: 78: 5'-UGCCUUAGCAGUCUUAUCUAAUUUU-3', antisense strand: SEQ ID NO: 79: 5'-AAUUAGAUAAGACUGCUAAGGCAUU-3';
HBV: sense strand: SEQ ID NO: 56: 5'-GGACUUCUCUCAAUUUUCUUU-3', antisense strand: SEQ ID NO: 57: 5'-AGAAAAUUGAGAGAAGUCCUU-3'; Preferably, the **HBV** includes a 2'-F modifications at C and U;
HSP: sense strand: SEQ ID NO: 58: 5'-CGCAGAACACCGUGUUCGAUU-3', antisense strand: SEQ ID NO: 59: 5'-UCGAACACGGUGUUCUGCGUU-3'; Preferably, in **HSP,** the two adjacent bases between the 1st and 3rd bases at the 5' end of the sense strand and the two adjacent bases between the 1st and 3rd bases at the 3' end of the antisense strand are sulfur-modified;

HS70: the sense strand is SEQ ID NO: 60: 5'-GGCCAACAAGAUCACCAUCUU-3', the antisense strand is SEQ ID NO: 61: 5'-GAUGGUGAUCUUGUUGGCCUU-3'; Preferably, in **HS70,** the two adjacent bases between the 1st and 3rd bases at the 5' end of the antisense strand and the two adjacent bases between the 1st and 3rd bases at the 3' end of the antisense strand are sulfur-modified;
PD-L1: the sense strand is SEQ ID NO: 62: 5'-CCAGCACACUGAGAAUCAAUU-3', the antisense strand is SEQ ID NO: 63: 5'-UUGAUUCUCAGUGUGCUGGUU-3'; alternatively, the sense strand is SEQ ID NO: 64: 5'-AGACGUAAGCAGUGUUGAATT-3', the antisense strand is SEQ ID NO: 65: 5'-UUCAACACUGCUUACGUCUTT-3';
PAPP-1: the sense strand is SEQ ID NO: 66: 5'-GAGGAAGGUAUCAACAAAUTT-3', the antisense strand is SEQ ID NO: 67: 5'-AUUUGUUGAUACCUUCCUCTT-3';
Survivin: The sense strand is SEQ ID NO: 70: 5'-GCAGGUUCCUUAUCUGUCACAUU-3', the antisense strand is SEQ ID NO: 71: 5'-UGUGACAGAUAAGGAACCUGCAGUU-3'; Alternatively,

The sense strand is SEQ ID NO: 72: 5'-GGAAUUGGAAGGCUGGGAACCUU-3', the antisense strand is SEQ ID NO: 73: 5'-GGUUCCCAGCCUUCCAAUUCCUU-3'; Preferably, the two adjacent bases between the 1st and 3rd bases at the 5' end of the sense strand are sulfur-modified, the two adjacent bases between the 1st and 3rd bases at the 5' end of the antisense strand are sulfur-modified, and the two adjacent bases between the 1st and 2nd bases at the 3' end of the antisense strand are sulfur-modified; Alternatively,
The sense strand is SEQ ID NO: 74: 5'-UGCAGGUUCCUUAUCUGUCATT-3', the antisense strand is SEQ ID NO: 75: 5'-UGACAGAUAAGGAACCUGCTT-3'; Alternatively,
The sense strand is SEQ ID NO: 76: 5'-CUGCAGGUUCCUUAUCUGUCACAUU-3', the antisense strand is SEQ ID NO: 77: 5'-UGUGACAGAUAAGGAACCUGCAGUU-3'; Preferably, the two adjacent bases between the 1st and 3rd bases at the 5' end of the sense strand and the 3' end of the sense strand are sulfur-modified, and the two adjacent bases between the 1st and 3rd bases at the 5' end of the antisense strand and the 3' end of the antisense strand are sulfur-modified;

TAP:

The sense strand is SEQ ID NO: 80: 5'-GCUGCACACGGUUCAGAAUUU-3', the antisense strand is SEQ ID NO: 81: 5'-AUUCUGAACCGUGUGCAGCUU-3'; Preferably, the two adjacent bases between the 1st and 3rd bases at the 5' end of the sense strand and the two adjacent bases between the 1st and 3rd bases at the 5' end of the antisense strand and the 3' end of the antisense strand are sulfur-modified; Alternatively, ,
The sense strand is SEQ ID NO: 82: 5'-CAGGAUGAGUUACUUGAAAUU -3', the antisense strand is SEQ ID NO: 83: 5'- UUUCAAGUAACUCAUCCUGUU -3';

TIM-3: the sense strand is SEQ ID NO: 86: 5'-GUGCUCAGGACUGAUGAAATT-3', the antisense strand is SEQ ID NO: 87: 5'-UUUCAUCAGUCCUGAGCACTT-3';
TGF-β1: the sense strand is SEQ ID NO: 88: 5'-GUCAACUGUGGAGCAACACUU-3', the antisense strand is SEQ ID NO: 89: 5'-GUGUUGCUCCACAGUUGACUU-3'; Alternatively, the sense strand is SEQ ID NO: 90: 5'-GCAACAACGCCAUCUAUGATT-3', the antisense strand is SEQ ID NO: 91: 5'-UCAUAGAUGGCGUUGUUGCTT-3';
VEGF-C:

The sense strand is SEQ ID NO: 92: 5'-GCAAGACGUUGUUUGAAAUUAUU-3', the antisense strand is SEQ ID NO: 93: 5'-UAAUUUCAAACAACGUCUUGCUU-3'; Preferably, the two adjacent bases between the 1st and 3rd bases at the 5' end of the sense strand are sulfur-modified, the two adjacent bases between the 1st and 3rd bases at the 5' end of the antisense strand are sulfur-modified, and the two adjacent bases between the 1st and 3rd bases at the 3' end of the antisense strand are sulfur-modified; Alternatively,
The sense strand is SEQ ID NO: 84: 5'-CAGCAAGACGUUGUUUGAAAUUAUU -3', the antisense strand is SEQ ID NO: 85: 5'- UAAUUUCAAACAACGUCUUGCUGUU -3';

Alternatively,
The sense strand is SEQ ID NO: 94: 5'-CAGGAUGGUAAAGACUACAUU-3', the antisense strand is SEQ ID NO: 95: 5'-UGUAGUCUUUACCAUCCUGUU-3'; Preferably, the two adjacent bases between the 1st and 3rd bases at the 5' end of the sense strand are sulfur-modified, the two adjacent bases between the 1st and 3rd bases at the 5' end of the antisense strand are sulfur-modified, and the two adjacent bases between the 1st and 3rd bases at the 3' end of the antisense strand are sulfur-modified;
Preferably, the miRNA includes one or more of miR-34, miR-542, miR-126-3p, and miR-122;

Preferably, the ASO includes one or more of A-miR21, A-miR-10a, A-miR-30c, and A-miR-1306;
The sequence structure of each miRNA and ASO from 5' to 3' is as follows:

A-miR21:

5'-GATAAGCT-3' ; Preferably, each base is locked nucleic acid-modified; Alternatively,
5'-GAUAAGCU-3' ; Preferably, each base is locked nucleic acid-modified;

Alternatively,

SEQ ID NO: 96: 5'-GTCAACATCAGTCTGATAAGCTA-3'; Alternatively,
SEQ ID NO: 97: 5'-GUCAACAUCAGUCUGAUAAGCUA-3'; Alternatively,
SEQ ID NO: 98: 5'-TCAACATCAGTCTGATAAGCTA; Alternatively,
5'-GATAAGCT-3'; Preferably, sulfur-modified between adjacent bases;

A-miR-10a: 5'-ACAGGGTA-3'; Preferably, each base is locked nucleic acid-modified;
A-miR-30c: SEQ ID NO: 99: 5'-GCTGAGAGTGTAGGATGTTTACA-3';
miR-34: 5'-TGTGACAG-3';
miR-542: 5'-TGGCAGTGT-3';
miR-126-3p: 5'-UCGUACC-3'; Preferably, sulfur-modified between adjacent bases;
miR-122: 5'-GGAAGTGT-3;
AmiR-1306: SEQ ID NO: 100: 5'-CATCACCACCAGAGCCAACGTC-3'; Preferably, sulfur-modified between the two adjacent bases between the 1st to 5th bases at the 5' end;

The sequence structure of each nucleic acid aptamer from 5' to 3' is as follows:

A1: SEQ ID NO: 101:
5'-GGTTGCATGCCGTGGGGAGGGGGGTGGGTTTTATAGCGTACTCAG-3';
A15: SEQ ID NO: 102: 5'-CCCTCCTACATAGGG-3'; Alternatively, SEQ ID NO: 227: 5'-CCCUCCUACAUAGGG-3';
AS1411: SEQ ID NO: 103: 5'-GGTGGTGGTGGTTGTGGTGGTGGTGG-3'; Alternatively, SEQ ID NO: 228: 5'-GGUGGUGGUGGUUGUGGUGGUGGUGG-3'; Alternatively, SEQ ID NO: 229:

AUUCUGAACCGUGUGCAGCACCACGCUGCACACGGUUCAGAAUACACACGAGGCTATC
TAGAATGTAC-3';

AFP: SEQ ID NO: 104:
5'-GGCAGGAAGACAAACAAGCTTGGCGGCGGGAAGGTGTTTAAATTCCCGGGTCTGCGT GGTCTGTGGTGCTGT-3'; Alternatively, SEQ ID NO: 105: 5'-ACCTGGGGAGTATTGGGGAGGAAGG-3';
ATP: SEQ ID NO: 106: 5'-ACCTGGGGAGTATTGGGGAGGAAGG-3'; Alternatively, SEQ ID NO: 107: 5'-GGGAGGACGATGCGGAGGAAGGGTAGG-3', Preferably, sulfur-modified between the two adjacent bases between the 1st to 4th bases at the 5' end;
Act-12c: SEQ ID NO: 108: 5'-CGGGGAAAGTCACGGGGGGTTTCAGATGTTCTGATCGGTGTGGAG-3';
A18: SEQ ID NO: 109: 5'-CCAGATAGTCCCTGG-3';
BAF7-1: SEQ ID NO: 110: 5'-GATAACGGGCACGAATTCGGAGTG-3';
C-Met-SL1: SEQ ID NO: 111: 5'-ATCAGGCTGGATGGTAGCTCGGTCGGGGTGGGTGGGTTGGCAAGT CTGAT-3';
CH6: SEQ ID NO: 112: 5'-AGTCTGTTGGACCGAATCCCGTGGACGCACCCTTTGGACG-3';
CA2: SEQ ID NO: 113: 5'-CCCACGTCTGCGCTTAGCTCCTGGCCTGGATGGGC-3';
C12: SEQ ID NO: 114: 5'-GTGGATTGTTGTGTTCTGTTGGTTTTTGTGTTGTC-3'; Preferably, sulfur-modified between the two adjacent bases between the 1st to 3rd bases at the 5' end;
CRAC Orail: SEQ ID NO: 115: 5'-CCAGTAGCCATACCGGTTTGTGGATGGGGTGTATGCGAGT-3';
CEA: SEQ ID NO: 116: 5'-CTAGGATCCCCACTCACCATCTCTCAGCTTGCTTCCTAGC-3'; Alternatively, SEQ ID NO: 234: 5'-CUAGGAUCCCCACUCACCAUCUCUCAGCUUGCUUCCUAGC-3'; CEA-18: SEQ ID NO: 117: 5'-TTAACTTATTCGACCATA-3';
CEA-T84: SEQ ID NO: 118:

5’-TCGCGCGAGTCGTCTGGGGAACCATCGAGTTACACCGACCTTCTATGTGCGGCCCCC CGCATCGTCCTCCC-3’;

CSC1: SEQ ID NO: 119:

5’-ACCTTGGCTGTCGTGTTGTAGGTGGTTTGCTGCGGTGGGCTCAAGAAGAAAGCGCAA AGAGGTCAGTGGTCAGAGCGT-3’;

CSC13: SEQ ID NO: 120:

5’-ACCTTGGCTGTCGTGTTGTGGGGTGTCGTATCTTTCGTGTCTTATTATTTTCTAGGTGGA GGTCAGTGGTCAGAGCGT-3’;

CD40: SEQ ID NO: 121: 5’-CCAACGAGTAGGCGATAGCGCGTGG-3’; Alternatively, SEQ ID NO: 122: 5’-AGAGACGATGCGGCCAACGAGTAGGCGATAGCGCGTGGCAGAGCGTCGCT-3’;
CD16a: SEQ ID NO: 123: 5’-CCACTGCGGGGGTCTATACGTGAGGAAGAAGTGG-3’;
CD19: SEQ ID NO: 124: 5’-TGCGTGTGTAGTGTGTGTCGTTTCTCCTTTTTTTGGTTGCTGCTCTTAGG GATTTGGGC GG-3’; Alternatively, SEQ ID NO: 125:

5’-TGCGTGTGTAGTGTGTCTGTTCTCCTTTTTTTGGTTGCTGCTCTTAGGGATTTGGGCGG -3’;

CD3-4: SEQ ID NO: 126:

5’-TCTCGGACGCGTGTGGTCGGCCGAGTGGCCCACGGTAGAAGGGTTAGAACTGCTGGT TGGTGAATCTCGCTGCCTGGCCCTAGAGTG-3’;

CD44:

SEQ ID NO: 127: 5’-CCAAGGCCTGCAAGGGAACCAAGGACACAG-3’; Alternatively,
SEQ ID NO: 128: 5’-CCAAGGCCTGCAAGGGAACCAAGGACACAG-3’; preferably, phosphorothioate modifications are introduced at the internucleoside phosphate linkages between adjacent nucleotides within nucleotides 3 to 5 (inclusive) counted from the 5’ terminus; at the internucleoside phosphate linkages between adjacent nucleotides within nucleotides 12 to 14 (inclusive) counted from the 5’ terminus; at the internucleoside phosphate linkage between nucleotides 1 and 2 counted from the 3’ terminus; at the internucleoside phosphate linkage between nucleotides 3 and 4 counted from the 3’ terminus; at the internucleoside phosphate linkage between nucleotides 5 and 6 counted from the 3’ terminus; and at the internucleoside phosphate linkages between adjacent nucleotides within nucleotides 12 to 14 (inclusive) counted from the 3’ terminus;

Alternatively,
SEQ ID NO: 237:

5’-GGGAUGGAUCCAAGCUUACUGGCAUCUGGAUUUGCGCGUGCCAGAAUAAAGAG UAUAACGUGUGAAUGGGAAGCUUCGAUAGGAAUUCGG-3’;

CD12 (HDLBP): SEQ ID NO: 129: 5’-GTGGATTGTTGTGTTCTGTTGGTTTTTGTGTTGTC-3’;
CD20: SEQ ID NO: 130: 5’-TGCGTGTGTAGTGTGTCTGTTTTTTATCTTCTTTTATCTACTCTTAGGGAT TTGGGCGG-3’ ;
CD24: SEQ ID NO: 131: 5’-TATGTGGGTGGGTGGGCGGTTATGCTGAGTCAGCCTTGCT-3’; Alternatively, SEQ ID NO: 132:

5'-ATCCAGAGTGACGCAGCATATGTGGGTGGGTGGGCGGTTATGCTGAGTCAGCCTTGCT TGGACACGGTGGCTTAGT-3';

CD33: SEQ ID NO: 133: 5'-TACCAGTGCGATGCTCAGCACGCTTATAGGGGCTGGACAAAATTCTACC CAGCCTTT-3';
CD38: SEQ ID NO: 134: 5'-TACGTGAATCTCGTACGATACTCTGTAAGCGT-3';
CD105: SEQ ID NO: 135: 5'-GATCAGTTTTCCATGCCAGTTGGTATTCCGCGACAGTTTGATCTC-3';
CD117: SEQ ID NO: 136: 5'-GGGGCCGGGGCAAGGGGGGGGTACCGTGGTAGGAC-3';
CD63: SEQ ID NO: 137: 5'-CACCCCACCTCGCTCCCGTGACACTAATGCTA-3';
CD123: SEQ ID NO: 138:

5'-TGCGTGTGTAGTGTGTCTGGGCTACATCGATGAGCTGCCTAGGGTCCCTCTTAGGGAT TTGGGCGG-3';

EGFR: SEQ ID NO: 139: 5'-GCCTTAGTAACGTGCTTTGATGTCGATTCGACAGGAGGC-3'; Alternatively, SEQ ID NO: 239: 5'-GCCUUAGUAACGUGCUUUGAUGUCGAUUCGACAGGAGGC-3'; Alternatively, SEQ ID NO: 240: 5'-UGCCGCUAUAAUGCACGGAUUUAAUCGCCGUAGAAAAGCAUGUCAAAGCCG UU-3';
EpCAM: SEQ ID NO: 140: 5'-CACTACAGAGGTTGCGTCTGTCCCACGTTGTCATGGGGGGTTGGCCT G-3'; Alternatively, SEQ ID NO: 141: 5'-GACAAACGGGGGAAGATTTGACGTCGACGAC-3'; Alternatively, SEQ ID NO: 241: 5'-GCGACUGGUUACCCGGUCG-3';
EcR: SEQ ID NO: 142: 5'-GCAGGTCCACTGCGGGGGTCTATACGTGAGGAAGAAGTGGGCAGGTC-3';
FAP: SEQ ID NO: 143: 5'-TGGGGGTTGAGGCTAAGCCGA-3';
Anti-FAP: SEQ ID NO: 144: 5'-CCGCTCGAGCTAGTCTGACAAAGAGAAACAC-3'; GPC-1: SEQ ID NO: 145: 5'-AACGGAGTGTGGCTAACTCGA-3';
GSK836: SEQ ID NO: 147: 5'-GCAGAGGTGAAGCGAAGTCG-3'; Preferably, phosphorothioate modifications are present at the internucleoside phosphate linkages between adjacent nucleotides within nucleotides 1 to 6 (inclusive) counted from the 5' terminus;
GPC3 (APS63-1): SEQ ID NO: 148: 5'-TAACGCTGACCTTAGCTGCATGGCTTTACATGTTCCA-3'; preferably, phosphorothioate modifications are present at the internucleoside phosphate linkages between adjacent nucleotides within nucleotides 1 to 4 (inclusive) counted from the 5' terminus and within nucleotides 1 to 4 (inclusive) counted from the 3' terminus;
Her2: SEQ ID NO: 149: 5'-AGCCGCGAGGGGAGGGATAGGGTAGGGCGCGGCT-3'; Alternatively, SEQ ID NO: 150: 5'-GGGAGCTCAGAATAAACGCTCAAAGGGTCAAGCTGATTACACTTTGTCCACTATTGG GT CCTTCGACATGAGGCCCGGATC-3'; Alternatively, SEQ ID NO: 246: 5'-AGCCGCGAGGGGAGGGAUAGGGUAGGGCGCGGCU-3';
Her3: SEQ ID NO: 151: 5'-GGGAGCTCAGAATAAACGCTCAAGGCTAACAGCACGCAACGGGGGGGA GTAATCGTG TCTGTTCGACATGAGGCCCGGATC-3'; Alternatively, SEQ ID NO: 247: 5'-CAGCGAAAGUUGCGUAUGGGUCACAUCGCAGGCACAUGUCAUCUGGGCG-3'; Alternatively, SEQ ID NO: 248: 5'-GAAUUCCGCGUGUGCCAGCGAAAGUUGCGUAUGGGCCACAUCGCAGGCACAUGUC AUCUGGGCGGUCCGUUCGGGAUCC-3';
HMGA2: SEQ ID NO: 152: 5'-GGAAAAAATTTTTTAAAAAACCC-3'; preferably, phosphorothioate modifications are present at the internucleoside phosphate linkages between adjacent nucleotides;
H2: SEQ ID NO: 146: 5'-GGGCCGTCGAACACGAGCATGGTGCGTGGACCTAGGATGACCTGAGTACT GTCC-3';
HBsAg: SEQ ID NO: 153: 5'-CACAGCGAACAGCGGCGGACATAATAGTGCTTACTACGAC-3'; preferably, phosphorothioate modifications are present at the internucleoside phosphate linkages between adjacent nucleotides within nucleotides 1 to 4 (inclusive) counted from the 5' terminus;
IFN-y (B4): SEQ ID NO: 154:

5'-CCGCCCAAATCCCTAAGAGAAGACTGTAATGACATCAAACCAGACACACACACTACACA CGCA-3';

IL-4Ra: SEQ ID NO: 155: 5'-GGAGGACGAUGCGGAAAAAGCAACAGGGUGCUCCAUGCGCAUGGAA CCUGCGCGC AGACGACUCGCUGAGGAUCCGAGA-3'; Alternatively, SEQ ID NO: 156: 5'-AAAAAGCAACAGGGUGCUCCAUGCGCAUGGAACCUGCGCG-3';
IL-17: SEQ ID NO: 157: 5'-CTTGGATCACCATAGTCGCTAGTCGAGGCT-3'; Alternatively, SEQ ID NO: 158: 5'-GCGGCATCCTATCACGCATTGACC-3';
LZH8: SEQ ID NO: 159:

5'-ATCCAGAGTGACGCAGCATATTAGTACGGCTTAACCCCATGGTGGACACGGTGGCTTAGT-3';

MUC1: SEQ ID NO: 160: 5'-GCAGTTGATCCTTTGGATACCCTGG-3'; Alternatively, SEQ ID NO: 161: 5'-GAAGTGAAAATGACAGAACACAACA-3'; Alternatively, SEQ ID NO: 162: 5'-AACCGCCCAAATCTCTAAGAGTCGGACTGCAACCTATGCTATCGTTGATGTCTGTCCAA GCAACACAGACACACTACACACACGCACA-3'; Alternatively, SEQ ID NO: 163: 5'-AATGACAGAACACAACATT-3'; Alternatively, SEQ ID NO: 250: 5'-GCAGUUGAUCCUUUGGAUACCCUGG-3';
M5: SEQ ID NO: 164:

5'-AGCAGCACAGAGGTCAGATGCTTGGTTCCACCGTACTGACTGTAGTAAAATCTGATCACTCCTATGCGTGCTACCGTGAA-3';

M7: SEQ ID NO: 165:

5'-AGCAGCACAGAGGTCAGATGTAGTCGGTCTTCTTGTTTGAAACTGCTAATTTTGAAAAAACCTATGCGTGCTACCGTGAA-3';

M1: SEQ ID NO: 166:

5'-AGCAGCACAGAGGTCAGATGATATAACCTTAATAAATAAAATATAAATTATTTAATCTTACCTATGCGTGCTACCGTGAA-3';

N5: SEQ ID NO: 167: 5'-GATTGAGTAGATAGTGGTTCTGTACGTAGTGAAAGAGTGG-3';
N-G-Dua: SEQ ID NO: 168:

5'-CAAGTTGCTCGTCGCGATACGTTTGGTTGGTGTGGTTGGCAGTATCGCAGGTCCAAGTTGCTCGTCGCGATACAACGGAGTGTGGCTAACTCGA-3';

NKG2D (20-N-15): SEQ ID NO: 169: 5'-CAAGTTGCTCGTCGCGATACGTTTGGTTGGTGTGGTTGGCAGTATC-3';
NSE: SEQ ID NO: 170: 5'-TCACACGGACCTCTCTCTACATTAATTGCGCATTTCGTT-3';
Np-A15: SEQ ID NO: 171: 5'-GCTGGATGTTCATGCTGGCAAAATTCCTTAGGGGCACCGTTACTTTGACACATCCAGC-3 ';
Np-A48: SEQ ID NO: 172: 5'-GCTGGATGTCGCTTACGACAATATTCCTTAGGGGCACCGCTACATTGACACATCCAGC-3 ';
Np-A58: SEQ ID NO: 173: 5'-GCTGGATGTCACCGGATTGTCGGACATCGGATTGTCTGAGTCATATGACACATCCAGC-3';
Np-A61: SEQ ID NO: 174: 5'-GCTGGATGTTGACCTTTACAGATCGGATTCTGTGGGGCGTTAAACTGACACATCCAGC-3';
OX40: SEQ ID NO: 175: 5'-GGGAGGACGATGCGGCAGTCTGCATCGTAGGAATCGCCACCGTATACTTTCCCACCAG ACGACTCGCTGAGGATCCGAGA-3'; Alternatively, SEQ ID NO: 176: 5'-CAGTCTGCATCGTAGGATTAGCCACCGUATCTTTCCCAC-3'; Alternatively, SEQ ID NO: 177: 5'-CCAACGAGTAGGCGATAGCGCGTGG-3'; Alternatively, SEQ ID NO: 252: 5'-GGGAGGACGAUGCGGCAGUCUGCAUCGUAGGAAUCGCCACCGUAUACUUUCCCAC CAGACGACUCGCUGAGGAUCCGAGA-3'; Alternatively, SEQ ID NO: 253: 5'-GGGAGGACGAUGCGGCAGUCUGCAUCGUAGGAAUCGCCACCGUAUACUUUCCCAC CAGACGACUCGCUG-3'; Alternatively, SEQ ID NO: 254: 5'-CAGUCUGCAUCGUAGGAAUCGCCACCGUAUACUUUCCCAC-3'; Alternatively, SEQ ID NO: 255: 5'-GGGAUGCGGAAAAAAGAACACUUCCGAUUAGGGCCCACCCUAACGGCCGCAGAC-3';

PSMA: SEQ ID NO: 179: 5'-GGGAGGACGATGCGGATCAGCCATGTTTACGTCACTCCT-3'; Alternatively, SEQ ID NO: 180: 5'-GCGTTTTCGCTTTTGCGTTTTGGGTCATCTGCTTACGATAGCAATGCT-3'; Alternatively, SEQ ID NO: 256: 5'-GGGACCGAAAAAGACCUGACUUCUAUACUAAGUCUACGUUCCC-3'; Alternatively, SEQ ID NO: 257: 5'-GGGAGGACGAUGCGGAUCAGCCAUGUUUACGUCACUCCU-3';
PDGFRβ: SEQ ID NO: 181: 5'-TGTCGTGGGGCATCGAGTAAATGCAATTCGACA-3'; Alternatively, SEQ ID NO: 258: 5'-UGUCGUGGGGCAUCGAGUAAAUGCAAUUCGACA-3';
PDGF: SEQ ID NO: 182: 5'-CAGGCTACGGCACGTAGAGCATCACCATGATCCTG-3';
PD-L1: SEQ ID NO: 183: 5'-AACAACGTATAACAATGCCCACGTCACCAGAGTACTATGG-3'; Alternatively, SEQ ID NO: 184: 5'-GCCCCAGTTATGCTTTCCCCCTCTGTCTCTTTG-3'; Alternatively, SEQ ID NO: 185: 5'-ATCGCCCGCAGCACCCATTTGTTTTTTTTTG-3'; Alternatively, SEQ ID NO: 69: ACGGGCCACATCAACTCATTGATAGACAATGCGTCCACTGCCCGT; Alternatively, SEQ ID NO: 186: 5'-TGCCCGCACA TCAACTCATTGATAGACAATGCGTCCACTCGGGCA-3'; Alternatively, SEQ ID NO: 187: 5'-TGCCCGC ACATCAACTCATTGATAGACAATGCGTCCACTACGGGC-3'; Alternatively, SEQ ID NO: 188: 5'-CGGGC ACACATCAACTCATTGATAGACAATGCGTCCACTGCCCGT-3'; Alternatively, SEQ ID NO: 189: 5'-GTT GGTCACATCAACTCATTGATAGACAATGCGTCCACTACCAAC-3'; Alternatively, SEQ ID NO: 190: 5'-G GGCCACATCAACTCATTGATAGACAATGCGTCCACTGCCC -3'; Alternatively, SEQ ID NO: 191: 5'-TG GTTGCACATCAACTCATTGATAGACAATGCGTCCACTCAACCA-3'; Alternatively, SEQ ID NO: 200: 5'-TACAGGTTCTGGGGGGTGGGTGGGGAACCTGTT-3'; Alternatively, SEQ ID NO: 238: 5'-CTACGAGA CGAACTTATGCGTAATAATGACTGTCGTAG-3';
PD-1: SEQ ID NO: 192: 5'-GACGATAGCGGTGACGGCACAGACGGTACAGTTCCCGTCCCTGCACTAC ACGTATGCC GCTTCCGTCCGTCGCTC-3'; Alternatively, SEQ ID NO: 193: 5'-GAGCGACGGACGGAA GCGGCATACGTGTAGTGCAGGGACGGGAACTGTACCGTCTGT GCCGTCACCGCTATCGTC-3'; Alternatively, SEQ ID NO: 194: 5'-GGATCCTAGACGCATTGACCCGCTGCCTCTACTGAGGCTGTGTCAGT GTGCGGCTCG GACTGTTGAATTC-3'; Alternatively, SEQ ID NO: 195: 5'-AGCGGTGACGGCACAGAC GGTACAGTTCCCGTCCCTGCACTACACGTATGCCGCTGA GAGAGAGGGAGGC-3'; Alternatively, SEQ ID NO: 196: 5'-ACCGACAGTGAAGGACTCAGCGAACTCTCAGACTCGGTTC-3';
PTK-7: SEQ ID NO: 197: 5'-ATCTAACTGCTGCGCCGCCGGGAAAATACTGTACGGTTAGA-3';
ProGRP-48: SEQ ID NO: 198: 5'-CATGCGGAGTAGAGCGAGCCCAGATAGTCCCTGGTTATTTCCTTA GG-3';
SF: SEQ ID NO: 199: 5'-GATCTCTCTCTGCCCTAAGTCCGCACCCGTGCTTCCCTGT-3';
TBA15: SEQ ID NO: 201: 5'-GGTTGGTGTGGTTGG-3';
TBA29: SEQ ID NO: 202: 5'-AGTCCGTGGTAGGGCAGGTTGGGGTGACT-3';
TfRA4: SEQ ID NO: 203: 5'-GCGTGGTACCACGC-3'; Alternatively, SEQ ID NO: 259: 5'-GCGUGGUACCACGC-3';
TfRA3: SEQ ID NO: 204: 5'-GCGTGGTCACACGC-3'; Alternatively, SEQ ID NO: 205: 5'-GCGGCGCCC ACGAGCGTTCGCGTGGTCACACGCGTTCCGCCCTCCTACATAGGGCGC ATAGCCGTGGGCGCCG C-3'; Alternatively, SEQ ID NO: 260: 5'-GCGUGGUCACACGC-3';
TTA1: SEQ ID NO: 206: 5'-CCTGCACTTGGCTTGGATTTCAGAAGGGAGACCC-3'; Alternatively, SEQ ID NO: 286: 5'-CTGCACTTGGCTTGGATTTCAGAAGGGAGACCC-3'; Alternatively, SEQ ID NO: 261: 5'-CCUGCACUUGGCUUGGAUUUCAGAAGGGAGACCC-3';
TLS9a: SEQ ID NO: 207: 5'-AGTCCATTTTATTCCTGAATATTTGTTAACCTCATGGAC-3';
TGF-βII (S58): SEQ ID NO: 208:

5'-ACATTGCTGCGTGATCGCCTCACATGGGTTTGTCTGGTCGATTTGGAGGTGGTGGGTG GC-3';

TNF-a: SEQ ID NO: 209: 5'-GCGGCCGATAAGGTCTTTCCAAGCGAACGAAAA-3';
TNF: SEQ ID NO: 210: 5'-GCGCCACTACAGGGGAGCTGCCATTCGAATAGGTGGGCCGC-3';
T1: SEQ ID NO: 211:

5'-CGCTCGATAGATCGAGCTTCGCTCGATGTGGTGTTGTGGGGGCTTGTATTGGTCGATC ACGCTCTAGAGCACTG-3';

VEGF: SEQ ID NO: 212: 5'-TGTGGGGGTGGACTGGGTGGGTACC-3'; Alternatively, SEQ ID NO: 213: 5'-TGTGGGGGTGGACGGGCCGGGTAGA-3'; Alternatively, SEQ ID NO: 214: 5'-GGTGGGGGTGGACGG GCCGGGTAGA-3'; Alternatively, SEQ ID NO: 266: 5'-AUGCAGUUUGAGAAGUCGCGCAU-3'; preferably, a phosphorothioate modifications are present at the internucleoside phosphate linkage between nucleotides

6 and 7 counted from the 5' terminus;
VCAM-1: SEQ ID NO: 215: 5'-ATACCAGCTTATTCAATTGGACACGGCAAAGGGGTATAGCCTACCGGA CCGTGAACATG GAATGGTGTGCTGCGTGGAGATAGTAAGTGCAATCT-3'; Alternatively, SEQ ID NO: 216: 5'-GGACACGGCAAAGGGGTATAGCCTACCGGACCGTGAACATGGAATGGTGTGCTGCGT GG-3';
VCAM-12d: SEQ ID NO: 217: 5'-AGGGAATCTTGCCTAGGGAGGGAGTAGCGAAAGGGCTCA-3';
CH6: SEQ ID NO: 218: 5'-AGTCTGTTGGACCGAATCCCGTGGACGCACCCTTTGGACG-3';
PL-45: SEQ ID NO: 219: 5'-ACTCATAGGGTTAGGGGCTGCTGGCCAGATACTCAGATGGTAGGGTTAC TATGAGC-3';
EP66: SEQ ID NO: 220: 5'-AACAGAGGGACAAACGGGGGAAGATTTGACGTCGACGACA-3';
AGC: SEQ ID NO: 221: 5'-CGACCCGGCACAAACCCAGAACCATATACACGATCATTCGTCTCCTGGG CCG-3';
Karpas299: SEQ ID NO: 222: 5'-ATCCAGATGACGCAGCACCACCACCGTACAATTTTTTCATTACCTAC TCGGC-3';
SW620: SEQ ID NO: 223: 5'-CCCATCAATGTTACGACCCGCTAGGGCTGCTGTGCCATCGGGTAA-3';
MDA-MB-231: SEQ ID NO: 224: 5'-AGAATTCAGTCGGACAGCGAAGTAGTTTTCCTTCTAACCTAAGAA CCCGCGGCAGTTTA ATGTAGA-3';
MCF-7: SEQ ID NO: 225: 5'-GCATGGGGTTTCGGCGTTTCGTCTATCTTGTTTCTGTTAGCGTCT-3';
PC-3: SEQ ID NO: 226: 5'-TGCCACTACAGCTGGTTCGGTTTGGTGACTTCGTTCTTCGTTGTGGTGC TTAGTGGC-3' ;
BCMA: SEQ ID NO: 230: 5'-AGUGCAAGACGUUCGCAGAUUAGCGAAAAGAGGGUCUCAUUGACUA GUAC-3';
CTLA-4: SEQ ID NO: 231: 5'-GGUGGAAGAGGGAUGGGCCGACGUGCCGCAU-3'; Alternatively, SEQ ID NO: 232:

5'-TCCCTACGGCGCTAACGATGGTGAAAATGGGCCTAGGGTGGACGGTGCCACCGTGCT ACAAC-3';

CCL1: SEQ ID NO: 233: 5'-UGACUCCUCUGACAGCCUAAUUUCUCCCGAUUACCCUG-3';
CD4-3: SEQ ID NO: 235: 5'-GGGAGGACGAUGCGGUUUGGGGUUUUCCCGUGCCCCAGACGACUC GCCCGA-3';
CD28: SEQ ID NO: 236:

5'-GGGAGAGAGGAAGAGGGAUGGGGAUUAGACCAUAGGCUCCCAACCCCCCCGGGA GAGAGGAAGAGGGAUGGGGAUUAGACCAUAGGCUCCCAACCCCCGGG-3';

FGF2 (F2): SEQ ID NO: 242: 5'-GGGAUACUAGGGCAUUAAUGUUACCAGUGUAGUCCC-3';
FGF2: SEQ ID NO: 243: 5'-GGGAAACUAGGGCGUUAACGUGACCAGUGUUUCUCGA-3'; Alternatively, SEQ ID NO: 244: 5'-GGGAAACUAGGGCGUUAACGUGACCAGUGUUUCCC-3';
FGF5: SEQ ID NO: 245:

5'-GGGCGACCUCUCCGUACUGACCUACAGAGCGACAUACUAGUGUAUCCAGAUCGCCC -3';

LAG-3: SEQ ID NO: 249:

5'-GGGAGAGAGAUAUAAGGGCCUCCUGAUACCCGCUGCUAUCUGGACCGAUCCCAUUA CCAAAUUCUCUCCC-3';

MRP1: SEQ ID NO: 251: 5'-GGGAGAAUAGUCAACAAAUCGUUUGGGGCGACUUCUCCUUCCUUUC UCCC-3';
TIM3: SEQ ID NO: 262: 5'-GGGAGAGGACCAGUA-GCCACUAUGGUGUUGGAGCUAGCGG-CAGAGCGUCGCGGU CCCUCCC-3'; Alternatively, SEQ ID NO: 263: 5'-GGGAGAGGACCAGUA -CUGGUAGUUCUCUGUGCGACUCCUA-CAGAGCGUCGCGGUCCCUCCC-3';
TIMC-11: SEQ ID NO: 264: 5'-AAGCAACACUUAGUCGCGAUUGAUACGUGCGCAGUCAU-3'; Alternatively, SEQ ID NO: 265:

5’-GGAGGACGAUGCGGGGAAGCAACACUUAGUCGCGAUUGAUACGUGCGCAGUCAUC AGACGACUCGCUGAGGAUCCGAGA-3’;

VEGF165: SEQ ID NO: 267: 5’-CGGAAUCAGUGAAUGCUUA UACAUCCG-3’;
4-1BB: SEQ ID NO: 268:

5’-GGGAGAGAGGAAGAGGGAUGGGCGACCGAACGUGCCCUUCAAAGCCGUUCACUAA CCAGUGGCAUAACCCAGAGGUCGAUAGUACUGGAUCCCCCC-3’;

The sequences of each immunostimulant from 5’ to 3’ are as follows:

CPG2006:

SEQ ID NO: 269: 5’-TCGTCGTTTTGTCGTTTTGTCGTT-3’; Preferably, phosphorothioate modifications are present at the internucleoside phosphate linkages between adjacent nucleotides; Alternatively,
SEQ ID NO: 270: 5’-UCGUCGUUUUGUCGUUUUGUCGUU-3’;

CPG1826: SEQ ID NO: 271: TCCATGACGTTCCTGACG-3’; Preferably, phosphorothioate modifications are present at the internucleoside phosphate linkages between adjacent nucleotides;
CPG2216: SEQ ID NO: 272: 5’-GGGGGACGATCGTCGGGGGG-3’;
CPG2395: SEQ ID NO: 273: TCGTCGTTTTCGGCGCGCGCCG-3’;
CPG-ODNT7: SEQ ID NO: 274: 5’-TCGTCGTCGTCGTCGTCGTCG-3’; Alternatively, SEQ ID NO: 275: 5’-TCGTCGTCGTCGTCGTCGTCGTCG-3’;
CPG-ODN-PCIF1: 5’-AGCGAA-3’.

**5.** The targeted chemical drug according to claim 3 or 4, wherein, when the targeted chemical drug comprises the nucleic acid aptamer, the targeting moiety comprises the nucleic acid aptamer and/or a small-molecule targeting ligand; when the targeted chemical drug does not comprise the nucleic acid aptamer, the targeting moiety is a small-molecule targeting ligand; wherein the small-molecule targeting ligand is selected from the group consisting of folic acid, biotin, vitamin B12, and mannose.

**6.** The targeted chemical drug according to claim 5, wherein the small-molecule chemical drug is selected from the group consisting of anthracycline chemotherapeutics, pyrimidine chemotherapeutics, platinum chemotherapeutics, glutamic-acid-derivative chemotherapeutics, flavonoid chemotherapeutics, phytomedicines and derivatives thereof, folate chemotherapeutics, salicylic-acid chemotherapeutics, and acridine chemotherapeutics;

Preferably, the anthracycline chemotherapeutic is selected from the group consisting of doxorubicin, epirubicin, pirarubicin, daunorubicin, idarubicin, mitoxantrone, and valrubicin, or the free base or hydrochloride thereof;
Preferably, the pyrimidine chemotherapeutic is selected from gemcitabine, 5-fluorouracil, cytarabine, or capecitabine;
Preferably, the platinum chemotherapeutic is selected from cisplatin, oxaliplatin, carboplatin, nedaplatin, or lobaplatin;
Preferably, the glutamic-acid-derivative chemotherapeutic is selected from lenalidomide, thalidomide, or pomalidomide;
Preferably, the flavonoid chemotherapeutic is selected from flavones, flavonols, dihydroflavones, isoflavones, or chalcones;
Preferably, the phytomedicine or derivative thereof is selected from vincristine, dihydroartemisinin, paclitaxel, maytansine, docetaxel, or 10-hydroxycamptothecin;
Preferably, the folate chemotherapeutic is methotrexate;
Preferably, the salicylic-acid chemotherapeutic is selected from aspirin or sodium salicylate;
Preferably, the acridine chemotherapeutic is selected from tacrine or anacridine;
More preferably, the small-molecule chemical drug is selected from one or more of epirubicin or the free base or hydrochloride thereof, gemcitabine, 5-fluorouracil, paclitaxel, and maytansine.

**7.** The targeted chemical drug according to claim 6, wherein, when the small-molecule targeting ligand, the oligonucleotide effector molecule, and the immunostimulant are linked to the nucleic acid carrier, they are either directly linked to

terminal bases of the respective sequences of the nucleic acid carrier, or are linked to the terminal bases of the respective sequences of the nucleic acid carrier via a base linker comprising 1 to 10 nucleotides; preferably,

when the small-molecule chemical drug is a anthracycline chemotherapeutic or a acridine chemotherapeutic, the small-molecule chemical drug specifically intercalates between adjacent GC base pairs of the targeted nucleic acid carrier;
when the small-molecule chemical drug is a pyrimidine chemotherapeutic, the small-molecule targeting ligand, the oligonucleotide effector molecule, and the immunostimulant are linked via a base linker comprising 1 to 10 nucleotides to the terminal bases of the sequences of the nucleic acid carrier, and the small-molecule chemical drug is incorporated into the nucleic acid carrier by at least partially replacing nucleotides in the base linker and/or as an extended base and/or by substitution of nucleotides in the carrier backbone;
when the small-molecule chemical drug is a platinum chemotherapeutic, a glutamic-acid-derivative chemotherapeutic, a flavonoid chemotherapeutic, a phytomedicine or a derivative thereof, a folate chemotherapeutic, or a salicylic-acid chemotherapeutic, the small-molecule chemical drug is covalently linked to the nucleic acid carrier via a linker; preferably, the linker is selected from long-chain primary amine compounds, more preferably long-chain primary amine compounds having a C2-C14 carbon chain, and further preferably 5-(bromomethyl)-6-bromohexan-1-amine.

**8.** The targeted chemical drug according to claim 7, wherein the oligonucleotide effector molecule, the immunostimulant, and the small-molecule targeting ligand are connected to one or more sequences of the nucleic acid carrier by strand extension or by complementary strand pairing; preferably, the targeted chemical drug is any one of the following:

A)The targeted chemical drug comprises a DNA carrier, a small-molecule chemical drug, and a EGFR nucleic acid aptamer, and optionally biotin, linked to the DNA carrier; preferably, the small-molecule chemical drug is epirubicin hydrochloride or its free base; preferably, the DNA carrier comprises the first group of sequences; the EGFR nucleic acid aptamer is linked to the 5' terminus of the sequence **b**, and the molar ratio of the EGFR nucleic acid aptamer to the DNA carrier is 1: 1; preferably, the EGFR nucleic acid aptamer is linked to the 5' terminus of the sequence **b** via a base linker "TTTTT" (five thymidines); when the targeted chemical drug comprises the DNA carrier, the small-molecule chemical drug, the EGFR nucleic acid aptamer, and biotin linked to the DNA carrier, 2 to 4 biotin moieties are linked per DNA carrier, each biotin being linked to the 5' or 3' terminus of the sequence **a** or the sequence **c;** Alternatively,
B)The targeted chemical drug comprises a DNA carrier, a small-molecule chemical drug, and a DNA-form nucleic acid aptamer AS1411, a EGFR nucleic acid aptamer, a DNA-form nucleic acid aptamer A15, and optionally biotin linked to the DNA carrier; preferably, the small-molecule chemical drug is epirubicin hydrochloride or its free base; preferably, the DNA carrier comprises the first group of sequences; the DNA-form nucleic acid aptamer AS1411 is present in a molar ratio of 1: 1 relative to the DNA carrier and is linked to the 5' terminus of the sequence **a;** preferably, the AS1411 aptamer is linked to the 5' terminus of the sequence **a** via a base linker "TTTTT" (five thymidines); the EGFR nucleic acid aptamer is present in a molar ratio of 1: 1 relative to the DNA carrier and is linked to the 5' terminus of the sequence **b;** preferably, the EGFR nucleic acid aptamer is linked to the 5' terminus of the sequence **b** via a base linker "TTTTT"; the DNA-form nucleic acid aptamer A15 is present in a molar ratio of 1: 1 relative to the DNA carrier and is linked to the 5' terminus of the sequence **c;** preferably, the A15 aptamer is linked to the 5' terminus of the sequence **c** via a base linker "TTTTT"; when the targeted chemical drug comprises the DNA carrier, the small-molecule chemical drug, the DNA-form nucleic acid aptamer AS1411, the EGFR nucleic acid aptamer, the DNA-form nucleic acid aptamer A15, and biotin linked to the DNA carrier, 2 biotin moieties are linked per DNA carrier, the biotin moieties being respectively linked to the 3' terminus of the sequence **a** and the 3' terminus of the sequence c; Alternatively,
C)The targeted chemical drug comprises a DNA carrier, a small-molecule chemical drug, a DNA-form nucleic acid aptamer AS1411 and biotin linked to the DNA carrier; preferably, the small-molecule chemical drug is epirubicin hydrochloride or its free base; preferably, the DNA carrier comprises the first group of sequences; the DNA-form nucleic acid aptamer AS1411 is linked to the 5' terminus of the sequence **b,** and the molar ratio of the DNA-form nucleic acid aptamer AS1411 to the DNA carrier is 1: 1; preferably, the AS1411 aptamer is linked to the 5' terminus of the sequence **b** via a base linker "TTTTT" (five thymidines); 2 to 4 biotin moieties are linked per DNA carrier, each biotin being linked to the 5' or 3' terminus of the sequence **a** or the sequence **c;** Alternatively,
D)The targeted chemical drug comprises a DNA carrier, a small-molecule chemical drug, and a nucleic acid aptamer A1 and the DNA-form nucleic acid aptamer A15 linked to the DNA carrier; preferably, the small-molecule chemical drug is epirubicin hydrochloride or its free base; preferably, the DNA carrier comprises the first group of sequences; the nucleic acid aptamer A1 is present in a molar ratio of 1: 1 relative to the DNA carrier and is linked to the 5' terminus of the sequence **b;** preferably, the aptamer A1 is linked to the 5' terminus of the sequence **b** via a

base linker "TTTTT"; the DNA-form nucleic acid aptamer A15 is present in a molar ratio of 1: 1 relative to the DNA carrier and is linked to the 5' terminus of the sequence **c;** preferably, the aptamer A15 is linked to the 5' terminus of the sequence **b** via a base linker "TTTTT"; Alternatively,

E)The targeted chemical drug comprises a DNA carrier, the small-molecule chemical drug, a DNA-form nucleic acid aptamer AS1411, a EGFR nucleic acid aptamer, a DNA-form nucleic acid aptamer A15, optional biotin, and an oligonucleotide A-miR-21 linked to the DNA carrier; preferably, the small-molecule chemical drug is epirubicin hydrochloride or its free base; preferably, the DNA carrier comprises the first group of sequences; the DNA-form nucleic acid aptamer AS1411 is present in a molar ratio of 1: 1 relative to the DNA carrier and is linked to the 5' terminus of the sequence **a;** preferably, the AS1411 aptamer is linked to the 5' terminus of the sequence **a** via a base linker "TTTTT"; the EGFR nucleic acid aptamer is present in a molar ratio of 1: 1 relative to the DNA carrier and is linked to the 5' terminus of the sequence **b;** preferably, the EGFR aptamer is linked to the 5' terminus of the sequence **b** via a base linker "TTTTT"; the DNA-form nucleic acid aptamer A15 is present in a molar ratio of 1: 1 relative to the DNA carrier and is linked to the 5' terminus of the sequence **c;** preferably, the A15 aptamer is linked to the 5' terminus of the sequence **c** via a base linker "TTTTT"; the oligonucleotide A-miR-21 is present in a molar ratio of 1: 1 relative to the DNA carrier and is linked to the 3' terminus of the sequence **c;** optionally, 2 biotin moieties are linked per DNA carrier, the biotin moieties being respectively linked to the 3' terminus of the sequence **a** and to the end of the oligonucleotide A-miR-21 distal from the sequence **c;** Alternatively,

F)The targeted chemical drug comprises a DNA carrier, a small-molecule chemical drug, and a DNA-form nucleic acid aptamer TfRA4, a DNA-form nucleic acid aptamer AS1411, and a DNA-form nucleic acid aptamer A15 linked to the DNA carrier; preferably, the small-molecule chemical drug is epirubicin hydrochloride or its free base; preferably, the DNA carrier comprises the first group of sequences; the DNA-form nucleic acid aptamer TfRA4 is present in a molar ratio of 1: 1 relative to the DNA carrier and is linked to the 5' terminus of the sequence **a;** preferably, the TfRA4 aptamer is linked to the 5' terminus of the sequence **a** via a base linker "TTTTT"; the DNA-form nucleic acid aptamer AS1411 is present in a molar ratio of 1: 1 relative to the DNA carrier and is linked to the 5' terminus of the sequence **b;** preferably, the AS1411 aptamer is linked to the 5' terminus of the sequence **b** via a base linker "TTTTT"; the DNA-form nucleic acid aptamer A15 is present in a molar ratio of 1: 1 relative to the DNA carrier and is linked to the 5' terminus of the sequence **c;** preferably, the A15 aptamer is linked to the 5' terminus of the sequence **c** via a base linker "TTTTT"; Alternatively,

G)The targeted chemical drug comprises a DNA carrier, a small-molecule chemical drug, and a DNA-form nucleic acid aptamer TfRA4, a DNA-form nucleic acid aptamer AS1411, and a DNA-form nucleic acid aptamer A15 linked to the DNA carrier, wherein the small-molecule chemical drug is epirubicin hydrochloride or its free base; the DNA carrier comprises the eleventh group of sequences; the DNA-form nucleic acid aptamer TfRA4 is present in a molar ratio of 1: 1 relative to the DNA carrier and is linked to the 5' terminus of the sequence **a;** preferably, the TfRA4 aptamer is linked to the 5' terminus of the sequence **a** via a transition base segment (SEQ ID NO: 276: 5'-TTGCGGCGAGCGGCGA-3'), and the targeted chemical drug further comprises a complementary sequence (SEQ ID NO: 277: 5'-TCGCCGCTCGCCGCTT-3') which is complementary to the transition base segment and whose 3' terminus is joined to the 5' end of the nucleic acid aptamer TfRA4; the DNA-form nucleic acid aptamer AS1411 is present in a molar ratio of 1: 1 relative to the DNA carrier and is linked to the 5' terminus of the sequence **b;** preferably, the AS1411 aptamer is linked to the 5' terminus of the sequence **b** via a base linker "TTTTT"; the DNA-form nucleic acid aptamer A15 is present in a molar ratio of 1: 1 relative to the DNA carrier and is linked to the 5' terminus of the sequence **c;** preferably, the A15 aptamer is linked to the 5' terminus of the sequence **c** via a base linker "TTTTT".Alternatively,

H)The targeted chemical drug comprises a DNA carrier, a small-molecule chemical drug, and a DNA-form nucleic acid aptamer AS1411, a DNA-form nucleic acid aptamer TfRA3, and a DNA-form nucleic acid aptamer A15 linked to the DNA carrier; preferably, the small-molecule chemical drug is epirubicin hydrochloride or its free base; preferably, the DNA carrier comprises the first group of sequences; the DNA-form nucleic acid aptamer AS1411 is present in a molar ratio of 1: 1 relative to the DNA carrier and is linked to the 5' terminus of the sequence **a;** preferably, the AS1411 aptamer is linked to the 5' terminus of the sequence **a** via a base linker "TTTTT" (five thymidines); the DNA-form nucleic acid aptamer TfRA3 is present in a molar ratio of 1: 1 relative to the DNA carrier and is linked to the 5' terminus of the sequence **b;** preferably, the TfRA3 aptamer is linked to the 5' terminus of the sequence **b** via a base linker "TTTTT"; the DNA-form nucleic acid aptamer A15 is present in a molar ratio of 1: 1 relative to the DNA carrier and is linked to the 5' terminus of the sequence c; preferably, the A15 aptamer is linked to the 5' terminus of the sequence **b** via a base linker "TTTTT"; Alternatively,

I)The targeted chemical drug comprises a DNA carrier, a small-molecule chemical drug, and an immunostimulant CPG2006, a nucleic acid aptamer CD40, and a DNA-form nucleic acid aptamer PD-L1 linked to the DNA carrier; preferably, the small-molecule chemical drug is epirubicin hydrochloride or its free base; preferably, the DNA carrier comprises the ninth group of sequences; the immunostimulant CPG2006 is present in a molar ratio of 1: 1 relative to the DNA carrier and is linked to the 5' terminus of the sequence **a;** preferably, CPG2006 is linked to the 5'

terminus of the sequence **a** via a base linker "TTTTT"; the nucleic acid aptamer CD40 is present in a molar ratio of 1: 1 relative to the DNA carrier and is linked to the 5' terminus of the sequence **b;** preferably, the CD40 aptamer is linked to the 5' terminus of the sequence **b** via a base linker "TTTTT"; the DNA-form nucleic acid aptamer PD-L1 is present in a molar ratio of 1: 1 relative to the DNA carrier and is linked to the 5' terminus of the sequence **c;** preferably, the PD-L1 aptamer is linked to the 5' terminus of the sequence **c** via a base linker "TTTTTT" (six thymidines); Alternatively,

J)The targeted chemical drug comprises a DNA carrier, a small-molecule chemical drug, and an immunostimulant CPG2006, a nucleic acid aptamer CD40, a DNA-form nucleic acid aptamer PD-L1, and a nucleic acid aptamer C12 linked to the DNA carrier; wherein the DNA carrier further comprises complementary sequences d and e, the sequence d being SEQ ID NO: 278: 5'-GCCACCGTGCTACA-3' and the sequence e being SEQ ID NO: 279: 5'-CAGCAGCAGCAGCA-3'; the 3' terminus of the sequence b of the DNA carrier is joined to a transition base segment, SEQ ID NO: 280: 5'-GTAGCACGGTGGC-3'; the sequence d is complementarily hybridized to the transition base segment SEQ ID NO: 280: 5'-GTAGCACGGTGGC-3'; and the sequence e is complementarily hybridized to the sequence c of the DNA carrier; preferably, the small-molecule chemical drug is epirubicin hydrochloride or its free base; preferably, the DNA carrier comprises the tenth group of sequences; the immunostimulant CPG2006 is present in a molar ratio of 2: 1 relative to the DNA carrier and is linked to the 5' termini of the sequence a and the sequence c, respectively; preferably, CPG2006 is linked to the 5' terminus of the sequence a via a base linker "TTTTTT" and to the 5' terminus of the sequence c via a base linker "TTTTT"; the nucleic acid aptamer CD40 is present in a molar ratio of 1: 1 relative to the DNA carrier and is linked to the 5' terminus of the sequence b; preferably, the CD40 aptamer is linked to the 5' terminus of the sequence b via a base linker "TTTTT"; the DNA-form nucleic acid aptamer PD-L1 is present in a molar ratio of 1: 1 relative to the DNA carrier and is linked to the 5' terminus of the sequence d; preferably, the PD-L1 aptamer is linked to the 5' terminus of the sequence d via a base linker "TTTTTT"; the nucleic acid aptamer C12 is present in a molar ratio of 1: 1 relative to the DNA carrier and is linked to the 5' terminus of the sequence **e;** preferably, the C12 aptamer is linked to the 5' terminus of the sequence **e** via a base linker "TTTTT"; Alternatively,

K)The targeted chemical drug comprises a DNA carrier, a small-molecule chemical drug, and an immunostimulant CPG2006, a nucleic acid aptamer C12, a CD47 siRNA oligonucleotide, a DNA-form nucleic acid aptamer PD-L1, and a PD-L1 siRNA oligonucleotide linked to the DNA carrier; preferably, the small-molecule chemical drug is epirubicin hydrochloride or its free base; preferably, the DNA carrier comprises the ninth group of sequences; the PD-L1 siRNA oligonucleotide is present in a molar ratio of 1: 1 relative to the DNA carrier, the sense strand thereof serving as a transition base segment linked to the 5' terminus of the sequence **c** of the DNA carrier, the antisense strand serving as a complementary sequence complementarily hybridized to the sense strand, and the DNA-form nucleic acid aptamer PD-L1 being linked to the 5' end of the antisense strand of the PD-L1 siRNA oligonucleotide; preferably, the PD-L1 aptamer is linked to the 5' end of the antisense strand of the PD-L1 siRNA oligonucleotide via a base linker "TTTTT"; the immunostimulant CPG2006 is present in a molar ratio of 1: 1 relative to the DNA carrier and is linked to the 5' terminus of the sequence **a;** preferably, CPG2006 is linked to the 5' terminus of the sequence **a** via a base linker "TTTTT"; the CD47 siRNA oligonucleotide is present in a molar ratio of 1: 1 relative to the DNA carrier, the sense strand thereof serving as a transition base segment linked to the 5' terminus of the sequence **b** and the antisense strand serving as a complementary sequence complementarily hybridized to the sense strand; the nucleic acid aptamer C12 is present in a molar ratio of 1: 1 relative to the DNA carrier and is linked to the 5' end of the antisense strand of the CD47 siRNA oligonucleotide; preferably, the C12 aptamer is linked to the 5' end of the antisense strand of the CD47 siRNA oligonucleotide via a base linker "TTTTT"; Alternatively,

L)The targeted chemical drug comprises a DNA carrier, a small-molecule chemical drug, and an A-miR-21 miRNA oligonucleotide, a TGF-β1 siRNA oligonucleotide, a nucleic acid aptamer TfRA4, and a nucleic acid aptamer PD-L1 linked to the DNA carrier; preferably, the small-molecule chemical drug is epirubicin hydrochloride or its free base; preferably, the DNA carrier comprises the second group of sequences; the TGF-β1 siRNA oligonucleotide is present in a molar ratio of 1: 1 relative to the DNA carrier, the antisense strand thereof serving as a transition base segment linked to the 3' terminus of the sequence **c,** and the sense strand serving as a complementary sequence complementarily hybridized to the antisense strand; the nucleic acid aptamer TfRA4 is present in a molar ratio of 1: 1 relative to the DNA carrier and is linked to the 5' terminus of the sequence **a;** preferably, the TfRA4 aptamer is linked to the 5' terminus of the sequence **a** via a base linker "TTTTT"; the nucleic acid aptamer PD-L1 is present in a molar ratio of 1: 1 relative to the DNA carrier and is linked to the 5' terminus of the sequence **b;** preferably, the PD-L1 aptamer is linked to the 5' terminus of the sequence **b** via a base linker "TTTTT"; the A-miR-21 miRNA oligonucleotide is present in a molar ratio of 2: 1 relative to the DNA carrier and is sequentially (in tandem) linked to the 5' end of the sense strand of the TGF-β1 siRNA oligonucleotide; Alternatively,

M)The targeted chemical drug comprises a DNA carrier, a small-molecule chemical drug, and a nucleic acid

aptamer IL-4Ra, a CD47 siRNA oligonucleotide, a DNA-form nucleic acid aptamer TfRA4, a PD-L1 siRNA oligonucleotide, and a nucleic acid aptamer GPC-1 linked to the DNA carrier; preferably, the small-molecule chemical drug is epirubicin hydrochloride or its free base; the DNA carrier comprises the third group of sequences; the DNA-form nucleic acid aptamer TfRA4 is present in a molar ratio of 1: 1 relative to the DNA carrier and is linked to the 5' terminus of the sequence **a;** preferably, the TfRA4 aptamer is linked to the 5' terminus of the sequence **a** via a base linker "TTTTT"; the nucleic acid aptamer GPC-1 is present in a molar ratio of 1: 1 relative to the DNA carrier and is linked to the 5' terminus of the sequence **c;** the PD-L1 siRNA oligonucleotide is present in a molar ratio of 1: 1 relative to the DNA carrier, the sense strand thereof serving as a transition base segment linked to the 5' terminus of the sequence **b**, the antisense strand serving as a complementary sequence complementarily hybridized to the sense strand, and being linked at the 3' terminus of the sequence **a;** the CD47 siRNA oligonucleotide is present in a molar ratio of 1: 1 relative to the DNA carrier, the sense strand thereof serving as a transition base segment linked to the 3' terminus of the sequence **c** and the antisense strand serving as a complementary sequence complementarily hybridized to the sense strand; the nucleic acid aptamer IL-4Ra is present in a molar ratio of 1: 1 relative to the DNA carrier and is linked to the 5' end of the antisense strand of the CD47 siRNA oligonucleotide; preferably, the IL-4Ra aptamer is linked to the 5' end of the antisense strand of the CD47 siRNA oligonucleotide via a base linker "AAAA"; Alternatively,

N)The targeted chemical drug comprises a DNA carrier, a small-molecule chemical drug, and a DNA-form nucleic acid aptamer AS1411, a DNA-form nucleic acid aptamer EGFR, and a DNA-form nucleic acid aptamer A15 linked to the DNA carrier; preferably, the small-molecule chemical drug is gemcitabine; preferably, the DNA carrier comprises the fourth group of sequences; the DNA-form nucleic acid aptamer AS1411 is present in a molar ratio of 1: 1 relative to the DNA carrier and is linked to the 5' terminus of the sequence **a;** the DNA-form nucleic acid aptamer EGFR is present in a molar ratio of 1: 1 relative to the DNA carrier and is linked to the 5' terminus of the sequence **b;** the DNA-form nucleic acid aptamer A15 is present in a molar ratio of 1: 1 relative to the DNA carrier and is linked to the 5' terminus of the sequence c; preferably, gemcitabine is linked in the form of at least one inserted base at one or more of the following positions: between the DNA-form nucleic acid aptamer AS1411 and the sequence **a;** at the 3' terminus of the sequence **a;** between the DNA-form nucleic acid aptamer EGFR and the sequence **b;** at the 3' terminus of the sequence **b;** between the DNA-form nucleic acid aptamer A15 and the sequence **c;** or at the 3' terminus of the sequence **c;** Alternatively,

O)The targeted chemical drug comprises a RNA carrier, a small-molecule chemical drug, andDNA-form nucleic acid aptamers AS1411, EGFR, and A15 linked to the RNA carrier; preferably, the small-molecule chemical drug is 5-fluorouracil; preferably, the RNA carrier comprises the fifth group of sequences; the DNA-form nucleic acid aptamer AS1411 is present in a molar ratio of 1: 1 relative to the RNA carrier and is linked to the 5' terminus of the sequence **a;** the DNA-form nucleic acid aptamer EGFR is present in a molar ratio of 1: 1 relative to the RNA carrier and is linked to the 5' terminus of the sequence **b;** the DNA-form nucleic acid aptamer A15 is present in a molar ratio of 1: 1 relative to the RNA carrier and is linked to the 5' terminus of the sequence **c;** preferably, 5-fluorouracil is linked in the form of at least one inserted base at one or more of the following positions: between the DNA-form nucleic acid aptamer AS1411 and the sequence **a;** at the 3' terminus of the sequence **a;** between the DNA-form nucleic acid aptamer EGFR and the sequence **b;** at the 3' terminus of the sequence **b;** between the DNA-form nucleic acid aptamer A15 and the sequence **c;** or at the 3' terminus of the sequence **c;** Alternatively,

P)The targeted chemical drug comprises a DNA carrier, a small-molecule chemical drug, and DNA-form nucleic acid aptamers AS1411, EGFR, and A15 linked to the DNA carrier; preferably, the small-molecule chemical drug is paclitaxel; preferably, the DNA carrier comprises the sixth group of sequences; the DNA-form nucleic acid aptamer AS1411 is present in a molar ratio of 1: 1 relative to the DNA carrier and is linked to the 5' terminus of the sequence **a;** preferably, the AS1411 aptamer is linked to the 5' terminus of the sequence **a** via a base linker "TTTTT" (five thymidines); the DNA-form nucleic acid aptamer EGFR is present in a molar ratio of 1: 1 relative to the DNA carrier and is linked to the 5' terminus of the sequence **b;** preferably, the EGFR aptamer is linked to the 5' terminus of the sequence **b** via a base linker "TTTTT"; the DNA-form nucleic acid aptamer A15 is present in a molar ratio of 1: 1 relative to the DNA carrier and is linked to the 5' terminus of the sequence **c;** preferably, the A15 aptamer is linked to the 5' terminus of the sequence **c** via a base linker "TTTTT"; Alternatively,

Q)The targeted chemical drug comprises a DNA carrier, a small-molecule chemical drug, and DNA-form nucleic acid aptamers AS1411, EGFR, and A15 linked to the DNA carrier; preferably, the small-molecule chemical drug is maytansine; preferably, the DNA carrier comprises the seventh group of sequences; the DNA-form nucleic acid aptamer AS1411 is present in a molar ratio of 1: 1 relative to the DNA carrier and is linked to the 5' terminus of the sequence **a;** preferably, the AS1411 aptamer is linked to the 5' terminus of the sequence **a** via a base linker "TTTTT"; the DNA-form nucleic acid aptamer EGFR is present in a molar ratio of 1: 1 relative to the DNA carrier and is linked to the 5' terminus of the sequence **b;** preferably, the EGFR aptamer is linked to the 5' terminus of the sequence **b** via a base linker "TTTTT"; the DNA-form nucleic acid aptamer A15 is present in a molar ratio of 1: 1 relative to the DNA carrier and is linked to the 5' terminus of the sequence **c;** preferably, the A15 aptamer is linked

to the 5' terminus of the sequence **c** via a base linker "TTTTT"; Alternatively,

R)The targeted chemical drug comprises a DNA carrier, a small-molecule chemical drug, and the nucleic acid aptamers MUC1, AS1411, and GPC-1 linked to the DNA carrier; preferably, the small-molecule chemical drug is gemcitabine; preferably, the DNA carrier comprises the ninth group of sequences; the nucleic acid aptamer MUC1 is present in a molar ratio of 1: 1 relative to the DNA carrier and is linked to the 5' terminus of the sequence **a;** the nucleic acid aptamer AS1411 is present in a molar ratio of 1: 1 relative to the DNA carrier and is linked to the 5' terminus of the sequence **b;** and the nucleic acid aptamer GPC-1 is present in a molar ratio of 1: 1 relative to the DNA carrier and is linked to the 5' terminus of the sequence **c.**

**9.** A pharmaceutical composition, comprising a targeted chemical drug according to any one of claims 1 to 8 and a pharmaceutically acceptable carrier and/or excipients.

**10.** Use of a targeted chemical drug according to any one of claims **1** to 8 in the manufacture of a medicament for the treatment of a tumor.

**11.** The use according to claim 10, wherein the tumor is selected from the group consisting of andrological tumors, gynecologic tumors, respiratory system tumors, digestive system tumors, hematologic tumors, urinary system tumors, bone tumors, neurologic tumors, dermatologic tumors, general surgical tumors, and otorhinolaryngologic/ophthalmologic tumors; preferably, the andrological tumor is selected from prostate cancer, penile cancer, testicular tumor, or male urethral carcinoma; the gynecologic tumor is selected from ovarian cancer, cervical cancer, endometrial cancer, uterine fibroid, vulvar cancer, or malignant hydatidiform mole; the respiratory system tumor is selected from lung cancer, non-small cell lung cancer, small cell lung cancer, nasopharyngeal carcinoma, tracheal tumor, metastatic lung cancer, pulmonary inflammatory pseudotumor, or radiation-induced lung cancer; the digestive system tumor is selected from liver cancer, gastric cancer, colorectal cancer, gallbladder cancer, esophageal cancer, rectal cancer, pancreatic cancer, or colon cancer; the hematologic tumor is selected from leukemia, lymphoma, lymphosarcoma, or multiple myeloma; the urinary system tumor is selected from kidney cancer, bladder cancer, or urinary tract cancer; the bone tumor is selected from giant cell tumor of bone, osteochondroma, or osteosarcoma; the neurologic tumor is selected from brain tumor, meningioma, cerebral tuberculoma, pituitary tumor, neuroblastoma, glioblastoma, or glioma; the dermatologic tumor is selected from skin cancer or melanoma; the general surgical tumor is selected from breast cancer, lipoma, thyroid cancer, or thyroid tumor; and the otorhinolaryngologic/ophthalmologic tumor is selected from oral cancer, tongue cancer, laryngeal cancer, middle-ear cancer, gingival cancer, or orbital tumor;

Preferably, a route of administration of the targeted chemical drug is intratumoral administration, intravenous administration, or intraperitoneal administration;

Preferably, a daily dose of the targeted chemical drug is 0.1 $\mu$g/kg to 100 mg/kg.

**12.** A method for preparing a targeted chemical drug according to any one of claims 1 to 8, comprising:
Forming, by self-assembly of sequences, a targeted nucleic acid carrier optionally bearing one or more oligonucleotide effector molecules and/or immunostimulants; and linking the small-molecule chemical drug to the targeted nucleic acid carrier to obtain the targeted chemical drug.

**13.** The method according to claim 12, wherein the step of forming, by sequence self-assembly, the targeted nucleic acid carrier optionally bearing oligonucleotide effector molecules and immunostimulants comprises:

Dissolving in an assembly solution at least three sequences that bear optional oligonucleotide effector molecules, optional immunostimulants, and targeting molecules, subjecting the solution to a denaturation reaction to form a self-assembled crude product; and successively purifying, eluting, and evaporatively drying the self-assembled crude product to obtain the targeted nucleic acid carrier bearing optional oligonucleotide effector molecules and optional immunostimulants;

Preferably, the at least three sequences comprise at least the following three sequences:

Sequence **A:** sequence **a** whose terminus is directly linked to, or linked via a base linker to, optional oligonucleotide effector molecules, optional immunostimulants, and optional targeting molecules;

Sequence **B:** sequence **b** whose terminus is directly linked to, or linked via a base linker to, optional oligonucleotide effector molecules, optional immunostimulants, and optional targeting molecules;

Sequence **C:** sequence **c** whose terminus is directly linked to, or linked via a base linker to, optional oligonucleotide effector molecules, optional immunostimulants, and optional targeting molecules;

the molar ratio among the sequences a, b, c is 0.90-1.10 : 0.90-1.10 : 0.90-1.10, more preferably 1 : 1 : 1; when the at least three sequences further comprise one or more sequences other than the **sequences a, b and c,** each such sequence is present at a molar ratio relative to the sequence a of 0.90-1.10 : 0.90-1.10, more preferably 1 : 1;

Preferably, when the at least three sequences comprise sequences other than sequences a, b, and c, such sequences are referred to as complementary sequences, in which case any of the 5' or 3' segments of the sequences a, b, and c is independently linked to a transition base segment; the complementary sequence(s) and the transition base segment(s) are base-complementary so as to complementarily hybridize the complementary sequence(s) to the transition base segment(s) during self-assembly; more preferably, the complementary sequence(s) are themselves the sense or antisense strand(s) of siRNA and miRNA oligonucleotide effector molecules and/or provide loading sites for small-molecule targeting ligands, targeting aptamers, or immunostimulants;
Further preferably, when the at least three sequences comprise the complementary sequence(s), the complementary sequence(s) carrying optional small-molecule targeting ligands, optional targeting aptamers, and optional immunostimulants are added to the assembly solution simultaneously and subjected to the denaturation reaction;
Preferably, the assembly solution is a TMS aqueous solution, a sodium chloride solution, a magnesium chloride solution, or purified water;
Preferably, the denaturation reaction is conducted at 80-99 °C, more preferably at 85-99 °C, and further preferably at 90-99 °C;
Preferably, upon completion of the denaturation reaction, the reaction system is cooled to a holding temperature to perform an incubation (holding) step, and finally cooled to afford the assembled product; the holding temperature is 70-50 °C (more preferably 65-55 °C, and further preferably 63-57 °C); the holding time is 3-15 min (more preferably 3-10 min, and further preferably 3-5 min);
Preferably, during cooling of the reaction system to the holding temperature, the cooling rate is 2-10 °C/min (more preferably 2-6 °C/min, and further preferably 2-3 °C/min);
Preferably, the final cooling end-temperature is 0-25 °C (more preferably 0-15 °C, and further preferably 0-4 °C);
Preferably, the pH of the denaturation reaction is 5.4-8.8.

**14.** The method according to claim 13, wherein:

When the small-molecule chemical drug is an anthracycline chemotherapeutic or an acridine chemotherapeutic, the step of loading the small-molecule chemical drug onto the targeted nucleic acid carrier comprises: dissolving the small-molecule chemical drug, a coupling agent, and the targeted nucleic acid carrier bearing optional oligonucleotide effector molecules and optional immunostimulants in a first solvent and reacting under light-protection so as to allow the small-molecule chemical drug to specifically intercalate between adjacent GC base pairs of the targeted nucleic acid carrier to afford a crude reaction product; and subjecting the crude reaction product sequentially to crystallization, washing, and evaporation to dryness to obtain the targeted chemical drug;

Preferably, the coupling agent is formaldehyde and/or paraformaldehyde;
Preferably, the reaction temperature is 0-37 °C, more preferably 4-20 °C, and still more preferably 4-8 °C;
Preferably, the reaction pH is 6-8;
Preferably, the reaction time is 24-96 h, more preferably 48-72 h, and still more preferably 72 h;
Preferably, the first solvent is purified water and/or phosphate-buffered saline (PBS);
Preferably, the washing solvent is selected from halogenated alkane solvents, ether solvents, or benzene-type (aromatic) solvents, more preferably chloroform, dichloromethane, diethyl ether, toluene, or xylene;
Preferably, the solvent used for crystallization is a lower alcohol, more preferably methanol, ethanol, or isopropanol;
Preferably, the average number of small-molecule chemical drug moieties loaded per targeted nucleic acid carrier molecule (bearing optional oligonucleotide effector molecules and optional immunostimulants) is 1-50, more preferably 10-40, and still more preferably 20-35;

When the small-molecule chemical drug is a pyrimidine chemotherapeutic, wherein the small-molecule targeting ligand, the oligonucleotide effector molecule, and the immunostimulant are linked to terminal bases of the respective sequences of the nucleic acid carrier via base linkers comprising 1 to 10 nucleotides, the step of connecting the small-molecule chemical drug to the targeted nucleic acid carrier bearing optional oligonucleotide effector molecules and optional immunostimulants comprises: during the step of forming the targeted nucleic acid

carrier by sequence self-assembly, incorporating the small-molecule chemical drug into at least one sequence of the nucleic acid carrier in the form of at least partial substitution of bases in the base linker and/or as an extended base and/or as substitution of nucleotides of the carrier backbone, and thereafter completing the connection of the small-molecule chemical drug by sequence self-assembly; Preferably, the average number of small-molecule chemical drug moieties loaded per targeted nucleic acid carrier molecule is 1-30, more preferably 10-20, and still more preferably 12-18;

When the small-molecule chemical drug is a platinum chemotherapeutic, a glutamic-acid-derivative chemotherapeutic, a flavonoid chemotherapeutic, a phytomedicine or a derivative thereof, a folate chemotherapeutic, or a salicylic-acid chemotherapeutic, the step of connecting the small-molecule chemical drug to the targeted nucleic acid carrier bearing optional oligonucleotide effector molecules and optional immunostimulants comprises: in the course of synthesizing the respective sequences of the nucleic acid carrier to which the targeting molecule is attached, binding a linker to at least one sequence of the nucleic acid carrier bearing optional oligonucleotide effector molecules, optional immunostimulants, and the targeting molecule to obtain a linker-bearing targeted nucleic acid carrier; reacting the linker-bearing targeted nucleic acid carrier with the small-molecule chemical drug in a second solvent, and then cooling, purifying, washing, and evaporatively drying to obtain the targeted chemical drug;

Preferably, when the small-molecule chemical drug itself carries a succinimidyl ester (NHS ester) group, the reaction is performed by dissolving the linker-bearing targeted nucleic acid carrier and the small-molecule chemical drug in the second solvent; when the small-molecule chemical drug itself does not carry a succinimidyl ester group, prior to reacting in the second solvent, the method further comprises reacting the small-molecule chemical drug sequentially with bis(2-hydroxyethyl) disulfide and N, N-diisopropylethylamine so as to introduce a succinimidyl ester group onto the small-molecule chemical drug.

Fig. 1

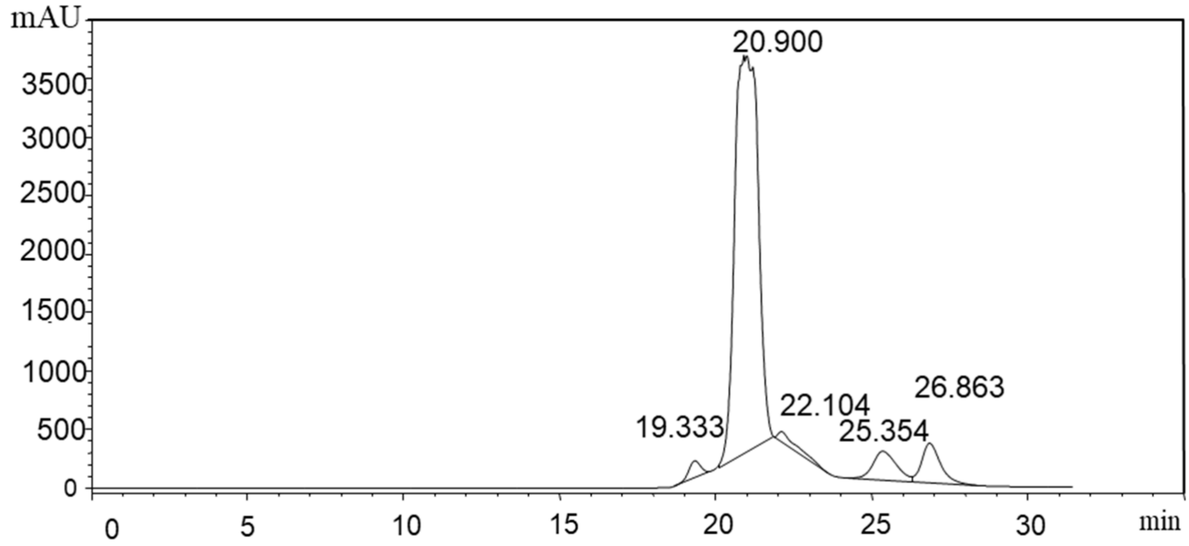
mAU
3500
3000
2500
2000
1500
1000
500
0
20.900
19.333
22.104
25.354
26.863
0
5
10
15
20
25
30
min

Fig. 2

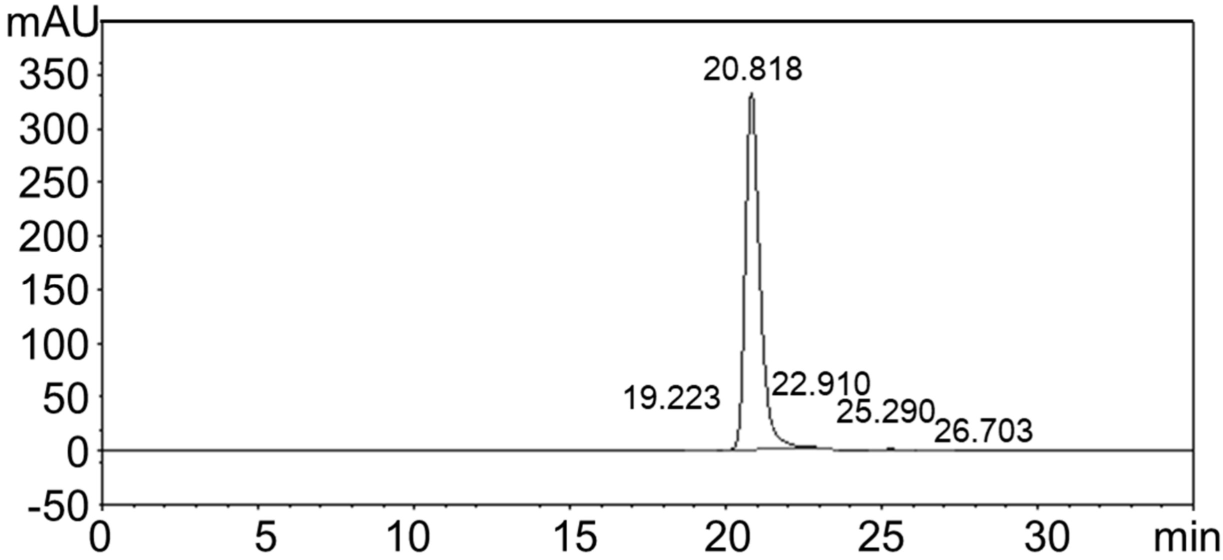
mAU
350
300
250
200
150
100
50
0
-50
20.818
19.223
22.910
25.290
26.703
0
5
10
15
20
25
30
min

Fig. 3

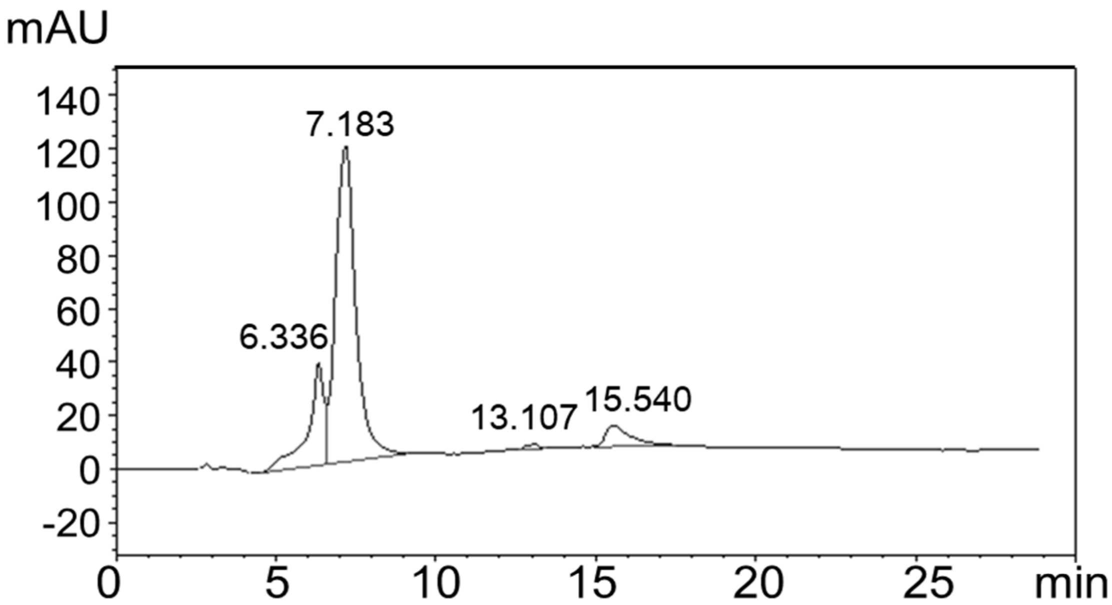


| # | Time | Peak Area | Peak Height | Peak Width | Symmetry Factor | Peak Area (%) |
|---|---|---|---|---|---|---|
| 1 | 6.336 | 1344.2 | 38.2 | 0.465 | 2.169 | 19.152 |
| 2 | 7.183 | 5200.4 | 118.4 | 0.6723 | 0.899 | 74.096 |
| 3 | 13.107 | 45.4 | 2 | 0.3071 | 2.366 | 0.647 |
| 4 | 15.54 | 428.5 | 8 | 0.892 | 9.18E-4 | 6.105 |

Fig. 4

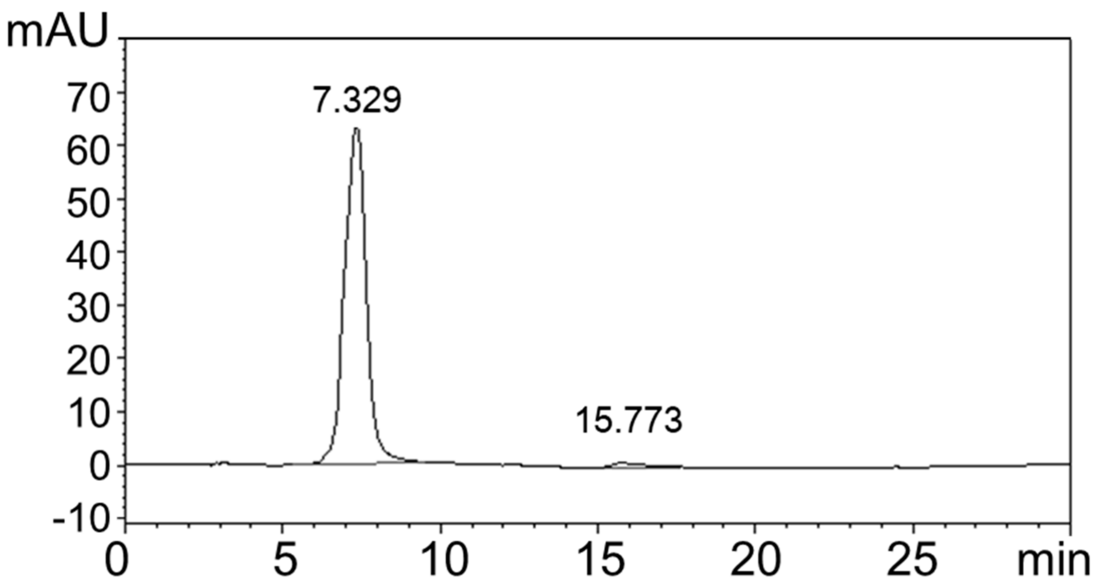


| # | Time | Peak Area | Peak Height | Peak Width | Symmetry Factor | Peak Area (%) |
|---|---|---|---|---|---|---|
| 1 | 7.329 | 3041.9 | 63.5 | 0.6986 | 1.112 | 98.296 |
| 2 | 15.773 | 52.7 | 7.4E-1 | 1.1936 | 0.363 | 1.704 |

Fig. 5

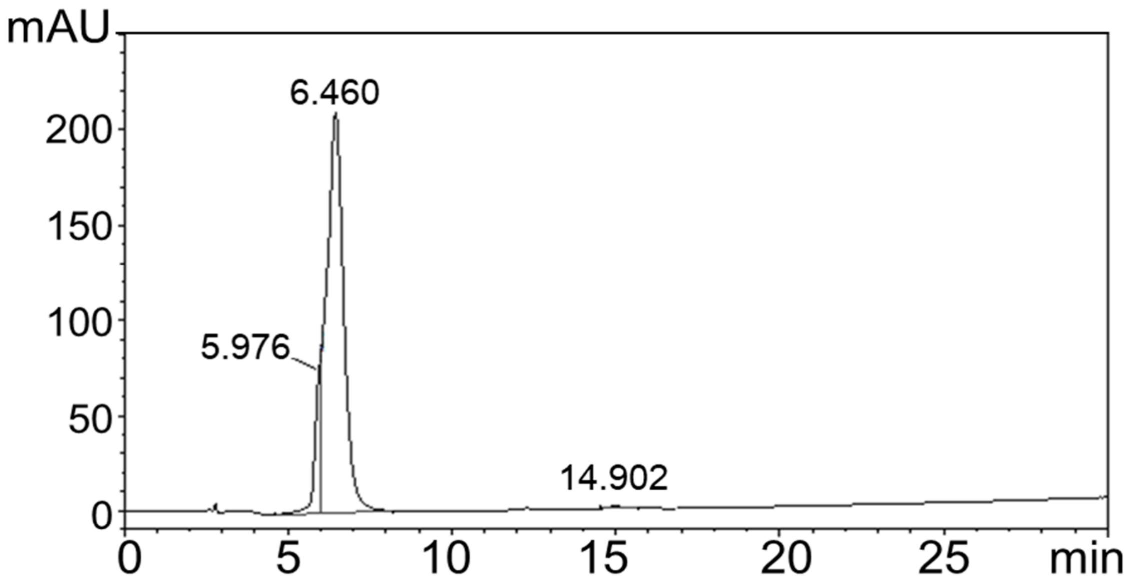


| # | Time | Peak Area | Peak Height | Peak Width | Symmetry Factor | Peak Area (%) |
|---|---|---|---|---|---|---|
| 1 | 5.976 | 822.7 | 78 | 0.1757 | 0 | 9.607 |
| 2 | 6.46 | 7712.6 | 209.6 | 0.6133 | 1.069 | 90.066 |
| 3 | 14.902 | 28 | 7.9E-1 | 0.5879 | 0.171 | 0.327 |

Fig. 6

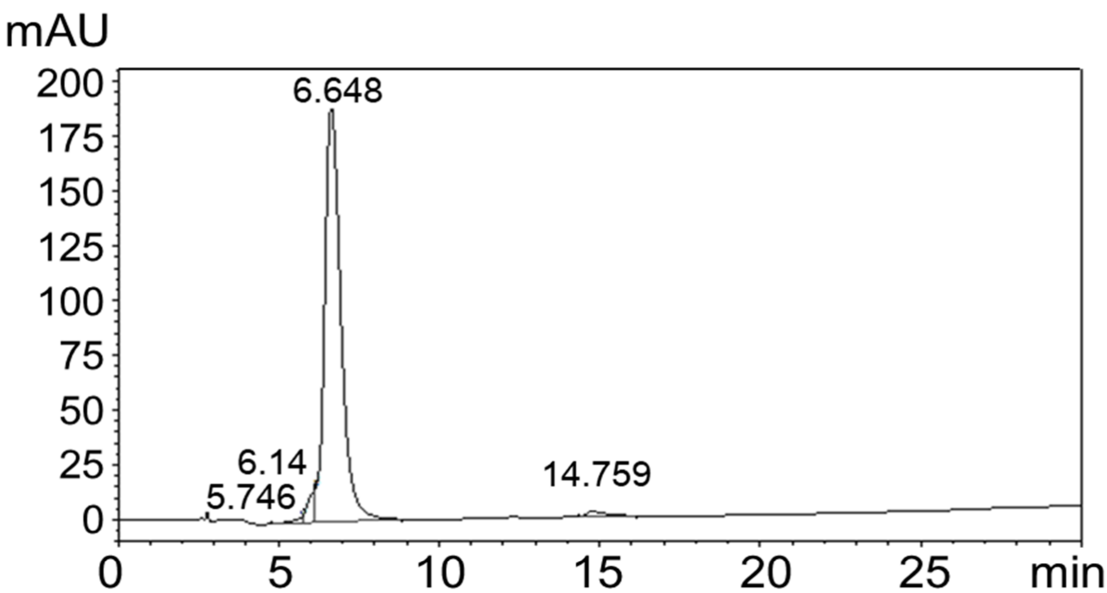


| # | Time | Peak Area | Peak Height | Peak Width | Symmetry Factor | Peak Area (%) |
|---|---|---|---|---|---|---|
| 1 | 5.746 | 50.1 | 2.5 | 0.331 | 2434.59 | 0.711 |
| 2 | 6.14 | 222.2 | 14.9 | 0.2484 | 0 | 3.155 |
| 3 | 6.648 | 6663 | 189.6 | 0.5856 | 0.676 | 94.615 |
| 4 | 14.759 | 107 | 2.3 | 0.6571 | 3.42E-2 | 1.519 |

Fig. 7

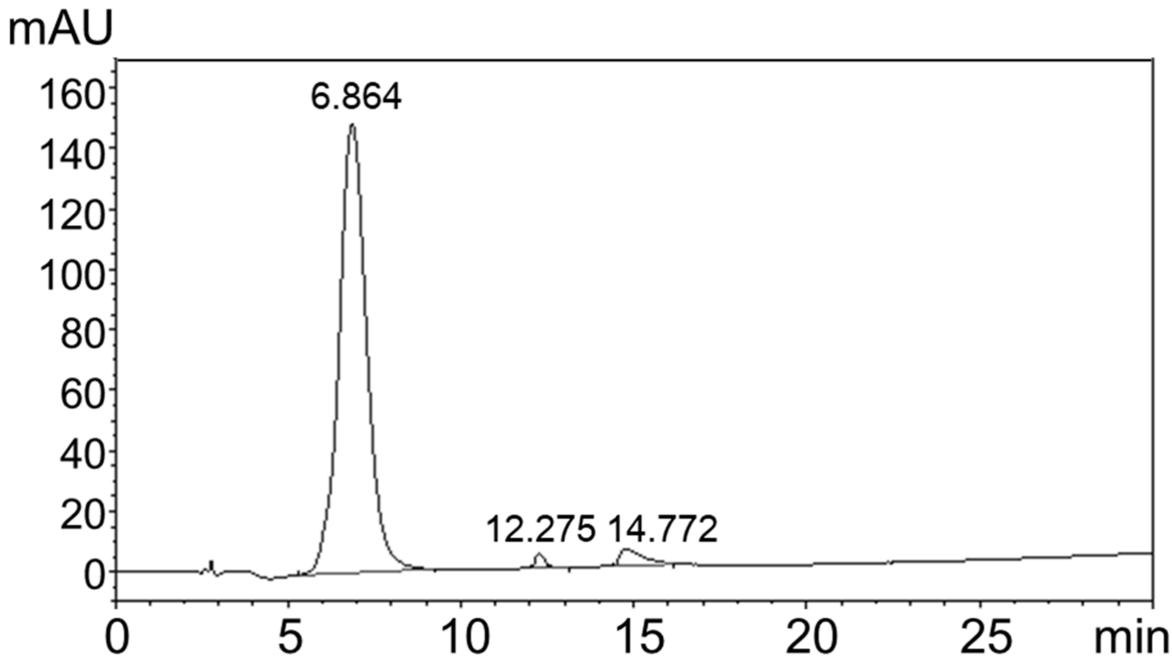


| # | Time | Peak Area | Peak Height | Peak Width | Symmetry Factor | Peak Area (%) |
|---|---|---|---|---|---|---|
| 1 | 6.864 | 8255.3 | 148.4 | 0.8431 | 0.889 | 96.015 |
| 2 | 12.275 | 90.3 | 4.6 | 0.2579 | 0.633 | 1.050 |
| 3 | 14.772 | 252.3 | 5.5 | 0.5411 | 0.331 | 2.935 |

Fig. 8

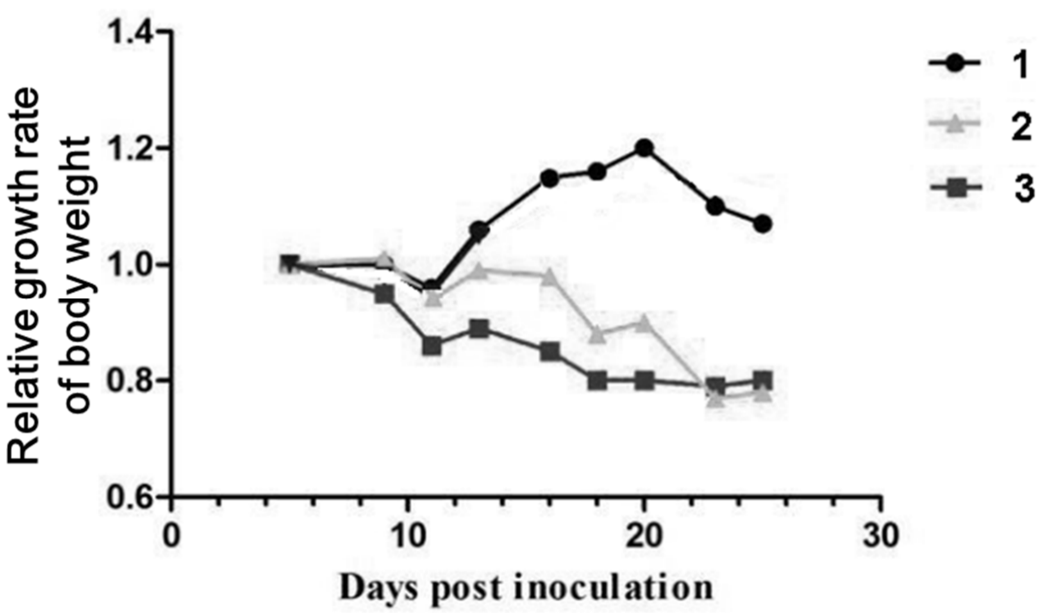

Fig. 9

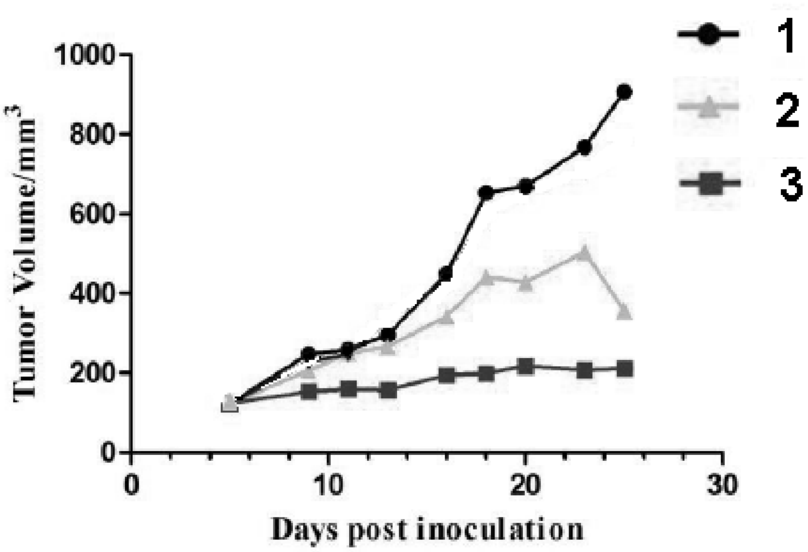
1
2
3
1000
800
600
400
200
0
Tumor Volume/mm3
0
10
20
30
Days post inoculation

Fig. 10

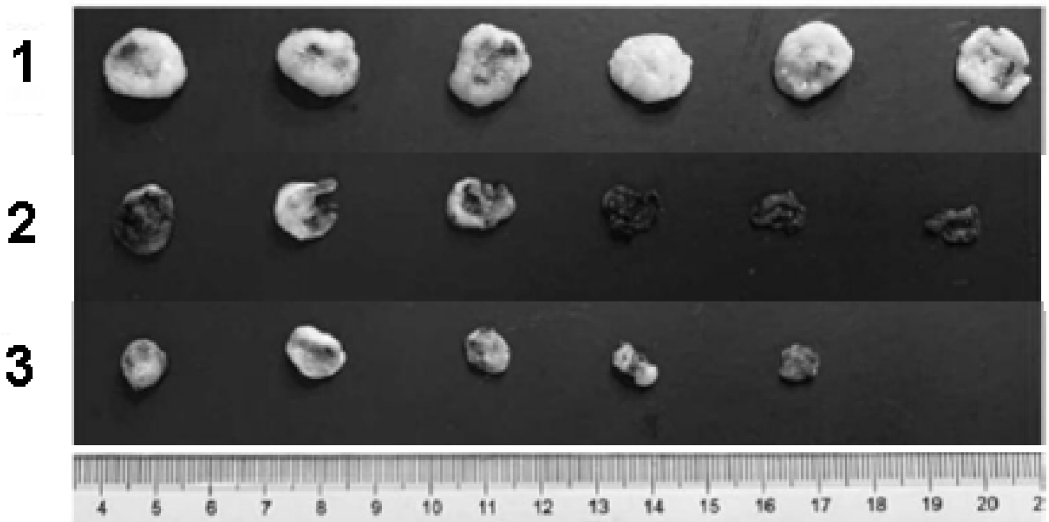
1
2
3
4 5 6 7 8 9 10 11 12 13 14 15 16 17 18 19 20

Fig. 11

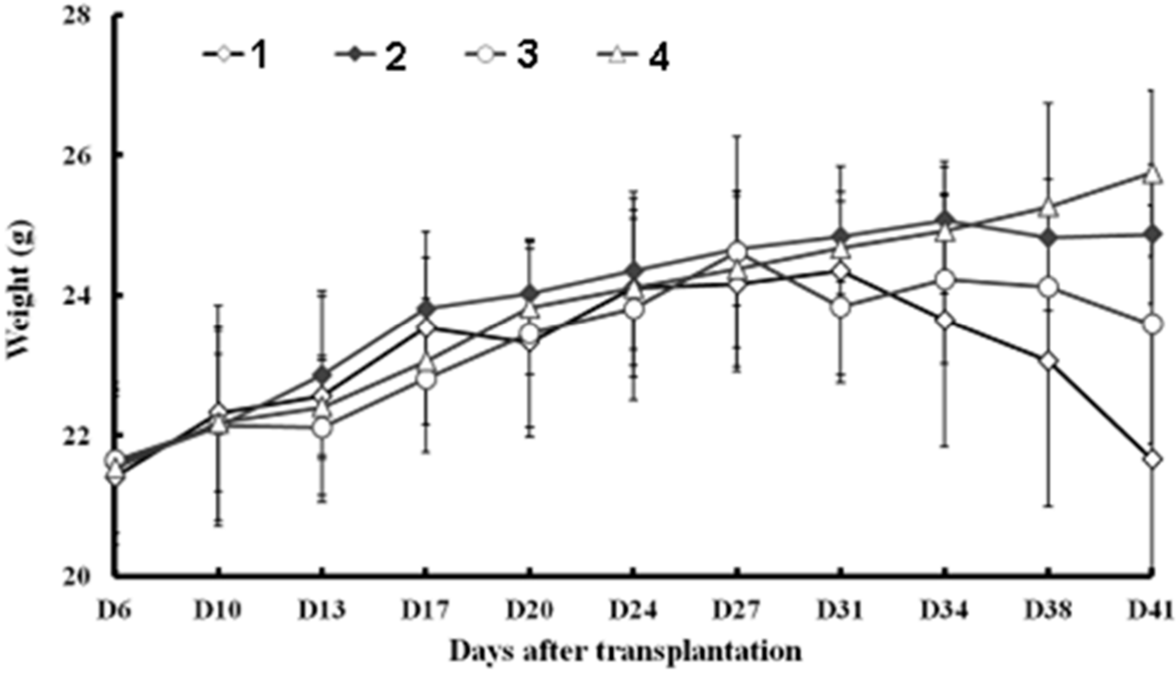
28
26
24
22
20
Weight (g)
1
2
3
4
D6
D10
D13
D17
D20
D24
D27
D31
D34
D38
D41
Days after transplantation

Fig. 12

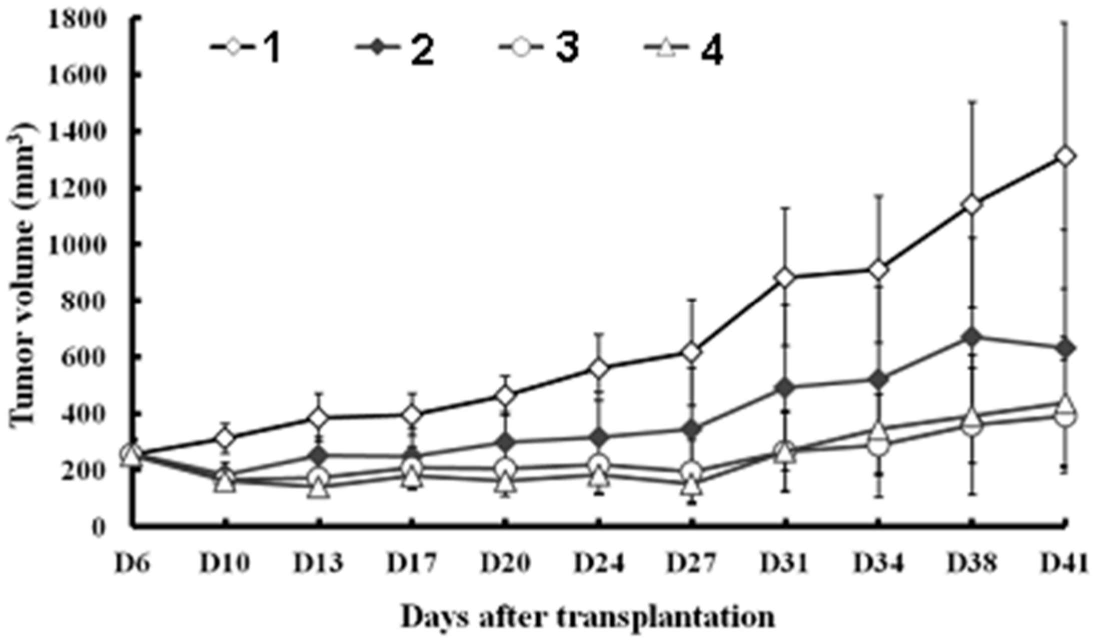
1800
1600
1400
1200
1000
800
600
400
200
0
Tumor volume (mm3)
1
2
3
4
D6
D10
D13
D17
D20
D24
D27
D31
D34
D38
D41
Days after transplantation

Fig. 13

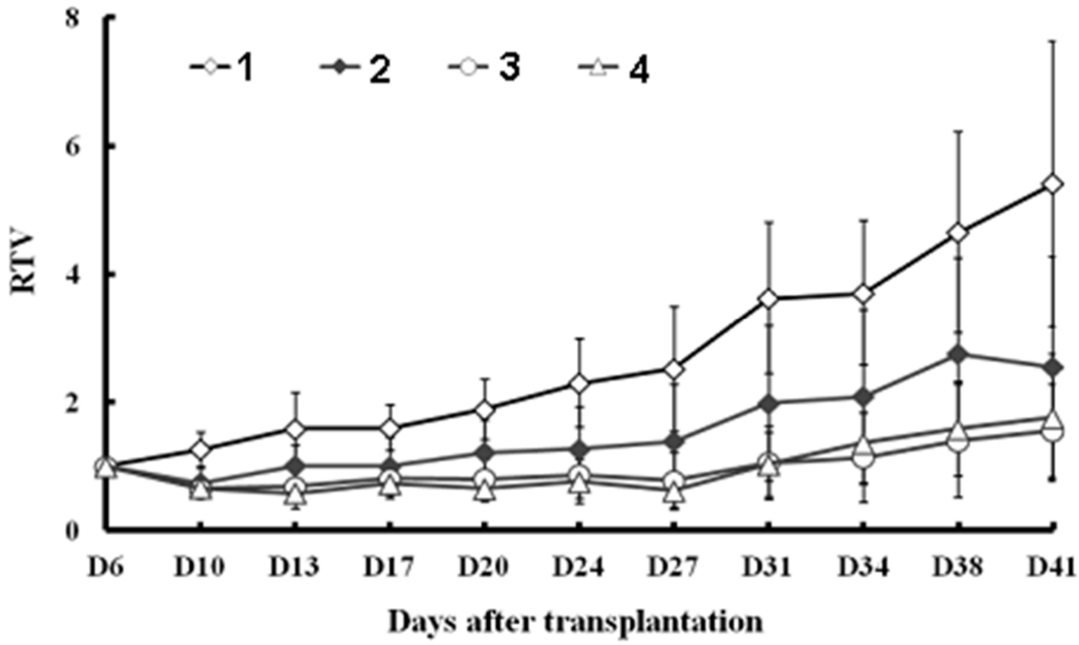
8
6
4
2
0
RTV
1
2
3
4
D6
D10
D13
D17
D20
D24
D27
D31
D34
D38
D41
Days after transplantation

Fig. 14

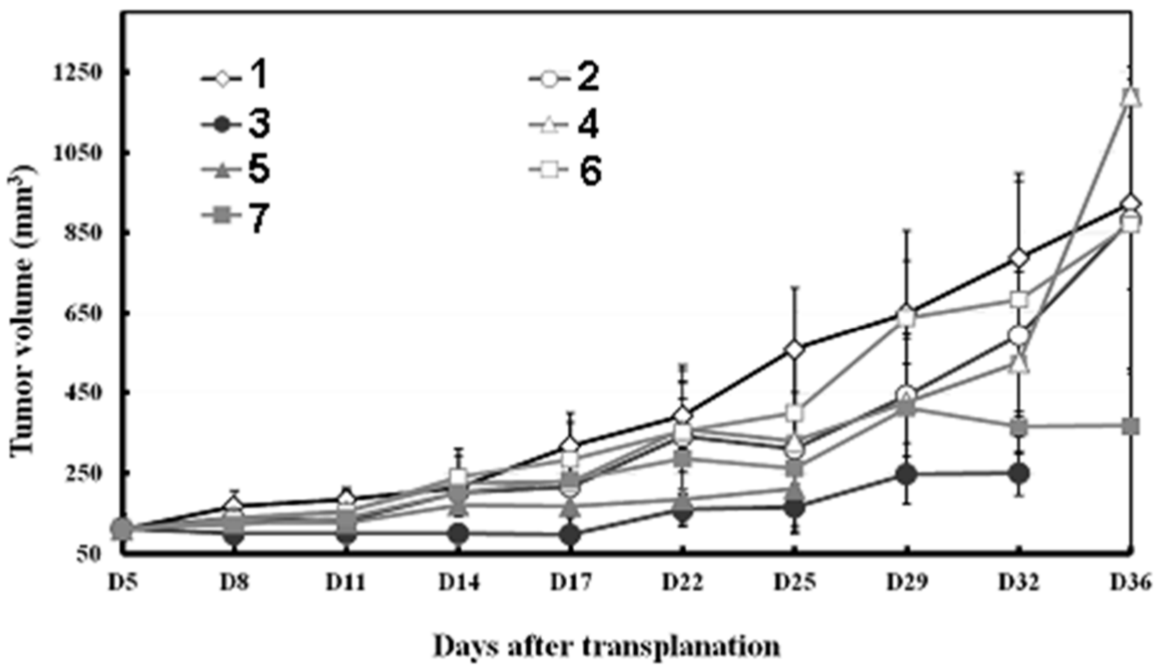
1250
1050
850
650
450
250
50
Tumor volume (mm³)
1
2
3
4
5
6
7
D5
D8
D11
D14
D17
D22
D25
D29
D32
D36
Days after transplanation

Fig. 15

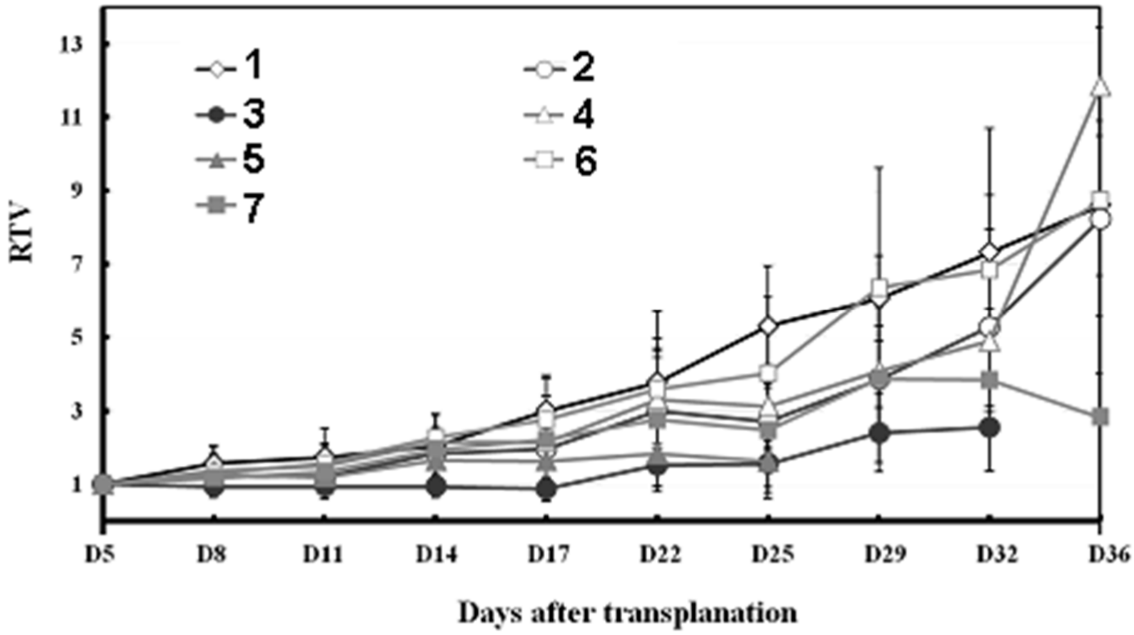
13
11
9
7
5
3
1
RTV
1
2
3
4
5
6
7
D5
D8
D11
D14
D17
D22
D25
D29
D32
D36
Days after transplanation

Fig. 16

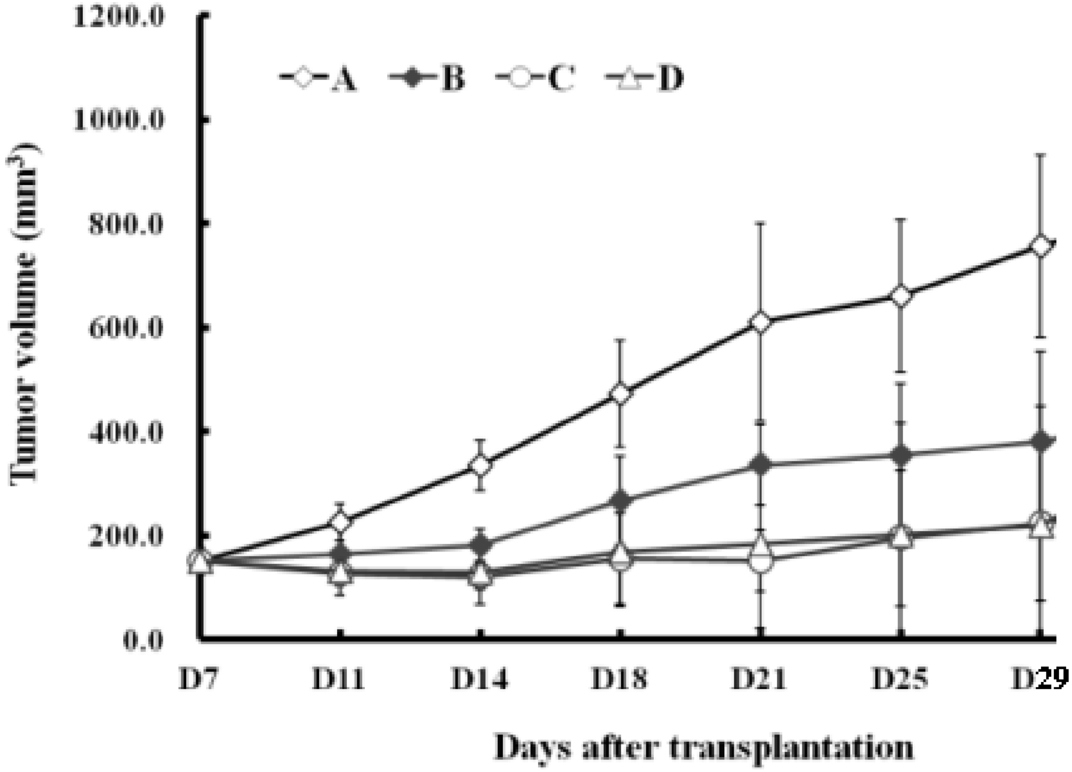
1200.0
1000.0
800.0
600.0
400.0
200.0
0.0
Tumor volume (mm3)
A
B
C
D
D7
D11
D14
D18
D21
D25
D29
Days after transplantation

Fig. 17

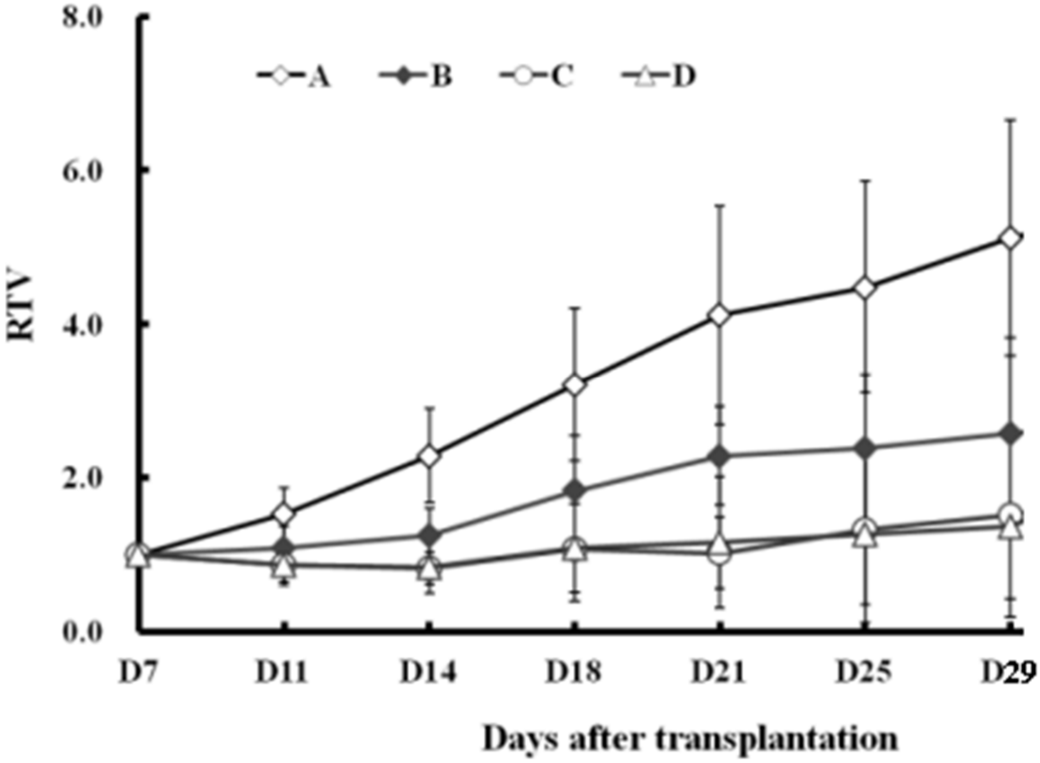
8.0
6.0
4.0
2.0
0.0
RTV
A
B
C
D
D7
D11
D14
D18
D21
D25
D29
Days after transplantation

Fig. 18

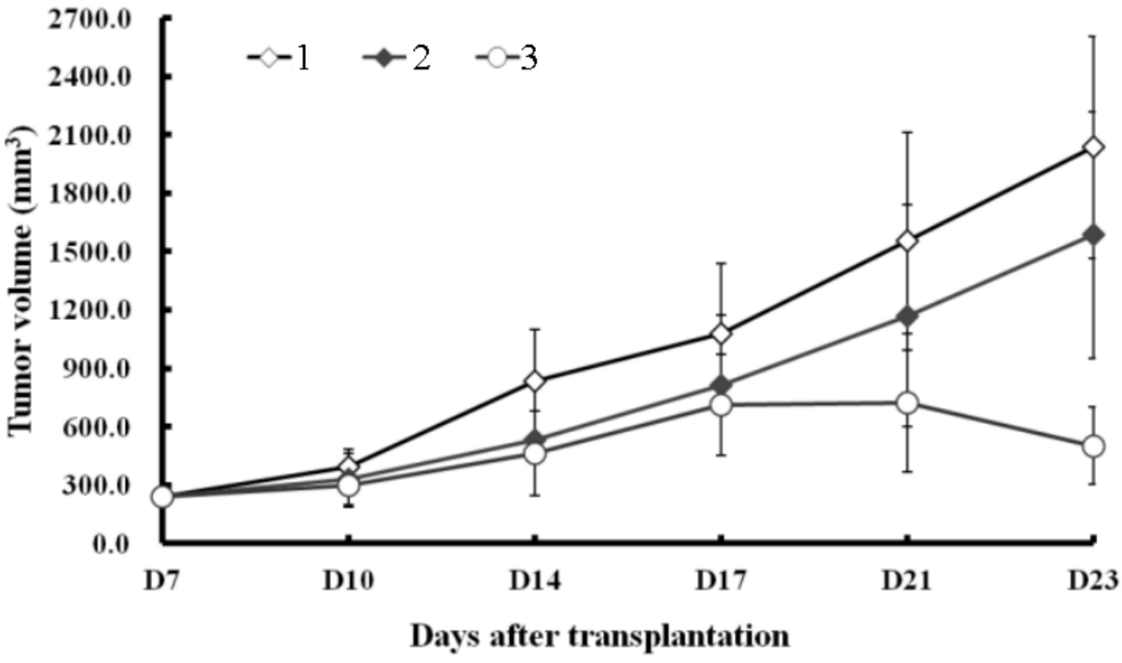
2700.0
2400.0
2100.0
1800.0
1500.0
1200.0
900.0
600.0
300.0
0.0
Tumor volume (mm3)
1
2
3
D7
D10
D14
D17
D21
D23
Days after transplantation

Fig. 19

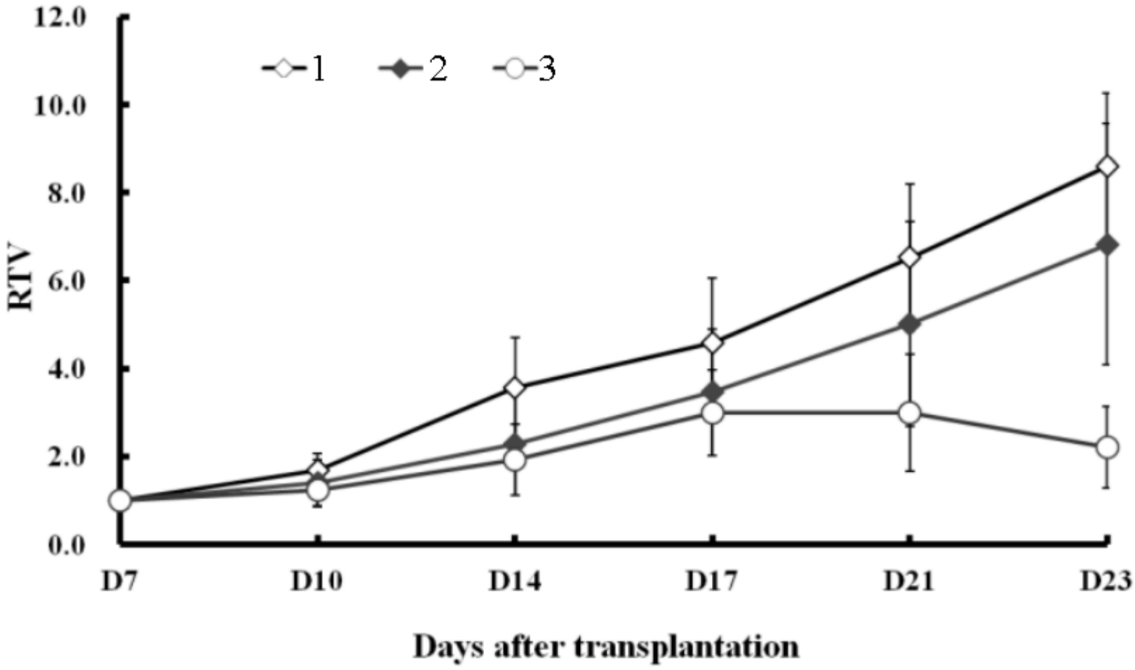
12.0
10.0
8.0
6.0
4.0
2.0
0.0
RTV
1
2
3
D7
D10
D14
D17
D21
D23
Days after transplantation

Fig. 20

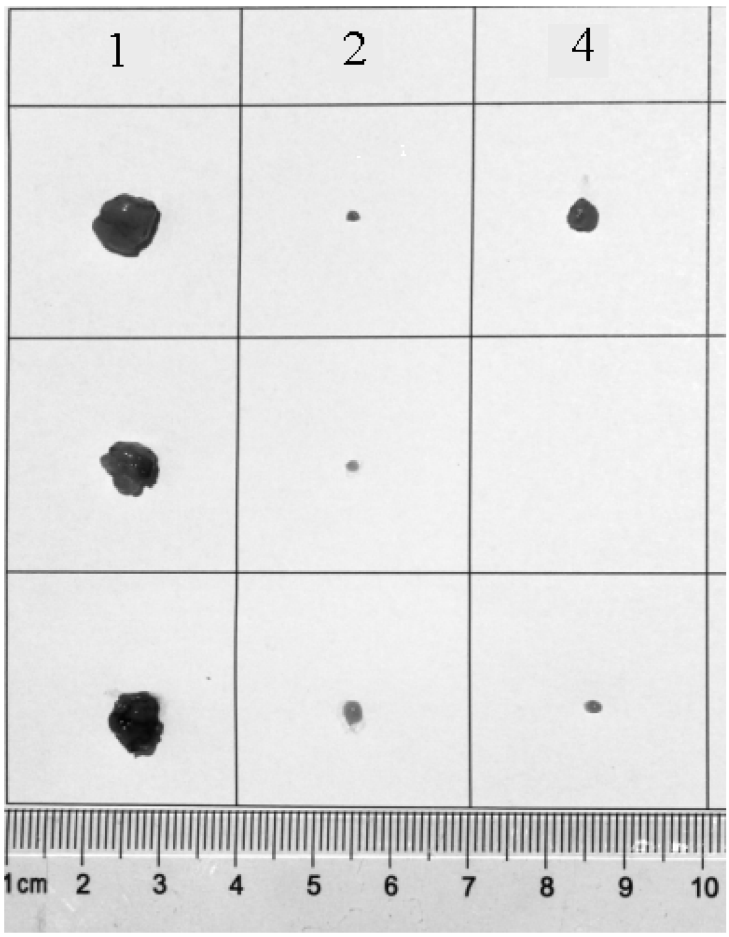
1
2
4
1cm 2 3 4 5 6 7 8 9 10

Fig. 21

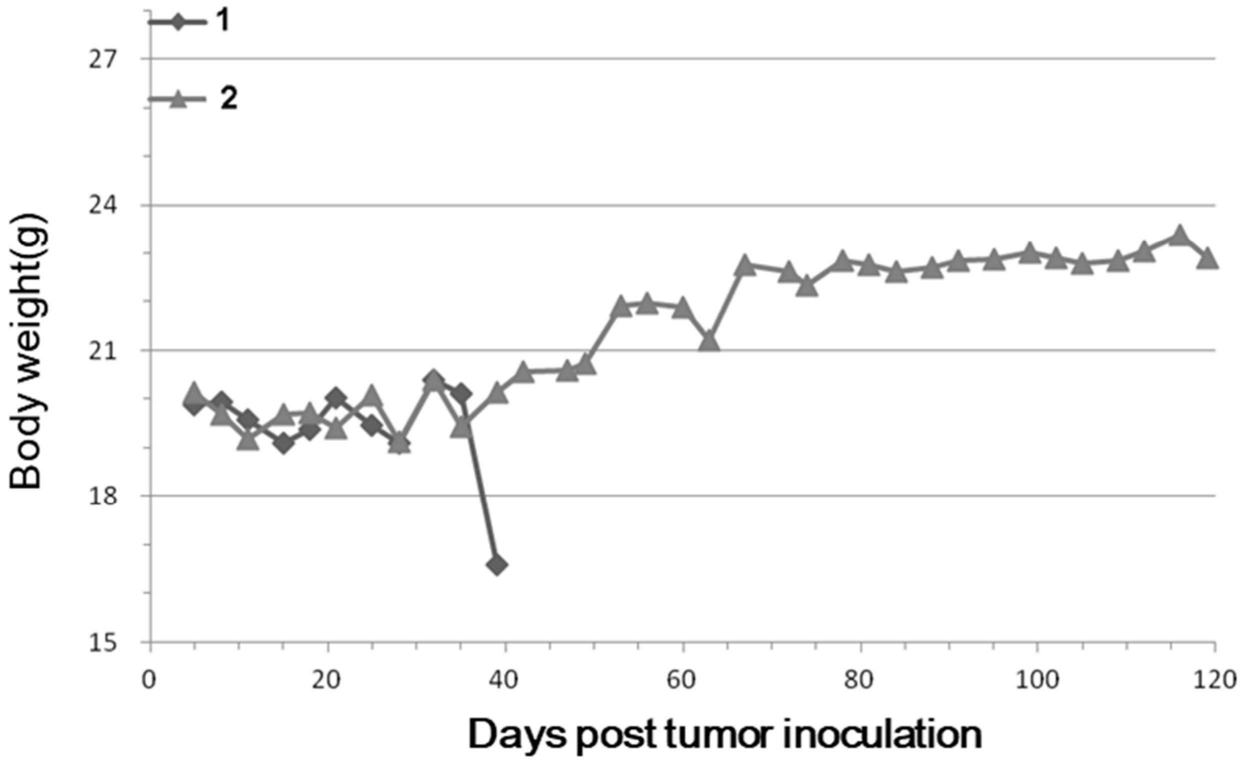
1
2
27
24
21
18
15
Body weight(g)
0
20
40
60
80
100
120
Days post tumor inoculation

Fig. 22

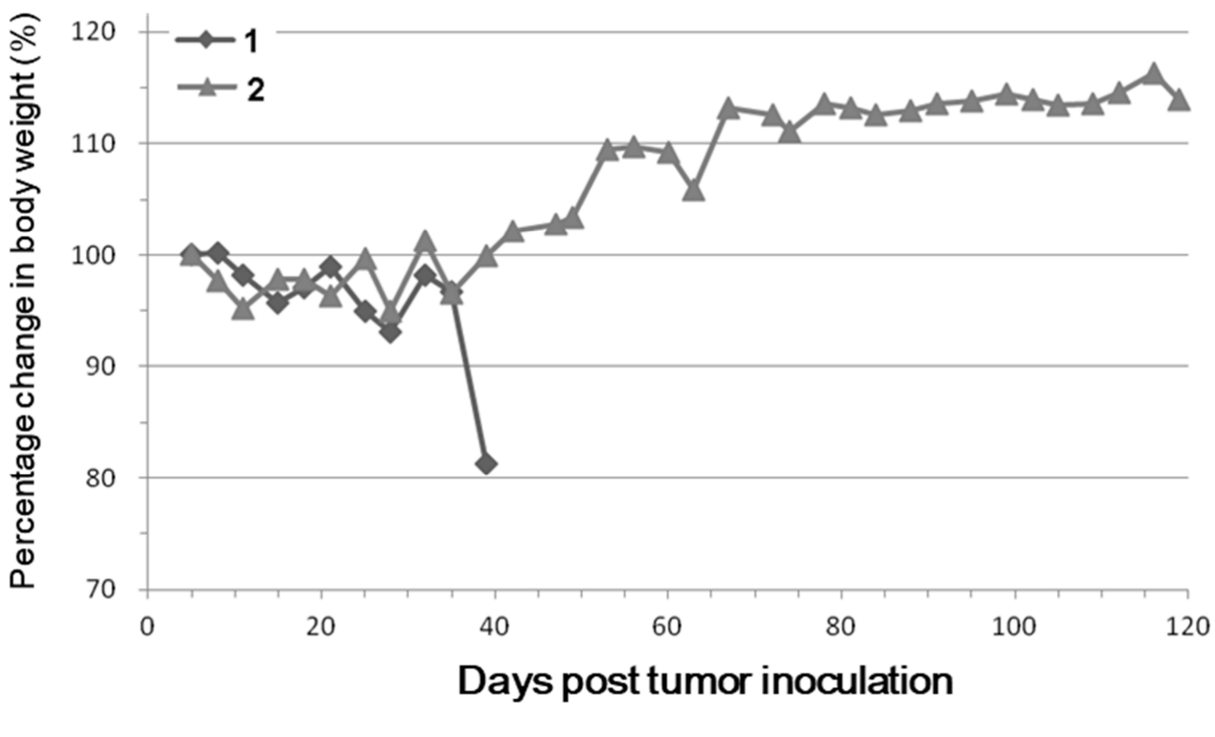
1
2
120
110
100
90
80
70
Percentage change in body weight (%)
0
20
40
60
80
100
120
Days post tumor inoculation

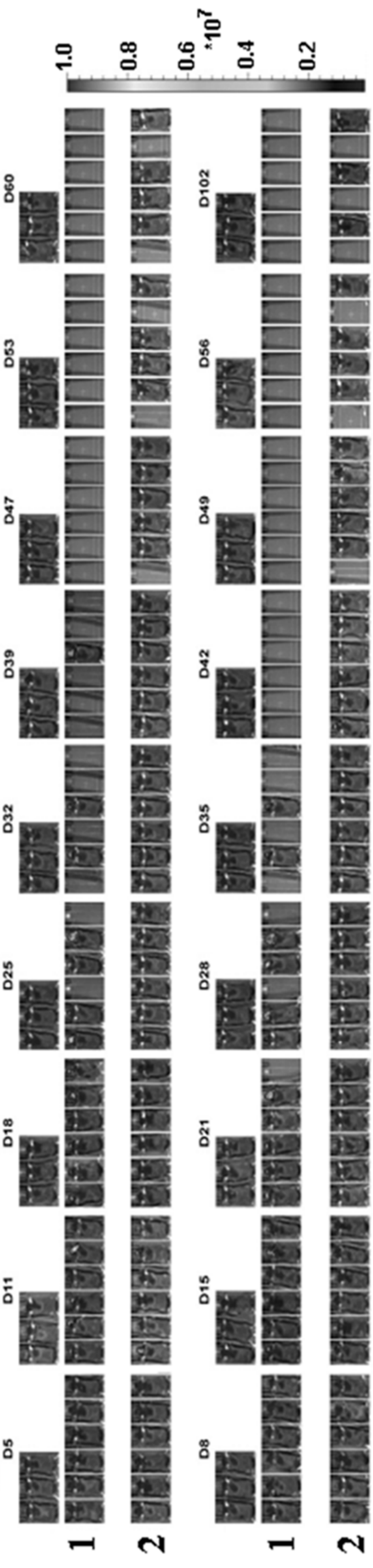
1.0
0.8
0.6
*10^7
0.4
0.2
D5
D11
D18
D25
D32
D39
D47
D53
D60
D8
D15
D21
D28
D35
D42
D49
D56
D102
1
2
1
2

Fig. 23

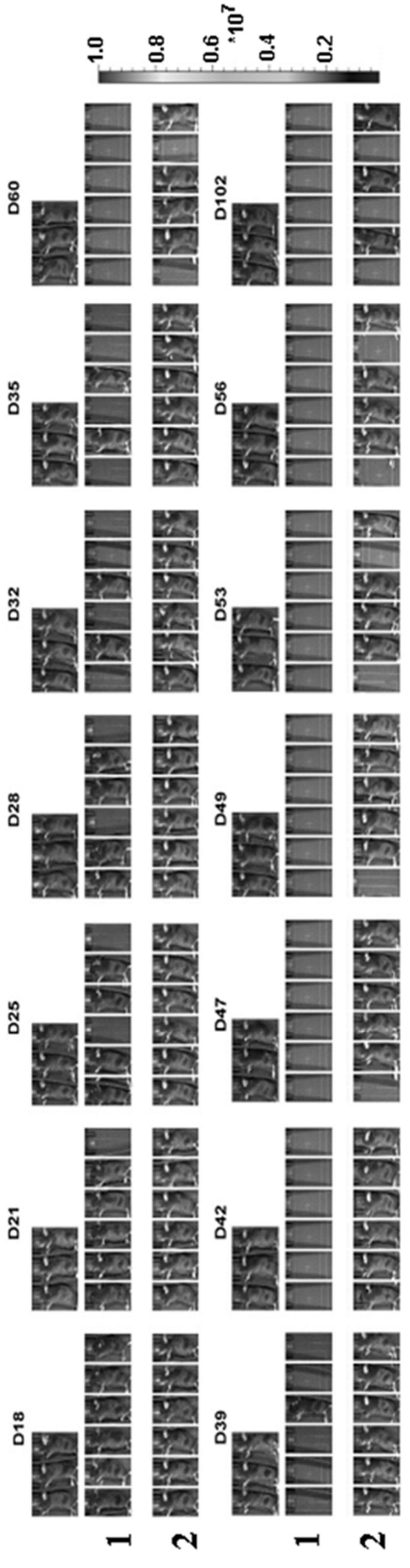
D18
D21
D25
D28
D32
D35
D60
D39
D42
D47
D49
D53
D56
D102
1
2
1
2
1.0
0.8
0.6
0.4
0.2
*10^7

Fig. 24

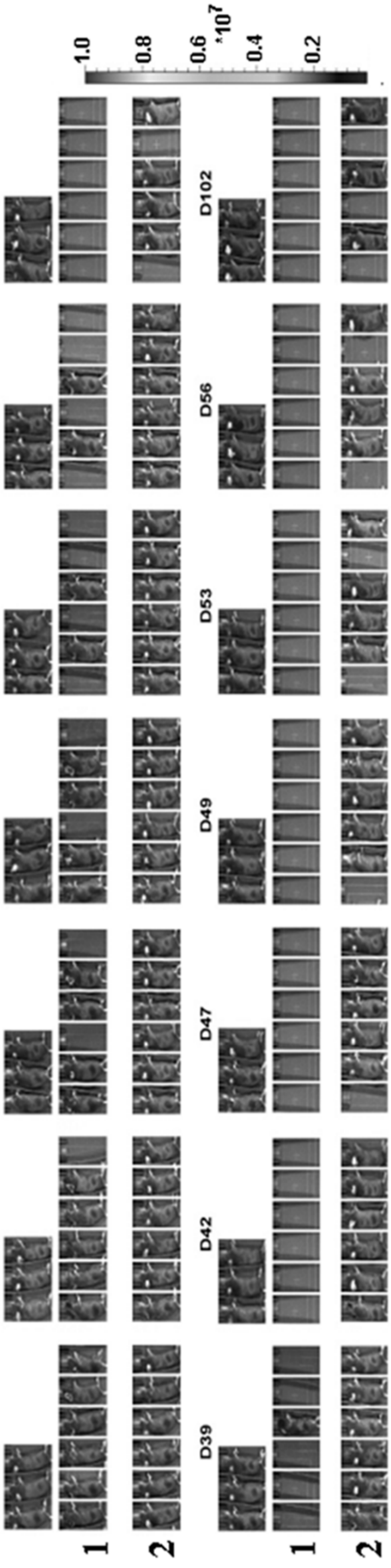
D18
D21
D25
D28
D32
D35
D60
D39
D42
D47
D49
D53
D56
D102
1
2
1
2
1.0
0.8
0.6
0.4
0.2
x10^7

Fig. 25

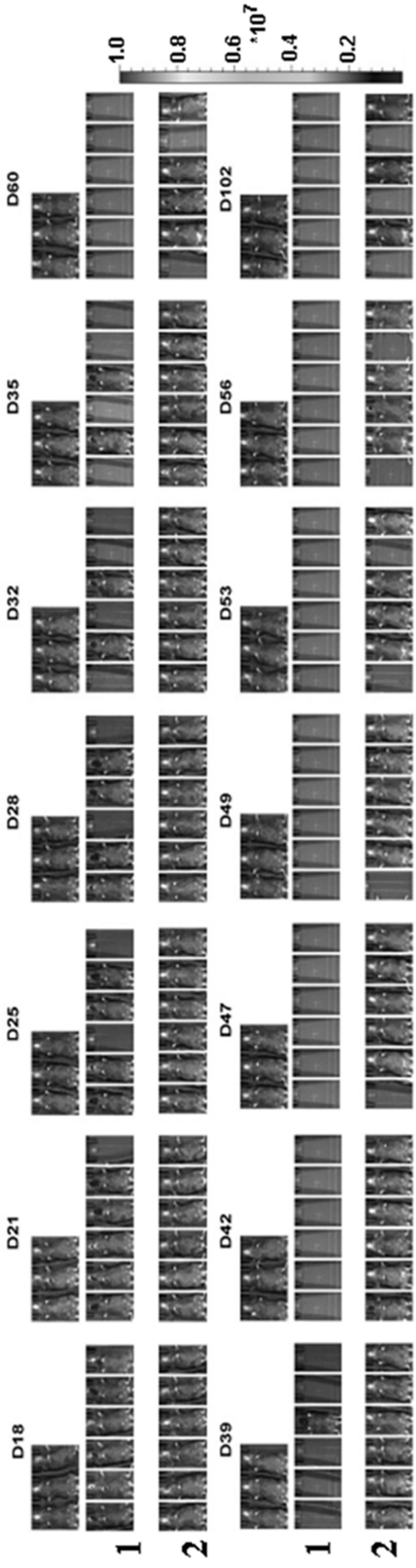
D18
D21
D25
D28
D32
D35
D60
D39
D42
D47
D49
D53
D56
D102
1
2
1
2
1.0
0.8
0.6
0.4
0.2
*10^7

Fig. 26

Fig. 27

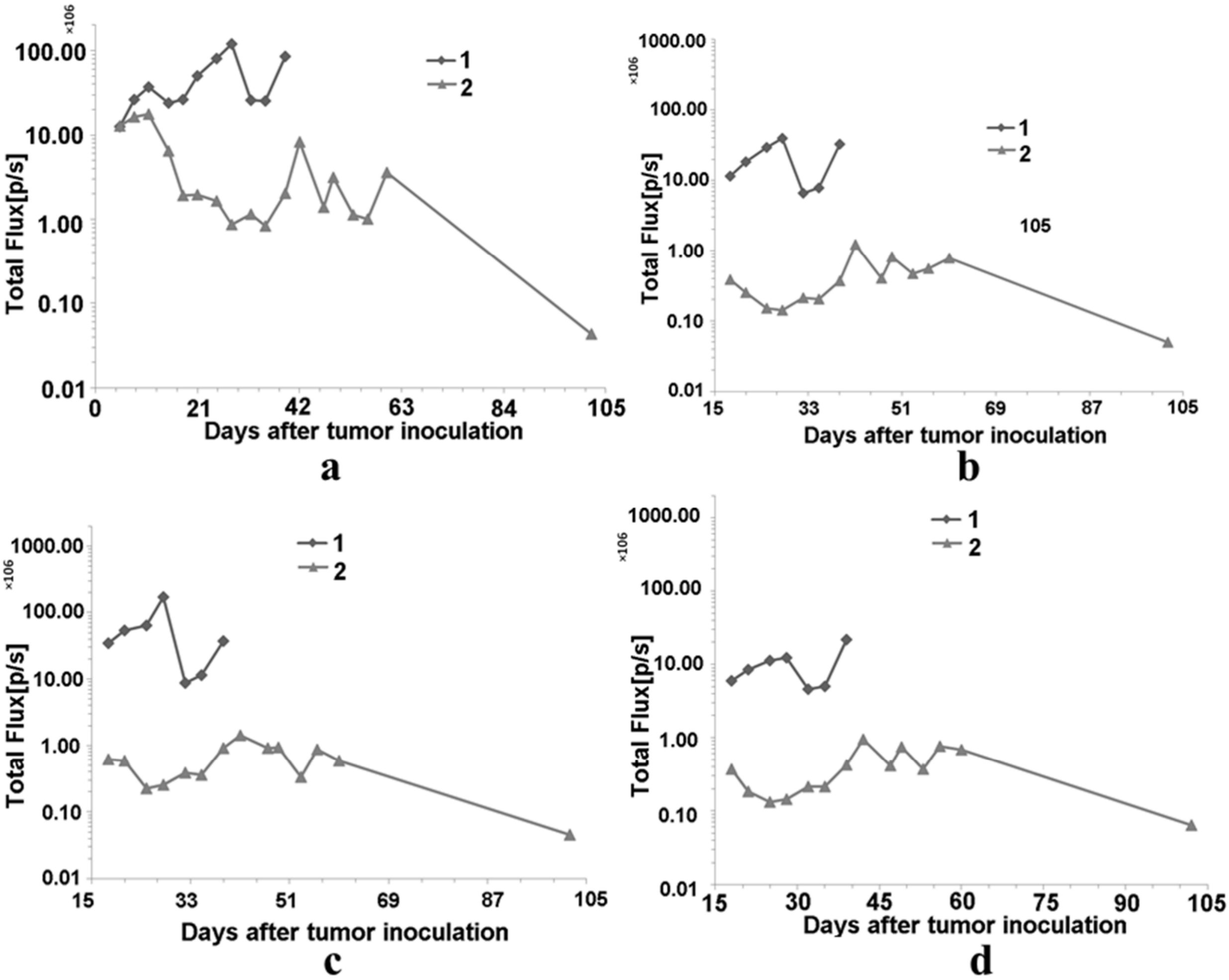
×106
100.00
10.00
1.00
0.10
0.01
0
21
42
63
84
105
Total Flux[p/s]
Days after tumor inoculation
1
2
a
1000.00
100.00
10.00
1.00
0.10
0.01
15
33
51
69
87
105
×106
Total Flux[p/s]
Days after tumor inoculation
1
2
105
b
1000.00
100.00
10.00
1.00
0.10
0.01
15
33
51
69
87
105
×106
Total Flux[p/s]
Days after tumor inoculation
1
2
c
1000.00
100.00
10.00
1.00
0.10
0.01
15
30
45
60
75
90
105
×106
Total Flux[p/s]
Days after tumor inoculation
1
2
d

Fig. 28

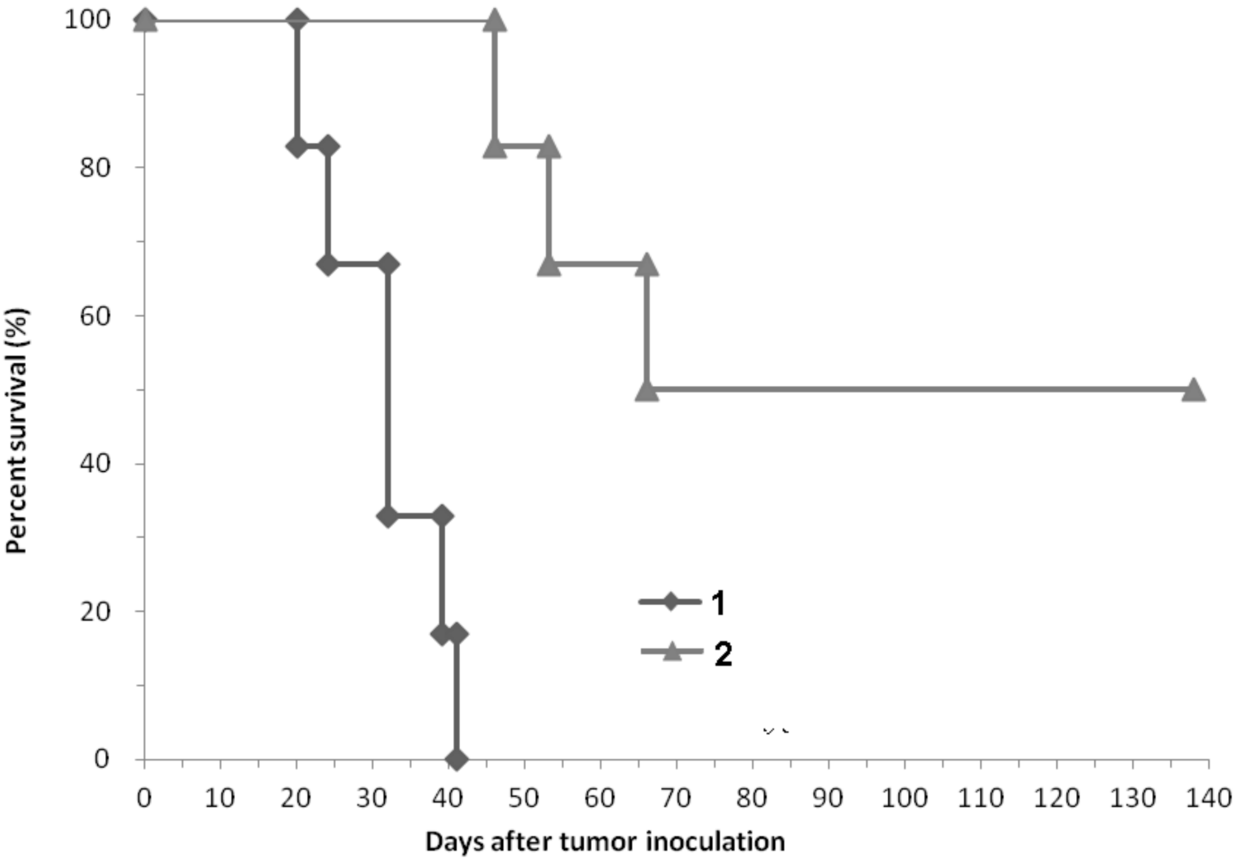
100
80
60
40
20
0
Percent survival (%)
0
10
20
30
40
50
60
70
80
90
100
110
120
130
140
Days after tumor inoculation
1
2

**INTERNATIONAL SEARCH REPORT**

International application No.
**PCT/CN2024/112770**

**A. CLASSIFICATION OF SUBJECT MATTER**

A61K 47/54(2017.01)i; A61K 45/00(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC:A61K,A61P

Documentation searched other than minimum documentation to the extent that such documents are included in the fields searched

Electronic data base consulted during the international search (name of data base and, where practicable, search terms used)

WPABSC, CNTXT, CNABS, WPABS, DWPI, ENTXTC, ISI_Web of Science, STNext, CNKI, 万方, WANFANG: 表柔比星, 表阿霉素, epirubicin, EPI, 核酸, 载体, 靶向, 合, conjugation, coupling, linker, ADC, linker, ADC, antibody drug conjugate

**C. DOCUMENTS CONSIDERED TO BE RELEVANT**

| Category* | Citation of document, with indication, where appropriate, of the relevant passages | Relevant to claim No. |
|---|---|---|
| PX | CN 116785445 A (BAIYAO ZHIDA (BEIJING) NANO BIOTECHNOLOGY CO., LTD.) 22 September 2023 (2023-09-22)<br>claims 1-14 | 1-14 |
| X | CN 110711253 A (BAIYAO ZHIDA (BEIJING) NANO BIOTECHNOLOGY CO., LTD.) 21 January 2020 (2020-01-21)<br>claim 10, and description, paragraphs 24, 26-27, 138, 40-42, 134, 139, 158-164, 5, 36, 39, and 176-181 | 1-14 |
| X | CN 107050464 A (CHINA PHARMACEUTICAL UNIVERSITY) 18 August 2017 (2017-08-18)<br>description, paragraphs 2-12 and 29-31 | 1-14 |
| X | CN 111053765 A (BAIYAO ZHIDA (BEIJING) NANO BIOTECHNOLOGY CO., LTD.) 24 April 2020 (2020-04-24)<br>description, paragraphs 28-45 and 131-196 | 1-14 |
| X | CN 111053913 A (BAIYAO ZHIDA (BEIJING) NANO BIOTECHNOLOGY CO., LTD.) 24 April 2020 (2020-04-24)<br>description, paragraphs 23-45 and 137-192 | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 November 2024** | **21 November 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/112770**

**C. DOCUMENTS CONSIDERED TO BE RELEVANT**

| Category* | Citation of document, with indication, where appropriate, of the relevant passages | Relevant to claim No. |
|---|---|---|
| X | CN 111150852 A (BAIYAO ZHIDA (BEIJING) NANO BIOTECHNOLOGY CO., LTD.) 15 May 2020 (2020-05-15) description, paragraphs 21-45, 157-176, and 190-194 | 1-14 |
| X | CN 115944606 A (JIANG BINCAN) 11 April 2023 (2023-04-11) description, paragraphs 5-24 | 1-14 |
| A | US 2007258986 A1 (GOVERNMENT OF THE UNITED STATES OF AMERICA AS REPRESENTED BY THE SECRETARY OF THE DEPARTMENT OF HEALTH AND HUMAN SERVICES et al.) 08 November 2007 (2007-11-08) description, paragraphs 1-193 | 1-14 |

Form PCT/ISA/210 (second sheet) (July 2022)

**INTERNATIONAL SEARCH REPORT**

International application No.
**PCT/CN2024/112770**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/112770**

| Patent document cited in search report | | | Publication date (day/month/year) | Patent family member(s) | | | Publication date (day/month/year) |
|---|---|---|---|---|---|---|---|
| CN | 116785445 | A | 22 September 2023 | None | | | |
| CN | 110711253 | A | 21 January 2020 | None | | | |
| CN | 107050464 | A | 18 August 2017 | None | | | |
| CN | 111053765 | A | 24 April 2020 | None | | | |
| CN | 111053913 | A | 24 April 2020 | None | | | |
| CN | 111150852 | A | 15 May 2020 | None | | | |
| CN | 115944606 | A | 11 April 2023 | None | | | |
| US | 2007258986 | A1 | 08 November 2007 | WO | 2005051429 | A2 | 09 June 2005 |
| | | | | WO | 2005051429 | A3 | 13 April 2006 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202310871629